# EUROPEAN PATENT SPECIFICATION

(11) **EP 1 499 349 B1**
(45) Date of publication and mention of the grant of the patent: **18.11.2009**
(21) Application number: 02713767.8
(22) Date of filing: 04.03.2002
(51) Int. Cl.: A61K 39/35, C12Q 1/00, C12P 21/06, C07H 21/04, C07K 1/00, C07K 14/435

(54) **RECOMBINANT HYBRID ALLERGEN CONSTRUCTS WITH REDUCED ALLERGENICITY THAT RETAIN IMMUNOGENICITY OF THE NATURAL ALLERGEN**
REKOMBINANTE HYBRID-ALLERGENKONSTRUKTE MIT VERRINGERTER ALLERGENITÄT UNTER BEIBEHALTUNG DER IMMUNOGENITÄT DES NATÜRLICHEN ALLERGENS
HYBRIDES RECOMBINES D'ALLERGENES A ALLERGENICITE REDUITE CONSERVANT L'IMMUNOGENICITE DES ALLERGENES NATURELS

(30) Priority: 02.03.2001 US 272818 P
(43) Date of publication of application: 26.01.2005
(73) Proprietor: THE ROCKEFELLER UNIVERSITY, New York, NY 10024 (US); Alk Abello A/S, 2970 Horsholm (DK)
(72) Inventor: KING, Te, Piao, New York, NY 10021 (US); SPANGFORT, Michael, Dho, S-254 37 Helsingborg (SE)
(74) Representative: Olsen, Lars Pallisgaard
(86) International application number: PCT/US2002/006765
(87) International publication number: WO 2002/070665

(56) References cited:
- WO-A-99/47680
- KING T P ET AL: "Recombinant allergens with reduced allergenicity but retaining immunogenicity of the natural allergens: hybrids of yellow jacket and paper wasp venom allergen antigen 5s" JOURNAL OF IMMUNOLOGY, THE WILLIAMS AND WILKINS CO. BALTIMORE, US, vol. 166, no. 10, 15 May 2001 (2001-05-15), pages 6057-6065, XP002245357 ISSN: 0022-1767
- LU G ET AL: "SEQUENCE ANALYSIS AND ANTIGENIC CROSS-REACTIVITY OF A VERNON ALLERGEN, ANTIGEN 5, FROM HORNETS, WASPS, AND YELLOW JACKETS" JOURNAL OF IMMUNOLOGY, THE WILLIAMS AND WILKINS CO. BALTIMORE, US, vol. 150, no. 7, 1 April 1993 (1993-04-01), pages 2823-2830, XP000867503 ISSN: 0022-1767
- AALBERSE ROB C: "Structural biology of allergens" JOURNAL OF ALLERGY AND CLINICAL IMMUNOLOGY, vol. 106, no. 2, August 2000 (2000-08), pages 228-238, XP002343238 ISSN: 0091-6749
- KING ET AL.: 'Allergenicity of hybrid Ag 5s of yellow jacket and paper wasp venoms' INTERNATIONAL ARCHIVES OF ALLERGY AND IMMUNOLOGY January 2001, pages 85 - 86, XP008049267
- [Online] Retrieved from the Internet: URL:http://content.karger.com> [retrieved on 2007-08-30] * abstract *
- [Online] Retrieved from the Internet: URL:http://content.karger.com> [retrieved on 2007-08-30] * abstract *

### SEQUENCE LISTING

<110> The Rockefeller University
   ALK Abelló
<120> RECOMBINANT HYBRID ALLERGEN CONSTRUCTS WITH REDUCED ALLERGENICITY THAT RETAIN IMMUNOGENICITY OF THE NATURAL ALLERGEN
<130> 2313/2H587WO0
<150> US 60/272,818
   <151> 2001-03-02
<160> 98
<170> PatentIn version 3.1
<210> 1
   <211> 8
   <212> PRT
   <213> Vespula vulgaris
<400> 1
   Asn Asn Tyr Cys Lys Ile Lys Cys
   1               5
<210> 2
   <211> 18
   <212> PRT
   <213> Vespula vulgaris
<400> 2
   Asn Asn Tyr Cys Lys Ile Lys Cys Leu Lys Gly Gly Val His Thr Ala
   1               5                   10                  15
   Cys Lys
<210> 3
   <211> 24
   <212> PRT
   <213> Vespula vulgaris
<400> 3
   Asn Asn Tyr Cys Lys Ile Lys Cys Leu Lys Gly Gly Val His Thr Ala
   1               5                   10                  15
   Cys Lys Tyr Gly Ser Leu Lys Pro
               20
<210> 4
   <211> 32
   <212> PRT
   <213> Vespula vulgaris
<400> 4
   Asn Asn Tyr Cys Lys Ile Lys Cys Leu Lys Gly Gly Val His Thr Ala
   1               5                   10                  15
   Cys Lys Tyr Gly Ser Leu Lys Pro Asn Cys Gly Asn Lys Val Val Val
               20                  25                  30
<210> 5
   <211> 39
   <212> PRT
   <213> Vespula vulgaris
<400> 5
   Asn Asn Tyr Cys Lys Ile Lys Cys Leu Lys Gly Gly Val His Thr Ala
   1               5                   10                  15
   Cys Lys Tyr Gly Ser Leu Lys Pro Asn Cys Gly Asn Lys Val Val Val
               20                  25                  30
   Ser Tyr Gly Leu Thr Lys Gln
           35
<210> 6
   <211> 46
   <212> PRT
   <213> Vespula vulgaris
<400> 6
   Asn Asn Tyr Cys Lys Ile Lys Cys Leu Lys Gly Gly Val His Thr Ala
   1               5                   10                  15
   Cys Lys Tyr Gly Ser Leu Lys Pro Asn Cys Gly Asn Lys Val Val Val
               20                  25                  30
   Ser Tyr Gly Leu Thr Lys Gln Glu Lys Gln Asp Ile Leu Lys
           35                  40                  45
<210> 7
   <211> 48
   <212> PRT
   <213> Vespula vulgaris
<400> 7
   Gln Val Gly Gln Asn Val Ala Leu Thr Gly Ser Thr Ala Ala Lys Tyr
   1               5                   10                  15
   Asp Asp Pro Val Lys Leu Val Lys Met Trp Glu Asp Glu Val Lys Asp
               20                  25                  30
   Tyr Asn Pro Lys Lys Lys Phe Ser Gly Asn Asp Phe Leu Lys Thr Gly
           35                  40                  45
<210> 8
   <211> 49
   <212> PRT
   <213> Vespula vulgaris
<400> 8
   His Tyr Thr Gln Met Val Trp Ala Asn Thr Lys Glu Val Gly Cys Gly
   1               5                   10                  15
   Ser Ile Lys Tyr Ile Gln Glu Lys Trp His Lys His Tyr Leu Val Cys
               20                  25                  30
   Asn Tyr Gly Pro Ser Gly Asn Phe Lys Asn Glu Glu Leu Tyr Gln Thr
           35                  40                  45
   Lys
<210> 9
   <211> 11
   <212> PRT
   <213> Vespula vulgaris
<400> 9
   Leu Lys Pro Asn Cys Gly Asn Lys Val Val Val
   1               5                   10
<210> 10
   <211> 11
   <212> PRT
   <213> Vespula vulgaris
<400> 10
   Leu Thr Gly Ser Thr Ala Ala Lys Tyr Asp Asp
   1               5                   10
<210> 11
   <211> 9
   <212> PRT
   <213> Vespula vulgaris
<400> 11
   Pro Lys Lys Lys Phe Ser Gly Asn Asp
   1 5
<210> 12
   <211> 7
   <212> PRT
   <213> Vespula vulgaris
<400> 12
   Ile Gln Ile Lys Trp His Lys
   1 5
<210> 13
   <211> 10
   <212> PRT
   <213> Vespula vulgaris
<400> 13
   Phe Lys Asn Glu Glu Leu Tyr Gln Thr Lys
   1 5 10
<210> 14
   <211> 615
   <212> DNA
   <213> Vespula vulgaris
<400> 14
   aacaattatt gtaaaataaa atgtttgaaa ggaggtgtcc atactgcctg caaatatgga 60
   agtcttaaac cgaattgcgg taataaggta gtggtatcct atggtctaac gaaacaagag 120
   aaacaagaca tcttaaagga gcacaatgac tttagacaaa aaattgcacg aggattggag 180
   actagaggta atcctggacc acagcctcca gcgaagaata tgaaaaattt ggtatggaac 240
   gacgagttag cttatgtcgc ccaagtgtgg gctaatcaat gtcaatatgg tcacgatact 300
   tgcagggatg tagcaaaata tcaggttgga caaaacgtag ccttaacagg tagcacggct 360
   gctaaatacg atgatccagt taaactagtt aaaatgtggg aagatgaagt gaaagattat 420
   aatcctaaga aaaagttttc gggaaacgac tttctgaaaa ccggccatta cactcaaatg 480
   gtttgggcta acaccaagga agttggttgt ggaagtataa aatacattca agagaaatgg 540
   cacaaacatt accttgtatg taattatgga cccagcggaa actttaagaa tgaggaactt 600
   tatcaaacaa agtaa 615
<210> 15
   <211> 618
   <212> DNA
   <213> Polistes annularis
<400> 15
   gttgattatt gtaaaataaa gtgtccaagt ggtatccata cagtctgcca atatggagaa     60
   tcgacaaaac caagcaagaa ttgtgccggt aaagtaatca aatcggttgg tccaacggaa    120
   gaggagaaaa aattaatcgt aagcgagcat aatcggttta gacaaaaagt tgcacagggg    180
   ttggaaacaa gaggtaatcc tggaccacaa cctgctgcct cggacatgaa tgatttggta    240
   tggaacgatg aattagcaca tatcgcgcaa gtatgggcca gccaatgcca atttctcgta    300
   cacgacaaat gcaggaatac cgcaaaatat ccagttggac aaaatatagc gtatgcaggt    360
   ggttctaact taccagatgt agtcagtcta atcaaacttt gggaaaacga agtgaaagat    420
   tttaattaca atacaggaat aacaaaacaa aactttgcta aaattggcca ttacactcaa    480
   atggtttggg gtaaaactaa agaaattggt tgtggatctc taaaatatat ggaaaataat    540
   atgcaaaatc attacctcat atgtaattat ggaccagctg gaaattactt gggtcaacta    600
   ccttatacaa aaaaataa                                                  618
<210> 16
   <211> 204
   <212> PRT
   <213> Vespula vulgaris
<400> 16
   Asn Asn Tyr Cys Lys Ile Lys Cys Leu Lys Gly Gly Val His Thr Ala
   1               5                   10                  15
   Cys Lys Tyr Gly Ser Leu Lys Pro Asn Cys Gly Asn Lys Val Val Val
               20                  25                  30
   Ser Tyr Gly Leu Thr Lys Gln Glu Lys Gln Asp Ile Leu Lys Glu His
           35                  40                  45
   Asn Asp Phe Arg Gln Lys Ile Ala Arg Gly Leu Glu Thr Arg Gly Asn
       50                  55                  60
   Pro Gly Pro Gln Pro Pro Ala Lys Asn Met Lys Asn Leu Val Trp Asn
   65                  70                  75                  80
   Asp Glu Leu Ala Tyr Val Ala Gln Val Trp Ala Asn Gln Cys Gln Tyr
                   85                  90                  95
   Gly His Asp Thr Cys Arg Asp Val Ala Lys Tyr Gln Val Gly Gln Asn
               100                 105                 110
   Val Ala Leu Thr Gly Ser Thr Ala Ala Lys Tyr Asp Asp Pro Val Lys
           115                 120                 125
   Leu Val Lys Met Trp Glu Asp Glu Val Lys Asp Tyr Asn Pro Lys Lys
       130                 135                 140
   Lys Phe Ser Gly Asn Asp Phe Leu Lys Thr Gly His Tyr Thr Gln Met
   145                 150                 155                 160
   Val Trp Ala Asn Thr Lys Glu Val Gly Cys Gly Ser Ile Lys Tyr Ile
                   165                 170                 175
   Gln Glu Lys Trp His Lys His Tyr Leu Val Cys Asn Tyr Gly Pro Ser
               180                 185                 190
   Gly Asn Phe Lys Asn Glu Glu Leu Tyr Gln Thr Lys
           195                 200
<210> 17
   <211> 205
   <212> PRT
   <213> Polistes annularis
<400> 17
   Val Asp Tyr Cys Lys Ile Lys Cys Pro Ser Gly Ile His Thr Val Cys
   1               5                   10                  15
   Gln Tyr Gly Glu Ser Thr Lys Pro Ser Lys Asn Cys Ala Gly Lys Val
               20                  25                  30
   Ile Lys Ser Val Gly Pro Thr Glu Glu Glu Lys Lys Leu Ile Val Ser
           35                  40                  45
   Glu His Asn Arg Phe Arg Gln Lys Val Ala Gln Gly Leu Glu Thr Arg
       50                  55                  60
   Gly Asn Pro Gly Pro Gln Pro Ala Ala Ser Asp Met Asn Asp Leu Val
   65                  70                  75                  80
   Trp Asn Asp Glu Leu Ala His Ile Ala Gln Val Trp Ala Ser Gln Cys
                   85                  90                  95
   Gln Phe Leu Val His Asp Lys Cys Arg Asn Thr Ala Lys Tyr Pro Val
               100                 105                 110
   Gly Gln Asn Ile Ala Tyr Ala Gly Gly Ser Asn Leu Pro Asp Val Val
           115                 120                 125
   Ser Leu Ile Lys Leu Trp Glu Asn Glu Val Lys Asp Phe Asn Tyr Asn
       130                 135                 140
   Thr Gly Ile Thr Lys Gln Asn Phe Ala Lys Ile Gly His Tyr Thr Gln
   145                 150                 155                 160
   Met Val Trp Gly Lys Thr Lys Glu Ile Gly Cys Gly Ser Leu Lys Tyr
                   165                 170                 175
   Met Glu Asn Asn Met Gln Asn His Tyr Leu Ile Cys Asn Tyr Gly Pro
               180                 185                 190
   Ala Gly Asn Tyr Leu Gly Gln Leu Pro Tyr Thr Lys Lys
           195                 200                 205
<210> 18
   <211> 24
   <212> DNA
   <213> Vespula vulgaris
<400> 18
   aacaattatt gtaaaataaa atgt 24
<210> 19
   <211> 54
   <212> DNA
   <213> Vespula vulgaris
<400> 19
   aacaattatt gtaaaataaa atgtttgaaa ggaggtgtcc atactgcctg caaa 54
<210> 20
   <211> 72 <212> DNA
   <213> Vespula vulgaris
<400> 20
   aacaattatt gtaaaataaa atgtttgaaa ggaggtgtcc atactgcctg caaatatgga 60
   agtcttaaac cg 72
<210> 21
   <211> 96
   <212> DNA
   <213> Vespula vulgaris
<400> 21
   aacaattatt gtaaaataaa atgtttgaaa ggaggtgtcc atactgcctg caaatatgga 60
   agtcttaaac cgaattgcgg taataaggta gtggta 96
<210> 22
   <211> 117
   <212> DNA
   <213> Vespula vulgaris
<400> 22
   aacaattatt gtaaaataaa atgtttgaaa ggaggtgtcc atactgcctg caaatatgga 60
   agtcttaaac cgaattgcgg taataaggta gtggtatcct atggtctaac gaaacaa 117
<210> 23
   <211> 138
   <212> DNA
   <213> Vespula vulgaris
<400> 23
   aacaattatt gtaaaataaa atgtttgaaa ggaggtgtcc atactgcctg caaatatgga 60
   agtcttaaac cgaattgcgg taataaggta gtggtatcct atggtctaac gaaacaagag 120
   aaacaagaca tcttaaag 138
<210> 24
   <211> 144
   <212> DNA
   <213> Vespula vulgaris
<400> 24
   caggttggac aaaacgtagc cttaacaggt agcacggctg ctaaatacga tgatccagtt 60
   aaactagtta aaatgtggga agatgaagtg aaagattata atcctaagaa aaagttttcg 120
   ggaaacgact ttctgaaaac cggc 144
<210> 25
   <211> 147
   <212> DNA
   <213> Vespula vulgaris
<400> 25
   cattacactc aaatggtttg ggctaacacc aaggaagttg gttgtggaag tataaaatac 60
   attcaagaga aatggcacaa acattacctt gtatgtaatt atggacccag cggaaacttt 120
   aagaatgagg aactttatca aacaaag 147
<210> 26
   <211> 33
   <212> DNA
   <213> Vespula vulgaris
<400> 26
   cttaaaccga attgcggtaa taaggtagtg gta 33
<210> 27
   <211> 33
   <212> DNA
   <213> Vespula vulgaris
<400> 27
   ttaacaggta gcacggctgc taaatacgat gat 33
<210> 28
   <211> 27
   <212> DNA
   <213> Vespula vulgaris
<400> 28
   cctaagaaaa agttttcggg aaacgac 27
<210> 29
   <211> 21
   <212> DNA
   <213> Vespula vulgaris
<400> 29
   attcaagaga aatggcacaa a 21
<210> 30
   <211> 30
   <212> DNA
   <213> Vespula vulgaris
<400> 30
   tttaagaatg aggaacttta tcaaacaaag 30
<210> 31
   <211> 30
   <212> DNA
   <213> Artificial Sequence
<220>
   <223> Ves v 5' EA sense PCR primer 1
<400> 31
   cgtgaattca acaattattg taaaataaaa 30
<210> 32
   <211> 36
   <212> DNA
   <213> Artificial Sequence
<220>
   <223> Ves v 5' KR sense PCR primer 2
<400> 32
   cgtctcgaga aaagaaacaa ttattgtaaa ataaaa 36
<210> 33
   <211> 30
   <212> DNA
   <213> Artificial Sequence
<220>
   <223> Ves v 3' downstream antisense PCR primer 3
<400> 33
   cgttctagat tactttgttt gataaagttc 30
<210> 34
   <211> 30
   <212> DNA
   <213> Artificial Sequence
<220>
   <223> Pol a 5' EA sense PCR primer 4
<400> 34
   cgtgaattcg ttgattattg taaaataaaa 30
<210> 35
   <211> 36
   <212> DNA
   <213> Artificial Sequence
<220>
   <223> Pol a 5' KR sense PCR primer 5
<400> 35
   cgtctcgaga aaagagttga ttattgtaaa ataaaa 36
<210> 36
   <211> 30
   <212> DNA
   <213> Artificial Sequence
<220>
   <223> Pol a 3' downstream antisense primer 6
<400> 36
   cgttctagat tatttttttg tataaggtag 30
<210> 37
   <211> 21
   <212> DNA
   <213> Artificial Sequence
<220>
   <223> Pol a aa 49-50 EH-mutagenic sense PCR primer 7
<400> 37
   gtaagcgagc acaatcggtt t 21
<210> 38
   <211> 21
   <212> DNA
   <213> Artificial Sequence
<220>
   <223> Pol a aa 49-50 EH-mutagenic antisense PCR primer 8
<400> 38
   aaaccgattg tgctcgctta c 21
<210> 39
   <211> 21
   <212> DNA
   <213> Artificial Sequence
<220>
   <223> Ves v ApoI conversion PCR primer 9
<400> 39
   gtagcaaaat ttcaggttgg a 21
<210> 40
   <211> 21
   <212> DNA
   <213> Artificial Sequence
<220>
   <223> Ves v ApoI conversion PCR primer 10
<400> 40
   tccaacctga aattttgcta c 21
<210> 41
   <211> 21
   <212> DNA
   <213> Artificial Sequence
<220>
   <223> Pol a ApoI conversion PCR primer 11
<400> 41
   accgcaaaat ttccagttgg a 21
<210> 42
   <211> 21
   <212> DNA
   <213> Artificial Sequence
<220>
   <223> Pol a ApoI conversion PCR primer 12
<400> 42
   tccaactgga aattttgcgg t 21
<210> 43
   <211> 72
   <212> DNA
   <213> Artificial Sequence
<220>
   <223> PV1-18 sense PCR primer 13
<400> 43
   cgtgaattca acaattattg taaaataaaa tgtttgaaag gaggtgtcca tactgcctgc 60
   aaatatggag aa 72
<210> 44
   <211> 54
   <212> DNA
   <213> Artificial Sequence
<220>
   <223> PV195-204 antisense PCR primer 14
<400> 44
   cgttctagat tactttgttt gataaagttc ctcattctta aaatttccag ctgg 54
<210> 45
   <211> 33
   <212> DNA
   <213> Artificial Sequence
<220>
   <223> PV18-24 and PV1-24 antisense PCR primer 15
<400> 45
   ggcacaattc ttgctcggtt taagacttcc ata 33
<210> 46
   <211> 33
   <212> DNA
   <213> Artificial Sequence
<220>
   <223> PV18-24 and PV 1-24 sense PCR primer 16
<400> 46
   tatggaagtc ttaaaccgag caagaattgt gcc 33
<210> 47
   <211> 45
   <212> DNA
   <213> Artificial Sequence
<220>
   <223> PV22-32 and PV1-32 sense PCR primer 17
<400> 47
   cttaaaccga attgcggtaa taaggtagtg gtatcggttg gtcca 45
<210> 48
   <211> 45
   <212> DNA
   <213> Artificial Sequence
<220>
   <223> PV22-32 and PV1-32 antisense PCR primer 18
<400> 48
   tggaccaacc gataccacta ccttattacc gcaattcggt ttaag 45
<210> 49
   <211> 36
   <212> DNA
   <213> Artificial Sequence
<220>
   <223> PV1-39 PCR primer 19
<400> 49
   tatggtctaa cgaaacaaga gaaaaaatta atcgta 36
<210> 50
   <211> 36
   <212> DNA
   <213> Artificial Sequence
<220>
   <223> PV1-39 PCR primer 20
<400> 50
   tacgattaat tttttctctt gtttcgttag accata 36
<210> 51
   <211> 45
   <212> DNA
   <213> Artificial Sequence
<220>
   <223> PV115-125 sense PCR primer 21
<400> 51
   ttaacaggta gcacggctgc taaatacgat gatgtagtca gtcta 45
<210> 52
   <211> 45
   <212> DNA
   <213> Artificial Sequence
<220>
   <223> PV115-125 antisense PCR primer 22
<400> 52
   atcatcgtat ttagcagccg tgctacctgt taacgctata ttttg 45
<210> 53
   <211> 42
   <212> DNA
   <213> Artificial Sequence
<220>
   <223> PV142-150 sense PCR primer 23
<400> 53
   cctaagaaaa agttttcggg aaacgacttt gctaaaattg gc 42
<210> 54
   <211> 42
   <212> DNA
   <213> Artificial Sequence
<220>
   <223> PV142-150 antisense PCR primer 24
<400> 54
   gtcgtttccc gaaaactttt tcttaggatt aaaatctttc ac 42
<210> 55
   <211> 33
   <212> DNA
   <213> Artificial Sequence
<220>
   <223> PV176-182 sense PCR primer 25
<400> 55
   attcaagaga aatggcacaa acattacctc ata 33
<210> 56
   <211> 33
   <212> DNA
   <213> Artificial Sequence
<220>
   <223> PV176-182 antisense PCR primer 26
<400> 56
   tttgtgccat ttctcttgaa tatattttag aga 33
<210> 57
   <211> 24
   <212> DNA
   <213> Artificial Sequence
<220>
   <223> PV1-50 antisense PCR primer 27
<400> 57
   gagcacaatg actttagaca aaaa 24
<210> 58
   <211> 24
   <212> DNA
   <213> Artificial Sequence
<220>
   <223> PV1-57 antisense PCR primer 28
<400> 58
   tttttgtcta aagtcattgt gctc 24
<210> 59
   <211> 24
   <212> DNA
   <213> Artificial Sequence
<220>
   <223> PV1-76 antisense PCR primer 29
<400> 59
   aaaattgcac gagggttgga aaca 24
<210> 60
   <211> 24
   <212> DNA
   <213> Artificial Sequence
<220>
   <223> PCR primer
<400> 60
   tgtttccaac cctcgtgcaa tttt 24
<210> 61
   <211> 24
   <212> DNA
   <213> Artificial Sequence
<220>
   <223> PCR primer
<400> 61
   aatatgaaaa atttggtatg gaac 24
<210> 62
   <211> 24
   <212> DNA
   <213> Artificial Sequence
<220>
   <223> PCR primer
<400> 62
   gttccatacc aaatttttca tatt 24
<210> 63
   <211> 204
   <212> PRT
   <213> Vespula maculifrons
<400> 63
   Asn Asn Tyr Cys Lys Ile Lys Cys Leu Lys Gly Gly Val His Thr Ala
   1               5                   10                  15
   Cys Lys Tyr Gly Ser Leu Lys Pro Asn Cys Gly Asn Lys Lys Val Val
               20                  25                  30
   Ser Tyr Gly Leu Thr Lys Gln Glu Lys Gln Asp Ile Leu Lys Glu His
           35                  40                  45
   Asn Asp Phe Arg Gln Lys Ile Ala Arg Gly Leu Glu Thr Arg Gly Asn
       50                  55                  60
   Pro Gly Pro Gln Pro Pro Ala Lys Asn Met Lys Asn Leu Val Trp Ser
   65                  70                  75                  80
   Asp Glu Leu Ala Tyr Ile Ala Gln Val Trp Ala Asn Gln Cys Gln Tyr
                   85                  90                  95
   Gly His Asp Thr Cys Arg Asp Val Ala Lys Tyr Gln Val Gly Gln Asn
               100                 105                 110
   Val Ala Leu Thr Gly Ser Thr Ala Ala Val Tyr Asn Asp Pro Val Lys
           115                 120                 125
   Leu Val Lys Met Trp Glu Asp Glu Val Lys Asp Tyr Asn Pro Lys Lys
       130                 135                 140
   Lys Phe Ser Glu Asn Asn Phe Leu Lys Ile Gly His Tyr Thr Gln Met
   145                 150                 155                 160
   Val Trp Ala Asn Thr Lys Glu Val Gly Cys Gly Ser Ile Lys Tyr Ile
                   165                 170                 175
   Gln Glu Asn Trp His Lys His Tyr Leu Val Cys Asn Tyr Gly Pro Ser
               180                 185                 190
   Gly Asn Phe Gln Asn Glu Glu Leu Tyr Gln Thr Lys
           195                 200
<210> 64
   <211> 204
   <212> PRT
   <213> Vespula vulgaris
<400> 64
   Asn Asn Tyr Cys Lys Ile Lys Cys Leu Lys Gly Gly Val His Thr Ala
   1 5 10 15
   Cys Lys Tyr Gly Ser Leu Lys Pro Asn Cys Gly Asn Lys Val Val Val
   20 25 30
   Ser Tyr Gly Leu Thr Lys Gln Glu Lys Gln Asp Ile Leu Lys Glu His
   35 40 45
   Asn Asp Phe Arg Gln Lys Ile Ala Arg Gly Leu Glu Thr Arg Gly Asn
   50 55 60
   Pro Gly Pro Gln Pro Pro Ala Lys Asn Met Lys Asn Leu Val Trp Asn
   65 70 75 80
   Asp Glu Leu Ala Tyr Val Ala Gln Val Trp Ala Asn Gln Cys Gln Tyr
   85 90 95
   Gly His Asp Thr Cys Arg Asp Val Ala Lys Tyr Gln Val Gly Gln Asn
   100 105 110
   Val Ala Leu Thr Gly Ser Thr Ala Ala Lys Tyr Asp Asp Pro Val Lys
   115 120 125
   Leu Val Lys Met Trp Glu Asp Glu Val Lys Asp Tyr Asn Pro Lys Lys
   130 135 140
   Lys Phe Ser Gly Asn Asp Phe Leu Lys Thr Gly His Tyr Thr Gln Met
   145 150 155 160
   Val Trp Ala Asn Thr Lys Glu Val Gly Cys Gly Ser Ile Lys Tyr Ile
   165 170 175
   Gln Glu Lys Trp His Lys His Tyr Leu Val Cys Asn Tyr Gly Pro Ser
   180 185 190
   Gly Asn Phe Met Asn Glu Glu Leu Tyr Gln Thr Lys
   195 200
<210> 65
   <211> 204
   <212> PRT
   <213> Vespula flavopilosa
<400> 65
   Asn Asn Tyr Cys Lys Ile Lys Cys Leu Lys Gly Gly Val His Thr Ala
   1               5                   10                  15
   Cys Lys Tyr Gly Ser Leu Lys Pro Asn Cys Gly Asn Lys Val Val Val
               20                  25                  30
   Ser Tyr Gly Leu Thr Lys Gln Glu Lys Gln Asp Ile Leu Lys Glu His
           35                  40                  45
   Asn Asp Phe Arg Gln Lys Ile Ala Arg Gly Leu Glu Thr Arg Gly Asn
       50                  55                  60
   Pro Gly Pro Gln Pro Pro Ala Lys Asn Met Lys Asn Leu Val Trp Asn
   65                  70                  75                  80
   Asp Glu Leu Ala Tyr Val Ala Gln Val Trp Ala Asn Gln Cys Gln Tyr
                   85                  90                  95
   Gly His Asp Thr Cys Arg Asp Ile Ala Lys Tyr Gln Val Gly Gln Asn
               100                 105                 110
   Val Ala Leu Thr Gly Ser Thr Ala Ala Lys Tyr Asp Asp Pro Val Lys
           115                 120                 125
   Leu Val Lys Met Trp Glu Asp Glu Val Lys Asp Tyr Asn Pro Lys Lys
       130                 135                 140
   Lys Phe Ser Gly Asn Asn Phe Leu Lys Thr Gly His Tyr Thr Gln Met
   145                 150                 155                 160
   Val Trp Ala Asn Thr Lys Glu Val Gly Cys Gly Ser Ile Lys Phe Ile
                   165                 170                 175
   Gln Glu Lys Trp His Lys His Tyr Leu Val Cys Asn Tyr Gly Pro Ser
               180                 185                 190
   Gly Asn Phe Gln Asn Glu Glu Leu Tyr Gln Thr Lys
           195                 200
<210> 66
   <211> 204
   <212> PRT
   <213> Vespula pensylvanica
<400> 66
   Asn Asn Tyr Cys Lys Ile Lys Cys Leu Lys Gly Gly Val His Thr Ala
   1               5                   10                  15
   Cys Lys Tyr Gly Ser Leu Lys Pro Asn Cys Gly Asn Lys Ile Val Val
               20                  25                  30
   Ser Tyr Gly Leu Thr Lys Glu Glu Lys Gln Asp Ile Leu Lys Glu His
           35                  40                  45
   Asn Asp Phe Arg Gln Lys Ile Ala Arg Gly Leu Glu Thr Arg Gly Asn
       50                  55                  60
   Pro Gly Pro Gln Pro Pro Ala Lys Asn Met Lys Asn Leu Val Trp Asn
   65                  70                  75                  80
   Asp Glu Leu Ala Tyr Val Ala Gln Val Trp Ala Asn Gln Cys Gln Tyr
                   85                  90                  95
   Gly His Asp Thr Cys Arg Asp Val Ala Lys Tyr Pro Val Gly Gln Asn
               100                 105                 110
   Val Ala Leu Thr Gly Ser Thr Ala Asp Lys Tyr Asp Asn Pro Val Lys
           115                 120                 125
   Leu Val Lys Met Trp Glu Asp Glu Val Lys Asp Tyr Asn Pro Lys Lys
       130                 135                 140
   Lys Phe Ser Glu Asn Asn Phe Asn Lys Ile Gly His Tyr Thr Gln Met
   145                 150                 155                 160
   Val Trp Ala Asn Thr Lys Glu Ile Gly Cys Gly Ser Ile Lys Tyr Ile
                   165                 170                 175
   Gln Asn Glu Trp His Lys His Tyr Leu Val Cys Asn Tyr Gly Pro Ser
               180                 185                 190
   Gly Asn Phe Gly Asn Glu Glu Leu Tyr Gln Thr Lys
           195                 200
<210> 67
   <211> 204
   <212> PRT
   <213> Vespula germanica
<400> 67
   Asn Asn Tyr Cys Lys Ile Lys Cys Leu Lys Gly Gly Val His Thr Ala
   1               5                   10                  15
   Cys Lys Tyr Glu Ser Leu Lys Pro Asn Cys Ala Asn Lys Lys Val Val
               20                  25                  30
   Ala Tyr Gly Leu Thr Lys Gln Glu Lys Gln Asp Ile Leu Lys Glu His
           35                  40                  45
   Asn Asp Phe Arg Gln Lys Ile Ala Arg Gly Leu Glu Thr Arg Gly Asn
       50                  55                  60
   Pro Gly Pro Gln Pro Pro Ala Lys Asn Met Lys Asn Leu Val Trp Ser
   65                  70                  75                  80
   Asp Glu Leu Ala Tyr Ile Ala Gln Val Trp Ala Asn Gln Cys Gln Tyr
                   85                  90                  95
   Gly His Asp Thr Cys Arg Asp Val Ala Lys Tyr Pro Val Gly Gln Asn
               100                 105                 110
   Val Ala Leu Thr Gly Ser Thr Ala Ala Lys Tyr Asp Asn Pro Val Lys
           115                 120                 125
   Leu Val Lys Met Trp Glu Asp Glu Val Lys Asp Tyr Asn Pro Lys Lys
       130                 135                 140
   Lys Phe Ser Glu Asn Asn Phe Leu Lys Ile Gly His Tyr Thr Gln Met
   145                 150                 155                 160
   Val Trp Ala Asn Thr Lys Glu Val Gly Cys Gly Ser Ile Lys Tyr Ile
                   165                 170                 175
   Gln Asp Lys Trp His Lys His Tyr Leu Val Cys Asn Tyr Gly Pro Ser
               180                 185                 190
   Gly Asn Phe Gly Asn Glu Glu Leu Tyr Gln Thr Lys
           195                 200
<210> 68
   <211> 206
   <212> PRT
   <213> Vespula vidua
<400> 68
   Lys Val Asn Tyr Cys Lys Ile Lys Cys Leu Lys Gly Gly Val His Thr
   1               5                   10                  15
   Ala Cys Lys Tyr Gly Thr Ser Thr Lys Pro Asn Cys Gly Lys Met Val
               20                  25                  30
   Val Lys Ala Tyr Gly Leu Thr Glu Ala Glu Lys Gln Glu Ile Leu Lys
           35                  40                  45
   Val His Asn Asp Phe Arg Gln Lys Val Ala Lys Gly Leu Glu Thr Arg
       50                  55                  60
   Gly Asn Pro Gly Pro Gln Pro Pro Ala Lys Asn Met Asn Asn Leu Val
   65                  70                  75                  80
   Trp Asn Asp Glu Leu Ala Asn Ile Ala Gln Val Trp Ala Ser Gln Cys
                   85                  90                  95
   Asn Tyr Gly His Asp Thr Cys Lys Asp Thr Glu Lys Tyr Pro Val Gly
               100                 105                 110
   Gln Asn Ile Ala Lys Arg Ser Thr Thr Ala Ala Leu Phe Asp Ser Pro
           115                 120                 125
   Gly Lys Leu Val Lys Met Trp Glu Asn Glu Val Lys Asp Phe Asn Pro
       130                 135                 140
   Asn Ile Glu Trp Ser Lys Asn Asn Leu Lys Lys Thr Gly His Tyr Thr
   145                 150                 155                 160
   Gln Met Val Trp Ala Lys Thr Lys Glu Ile Gly Cys Gly Ser Val Lys
                   165                 170                 175
   Tyr Val Lys Asp Glu Trp Tyr Thr His Tyr Leu Val Cys Asn Tyr Gly
               180                 185                 190
   Pro Ser Gly Asn Phe Arg Asn Glu Lys Leu Tyr Glu Lys Lys
           195                 200                 205
<210> 69
   <211> 205
   <212> PRT
   <213> Vespula squamosa
<400> 69
   Val Asp Tyr Cys Lys Ile Lys Cys Leu Lys Gly Gly Val His Thr Ala
   1               5                   10                  15
   Cys Lys Tyr Gly Thr Ser Thr Lys Pro Asn Cys Gly Asn Met Val Val
               20                  25                  30
   Lys Ser Tyr Gly Val Thr Gln Ala Glu Lys Gln Glu Ile Leu Lys Ile
           35                  40                  45
   His Asn Asp Phe Arg Asn Lys Val Ala Arg Gly Leu Glu Thr Arg Gly
       50                  55                  60
   Asn Pro Gly Pro Gln Pro Pro Ala Lys Asn Met Asn Asn Leu Val Trp
   65                  70                  75                  80
   Asn Asn Glu Leu Ala Asn Ile Ala Gln Ile Trp Ala Ser Gln Cys Lys
                   85                  90                  95
   Tyr Gly His Asp Thr Cys Lys Asp Thr Thr Lys Tyr Asn Val Gly Gln
               100                 105                 110
   Asn Ile Ala Val Ser Ser Ser Thr Ala Ala Val Tyr Glu Asn Val Gly
           115                 120                 125
   Asn Leu Val Lys Ala Trp Glu Asn Glu Val Lys Asp Phe Asn Pro Thr
       130                 135                 140
   Ile Ser Trp Glu Gln Asn Glu Phe Lys Lys Ile Gly His Tyr Thr Gln
   145 150 155 160
   Met Val Trp Ala Lys Thr Lys Glu Ile Gly Cys Gly Ser Ile Lys Tyr
   165 170 175
   Val Asp Asn Asn Trp Tyr Thr His Tyr Leu Val Cys Asn Tyr Gly Pro
   180 185 190
   Ala Gly Asn Phe Gly Asn Gln Glu Val Tyr Glu Arg Lys
   195 200 205
<210> 70
   <211> 204
   <212> PRT
   <213> Dolichovespula maculata
<400> 70
   Asn Asn Tyr Cys Lys Ile Lys Cys Arg Lys Gly Ile His Thr Leu Cys
   1 5 10 15
   Lys Phe Gly Thr Ser Met Lys Pro Asn Cys Gly Arg Asn Val Val Lys
   20 25 30
   Ala Tyr Gly Leu Thr Asn Asp Glu Lys Asn Glu Ile Leu Lys Arg His
   35 40 45
   Asn Asp Phe Arg Gln Asn Val Ala Lys Gly Leu Glu Thr Arg Gly Lys
   50 55 60
   Pro Gly Pro Gln Pro Pro Ala Lys Asn Met Asn Val Leu Val Trp Asn
   65 70 75 80
   Asp Glu Leu Ala Lys Ile Ala Gln Thr Trp Ala Asn Gln Cys Asp Phe
   85 90 95
   Asn His Asp Asp Cys Arg Asn Thr Ala Lys Tyr Gln Val Gly Gln Asn
   100 105 110
   Ile Ala Ile Ser Ser Thr Thr Ala Thr Gln Phe Asp Arg Pro Ser Lys
   115 120 125
   Leu Ile Lys Gln Trp Glu Asp Glu Val Thr Glu Phe Asn Tyr Lys Val
   130 135 140
   Gly Leu Gln Asn Ser Asn Phe Arg Lys Val Gly His Tyr Thr Gln Met
   145 150 155 160
   Val Trp Gly Lys Thr Lys Glu Ile Gly Cys Gly Ser Ile Lys Tyr Ile
   165 170 175
   Glu Asp Asn Trp Tyr Thr His Tyr Leu Val Cys Asn Tyr Gly Pro Gly
   180 185 190
   Gly Asn Asp Phe Asn Gln Pro Ile Tyr Glu Arg Lys
   195 200
<210> 71
   <211> 203
   <212> PRT
   <213> Dolichovespula arenaria
<400> 71
   Asn Asn Tyr Cys Lys Ile Cys Pro Lys Gly Thr His Thr Leu Cys Lys
   1 5 10 15
   Tyr Gly Thr Ser Met Lys Pro Asn Cys Gly Gly Lys Ile Val Lys Ser
   20 25 30
   Tyr Gly Val Thr Asn Asp Glu Lys Asn Glu Ile Val Lys Arg His Asn
   35 40 45
   Glu Phe Arg Gln Lys Val Ala Gln Gly Leu Glu Thr Arg Gly Asn Pro
   50 55 60
   Gly Pro Gln Pro Pro Ala Lys Asn Met Asn Leu Leu Val Trp Asn Asp
   65 70 75 80
   Glu Leu Ala Lys Ile Ala Gln Thr Trp Ala Asn Gln Cys Asn Phe Gly
   85 90 95
   His Asp Gln Cys Arg Asn Thr Ala Lys Tyr Pro Val Gly Gln Asn Val
   100 105 110
   Ala Ile Ala Ser Thr Thr Gly Asn Ser Tyr Gln Thr Met Ser Tyr Leu
   115 120 125
   Ile Lys Met Trp Glu Asp Glu Val Lys Asp Tyr Asn Pro His Lys Asp
   130 135 140
   Leu Met His Asn Asn Phe Ser Lys Val Gly His Tyr Thr Gln Met Val
   145 150 155 160
   Trp Gly Lys Thr Lys Glu Ile Gly Cys Gly Ser Val Lys Tyr Ile Glu
   165 170 175
   Asn Lys Trp His Thr His Tyr Leu Val Cys Asn Tyr Gly Pro Ala Gly
   180 185 190
   Asn Tyr Met Asn Gln Pro Val Tyr Glu Arg Lys
   195 200
<210> 72
   <211> 205
   <212> PRT
   <213> Dolichovespula maculata
<400> 72
   Asn Asn Tyr Cys Lys Ile Lys Cys Ser Arg Gly Ile His Thr Leu Cys
   1 5 10 15
   Lys Phe Gly Thr Ser Met Lys Pro Asn Cys Gly Ser Lys Leu Val Lys
   20 25 30
   Val His Gly Val Ser Asn Asp Glu Lys Asn Glu Ile Val Asn Arg His
           35                  40                      45
   Asn Gln Phe Arg Gln Lys Val Ala Lys Gly Leu Glu Thr Arg Gly Asn
       50                  55                  60
   Pro Gly Pro Gln Pro Pro Ala Lys Asn Met Asn Val Leu Val Trp Asn
   65                  70                  75                  80
   Asp Glu Leu Ala Lys Ile Ala Gln Thr Trp Ala Asn Gln Cys Ser Phe
                   85                  90                  95
   Gly His Asp Gln Cys Arg Asn Thr Glu Lys Tyr Gln Val Gly Gln Asn
               100                 105                 110
   Val Ala Ile Ala Ser Thr Thr Gly Asn Ser Tyr Ala Thr Met Ser Lys
           115                 120                 125
   Leu Ile Glu Met Trp Glu Asn Glu Val Lys Asp Phe Asn Pro Lys Lys
       130                 135                 140
   Gly Thr Met Gly Asp Asn Asn Phe Ser Lys Val Gly His Tyr Thr Gln
   145                 150                 155                 160
   Met Val Trp Gly Lys Thr Lys Glu Ile Gly Cys Gly Ser Val Lys Tyr
                   165                 170                 175
   Ile Glu Asn Asn Trp His Thr His Tyr Leu Val Cys Asn Tyr Gly Pro
               180                 185                 190
   Ala Gly Asn Tyr Met Asp Gln Pro Ile Tyr Glu Arg Lys
           195                 200                 205
<210> 73
   <211> 202
   <212> PRT
   <213> Vespa mandarinia
<400> 73
   Asn Asn Tyr Cys Lys Ile Lys Cys Arg Ser Gly Ile His Thr Leu Cys
   1 5 10 15
   Lys Phe Gly Ile Ser Thr Lys Pro Asn Cys Gly Lys Asn Val Val Lys
   20 25 30
   Ala Ser Gly Leu Thr Lys Ala Glu Lys Leu Glu Ile Leu Lys Gln His
   35 40 45
   Asn Glu Phe Arg Gln Lys Val Ala Arg Gly Leu Glu Thr Arg Gly Lys
   50 55 60
   Pro Gly Pro Gln Pro Pro Ala Lys Ser Met Asn Thr Leu Val Trp Asn
   65 70 75 80
   Asp Glu Leu Ala Gln Ile Ala Gln Val Trp Ala Gly Gln Cys Asp Tyr
   85 90 95
   Gly His Asp Val Cys Arg Asn Thr Ala Lys Tyr Ser Val Gly Gln Asn
   100 105 110
   Ile Ala Glu Asn Gly Ser Thr Ala Ala Ser Phe Ala Ser Val Ser Asn
   115 120 125
   Met Val Gln Met Trp Ala Asp Glu Val Lys Asn Tyr Gln Tyr Gly Ser
   130 135 140
   Thr Lys Asn Lys Leu Ile Glu Val Gly His Tyr Thr Gln Met Val Trp
   145 150 155 160
   Ala Lys Thr Lys Glu Ile Gly Cys Gly Ser Ile Lys Tyr Ile Glu Asn
   165 170 175
   Gly Trp His Arg His Tyr Leu Val Cys Asn Tyr Gly Pro Ala Gly Asn
   180 185 190
   Ile Gly Asn Glu Pro Ile Tyr Glu Arg Lys
   195 200
<210> 74
   <211> 202
   <212> PRT
   <213> Vespa crabro
<400> 74
   Asn Asn Tyr Cys Lys Ile Lys Cys Arg Ser Gly Ile His Thr Leu Cys
   1               5                   10                  15
   Lys Tyr Gly Thr Ser Thr Lys Pro Asn Cys Gly Lys Asn Val Val Lys
               20                  25                  30
   Ala Ser Gly Leu Thr Lys Gln Glu Asn Leu Glu Ile Leu Lys Gln His
           35                  40                  45
   Asn Glu Phe Arg Gln Lys Val Ala Arg Gly Leu Glu Thr Arg Gly Asn
       50                  55                  60
   Pro Gly Pro Gln Pro Pro Ala Lys Ser Met Asn Thr Leu Val Trp Asn
   65                  70                  75                  80
   Asp Glu Leu Ala Gln Ile Ala Gln Val Trp Ala Asn Gln Cys Asn Tyr
                   85                  90                  95
   Gly His Asp Asn Cys Arg Asn Ser Ala Lys Tyr Ser Val Gly Gln Asn
               100                 105                 110
   Ile Ala Glu Gly Ser Thr Thr Ala Asp Asn Phe Gly Ser Val Ser Asn
           115                 120                 125
   Met Val Lys Met Trp Glu Asp Glu Val Lys Asp Tyr Gln Tyr Gly Ser
       130                 135                 140
   Pro Lys Asn Lys Leu Asn Lys Val Gly His Tyr Thr Gln Met Val Trp
   145                 150                 155                 160
   Ala Lys Thr Lys Glu Ile Gly Cys Gly Ser Ile Lys Tyr Ile Glu Asn
                   165                 170                 175
   Gly Trp His Arg His Tyr Leu Val Cys Asn Tyr Gly Pro Ala Gly Asn
               180                 185                 190
   Val Gly Asn Glu Pro Ile Tyr Glu Arg Lys
           195                 200
<210> 75
   <211> 202
   <212> PRT
   <213> Vespa crabro
<400> 75
   Asn Asn Tyr Cys Lys Ile Lys Cys Arg Ser Gly Ile His Thr Leu Cys
   1               5                   10                  15
   Lys Tyr Gly Thr Ser Thr Lys Pro Asn Cys Gly Lys Asn Val Val Lys
               20                  25                  30
   Ala Ser Gly Leu Thr Lys Gln Glu Asn Leu Glu Ile Leu Lys Gln His
           35                  40                  45
   Asn Glu Phe Arg Gln Lys Val Ala Arg Gly Leu Glu Thr Arg Gly Asn
       50                  55                  60
   Pro Gly Pro Gln Pro Pro Ala Lys Ser Met Asn Thr Leu Val Trp Asn
   65                  70                  75                  80
   Asp Glu Leu Ala Gln Ile Ala Gln Val Trp Ala Asn Gln Cys Asn Tyr
                   85                  90                  95
   Gly His Asp Asn Cys Arg Asn Ser Ala Lys Tyr Ser Val Gly Gln Asn
               100                 105                 110
   Ile Ala Glu Gly Ser Thr Ser Ala Asp Asn Phe Val Asn Val Ser Asn
           115                 120                 125
   Met Val Lys Met Trp Glu Asp Glu Val Lys Asp Tyr Gln Tyr Gly Ser
       130                 135                     140
   Pro Lys Asn Lys Leu Asn Lys Val Gly His Tyr Thr Gln Met Val Trp
   145                 150                 155                 160
   Ala Lys Thr Lys Glu Ile Gly Cys Gly Ser Glu Asp Tyr Ile Glu Asp
                   165                 170                 175
   Gly Trp His Arg His Tyr Leu Val Cys Asn Tyr Gly Pro Ala Gly Asn
               180                 185                 190
   Val Gly Asn Glu Pro Ile Tyr Glu Arg Lys
           195                 200
<210> 76
   <211> 205
   <212> PRT
   <213> Polistes fuscatus
<400> 76
   Val Asp Tyr Cys Lys Ile Lys Cys Ser Ser Gly Ile His Thr Val Cys
   1               5                   10                  15
   Gln Tyr Gly Glu Ser Thr Lys Pro Ser Lys Asn Cys Ala Asp Lys Val
               20                  25                  30
   Ile Lys Ser Val Gly Pro Thr Glu Glu Glu Lys Lys Leu Ile Val Asn
           35                  40                  45
   Glu His Asn Arg Phe Arg Gln Lys Val Ala Gln Gly Leu Glu Thr Arg
       50                  55                  60
   Gly Asn Pro Gly Pro Gln Pro Ala Ala Ser Asp Met Asn Asn Leu Val
   65                  70                  75                  80
   Trp Asn Asp Glu Leu Ala His Ile Ala Gln Val Trp Ala Ser Gln Cys
                   85                  90                  95
   Gln Ile Leu Val His Asp Lys Cys Arg Asn Thr Ala Lys Tyr Gln Val
               100                 105                 110
   Gly Gln Asn Ile Ala Tyr Ala Gly Gly Ser Lys Leu Pro Asp Val Val
           115                 120                 125
   Ser Leu Ile Lys Leu Trp Glu Asn Glu Val Lys Asp Phe Asn Tyr Asn
       130                 135                 140
   Lys Gly Ile Thr Lys Gln Asn Phe Gly Lys Val Gly His Tyr Thr Gln
   145                 150                 155                 160
   Met Ile Trp Ala Lys Thr Lys Glu Ile Gly Cys Gly Ser Leu Lys Tyr
                   165                 170                 175
   Met Lys Asn Asn Met Gln His His Tyr Leu Ile Cys Asn Tyr Gly Pro
               180                 185                 190
   Ala Gly Asn Tyr Leu Gly Gln Leu Pro Tyr Thr Lys Lys
           195                 200                 205
<210> 77
   <211> 205
   <212> PRT
   <213> Polistes exclamans
<400> 77
   Val Asp Tyr Cys Lys Ile Lys Cys Pro Ser Gly Ile His Thr Val Cys
   1               5                   10                  15
   Gln Tyr Gly Glu Ser Thr Lys Pro Ser Lys Asn Cys Ala Gly Lys Val
               20                  25                  30
   Ile Lys Ser Val Gly Pro Thr Glu Glu Glu Lys Lys Leu Ile Val Ser
           35                  40                  45
   Glu His Asn Arg Phe Arg Gln Lys Val Ala Gln Gly Leu Glu Thr Arg
       50                  55                      60
   Gly Asn Pro Gly Pro Gln Pro Ala Ala Ser Asp Met Asn Asp Leu Val
   65                  70                  75                  80
   Trp Asn Asp Glu Leu Ala His Ile Ala Gln Val Trp Ala Ser Gln Cys
                   85                  90                  95
   Gln Phe Leu Val His Asp Lys Cys Arg Asn Thr Ala Lys Tyr Pro Val
               100                 105                 110
   Gly Gln Asn Ile Ala Tyr Ala Gly Gly Ser Lys Leu Pro Asp Val Val
           115                 120                 125
   Ser Leu Ile Lys Leu Trp Glu Asn Glu Val Lys Asp Phe Asn Tyr Asn
       130                 135                 140
   Thr Gly Ile Thr Lys Gln Asn Phe Ala Lys Ile Gly His Tyr Thr Gln
   145                 150                 155                 160
   Met Val Trp Gly Lys Thr Lys Glu Ile Gly Cys Gly Ser Leu Lys Tyr
                   165                 170                 175
   Ile Glu Asn Lys Met Gln Asn His Tyr Leu Ile Cys Asn Tyr Gly Pro
               180                 185                 190
   Ala Gly Asn Tyr Leu Gly Gln Leu Pro Tyr Thr Lys Lys
           195                 200                 205
<210> 78
   <211> 205
   <212> PRT
   <213> Polistes annularis
<400> 78
   Val Asp Tyr Cys Lys Ile Lys Cys Pro Ser Gly Ile His Thr Val Cys
   1               5                   10                  15
   Gln Tyr Gly Glu Ser Thr Lys Pro Ser Lys Asn Cys Ala Gly Lys Val
               20                  25                  30
   Ile Lys Ser Val Gly Pro Thr Glu Glu Glu Lys Lys Leu Ile Val Ser
           35                  40                  45
   Glu His Asn Arg Phe Arg Gln Lys Val Ala Gln Gly Leu Glu Thr Arg
       50                  55                  60
   Gly Asn Pro Gly Pro Gln Pro Ala Ala Ser Asp Met Asn Asp Leu Val
   65                  70                  75                  80
   Trp Asn Asp Glu Leu Ala His Ile Ala Gln Val Trp Ala Ser Gln Cys
                   85                  90                  95
   Gln Phe Leu Val His Asp Lys Cys Arg Asn Thr Ala Lys Tyr Pro Val
               100                 105                 110
   Gly Gln Asn Ile Ala Tyr Ala Gly Gly Ser Asn Leu Pro Asp Val Val
           115                 120                 125
   Ser Leu Ile Lys Leu Trp Glu Asn Glu Val Lys Asp Phe Asn Tyr Asn
       130                 135                 140
   Thr Gly Ile Thr Lys Gln Asn Phe Ala Lys Ile Gly His Tyr Thr Gln
   145                 150                 155                 160
   Met Val Trp Gly Lys Thr Lys Glu Ile Gly Cys Gly Ser Leu Lys Tyr
                   165                 170                 175
   Met Glu Asn Asn Met Gln Asn His Tyr Leu Ile Cys Asn Tyr Gly Pro
               180                 185                 190
   Ala Gly Asn Tyr Leu Gly Gln Leu Pro Tyr Thr Lys Lys
           195                 200                 205
<210> 79 <211> 212
   <212> PRT
   <213> Solenopsis invicta
<400> 79
   Thr Asn Tyr Cys Asn Leu Gln Ser Cys Lys Arg Asn Asn Ala Ile His
   1               5                   10                  15
   Thr Met Cys Gln Tyr Thr Ser Pro Thr Pro Gly Pro Met Cys Leu Glu
               20                  25                  30
   Tyr Ser Asn Val Gly Phe Thr Asp Ala Glu Lys Asp Ala Ile Val Asn
           35                  40                  45
   Lys His Asn Glu Leu Arg Gln Arg Val Ala Ser Gly Lys Glu Met Arg
       50                  55                  60
   Gly Thr Asn Gly Pro Gln Pro Pro Ala Val Lys Met Pro Asn Leu Thr
   65                  70                  75                  80
   Trp Asp Pro Glu Leu Ala Thr Ile Ala Gln Arg Trp Ala Asn Gln Cys
                   85                  90                  95
   Thr Phe Glu His Asp Ala Cys Arg Asn Val Glu Arg Phe Ala Val Gly
               100                 105                 110
   Gln Asn Ile Ala Ala Thr Ser Ser Ser Gly Lys Asn Lys Ser Thr Pro
           115                 120                 125
   Asn Glu Met Ile Leu Leu Trp Tyr Asn Glu Val Lys Asp Phe Asp Asn
       130                 135                 140
   Arg Trp Ile Ser Ser Phe Pro Ser Asp Asp Asn Ile Leu Met Lys Val
   145                 150                 155                 160
   Glu His Tyr Thr Gln Ile Val Trp Ala Lys Thr Ser Lys Ile Gly Cys
                   165                 170                 175
   Ala Arg Ile Met Phe Lys Glu Pro Asp Asn Trp Thr Lys His Tyr Leu
               180                 185                 190
   Val Cys Asn Tyr Gly Pro Ala Gly Asn Val Leu Gly Ala Pro Ile Tyr
           195                 200                 205
   Glu Ile Lys Lys
       210
<210> 80
   <211> 211
   <212> PRT
   <213> Solenopsis richteri
<400> 80
   Thr Asn Tyr Cys Asn Leu Gln Ser Cys Lys Arg Asn Asn Ala Ile His
   1               5                   10                  15
   Thr Met Cys Gln Tyr Thr Ser Pro Thr Pro Gly Pro Met Cys Leu Glu
               20                  25                  30
   Tyr Ser Asn Val Gly Phe Thr Asp Ala Glu Lys Asp Ala Ile Val Asn
           35                  40                  45
   Lys His Asn Glu Leu Arg Gln Arg Val Ala Ser Gly Lys Glu Met Arg
       50                  55                  60
   Gly Thr Asn Gly Pro Gln Pro Pro Ala Val Lys Met Pro Asn Leu Thr
   65                  70                  75                  80
   Trp Asp Pro Glu Leu Ala Thr Ile Ala Gln Arg Trp Ala Asn Gln Cys
                   85                  90                  95
   Thr Phe Glu His Asp Ala Cys Arg Asn Val Glu Arg Phe Ala Val Gly
               100                 105                 110
   Gln Asn Ile Ala Ala Thr Ser Ser Ser Gly Lys Asn Lys Ser Thr Leu
           115                 120                 125
   Ser Asp Met Ile Leu Leu Trp Tyr Asn Glu Val Lys Asp Phe Asp Asn
   130 135 140
   Arg Trp Ile Ser Ser Phe Pro Ser Asp Gly Asn Ile Leu Met His Val
   145 150 155 160
   Gly His Tyr Thr Gln Ile Val Trp Ala Lys Thr Lys Lys Ile Gly Cys
   165 170 175
   Gly Arg Ile Met Phe Lys Glu Asp Asn Trp Asn Lys His Tyr Leu Val
   180 185 190
   Cys Asn Tyr Gly Pro Ala Gly Asn Val Leu Gly Ala Gln Ile Tyr Glu
   195 200 205
   Ile Lys Lys
   210
<210> 81
   <211> 204
   <212> PRT
   <213> Vespula vulgaris
<400> 81
   Asn Asn Tyr Cys Lys Ile Lys Cys Leu Lys Gly Gly Val His Thr Ala
   1 5 10 15
   Cys Lys Tyr Gly Ser Leu Lys Pro Asn Cys Gly Asn Lys Val Val Val
   20 25 30
   Ser Tyr Gly Leu Thr Lys Gln Glu Lys Gln Asp Ile Leu Lys Glu His
   35 40 45
   Asn Asp Phe Arg Gln Lys Ile Ala Arg Gly Leu Glu Thr Arg Gly Asn
   50 55 60
   Pro Gly Pro Gln Pro Pro Ala Lys Asn Met Lys Asn Leu Val Trp Asn
   65 70 75 80
   Asp Glu Leu Ala Tyr Val Ala Gln Val Trp Ala Asn Gln Cys Gln Tyr
   85 90 95
   Gly His Asp Thr Cys Arg Asp Val Ala Lys Tyr Gln Val Gly Gln Asn
   100 105 110
   Val Ala Leu Thr Gly Ser Thr Ala Ala Lys Tyr Asp Asp Pro Val Lys
   115 120 125
   Leu Val Lys Met Trp Glu Asp Glu Val Lys Asp Tyr Asn Pro Lys Lys
   130 135 140
   Lys Phe Ser Gly Asn Asp Phe Leu Lys Thr Gly His Tyr Thr Gln Met
   145 150 155 160
   Val Trp Ala Asn Thr Lys Glu Val Gly Cys Gly Ser Ile Lys Tyr Ile
   165 170 175
   Gln Glu Lys Trp His Lys His Tyr Leu Val Cys Asn Tyr Gly Pro Ser
   180 185 190
   Gly Asn Phe Met Asn Glu Glu Leu Tyr Gln Thr Lys
   195 200
<210> 82
   <211> 212
   <212> PRT
   <213> Solenopsis invicta
<400> 82
   Thr Asn Tyr Cys Asn Leu Gln Ser Cys Lys Arg Asn Asn Ala Ile His
   1 5 10 15
   Thr Met Cys Gln Tyr Thr Ser Pro Thr Pro Gly Pro Met Cys Leu Glu
   20 25 30
   Tyr Ser Asn Val Gly Phe Thr Asp Ala Glu Lys Asp Ala Ile Val Asn
   35 40 45
   Lys His Asn Glu Leu Arg Gln Arg Val Ala Ser Gly Lys Glu Met Arg
   50 55 60
   Gly Thr Asn Gly Pro Gln Pro Pro Ala Val Lys Met Pro Asn Leu Thr
   65 70 75 80
   Trp Asp Pro Glu Leu Ala Thr Ile Ala Gln Arg Trp Ala Asn Gln Cys
   85 90 95
   Thr Phe Glu His Asp Ala Cys Arg Asn Val Glu Arg Phe Ala Val Gly
   100 105 110
   Gln Asn Ile Ala Ala Thr Ser Ser Ser Gly Lys Asn Lys Ser Thr Pro
   115 120 125
   Asn Glu Met Ile Leu Leu Trp Tyr Asn Glu Val Lys Asp Phe Asp Asn
   130 135 140
   Arg Trp Ile Ser Ser Phe Pro Ser Asp Asp Asn Ile Leu Met Lys Val
   145 150 155 160
   Glu His Tyr Thr Gln Ile Val Trp Ala Lys Thr Ser Lys Ile Gly Cys
   165 170 175
   Ala Arg Ile Met Phe Lys Glu Pro Asp Asn Trp Thr Lys His Tyr Leu
   180 185 190
   Val Cys Asn Tyr Gly Pro Ala Gly Asn Val Leu Gly Ala Pro Ile Tyr
   195 200 205
   Glu Ile Lys Lys
   210
<210> 83
   <211> 136
   <212> PRT
   <213> Lycopersicon esculentum
<400> 83
   Gln Asn Ser Pro Gln Asp Tyr Leu Ala Val His Asn Asp Ala Arg Ala
   1 5 10 15
   Gln Val Gly Val Gly Pro Met Ser Trp Asp Ala Asn Leu Ala Ser Arg
   20 25 30
   Ala Gln Asn Tyr Ala Asn Ser Arg Ala Gly Asp Cys Asn Leu Ile His
   35 40 45
   Ser Gly Ala Gly Glu Asn Leu Ala Lys Gly Gly Gly Asp Phe Thr Gly
   50 55 60
   Arg Ala Ala Val Gln Leu Trp Val Ser Glu Arg Pro Ser Tyr Asn Tyr
   65 70 75 80
   Ala Thr Asn Gln Cys Val Gly Gly Lys Lys Cys Arg His Tyr Thr Gln
   85 90 95
   Val Val Trp Arg Asn Ser Val Arg Leu Gly Cys Gly Arg Ala Arg Cys
   100 105 110
   Asn Asn Asn Gly Trp Trp Phe Ile Ser Cys Asn Tyr Asp Pro Val Gly
   115 120 125
   Asn Trp Ile Gly Gln Arg Pro Tyr
   130 135
<210> 84
   <211> 187
   <212> PRT
   <213> Schizophyllum commune
<400> 84
   Ser Pro Ala Pro Val Asp Val Asp Ala Arg Ala Pro Val Ala Leu Asp
   1 5 10 15
   Ser Arg Ser Ile Asp Ile Asp Ser Arg Ser Ala Asp Ala Leu Ala Asn
               20                  25                  30
   Arg Ala Ala Pro Pro Gln Ser Glu Ile Asp Gln Trp Leu Lys Ala His
           35                  40                  45
   Asn Asn Glu Arg Ala Gln His Gly Ala Val Ala Leu Val Trp Asn Gln
       50                  55                  60
   Thr Leu Ser Asp Lys Ala Ala Asp Trp Ala Ser Gln Cys Ile Trp Glu
   65                  70                  75                  80
   His Ser Asn Ser Gly Gln Asn Leu Ala Ala Trp Phe Ser Pro Gln Ala
                   85                  90                  95
   Asn Lys Pro Met Asn Ile Ser Gln Gly Val Gly Gly Trp Asn Ala Glu
               100                 105                 110
   Glu Pro Asp Tyr Asn Thr Thr Thr Tyr Ser Gly Ala Gly His Trp Thr
           115                 120                 125
   Gln Val Val Trp Lys Ser Thr Thr Ser Val Gly Cys Ala Ala Tyr Ser
       130                 135                 140
   Cys Pro Pro Gly Thr Leu Gly Arg Lys Pro Thr Asp Pro Trp Lys Thr
   145                 150                 155                 160
   Leu Trp Tyr Tyr Val Cys Asn Tyr Tyr Arg Pro Gly Asn Val Ser Pro
                   165                 170                 175
   Arg Asp Lys Tyr Tyr Pro Ile Asn Val Gln Pro
               180                 185
<210> 85
   <211> 239
   <212> PRT
   <213> Homo sapiens
<400> 85
   Ser Thr Val Val Leu Leu Asn Ser Thr Asp Ser Ser Pro Pro Thr Asn
   1 5 10 15
   Asn Phe Thr Asp Ile Glu Ala Ala Leu Lys Ala Gln Leu Asp Ser Ala
   20 25 30
   Asp Ile Pro Lys Ala Arg Arg Lys Arg Tyr Ile Ser Gln Asn Asp Met
   35 40 45
   Ile Ala Ile Leu Asp Tyr His Asn Gln Val Arg Gly Lys Val Phe Pro
   50 55 60
   Pro Ala Ala Asn Met Glu Tyr Met Val Trp Asp Glu Asn Leu Ala Lys
   65 70 75 80
   Ser Ala Glu Ala Trp Ala Ala Thr Cys Ile Trp Asp His Gly Pro Ser
   85 90 95
   Tyr Leu Leu Arg Phe Leu Gly Gln Asn Leu Ser Val Arg Thr Gly Arg
   100 105 110
   Tyr Arg Ser Ile Leu Gln Leu Val Lys Pro Trp Tyr Asp Glu Val Lys
   115 120 125
   Asp Tyr Ala Phe Pro Tyr Pro Gln Asp Cys Asn Pro Arg Cys Pro Met
   130 135 140
   Arg Cys Phe Gly Pro Met Cys Thr His Tyr Thr Gln Met Val Trp Ala
   145 150 155 160
   Thr Ser Asn Arg Ile Gly Cys Ala Ile His Thr Cys Gln Asn Met Asn
   165 170 175
   Val Trp Gly Ser Val Trp Arg Arg Ala Val Tyr Leu Val Cys Asn Tyr
   180 185 190
   Ala Pro Lys Gly Asn Trp Ile Gly Glu Ala Pro Tyr Lys Val Gly Val
   195 200 205
   Pro Cys Ser Ser Cys Pro Pro Ser Tyr Gly Gly Ser Cys Thr Asp Asn
   210 215 220
   Leu Cys Phe Pro Gly Val Thr Ser Asn Tyr Leu Tyr Trp Phe Lys
   225 230 235
<210> 86
   <211> 245
   <212> PRT
   <213> Homo sapiens
<400> 86
   Ala Asn Ile Leu Pro Asp Ile Glu Asn Glu Asp Phe Ile Lys Asp Cys
   1 5 10 15
   Val Arg Ile His Asn Lys Phe Arg Ser Glu Val Lys Pro Thr Ala Ser
   20 25 30
   Asp Met Leu Tyr Met Thr Trp Asp Pro Ala Leu Ala Gln Ile Ala Lys
   35 40 45
   Ala Trp Ala Ser Asn Cys Gln Phe Ser His Asn Thr Arg Leu Lys Pro
   50 55 60
   Pro His Lys Leu His Pro Asn Phe Thr Ser Leu Gly Glu Asn Ile Trp
   65 70 75 80
   Thr Gly Ser Val Pro Ile Phe Ser Val Ser Ser Ala Ile Thr Asn Trp
   85 90 95
   Tyr Asp Glu Ile Gln Asp Tyr Asp Phe Lys Thr Arg Ile Cys Lys Lys
   100 105 110
   Val Cys Gly His Tyr Thr Gln Val Val Trp Ala Asp Ser Tyr Lys Val
   115 120 125
   Gly Cys Ala Val Gln Phe Cys Pro Lys Val Ser Gly Phe Asp Ala Leu
   130 135 140
   Ser Asn Gly Ala His Phe Ile Cys Asn Tyr Gly Pro Gly Gly Asn Tyr
   145 150 155 160
   Pro Thr Trp Pro Tyr Lys Arg Gly Ala Thr Cys Ser Ala Cys Pro Asn
   165 170 175
   Asn Asp Lys Cys Leu Asp Asn Leu Cys Val Asn Arg Gln Arg Asp Gln
   180 185 190
   Val Lys Arg Tyr Tyr Ser Val Val Tyr Pro Gly Trp Pro Ile Tyr Pro
   195 200 205
   Arg Asn Arg Tyr Thr Ser Leu Phe Leu Ile Val Asn Ser Val Ile Leu
   210 215 220
   Ile Leu Ser Val Ile Ile Thr Ile Leu Val Gln Leu Lys Tyr Pro Asn
   225 230 235 240
   Leu Val Leu Leu Asp
   245
<210> 87
   <211> 223
   <212> PRT
   <213> Heloderma horridum
<400> 87
   Glu Ala Ser Pro Lys Leu Pro Gly Leu Met Thr Ser Asn Pro Asp Gln
   1 5 10 15
   Gln Thr Glu Ile Thr Asp Lys His Asn Asn Leu Arg Arg Ile Val Glu
   20 25 30
   Pro Thr Ala Ser Asn Met Leu Lys Met Thr Trp Ser Asn Lys Ile Ala
   35 40 45
   Gln Asn Ala Gln Arg Ser Ala Asn Gln Cys Thr Leu Glu His Thr Ser
       50                  55                  60
   Lys Glu Glu Arg Thr Ile Asp Gly Val Glu Cys Gly Glu Asn Leu Phe
   65                  70                  75                      80
   Phe Ser Ser Ala Pro Tyr Thr Trp Ser Tyr Ala Ile Gln Asn Trp Phe
                   85                  90                  95
   Asp Glu Arg Lys Tyr Phe Arg Phe Asn Tyr Gly Pro Thr Ala Gln Asn
               100                 105                 110
   Val Met Ile Gly His Tyr Thr Gln Val Val Trp Tyr Arg Ser Tyr Glu
           115                 120                 125
   Leu Gly Cys Ala Ile Ala Tyr Cys Pro Asp Gln Pro Thr Tyr Lys Tyr
       130                 135                 140
   Tyr Gln Val Cys Gln Tyr Cys Pro Gly Gly Asn Ile Arg Ser Arg Lys
   145                 150                 155                 160
   Tyr Thr Pro Tyr Ser Ile Gly Pro Pro Cys Gly Asp Cys Pro Asp Ala
                   165                 170                 175
   Cys Asp Asn Gly Leu Cys Thr Asn Pro Cys Lys Gln Asn Asp Val Tyr
               180                 185                 190
   Asn Asn Cys Pro Asp Leu Lys Lys Gln Val Gly Cys Gly His Pro Ile
           195                 200                 205
   Met Lys Asp Cys Met Ala Thr Cys Lys Cys Leu Thr Glu Ile Lys
       210                 215                 220
<210> 88
   <211> 222
   <212> PRT
   <213> Homo sapiens
<400> 88
   Lys Asp Pro Ala Phe Thr Ala Leu Leu Thr Thr Gln Leu Gln Val Gln
   1               5                   10                  15
   Arg Glu Ile Val Asn Lys His Asn Glu Leu Arg Lys Ala Val Ser Pro
               20                  25                  30
   Pro Ala Ser Asn Met Leu Lys Met Glu Trp Ser Arg Glu Val Thr Thr
           35                  40                  45
   Asn Ala Gln Arg Trp Ala Asn Lys Cys Thr Leu Gln His Ser Asp Pro
       50                  55                  60
   Glu Asp Arg Lys Thr Ser Thr Arg Cys Gly Glu Asn Leu Tyr Met Ser
   65                  70                  75                  80
   Ser Asp Pro Thr Ser Trp Ser Ser Ala Ile Gln Ser Trp Tyr Asp Glu
                   85                  90                  95
   Ile Leu Asp Phe Val Tyr Gly Val Gly Pro Lys Ser Pro Asn Ala Val
               100                 105                 110
   Val Gly His Tyr Thr Gln Leu Val Trp Tyr Ser Thr Tyr Gln Val Gly
           115                 120                 125
   Cys Gly Ile Ala Tyr Cys Pro Asn Gln Asp Ser Leu Lys Tyr Tyr Tyr
       130                 135                 140
   Val Cys Gln Tyr Cys Pro Ala Gly Asn Asn Met Asn Arg Lys Asn Thr
   145                 150                 155                 160
   Pro Tyr Gln Gln Gly Thr Pro Cys Ala Gly Cys Pro Asp Asp Cys Asp
                   165                 170                 175
   Lys Gly Leu Cys Thr Asn Ser Cys Gln Tyr Gln Asp Leu Leu Ser Asn
               180                 185                 190
   Cys Asp Ser Leu Lys Asn Thr Ala Gly Cys Glu His Glu Leu Leu Lys
   195 200 205
   Glu Lys Cys Lys Ala Thr Cys Leu Cys Glu Asn Lys Ile Tyr
   210 215 220
<210> 89
   <211> 6
   <212> PRT
   <213> Artificial Sequence
<220>
   <223> peptide
<400> 89
   Glu Ala Glu Ala Glu Phe
   1 5
<210> 90
   <211> 4
   <212> PRT
   <213> Artificial Sequence
<220>
   <223> peptide
<400> 90
   Glu Ala Glu Phe
   1
<210> 91
   <211> 7
   <212> PRT
   <213> Artificial Sequence
<220>
   <223> peptide
<400> 91
   Arg Glu Ala Glu Ala Glu Phe
   1 5
<210> 92
   <211> 9
   <212> PRT
   <213> Artificial Sequence
<220>
   <223> peptide
<400> 92
   Glu Glu Gly Val Ser Leu Glu Lys Arg
   1               5
<210> 93
   <211> 50
   <212> PRT
   <213> Vespula vulgaris
<400> 93
   Asn Asn Tyr Cys Lys Ile Lys Cys Leu Lys Gly Gly Val His Thr Ala
   1               5                   10                  15
   Cys Lys Tyr Gly Ser Leu Lys Pro Asn Cys Gly Asn Lys Val Val Val
               20                  25                  30
   Ser Tyr Gly Leu Thr Lys Gln Glu Lys Gln Asp Ile Leu Lys Glu His
           35                  40                  45
   Asn Asp
       50
<210> 94
   <211> 57
   <212> PRT
   <213> Vespula vulgaris
<400> 94
   Asn Asn Tyr Cys Lys Ile Lys Cys Leu Lys Gly Gly Val His Thr Ala
   1               5                   10                  15
   Cys Lys Tyr Gly Ser Leu Lys Pro Asn Cys Gly Asn Lys Val Val Val
   20 25 30
   Ser Tyr Gly Leu Thr Lys Gln Glu Lys Gln Asp Ile Leu Lys Glu His
   35 40 45
   Asn Asp Phe Arg Gln Lys Ile Ala Arg
   50 55
<210> 95
   <211> 76
   <212> PRT
   <213> Vespula vulgaris
<400> 95
   Asn Asn Tyr Cys Lys Ile Lys Cys Leu Lys Gly Gly Val His Thr Ala
   1 5 10 15
   Cys Lys Tyr Gly Ser Leu Lys Pro Asn Cys Gly Asn Lys Val Val Val
   20 25 30
   Ser Tyr Gly Leu Thr Lys Gln Glu Lys Gln Asp Ile Leu Lys Glu His
   35 40 45
   Asn Asp Phe Arg Gln Lys Ile Ala Arg Gly Leu Glu Thr Arg Gly Asn
   50 55 60
   Pro Gly Pro Gln Pro Pro Ala Lys Asn Met Lys Asn
   65 70 75
<210> 96
   <211> 150
   <212> DNA
   <213> Vespula vulgaris
<400> 96
   aacaattatt gtaaaataaa atgtttgaaa ggaggtgtcc atactgcctg caaatatgga 60
   agtcttaaac cgaattgcgg taataaggta gtggtatcct atggtctaac gaaacaagag 120
   aaacaagaca tcttaaagga gcacaatgac 150
<210> 97
   <211> 171
   <212> DNA
   <213> Vespula vulgaris
<400> 97
   aacaattatt gtaaaataaa atgtttgaaa ggaggtgtcc atactgcctg caaatatgga 60
   agtcttaaac cgaattgcgg taataaggta gtggtatcct atggtctaac gaaacaagag 120
   aaacaagaca tcttaaagga gcacaatgac tttagacaaa aaattgcacg a 171
<210> 98
   <211> 228
   <212> DNA
   <213> Vespula vulgaris
<400> 98
   aacaattatt gtaaaataaa atgtttgaaa ggaggtgtcc atactgcctg caaatatgga 60
   agtcttaaac cgaattgcgg taataaggta gtggtatcct atggtctaac gaaacaagag 120
   aaacaagaca tcttaaagga gcacaatgac tttagacaaa aaattgcacg aggattggag 180
   actagaggta atcctggacc acagcctcca gcgaagaata tgaaaaat 228

## Description

### FIELD OF THE INVENTION

The present invention is directed to recombinant hybrid proteins having native conformation and containing at least one antigenic peptide sequence introduced into a scaffold protein. The invention is further directed to recombinant nucleic acids and vectors encoding the recombinant vespid hybrid proteins and cells containing the recombinant vectors. Such recombinant hybrid proteins are useful for eliciting an immune response without cliciting an allergenic response, and arc therefore particularly useful for therapeutic treatment of allergy.

### BACKGROUND OF THE INVENTION

Genetically predisposed individuals become sensitized (allergic) to antigens originating from a variety of environmental sources, to the allergens of which the individuals are exposed. The allergic reaction occurs when a previously sensitized individual is re-exposed to the same or a homologous allergen. Allergic responses range from hay fever, rhinoconductivitis, rhinitis and asthma to systemic anaphylaxis and death in response to, e.g., bee or hornet sting or insect bite. The reaction is immediate and can be caused by a variety of allergens such as compounds originating from grasses, trees, weeds, insects, food, drugs, chemicals and perfumes.

### Biochemical Aspects of Allergens

Insect sting allergy to bees and vespids is of common occurrence. The vespids include hornets, yellow jackets and wasps (Golden et al., 1989, Am. Med. Assoc. 262:240). Susceptible people can be sensitized on exposure to minute amounts of venom proteins; as little as 2-10 µg of protein is injected into the skin on a single sting by a vespid (Hoffman and Jacobson, 1984, Ann. Allergy. 52:276).

There are many species of hornets (genus Dolichovespula), yellowjackets (genus Vespula) and wasp (genus Polistes) in North America (Akre et al., 1980, "Yellowjackets of America North of Mexico," Agriculture Handbook No. 552, US Department of Agriculture). The vespids have similar venom compositions (King et al., 1978, Biochemistry 17:5165; King et al., 1983, Mol. Immunol. 20:297; King et al., 1984, Arch. Biochem. Biophys. 230:1; King et al., 1985, J. Allergy and Clin. Immunol. 75:621; King, 1987, J. Allergy Clin. Immunol. 79:113; Hoffman, 1985, J. Allergy and Clin. Immunol. 75:611). Their venom each contains three major venom allergens, phospholipase (37 kD), hyaluronidase (43 kD) and antigen 5 (23 kD) of as yet unknown biological function.

In addition to the insect venom allergens described above, the complete amino acid sequence of several major allergens from different grass (Perez et al., 1990, J. Biol. Chem. 265:16210; Ansari et al., 1989, Biochemistry 26:8665; Silvanovich et al., 1991, J. Biol. Chcm. 266:1204), tree pollen (Breiteneder, 1989, EMBO J. 8:1935; Valenta et al., 1991, Science, 253:557), weed pollen (Rafnar et al., 1991, J. Biol. Chem. 266:1229; Griffith et al., 1991, Int. Arch. Allergy Appl. Immunol. 96:296), mites (Chua et al., 1988, J. Exp. Med. 167:175), cat dander (Griffith et al., 1992, Gene. 113:263), and mold (Aruda et al., 1990, J. Exp. Med. 172:1529; Han et al., 1991, J. Allergy Clin. Immunol. 87:327) have been reported. These major allergens are proteins of 10-40 kD and they have widely different biological functions. Nearly all allergens of known sequences have a varying extent of sequence similarity with other proteins in our environment. A comprehensive list of nearly all known allergens is maintained under the auspices of the World Health Organization (WHO) and International Union of Immunological Standards (IUIS) Sub-Committee for Allergen Nomenclature, available at Internet site allergen.org on the World Wide Web.

### T and B Cell Epitope of Allergens

Antibody responses to proteins require the collaboration of T helper and B lymphocytes and antigen presenting cells (APC). The antigen receptors ofB cells are the membrane-bound antibody (Ab) molecules, which recognize and bind immunogens directly. The antigen receptors ofT cells (TCR) only recognize and bind complexes of antigenic peptide-MHC class II molecule. Immunogens are first processed by APC into peptides that are presented on the surface of APC in association with the MHC class II molecules (Unanue, 1992, Current Opinion in Immunol 4:63). As MHC molecules are highly polymorphic in individuals, they have different specificity of binding antigenic peptides (Rothbard and Gefter, 1991, Ann. Rev. Immunol. 9:527). This is one mechanism for genetic control of immune response.

T helper cells are activated when the antigen receptor binds the peptide-MHC complex on the surface of APC. Activated T cells secrete lymphokines. In mice (Street and Mosmann, 1991, FASEB J. 5:171) and apparently in humans (Wierenga et al., 1990, J. Immunol. 144:4651; Parronchi et al., 1991, Proc. Natl. Acad. Sci. USA. 88:4538) the T helper cells can be divided into different types on the basis of their patterns of lymphokine production. Primarily, T helper cells divide into two groups: Th 1 cells producing IL-2 and IFN-γ and Th2 cells producing IL-4 and IL-5. These lymphokines in turn influence the antigen-activated B cells to differentiate and proliferate into plasma cells secreting Abs of different isotypes. IL-4 is one lymphokine known to influence IgE synthesis (Finkelman et al., 1990, Ann. Rev. Immunol. 8:303).

It is believed that the entire accessible surface of a protein molecule can be recognized as epitopes by the antigen receptors of B cells, although all epitopes are not necessarily recognized with equal likelihood (Benjamin et al., 1984, Ann. Rev. Immunol. 2:67). B cell epitopes of a protein are of two types: topographic and linear. The topographic type consists of amino acid residues which are spatially adjacent but may or may not be sequentially adjacent. The linear type consists of only sequentially adjacent residues. X-ray crystallographic data of Ag-Ab complexes indicate the size of their complementary binding region to have 16-17 amino acid residues (Amit et al., 1986, Science 233:747). Phospholipase, like other protein antigens, can have both types of B cell epitopes or only one. Vespid antigen 5s have both types. Bee venom melittin appears to have only one B cell epitope of linear type (King et al., 1984, J. Immunol. 133:2668).

T cell epitopes of proteins consist of only the linear type since they are peptides that have been processed in the lysosomes of APC by proteases (Unanue, 1992, Curr. Op. Immunol. 4:63). Analysis of naturally processed antigenic peptides bound to MHC class II molecules indicates that their size ranges from about 13 to 17 amino acid residues, but analysis of synthetic peptide-MHC class II molecule complex for their T cell proliferate response suggests a minimal size of about 8 amino acid residues (Cf. Rudensky et al., 1991, Nature 353:622). Studies suggest that T cell epitopes are distributed throughout the entire protein molecule, and they may function as major or minor determinants depending on the MHC haplotype of the immunized host (Roy et al., Science 244:572; Gammon et al., 1987, Immunol. Rev. 98:53; O'Hehir et al., 1991, Ann. Rev. Immunol. 9:67).

Hypersensitivity of the immediate type is known to be caused by the presence of allergen-specific IgE. IgE is found in the circulation and bound to specific IgE-Fc receptors on mast cells and basophils. Cross-linking of cell-bound IgE by allergens leads to release of histamine, leukotrienes and other chemical mediators that cause the allergic symptoms. IgE is one of the different isotypes of immunoglobulins. As pointed out above, lymphokines secreted by T cells influence isotype switch events in B cells.

Because of the central role of Th2 cells in determining the isotype switch event of B cells, the T cell epitopes of several allergens have been mapped (CA. O'Hehir *et al., supra*)*.* These allergens include ragweed Amb III, rye grass Lol p 1, cat Fel d 1, mouse urine Mus m I, midge Chi t I, bee venom phospholipase A2 (Dhillon et al., 1992, J. Allergy Clin. Immunol. 90:42) and melittin (Fehlner et al., 1991, J. Immunol. 146:799). The data do not reveal any unusual or common structural features. However, any conclusion from these data is qualified as these data are collected from humans and mice of different haplotypes.

### Modulation of T and B Cell Responses

Normally hosts are tolerant to the dominant B and T cell epitopes of self proteins by clonal deletion and anergy. However this tolerance can be broken under certain circumstances (Gammon et al., 1991, Immunol. Today 12:193; Basten et al., 1991, Immunol. Rev. 122:5). It has been suggested that self-tolerance is broken in autoimmune diseases through encounters with foreign proteins that are similar to host proteins. Therefore the sequence similarity of allergens with autologous proteins is of interest for closer investigation.

Mature B cells are activated in response to multivalent antigens, which can cross-link cell surface Ig receptors (DeFranco, 1987, Ann. Rev. Cell Biol. 3:143), and they are rendered anergic in response to mono-valent antigen (Basten *et al.,* 1991, supra). Antigen activation of T cells requires not only the integration of TCR with peptide-MHC complex but also with other co-stimulating signals on the surface of APC (Schwartz, 1990, Science 248:1349; Jenkins and Miller, 1992, FASEB J. 6:2428). Interaction of TCR with peptide-MHC complex in absence of co-stimulating signals can lead to T cell anergy.

Experimental autoimmune encephalomyelitis (EAE) in mice or rats is a well-studied model for multiple sclerosis. Many studies have identified immunodominant T cell determinants for myelin basic protein, which is used to induce this condition. Peptides that correspond to immunodominant epitopes of myelin basic protein can induce tolerance to the same peptide antigen or to the intact myelin basic protein. The same peptides that induced tolerance could also induce T cell anergy in an ongoing autoimmune response (Gaur et al., 1992, Science 259:1491-1494).

Early studies have shown that the physical state of the immunogen and the route of immunization are important variables in determining the outcome of an immune response. In the light of our current understanding, these variables may well influence antigen presentation so as to have T and B cell activation or anergy.

### Immunotherapy

One way to treat allergic diseases is by immunotherapy, which involves repeated subcutaneous injections of the offending allergen(s) into patients. For most patients following immunotherapy, allergen-specific IgG levels initially rise. A gradual decrease of allergen-specific IgE levels follows the IgG rise (Norman, 1993, Current Op. Immunol. 5:968). Treated patients also show changes in their T cell cytokine profile: IL-4 and IL-5 levels decreased and IFN-γ level increased (Secrist et al., 1993, J. Exp. Med. 178:2123.)

Studies have shown that immunotherapy with high doses of allergens is more effective for symptom reduction than that with low doses. However, effective dosages of allergens were limited by the potential danger of unwanted systemic allergic reaction in patients. Because of the undesirable systemic reaction on immunotherapy with native allergens, there has been continued interest in the development of modified allergens with reduced allergenic activities for immunotherapy (T.P. King, 1993, in "Bronchial Asthma," edited by E.B. Weiss and M. Stein, Little Brown, Boston, pp. 43-49; R.E. O'Hehir *et al.,* 1991, *supra*).

Allergenicity depends on the interaction of a multi-valent allergen with basophil or mast cell-bound IgE antibodies. Therefore, allergenicity of a protein can be reduced by decreasing its B cell epitope density. Reduction of B cell epitope density of a protein can be accomplished by several approaches. One approach is by partial or complete denaturation of allergens by chemical treatment or fragmentation (Takatsu et al., 1975, J Immunol 115:1469; Pesce et al., 1990, Int Arch Allergy Appl Immunol 92:88; Vrtala et al., 1997, J Clin Invest 99:1673) since the majority ofB cell epitopes are of the discontinuous type, i.e., dependent on the native conformation of proteins. For example, urea treatment of the major allergen from ragweed pollen led to irreversible denaturation with loss of the discontinuous B cell epitopes but retention of the continuous B and T cell epitopes (Takatsu et al., 1975, J Immunol 115:1469). Immunotherapy of patients with the fully denatured ragweed allergen showed no changes in specific IgE and IgG levels for the native allergen although the peripheral blood mononuclear cells of treated patients did show decreased proliferative response on antigen stimulation (Norman et al., 1980, J Allergy Clin Immunol 66:336). Use of partially denatured allergens has also been proposed. This is exemplified by the recombinant mite allergens, which lack the cysteine residues that are involved in maintaining the native structure of the protein (Smith et al., 1996, Mol Immunol 33:399; T. Takai et al., 1997, Nature Biothechnology 15:754).

Two reports have appeared on the use of T cell epitope peptides to modulate allergen-specific immune responses. One report is on the subcutaneous injection of mice with two peptides from the major cat allergen Fel d I to decrease T cell response to the entire molecule Fel d I (Briner et al., 1993, Proc. Natl. Acad. Sci. U.S.A. 90:7608-12). Another is on the intranasal therapy with a peptide from the major mite allergen Der p I to suppress allergen-specific response in naive or sensitized mice (Hoyne et al., 1993, J. Exp. Med. 178:1783-1788).

These findings suggested the use ofT cell peptides as immunotherapeutic reagents since T cell peptides are like the denatured allergens in that they lack the discontinuous B cell epitopes. The dominant T cell peptides of several allergens were tested in patients; cytokine level changes but not antibody level changes were observed (Muller et al., 1998, J Allergy Clin Immunol 101:747; Simons et al., 1996, Int Immunol 8:1937; Creticos et al., 1997, J Allergy Clin Immunol 99:401; Marcotte et al., 1997, J Allergy Clin Immunol 99:405). Importantly, these clinical findings with the urea-denatured allergen and T cell peptides suggest that the retention of the discontinuous B cell epitopes as well as the continuous B and T cell epitopes is required for modified allergens to be effective in modulating both antibody and cellular immune responses.

A second approach to reduce the accessibility ofB cell epitopes of allergen involves polymerization of the allergen by formaldehyde or glutaraldehyde treatment (Marsh, 1971, Int Arch Allergy Appl Immunol 41:199; Patterson et al., 1973, J Immunol 110:1413) or by attachment of non-immunogenic polymers (King et al., 1979, J Exp Med 149:424). Glutaraldehyde polymerized antigens were found to be processed differently from the natural antigens in mice, and they were processed by antigen-presenting cells that secrete cytokines promoting Thl responses (Gieni et al., 1993,. J Immunol 150:302). This second approach for improved immunotherapy had been tried with ragweed pollen allergens with immunological findings similar to those with natural allergens (Norman et al., 1982, J Allergy Clin Immunol 70:248; Norman, 1984, J Allergy Clin Immunol 73:787). One limitation of this approach was that near complete loss of the discontinuous B cell epitopes usually occurred,when allergens were modified to achieve greater than100-fold reduction in allergenicity.

A third approach is by site-directed mutagenesis to selectively alter the contact amino acid residues of B cell epitopes of allergens. If the key contact residues of B cell epitopes are known; this can be a useful approach. For example, a single residue mutation of Glu to Ser in the major birch allergen abolished its binding of a murine antibody, and resulted in a 40% decrease of its binding of IgEs from a serum pool of allergic patients (Mirza et al., 2000, J Immunol. 165:331). The different decreases probably reflect that the murine antibody and the human IgEs are respectively of monoclonal and polyclonal origins.

Since an MHC class II molecule of any one haplotype can bind a wide range of peptides in its binding groove, it may be possible to modulate T cell response by inhibition of allergen-derived T cell epitope binding to MHC molecules with other peptides. For example, a mouse lysozyme peptide which is not immunogenic by itself in H-2k mice inhibits T cell response to hen egg white lysozyme (Adorini and Nagy, 1990, Immunol. Today 11:21). Another example is the *in vitro* inhibition ofT cell response to a mite allergen by an influenza HA peptide (O'Hehir et al., 1991, J. Allergy Clin. Immunol. 87:1120).

Immune response to an immunogen/allergen thus depends in part on the genetic make-up of the host, the route and mode of immunization and the immunogen/allergen. The extent to which an allergen determines the outcome of IgE response is not known. How many B and T cell epitopes must each allergen have? Are immunodominant B or T cell epitopes of an allergen recognized by different or all susceptible individuals? Are there T cell epitopes which favor IgE class switch events in B cells? Does antigenic cross reactivity of allergens with host proteins play a role as to why some proteins are more allergenic than others are? Can tolerance to a multi-valent allergen be induced by treatment with a single or a combination of B or T cell epitopes?

U.S. Patent Nos. 5,593,877; 5,612,209, 5,804,201, 6,106,844, 6,270,763 and 6,287,559 and 6,372,471 (U.S, application Serial No. 09/166,205 to King) disclose the isolation of cDNAs encoding vespid venom proteins and the deduced amino acid sequences of proteins encoded by the cDNAs. The cDNAs allow the expression and purification of large quantities of vespid venom proteins and polypeptides for use in immunotherapy. Sequences, however, fail to yield information on the native structure of vespid venom. Hence, the cDNAs and deduced amino acid sequences do not yield information on discontinuous epitopes. Nor do the deduced vespid venom amino acid sequences predict epitopes that will be present on the surface of recombinantly produced vespid venom proteins. Consequently, the cDNA and deduced amino acid sequences alone cannot accurately predict which regions or peptides of vespid venom proteins will serve as efficient immunogens to stimulate a B cell-mediated immune response. Nor can the cDNA and deduced amino acid sequences alone predict the epitope density on the surface of a vespid venom protein, which is an important determinant of the potential to crosslink surface IgE molecules, and hence the allergenicity, of a vespid venom protein.

Thus, there is a need in the art to determine how modification ofB cell epitopes in the native structure of allergen proteins permits the design of improved therapeutics.

There is also a need in the art to provide allergen proteins that stimulate a B cell-mediated immune response without stimulating IgE mediated allergic responses. In particular, there is need in the art for providing allergens with a reduced density of epitopes that are efficient in stimulating an IgG production in B cells but are inefficient at crosslinking IgE antibodies specific for the native allergen bound to the surface of, for example and without limitation, mast cells or basophils.

There is also a need in the art to provide hybrid proteins bearing non-cross-reactive B cell epitopes that are effective in immunotherapy. In particular there is a need to for hybrid proteins that present allergen peptide epitope sequences in a conformation that is accessible to receptors on the surface of immune cells and soluble proteins, especially antibodies.

Hence, what are needed are agents, pharmaceutical compositions and methods for generating an IgG B cell response that provides protection against allergens, without eliciting an allergic reaction such as anaphylactic shock.

The citation of references herein shall not be construed as an admission that such is prior art to the present invention.

### SUMMARY OF THE INVENTION

The present invention relates to a new approach to prepare modified allergens. The modified allergens are hybrids consisting of a small portion of the "guest" allergen of interest and a large portion of a homologous but poorly cross-reacting "host" protein. The homologous host protein functions as a scaffold to maintain the native structure of the guest allergen of interest so that the conformation-dependent B cell epitopes of the guest allergen of interest are preserved in the hybrid, but at a reduced density. Homologous proteins of greater than 30% sequence identity and of similar functions are known to have closely similar three-dimensional structures (Chothia et al., 1990, Annual Review Biochem 59:1007; Russell, 1994, J Mol Biol 244:332), thus providing a plethora of guest/host proteins.

Thus, the present invention is directed to recombinant allergens, e.g., vespid venom allergens, of reduced allergenicity but that retain immunogenicity. Hence, the invention provides an allergen hybrid protein having reduced allergenicity but retaining immunogenicity in respect of a native allergen protein, comprising a peptide epitope sequence of the allergen protein and a scaffold protein that is structurally homologous to the allergen protein, wherein the peptide epitope sequence is substituted for the homologous region of the scaffold protein; and the scaffold protein in the hybrid protein has a native conformation and the peptide epitope sequence is present in a surface accessible loop or corner region of the hybrid protein corresponding to its position in the allergen protein, wherein the scaffold protein has SEQ ID NO: 17, and the peptide epitope sequence comprises a sequence selected from the group consisting of NNYCKIKCLKGGVHTACK (SEQ ID NO: 2); LTGSTAAKYDD (SEQ ID NO: 10); PKKKFSGND (SEQ ID NO: 11); and FKNEELYQTK (SEQ ID NO: 13).

The invention provides nucleic acids encoding such hybrid constructs, and methods that may be used to stimulate a therapeutic immune response to the allergens with reduced allergic response, i.e., an allergy immunotherapy. In particular, the recombinant hybrid proteins, nucleic acids and methods of the invention provide for stimulating a B cell-based response against the allergen, without triggering an IgE-based allergic response such as acute anaphylaxis.

The hybrid proteins of the present invention are present in a native conformation, and comprise the allergen peptide epitope sequence in a native conformation. More specifically, the scaffold protein and the native protein from which the allergen peptide epitope sequence is derived have the same native conformation.

In certain embodiments the hybrid proteins of the invention comprise a fusion peptide, such as a signal peptide or handle for purification. In other embodiments the hybrid proteins of the invention may comprise a protcasc processing site, e.g., for cleavage of the purification handle. Accordingly, the hybrid proteins of the invention comprise an allergen peptide epitope sequence, a scaffold protein sequence, and, optionally, either separately or in combination, a fused sequence and protease processing site.

The recombinant peptide epitope sequences are found on the surface of the native protein from which the sequence is derived, and the allergen peptide is a loop or corner region of the native protein.

It will be appreciated that hybrid proteins may comprise more than one peptide epitope sequence introduced into the scaffold protein sequence.

In the present invention each of the peptide antigen and the scaffold proteinarederived from vespid venom proteins Ag 5s. Disclosed allergen peptide epitope sequences derived from an Ag 5 peptide consist of
NNYCKIKC (SEQ ID: 1);
NNYCKIKCLKGGVHTACK (SEQ ID: 2);
NNYCKIKCLKGGVHTACKYGSLKP (SEQ ID: 3);
NNYCKIKCLKGGVHTACKYGSLKPNCGNKVVV (SEQ ID: 4);
NNYCKIKCLKGGVHTACKYGSLKPNCGNKVVVSYGLTKQ (SEQ ID: 5);
NNYCKIKCLKGGVHTACKYGSLKPNCGNKVWSYGLTKQEKQDILK (SEQ ID: 6);
LKPNCGNKWV (SEQ ID NO: 9);
LTGSTAAKYDD (SEQ ID NO: 10);
PKKKFSGND (SEQ ID NO: 11)
IQEKWHK (SEQ ID NO: 12); and
FKNEELYQTK (SEQ ID NO: 13); .

The present invention further extends to an isolated expression vector comprising a promoter operationally associated with a nucleic acid of the invention. Numerous promoters commercially available to the skilled artisan can be used in this aspect of the invention. Examples include, but are not limited to immediate early promoters of hCMV, early promoters of SV40, early promoters of adenovirus, early promoters of vaccinia, early promoters ofpolyoma, late promoters of SV40, late promoters of adenovirus, late promoters of vaccinia, late promoters of polyoma, the lac the trp system, the TAC system, the TRC system, the major operator and promoter regions ofphage lambda, control regions of fd coat protein, 3-phosphoglycerate kinase promoter, acid phosphatase promoter, or promoters of yeast a mating factor, to name only a few. Numerous examples of expression vectors having applications herein, and which are also readily available to the skilled artisan are described infra.

The invention also provides a method for preparing a nucleic acid that encodes an allergen hybrid protein of the invention. This method comprises substituting a nucleotide sequence encoding a peptide epitope sequence of an allergen protein into a nucleotide sequence encoding a scaffold protein that is structurally homologous to the allergen protein. The nucleotide sequence encoding the peptide epitope sequence is substituted in-frame with the nucleotide sequence encoding the scaffold protein, and in a location such that in the allergen hybrid protein the peptide epitope sequence is present in a surface accessible loop or corner region of the hybrid protein corresponding to its position in the allergen protein, wherein the scaffold protein has SEQ ID NO: 17, and the peptide epitope sequence comprises a sequence selected from the group consisting of NNYCKIKCLKGGVHTACK (SEQ ID NO: 2); LTGSTAAKYDD (SEQ ID NO: 10); PKKKFSGND (SEQ ID NO: 11); and FKNEELYQTK (SEQ ID NO: 13).

In one such embodiment, the nucleotide sequence encoding the scaffold protein is mutated to substitute the nucleotide sequence encoding the peptide epitope sequence. In another such embodiment, the nucleotide encoding the peptide epitope sequence is substituted by ligating fragments from nucleic acids comprising the nucleotide sequence encoding the peptide epitope sequence and the nucleotide sequence encoding the scaffold protein treated with an endonuclease. If necessary, endonuclease restriction sites can be introduced into the nucleic acids comprising such sequences using standard techniques in the art.

A method for producing a hybrid protein of the invention by expression of an isolated nucleic acid molecule of the invention is described. Such production provides a plentiful source of the hybrid protein for diagnosis and therapy. An example of such a method for producing a hybrid protein is culturing a host cell transformed or transfected with an expression vector of the invention so that the host cell produces the hybrid protein of the invention. Preferably, the hybrid protein of the invention so produced from the culture, the host cell, or both is recovered.

The present invention further extends to the use of a therapeutically effective amount of the hybrid protein or the expression vector of the invention in the preparation of a pharmaceutical compositions effective for the treatment or prevention of an allergen-specific allergic condition which composition is for administration to a patient who is allergic to the allergen protein or the scaffold protein, or both. In particular, the present invention extends to a pharmaceutical composition comprising a hybrid protein of the invention, or a nucleic acid preferably an expression vector, encoding such a hybrid protein, and a pharmaceutically acceptable carrier thereof. Pharmaceutical compositions containing a plurality of hybrid proteins of the invention, or containing a nucleic acid or nucleic acids encoding such a plurality are described.

Naturally, a method for treating allergen-specific allergic condition comprises administering a therapeutically effective amount of a pharmaceutical composition of the invention. Administration of a pharmaceutical composition of the invention can occur by any route, and particularly orally, pulmonarily, nasally, topically or parenterally. Other routes of administration are also possible.

As a result, administration of such a pharmaceutical composition modulates the immune system's ability to recognize and attack the immunogen. The immune system of the mammal to recognize and attack the immunogen is increased upon administration of the pharmaceutical composition relative to the ability of the subject's immune system to recognize and attack the immunogen prior to administration of a pharmaceutical composition of the invention.

### ABBREVIATIONS

- Dol m Dolichovespula maculata: white faced hornet
- Dol a D. arenaria: yellow hornet
- Pol a Polistcs annularis: wasp
- Pol e P. exclamans: wasp
- Ves m Vespula maculifrons: yellowjacket
- Ves v V. vulgaris: yellowjacket
- PCR: polymerase chain reaction
- RACE: rapid amplification of cDNA ends
- TCR: T cell receptor for antigen

### BRIEF DESCRIPTION OF THE DRAWINGS

**Fig. 1****.** Ves v 5 cDNA [SEQ ID NO: 14] and amino acid [SEQ ID NO: 16] sequences. Numbering at L refers to nucleotide position; numbering at R refers to amino acid position.
**Fig. 2****.** Pol a 5 cDNA [SEQ ID NO: 15] and amino acid [SEQ ID NO: 17]sequence. Numbering at L refers to nucleotide position; numbering at R refers to amino acid position.
**Fig. 3****.** Amino acid comparison of Ves v 5 (V) [SEQ ID NO: 16] and Pol a 5 (P) [SEQ ID NO: 17].
**Fig. 4****.** Schematic sequence representations of Ag 5s and hybrids. Residue numbers given for hybrids refer to those of Ves v 5.

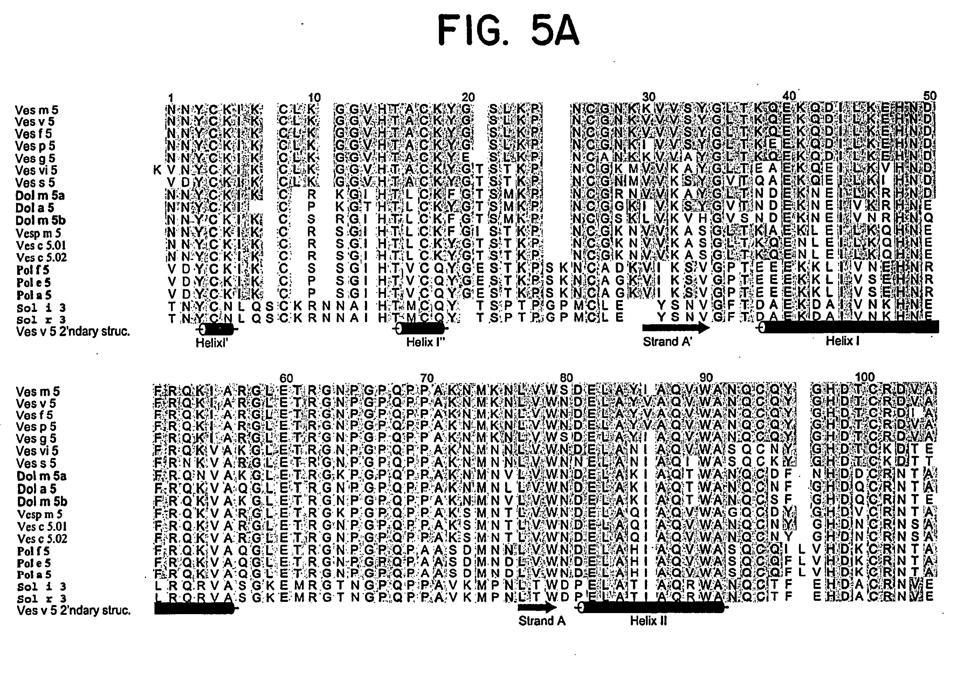

**Fig. 5A-B**. Alignment of Ves v 5 homologous proteins from insect venoms from Vespula maculifrons [Ves m 5, SEQ ID NO: 63]; Vespula vulgaris [Ves v 5, SEQ ID NO: 64]; Vespula flavopilosa [Ves f 5, SEQ ID NO: 65]; Vespula pensylvanica [Vcs p 5, SEQ ID NO: 66]; Vespula germanica [Ves g 5, SEQ ID NO: 67]; Vespula vidua [Ves vi 5, SEQ ID NO: 68]; Vespula squamosa [Ves s 5, SEQ ID NO: 69]; Dolichovespula maculata [Dol m 5a, SEQ ID NO: 70]; Dolichovespula arenaria [Dol a 5, SEQ ID NO: 71]; Dolichovespula maculata [Dol m 5b, SEQ ID NO: 72]; Vespa mandarinia [Vesp m 5, SEQ ID NO: 73]; Vespa crabro [Ves c 5.01, SEQ ID NO: 74]; Vespa crabro [Ves c 5.02, SEQ ID NO: 75]; Polistes fuscatus [Pol f 5, SEQ ID NO: 76]; Polistes exclamans [Pol e 5, SEQ ID NO: 77]; Polistes annularis [Pol a 5, SEQ ID NO: 78]; Solenopsis invicta [Sol i 3, SEQ ID NO: 79]; and Solenopsis richteri [Sol r 3, SEQ ID NO: 80].
**Fig. 6A-B**. SDS gel patterns of Ag 5s and hybrids.
**Fig. 7**. Circular dichroism (CD) spectra of Ves v 5 and hybrids.
**Fig. 8A-C**. Inhibition ELISA with mouse antibodies specific for natural Ves v 5 using (A) Ves v 5-specific antibodies isolated from BALB/c mice and depleted of Pol across reactive antibodies (B) antisera from ASW/n mice and (C) antisera from P/J mice.
**Fig. 9A-C**. Inhibition ELISA with sera from yellow jacket-sensitive patients.
**Fig. 10 A-C**. Binding of mouse Ves v 5-specific monoclonal antibodies to solid-phase Ves v 5 or hybrids.
**Fig.11 A-C**. Histamine release assay of Ves v 5, Pol a 5 and hybrids.
**Fig. 12A-B**. Alignment of Ves v 5-like proteins. Aligned proteins are Ves v 5 [SEQ ID NO: 81]; Sol i 3 [SEQ ID NO: 82]; Lycopersicon esculentum p14a [SEQ ID NO: 83]; Schizophyllum commune SC7 [SEQ ID NO: 84]; human trypsin inhibitor [SEQ ID NO: 85]; human glipr [SEQ ID NO: 86]; Heloderma horridum helothermine [SEQ ID NO: 87]; and human TPX-1 [SEQ ID NO: 88].

### DETAILED DESCRIPTION

The present invention is directed to an allergen hybrid protein having reduced allergenicity but retaining immunogenicity in respect of a native allergen protein, comprising a peptide epitope sequence of the allergen protein and a scaffold protein that is structurally homologous to the allergen protein, wherein the peptide epitope sequence is substituted for the homologous region of the scaffold protein; and the scaffold protein in the hybrid protein has a native conformation and the peptide epitope sequence is present in a surface accessible loop or corner region of the hybrid protein corresponding to its position in the allergen protein, wherein the scaffold protein has SEQ ID NO: 17, and the peptide epitope sequence comprises a sequence selected from the group consisting of NNYCKIKCLKGGVHTACK (SEQ ID NO: 2); LTGSTAAKYDD (SEQ ID NO: 10);PKKKFSGND (SEQ ID NO: 11); and FKNEELYQTK (SEQ ID NO: 13). The invention provides recombinant allergen hybrid protein constructs of reduced allergenicity and but retaining immunogenicity, the nucleic acid molecules encoding such allergens, and methods of use for such allergens in the therapy of allergy. The hybrid proteins of the invention comprise a surface, e.g., loop or corner region, peptide epitope sequence introduced into a scaffold protein sequence. The hybrid proteins, nucleic acids and methods of the invention provide for stimulating a B cell-based response against the allergen without triggering an IgE-based allergic response. The recombinant hybrid protein comprises a vespid venom surface corner or loop peptide antigen, Ves v 5, fused to a scaffold protein, Pol a 5.

The invention is further directed to expression vectors comprising nucleic acid molecules that include allergen hybrid proteins of decreased allergenicity that retain immunogenicity. Methods for producing such hybrid proteins of the invention by expressing and recovering such hybrid proteins are described.

The invention also provides pharmaceutical compositions effective for the treatment of an allergen-specific allergic condition comprising a hybrid protein of the invention or nucleic acid vector encoding such a hybrid protein, for use in methods for treating such allergic conditions comprising administering a therapeutically effective amount of such pharmaceutical compositions.

The hybrid proteins of the invention can also be useful for diagnosis of allergen-specific allergic conditions.

The present invention is based, in part, on the discovery that insertion of sequences from surface accessible regions of yellowjacket (*Vespula vulgaris*) antigen 5 into the corresponding region of *Polistes annularis* antigen 5 yielded a hybrid construct that retained the immunogenicity of the parent proteins, but showed significantly reduced allergenicity. Moreover, the most advantageous positions for introducing sequences were at surface accessible sites, especially loop and corner regions, as determined from the crystal structure of Ves v 5.

Earlier work established that hybrid constructs, in which one-quarter to one-third of the allergenic protein was introduced into the corresponding region of a homologous scaffold protein. However, these hybrid constructs lack the advantages and refinements of the present invention.

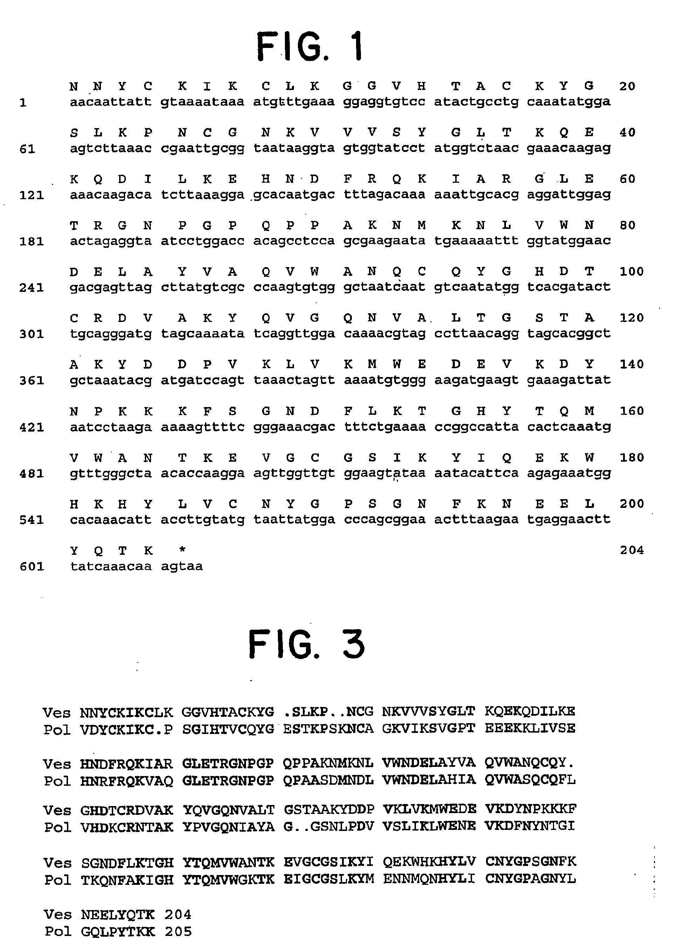

Clinical studies in patients and tests with experimental animals have shown that there is limited cross reactivity of antibodies specific for the yellow jacket and paper wasp venom proteins (Lichtenstein et al., 1979, J Allergy Clin Immunol 64:5; Lu et al., 1993, J Immunol 150:2823). These observations form the basis of a preferred embodiment of the present invention. A preferred guest allergen antigen 5 is Ves v 5, a yellow jacket venom protein of 23 kd. A preferred homologous host allergen, which serves as a scaffold protein, is Pol a 5, a paper wasp venom protein of similar size. Ves v 5 and Pol a 5 have 59% sequence identity (Fig. 3). Both can be expressed in yeast and the recombinant proteins were shown to have the native conformation of the natural proteins (Monsalve et al., 1999, Protein Expr. Purif. 16:410).

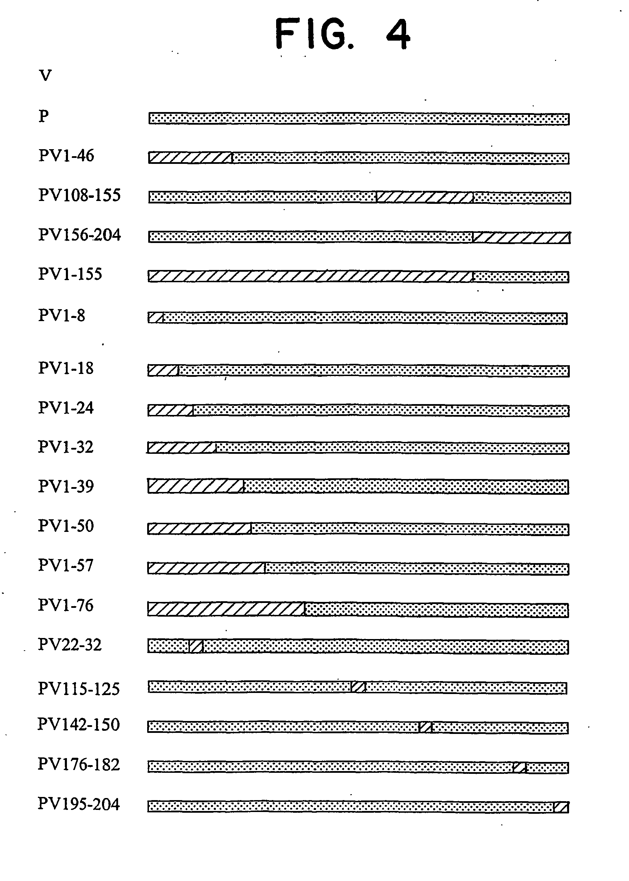

Immunochemical findings are reported for hybrids of Ves v 5 and Pol a 5. The sequence representations of these hybrids are shown schematically in Fig. 4. Hybrids PV 1-46, PV 109-155 and PV 156-204 contain respectively the first one-quarter (i.e., amino acids 1-46), the third one-quarter (i.e., amino acids 109-155) and the last one-quarter (i.e., amino acids 156-204) of the Ves v 5 molecule, together with portions of the Pol a 5 molecule to complete the hybrid Ag 5 molecule. A hybrid containing the second one-quarter of the Ves v 5 molecule was not prepared, as this is a region of high sequence identity of Ves v 5 and Pol a 5 (see Fig. 3). Hybrid PV1-155 has the opposite arrangement of the Ves v 5 and Pol a 5 amino-terminal and carboxy-terminal fragments, when compared to PV156-204.

Hybrids PV1-8, PV1-18, PV1-24, PV1-32, PV22-32, PV115-125, PV142-150, PV 176-182 and PV 195-204 were designed to contain the surface, loop or corner regions of Ves v 5. These hybrids include 7-32 amino acids of Ves v Ag 5 substituted for a homologous region of Pol a Ag 5.

Switching corresponding regions of homologous proteins, especially in surface accessible, e.g., loop and corner, regions predictably conserves native structure. Surface accessible regions especially loop and corner regions, tend to demonstrate more flexibility and better tolerate changes while retaining structure. This approach also finds a counterpart in directed evolution, where homologous enzymes are recombined to yield novel, functional enzyme chimeras.

The term "allergen hybrid protein" refers to a recombinant or synthetic protein that has the native structure of the scaffold protein, but includes one or more sequences from an allergen. The allergen is a structural homolog of the scaffold protein, thus permitting introduction of the allergen sequences into corresponding positions in the scaffold protein. A "corresponding position" is the same position in the primary sequence or same topological position in the native structure. The allergen sequences are selected from a surface accessible region of the allergen and inserted in the corresponding surface accessible region of the scaffold protein. Because B cell epitopes of proteins in their native conformation are surface accessible, the sequences from the allergen introduced into the scaffold protein can act as B cell epitopes, hence they are called "peptide epitope sequences" of an allergen protein.

In connection with the present invention the expression "reduced allergenicity" means a molecule or antigen exhibits significantly reduced allergenic activity in an in vitro assay designed to measure such allergenicity. Such *in vitro* assays are well known in the art and include, for example and without limitation, assay of histamine release from basophils of a allergen sensitive patient or experimental animal following challenge. Furthermore, "activity" as used herein may refer to any measurable parameter or result that is indicative of the allergenicity of a molecule or antigen, such as, for example and without limitation, the maximum response obtained in an assay or the amount or concentration of antigen required to elicit a defined result in an assay.

The term "retaining immunogenicity" (in any grammatical form) means that the hybrid protein elicits an immune response, particularly an IgG-predominated humoral immune response, that is comparable to the immune response elicited by the native allergen or scaffold protein (or both) and greater than the allergic (IgE) immune response they elicit. The hybrid-specific IgG will cross react with epitopes present on the allergen and the scaffold protein. This IgG response can block IgE binding, thus reducing or preventing allergic responses. In addition, the hybrid protein may elicit T cell anergy and other allergy suppressive immune responses.

In accordance with the present invention, proteins are "homologous" if, following alignment, they exhibit at least about 30 percent amino acid identity, as determined by programs that arc well know in the art, including, as non-limiting examples, the programs Gap, Bestfit and BLAST. More preferable is where homologous proteins exhibit at least 50 percent amino acid identity. However, in a specific embodiment the allergen protein and the scaffold protein do not have more than 70% sequence identity to reduce the possibility of a high degree of cross reactivity that might lead to an unaccepatable degree of allergenicity of the hybrid protein. Greater sequence identity can be tolerated, particularly where the peptide epitope sequence inserted in the scaffold protein is very dissimilar, e.g., less than 50% identical and preferably less than 30% identical, to the corresponding sequence from the scaffold protein that it replaces.

Proteins are structurally homologous when, due to primary sequence similarity, they adopt a similar core secondary and tertiary structure so that their three-dimensional structures can be superimposed with almost complete (greater than 70%) overlap. Their surface tertiary structure, however, may vary.

In a preferred embodiment of the present invention, peptide epitope sequences from the allergen are inserted into or replace sequences within "scaffold" proteins. Accordingly, a "scaffold protein" of the present invention is a protein which includes an allergen epitope sequence, either as an inserted sequence or as a replacement sequence for a homologous (corresponding) sequence of the scaffold protein. The scaffold protein adopts a native conformation. The allergen and scaffold can alternate positions; these terms are used to indicate the source of sequences (from the "allergen") introduced into the "scaffold". Because the "allergen" and "scaffold" are homologous, they are both likely to act as allergens, albeit to different populations. Thus, a "scaffold" can be an "allergen" if its surface accessible sequences are introduced into another structurally homologous protein.

The expression "native conformation" includes a functional conformation adopted by a non-recombinant, *i.e*., natural protein, polypeptide, or antigen, within its natural environment or following purification under conditions that maintain the functional conformation adopted in said natural environment. Native conformation can be measured, for example and without limitation, by determining the CD spectrum of a protein. Native conformation may also be determined by measuring enzymatic activity. It will be understood by the skilled artisan that, in cases where the functional conformation of a natural non-recombinant protein is unknown, "native conformation" will encompass forms of recombinant proteins that reproducibly exhibit a non-random defined conformation that includes secondary elements as typically found in properly folded functional proteins, such as for example, and without limitation, a helix and P sheet elements. It is also well known that, using recombinant techniques, additional amino acids may be joined to the amino or carboxyl end of a protein without disrupting the native conformation of the protein. Such additional amino acids may be short polypeptide "tags", which are typically 1-25 amino acids in length and which are typically disordered, or longer polypeptides which may form a distinct domain, which may itself be ordered or disordered.

The expression "surface-exposed amino acid" means that an amino acid residue is located at the surface of the three-dimensional structure in such a manner that when the allergen is in solution at least a part of at least one atom of the amino acid residue is accessible for contact with the surrounding solvent. Preferably, the amino acid residue in the three-dimensional structure has a solvent (water) accessibility of at least 20%, more preferably at least 30 %, still more preferably at least 40 % and most preferably at least 50%.

The expression "solvent accessibility" is defined as the area of the molecule accessible to a sphere with a radius comparable to a solvent (water, r = 1.4 A) molecule.

An "allergen" has its ordinary meaning, i.e., is any proteinacious molecule that elicits an allergic response, e.g., histamine release to anaphylactic shock. Allergens are well known; a representative group are listed in Table 8 ofthis specification. Examples of allergens according to the invention may suitably be an inhalation allergen originating, e.g., from trees, grasses, herbs, fungi, house dust mites, cockroaches and animal hair and dandruff. Important pollen allergens from trees, grasses and herbs are such originating from the taxonomic orders of *Fagales, Oleales* and *Pinales* including birch (*Betula*)*,* alder (*Alnus*), hazel (*Corylus*), hornbeam (*Carpinus*) and olive (*Olea*), the order of *Poales* including i.a. grasses of the genera *Lolium, Phleum, Poa, Cynodon, Dactylis* and *Secale,* the orders of *Asterales* and *Urticales* including herbs of the genera *Ambrosia* and *Artemisia.* Important inhalation allergens from fungi are such originating from the genera *Alternaria* and *Cladosporium.* Other important inhalation allergens are those from house dust mites of the genus *Dermatophagoides,* those from cockroaches and those from mammals such as cat, dog and horse. Further, recombinant allergens according to the invention may be mutants of venom allergens including such originating from stinging or biting insects such as those from the taxonomic order of *Hymenoptera* including bees (superfamily Apidae), wasps (superfamily Vespidea), and ants (superfamily Formicoidae). Specific allergen components include, e.g., *Bet v* 1 (*B. verrucosa,* birch), *Aln g* 1 (*Alnus glutinosa,* alder), *Cor a*1 (*Corylus avelana,* hazel) and *Car b* 1 (*Carpinus betulus,* hornbeam) of the *Fagales* order. Others are *Cry j* 1 (*Pinales*), *Amb a* 1 and 2, *Art v* 1 (*Asterales*)*, Par j* 1 (*Urticales*), *Ole e* 1 (*Oleales*), *Ave e 1, Cyn d* 1*, Dac g* 1, *Fes p* 1, *Hol l* 1, *Lol p* 1 and 5, *Pas n* 1, *Phl p* 1 and 5, *Poa p* 1, 2 and *5, Sec c* I and 5, and *Sor h* 1 (various grass pollens), *Alt* a 1 and *Cla h* 1 (fungi), *Der f* 1 and 2, *Der p* 1 and 2 (house dust mites, *D. farinae* and *D. pteronyssinus,* respectively), *Lep d* 1 and 2 (Lepidoglyphus destructor; storage mite), *Bla g* 1 and 2, *Per a* 1 (cockroaches, *Blatella germanica and Periplaneta americana, respectively*), *Fel d* 1 (cat), *Can f* 1 (*dog*)*, Equ c* 1, 2 and 3 (horse), *Apis m* 1 and 2 (honeybee), *Ves v* 1, 2 and 5, *Pol a* 1, 2 and 5 (all wasps) and *Sol i* 1, 2*,* 3 and 4 (fire ant). The term also includes all examples described in the "Background", *supra.*

For example, the term "vespid venom allergen" refers to a protein found in the venom of a vespid, to which susceptible people are sensitized on exposure to the sting of the insect. While most antigens are characterized by being reactive with specific IgG class antibodies, an allergen is characterized by also being reactive with IgE type antibodies. The IgE type antibodies are responsible for mediating the symptoms of an allergic condition, i.e., immediate-type hypersensitivity.

As used herein, the term "vespid" is used according to the practice of those in the field of allergy, and refers to insects belonging to the worldwide family of Vespidae, *i.e*., social wasps including hornets, yellowjackets, and paper wasps. In particular, vespids include the subfamilies Vespinae and Polistinae. More particularly, the vespids include the genera Vespa Linnaeus, Vespula Thomson, Dolichovespula Rohwer, and Polistes Latreille. Species in the genus Vespula include but are not limited to V. germanica (Fab.), V. squamosa (Drury), V. maculifrons (Buysson), V. flavopilosa (Jacobson), V. vulgaris (L.), and V. pensylvanica (Saussurc). Species in the genus Polistes include but are not limited to P. annularis (Linnaeus), P. exclamans (Viereck), P. metricus (Say), P. fuscatus (Fabricius), and P. apachus (Saussure). Species in the genus Dolichovespula include but are not limited to D. maculata (L.) and D. arenaria (Fab.). Species in the genus Vespa include but are not limited to V. crabro (L.) and V. orientalis (Linnaeus).

The taxonomic classification of Vespula vulgaris is as follows:

| | |
|---|---|
| Order: | Hymenoptera |
| Suborder: | Apocrita |
| Division: | Aculeata |
| Superfamily: | Vespoidea |
| Family: | Vespidae |
| Subfamily: | Vespinae |
| Genus: | Vespula |
| Species Group: | Vespula vulgaris species group |
| Species: | vulgaris |

The taxonomic classification for Polistes annularis is as follows:

| | |
|---|---|
| Order: | Hymenoptera |
| Suborder: | Apocrita |
| Division: | Aculeata |
| Superfamily: | Vespoidea |
| Family: | Vespidae |
| Subfamily: | Polistinae |
| Tribe: | Polistini |
| Genus: | Polistes |
| Subgenus: | Aphanilopterus |
| Species: | annularis |

As used herein, the term "immunomodulatory" refers to an ability to increase or decrease an antigen-specific immune response, either at the B cell or T cell level. Immunomodulatory activity can be detected, e.g., in T cell proliferation assays, by measurement of antibody production, lymphokines production or T cell responsiveness. In particular, in addition to affects on B cell responses, the immunomodulatory polypeptides of the invention may bind to molecules on the surface of T cells, and affect T cell responses as well.

As used herein, the phrase "immune system related disease or disorder" refers to a disease or disorder that evokes an immune response in a subject, or effects the ability of the immune system to respond to an immunogen. Hence, examples of immune system related diseases or disorders comprise a pathogenic disease or disorder; a viral disease or disorder, *e.g.*, HIV, Herpes Simplex virus, or papilloma virus; an autoimmune disease, e.g., arthritis or Lupus.

### Determining Allergen Structure

The three-dimensional structure of a protein may be determined by physical methods that are well known in the art, including and without limitation, x-ray crystallography, nmr spectroscopy and electron crystallography. Preferred, the three-dimensional structure of a protein is determined by x-ray crystallography. It is also preferred that such techniques yield a resolution of 5Å or better, at which resolution a trace of the α-carbons in the polypeptide backbone of a protein may be obtained, allowing the determination of protein secondary structure features, as for example, α-helix and β-sheet elements. More preferred is where the three dimensional structure of protein is determined at a resolution of 2Å or better, at which resolution the position of amino acid side chains may be ascertained. Structures of specific allergens are well known, as set forth in Table 9. These, or others, can be determined using the standard techniques set forth above.

The three dimensional structure of a protein may also be inferred by comparison to an homologous protein, whose structure has been determined empirically by a physical method, as for example by aligning and comparing amino acid sequences. Methods for comparing and aligning amino acid sequences are well known in the art and include, for example and without limitation, the Pileup, Gap, BestFit and Compare programs (Genetic Computer Group, Madison, WI). Such alignment and comparison allows the identification of regions of high amino acid identity or similarity, which may adopt similar or identical conformations in homologous proteins. In this manner, once the three dimensional structure is determined for one protein, the three-dimensional structure may be determined for many homologous proteins, which allows for the identification of surface and loop regions of homologous proteins.

The three dimensional structure and function of a proteins is typically effected to a lesser extent by changes in amino acids located in surface and loop regions of proteins, compared to effects observed due to changes in internally located amino acids. The amino acid residues of surface and loop regions arc therefore typically less conserved among homologous proteins, compared to internal residues. It will be appreciated by one of ordinary skill in the art, however, that surface and loop regions will occupy the same relative position in the native conformation of homologous proteins. The surface and loop regions therefore represent "conserved elements" or "homologous elements" within homologous proteins.

In addition, various spectroscopic techniques can be used to evaluate structure, particularly to confirm that the hybrid protein retains the native structure of the allergen and scaffold proteins. These techniques include, without limitation, circular dichroism spectroscopy, nmr spectroscopy (particularly at lower resolution), neutron diffraction, fluorescence spectroscopy (and other light absorption and transmission spectroscopic techniques), and the like. In particularly, evaluating identity of spectra can indicate the degree to which the hybrid protein adopts the native conformation. Circular dichroism spectroscopy provides a preferred tool for this type of evaluation.

### Molecular Biological Techniques

In accordance with the present invention there may be employed conventional molecular biology, microbiology, and recombinant DNA techniques within the skill of the art. Such techniques are explained fully in the literature. See, e.g., Sambrook, Fritsch & Maniatis, "Molecular Cloning: a Laboratory Manual," Second Edition (1989) Cold Spring Harbor Laboratory Press, Cold Spring Harbor, New York (herein "Sambrook *et al.,* 1989"); "DNA Cloning: a Practical Approach," Volumes I and II (D.N. Glover ed. 1985); "Oligonucleotide Synthesis" (M.J. Gait ed. 1984); "Nucleic Acid Hybridization" [B.D. Hames & S.J.Higgins eds. (1985)]; "Transcription And Translation" [B.D. Hames & S.J. Higgins, eds. (1984)]; "Animal Cell Culture" [R.I. Freshney, ed. (1986)]; "Immobilized Cells And Enzymes" [IRL Press, (1986)]; B. Perbal, "A Practical Guide To Molecular Cloning" (1984). Other techniques in accordance with the present invention may be found in U.S. Patent Nos. 5,593,877; 5,612,209, 5,804,201, 6,106,844, 6,270,763, 6,287,559, 6,372,471 (U.S. application Serial Nos. 08/484,388, 08/474,853, and 09/166,205 to King respectively) and in Monsalve et al. (1999, Protein Exp. Purif 16:410).

A "nucleic acid molecule" refers to the phosphate ester polymeric form of ribonucleosides (adenosine, guanosine, uridine or cytidine; "RNA molecules") or deoxyribonucleosides (deoxyadenosine, deoxyguanosine, deoxythymidine, or deoxycytidine; , "DNA molecules") in either single stranded form, or a double-stranded helix. Double stranded DNA-DNA, DNA-RNA and RNA-RNA helices arc possible. The term nucleic acid molecule, and in particular DNA or RNA molecule, refers only to the primary and secondary structure of the molecule, and does not limit it to any particular tertiary forms. Thus, this term includes double-stranded DNA found, inter alia, in linear or circular DNA molecules, restriction fragments, viruses, plasmids, and chromosomes. In discussing the structure of particular double-stranded DNA molecules, sequences may be described herein according to the normal convention of giving only the sequence in the 5' to 3' direction along the nontranscribed strand of DNA (*i.e.,* the strand having a sequence homologous to the mRNA). A "recombinant DNA molecule" is a DNA molecule that has undergone a molecular biological manipulation.

A nucleic acid molecule is "hybridizable" to another nucleic acid molecule, such as a cDNA, genomic DNA, or RNA, when a single stranded form of the nucleic acid molecule can anneal to the other nucleic acid molecule under the appropriate conditions of temperature and solution ionic strength (see Sambrook *et al.,* supra). The conditions of temperature and ionic strength determine the "stringency" of the hybridization. For preliminary screening for homologous nucleic acid molecules, low stringency hybridization conditions, corresponding to a Tm of 55E, can be used, e.g., 5x SSC, 0.1% SDS, 0.25% non-fat dry milk, and no formamide; or 30% formamide, 5x SSC, 0.5% SDS). Moderate stringency hybridization conditions correspond to a higher Tm, e.g., 40% formamide, with 5x or 6x SSC. High stringency hybridization conditions correspond to the highest Tm, e.g., 50% formamide, 5x or 6x SSC. Hybridization requires that the two nucleic acid molecules contain complementary sequences, although depending on the stringency of the hybridization, mismatches between bases are possible. The appropriate stringency for hybridizing nucleic acid molecules depends on the length of the nucleic acid molecules and the degree of complementation, variables well known in the art. The greater the degree of similarity or homology between two nucleotide sequences, the greater the value of Tm for hybrids of nucleic acid molecules having those sequences. The relative stability (corresponding to higher Tm) of nucleic acid hybridizations decreases in the following order: RNA:RNA, DNA:RNA, DNA:DNA. For hybrids of greater than 100 nucleotides in length, equations for calculating Tm have been derived (see Sambrook *et al.,* supra, 9.50-0.51). For hybridization with shorter nucleic acid molecules, i.e., oligonucleotides, the position of mismatches becomes more important, and the length of the oligonucleotide determines its specificity (see Sambrook *et al.,* supra, 11.7-11.8). Preferably a minimum length for a hybridizable nucleic acid molecule is at least about 10 nucleotide; more preferably the length is at least about 20 nucleotides; even more preferably at least about 30 nucleotides; and most preferably at least about 40 nucleotides.

In a specific embodiment, the term "standard hybridization conditions" refers to a Tm of 55EC, and utilizes conditions as set forth above. In a preferred embodiment, the Tm is 60EC; in a more preferred embodiment, the Tm is 65EC.

A DNA "coding sequence".or "encoding sequence" is a double-stranded DNA sequence which is transcribed and translated into a polypeptide in vivo when placed under the control of appropriate regulatory sequences. The boundaries of the coding sequence are determined by a start codon at the 5' (amino) terminus and a translation stop codon at the 3' (carboxyl) terminus. A coding sequence can include, but is not limited to, prokaryotic sequences, cDNA from eukaryotic mRNA, genomic DNA sequences from eukaryotic (e.g., mammalian) DNA, and even synthetic DNA sequences. If the coding sequence is intended for expression in a eukaryotic cell, a polyadenylation signal and transcription termination sequence will usually be located 3' to the coding sequence.

Transcriptional and translational control sequences are DNA regulatory sequences, such as promoters, enhancers, terminators, and the like, that provide for the expression of a coding sequence in a host cell. In eukaryotic cells, polyadenylation signals are control sequences.

A "promoter sequence" is a DNA regulatory region capable of binding RNA polymerase in a cell and initiating transcription of a downstream (3' direction) coding sequence. For purposes of defining the present invention, the promoter sequence is bounded at its 3' terminus by the transcription initiation site and extends upstream (5' direction) to include the minimum number of bases or elements necessary to initiate transcription at levels detectable above background. Within the promoter sequence will be found a transcription initiation site (conveniently defined for example, by mapping with nuclease S1), as well as protein binding domains (consensus sequences) responsible for the binding of RNA polymerase. Eukaryotic promoters will often, but not always, contain "TATA" boxes and "CAT" boxes.

A coding sequence is "under the control"of or "operationally associated" with transcriptional and translational control sequences in a cell when RNA polymerase transcribes the coding sequence into mRNA, which is then translated into the protein encoded by the coding sequence. A "signal sequence" can be included before the coding sequence. This sequence encodes a "signal peptide", N-terminal to the polypeptide, that directs the host cell to transport the polypeptide to the cell surface or secrete the polypeptide into the media. The signal peptide is usually selectively degraded by the cell upon exportation. Signal sequences can be found associated with a variety of proteins native to prokaryotes and eukaryotes.

A "nucleic acid molecule" refers to the phosphate ester polymeric form of ribonucleosides (adenosine, guanosine, uridine or cytidinc; "RNA molecules") or deoxyribonucleosides (deoxyadenosine, deoxyguanosine, deoxythymidine, or deoxycytidine; "DNA molecules") in either single stranded form, or a double-stranded helix. Double stranded DNA-DNA, DNA-RNA and RNA-RNA helices are possible. The term nucleic acid molecule, and in particular DNA or RNA molecule, refers only to the primary and secondary structure of the molecule, and does not limit it to any particular tertiary forms. Thus, this term includes double-stranded DNA found, inter alia, in linear or circular DNA molecules, restriction fragments, viruses, plasmids, and chromosomes. In discussing the structure of particular double-stranded DNA molecules, sequences may be described herein according to the normal convention of giving only the sequence in the 5' to 3' direction along the nontranscribed strand of DNA *(i.e.,* the strand having a sequence homologous to the mRNA). A "recombinant DNA molecule" is a DNA molecule that has undergone a molecular biological manipulation.

A nucleic acid molecule is "hybridizable" to another nucleic acid molecule, such as a cDNA, genomic DNA, or RNA, when a single stranded form of the nucleic acid molecule can anneal to the other nucleic acid molecule under the appropriate conditions of temperature and solution ionic strength (see Sambrook *et al.,* supra). The conditions of temperature and ionic strength determine the "stringency" of the hybridization. For preliminary screening for homologous nucleic acid molecules, low stringency hybridization conditions, corresponding to a Tm of 55E, can be used, *e.g*., 5x SSC, 0.1% SDS, 0.25% non-fat dry milk, and no formamide; or 30% formamide, 5x SSC, 0.5% SDS). Moderate stringency hybridization conditions correspond to a higher Tm, *e.g.*, 40% formamide, with 5x or 6x SSC. High stringency hybridization conditions correspond to the highest Tm, e.g., 50% formamide, 5x or 6x SSC. Hybridization requires that the two nucleic acid molecules contain complementary sequences, although depending on the stringency of the hybridization, mismatches between bases are possible. The appropriate stringency for hybridizing nucleic acid molecules depends on the length of the nucleic acid molecules and the degree of complementation, variables well known in the art. The greater the degree of similarity or homology between two nucleotide sequences, the greater the value of Tm for hybrids of nucleic acid molecules having those sequences. The relative stability (corresponding to higher Tm) of nucleic acid hybridizations decreases in the following order: RNA:RNA, DNA:RNA, DNA:DNA. For hybrids of greater than 100 nucleotides in length, equations for calculating Tm have been derived (see Sambrook *et al.,* supra, 9.50-0.51). For hybridization with shorter nucleic acid molecules, *i.e.*, oligonucleotides, the position of mismatches becomes more important, and the length of the oligonucleotide determines its specificity (see Sambrook *et al.,* supra, 11.7-11.8). Preferably a minimum length for a hybridizable nucleic acid molecule is at least about 10 nucleotide; more preferably the length is at least about 20 nucleotides; even more preferably at least about 30 nucleotides; and most preferably at least about 40 nucleotides.

In a specific embodiment, the term "standard hybridization conditions" refers to a Tm of 55EC, and utilizes conditions as set forth above. In a preferred embodiment, the Tm is 60EC; in a more preferred embodiment, the Tm is 65EC.

A DNA "coding sequence" or "encoding sequence" is a double-stranded DNA sequence which is transcribed and translated into a polypeptide in vivo when placed under the control of appropriate regulatory sequences. The boundaries of the coding sequence are determined by a start codon at the 5' (amino) terminus and a translation stop codon at the 3' (carboxyl) terminus. A coding sequence can include, but is not limited to, prokaryotic sequences, cDNA from eukaryotic mRNA, genomic DNA sequences from eukaryotic (*e.g*., mammalian) DNA, and even synthetic DNA sequences. If the coding sequence is intended for expression in a eukaryotic cell, a polyadenylation signal and transcription termination sequence will usually be located 3' to the coding sequence.

Transcriptional and translational control sequences are DNA regulatory sequences, such as promoters, enhancers, terminators, and the like, that provide for the expression of a coding sequence in a host cell. In eukaryotic cells, polyadenylation signals are control sequences.

A "promoter sequence" is a DNA regulatory region capable of binding RNA polymerase in a cell and initiating transcription of a downstream (3' direction) coding sequence. For purposes of defining the present invention, the promoter sequence is bounded at its 3' terminus by the transcription initiation site and extends upstream (5' direction) to include the minimum number of bases or elements necessary to initiate transcription at levels detectable above background. Within the promoter sequence will be found a transcription initiation site (conveniently defined for example, by mapping with nuclease S1), as well as protein binding domains (consensus sequences) responsible for the binding of RNA polymerase. Eukaryotic promoters will often, but not always, contain "TATA" boxes and "CAT" boxes.

A coding sequence is "under the control"of or "operationally associated" with transcriptional and translational control sequences in a cell when RNA polymerase transcribes the coding sequence into mRNA, which is then translated into the protein encoded by the coding sequence. A "signal sequence" can be included before the coding sequence. This sequence encodes a "signal peptide", N-terminal to the polypeptide, that directs the host cell to transport the polypeptide to the cell surface or secrete the polypeptide into the media. The signal peptide is usually selectively degraded by the cell upon exportation. Signal sequences can be found associated with a variety of proteins native to prokaryotes and eukaryotes.

### Nucleic Acid Molecules Encoding Hybrid Proteins

The invention relates to isolated nucleic acid molecules encoding recombinant allergen hybrid proteins. The invention further relates to a cell line stably containing a recombinant nucleic acid molecule encoding a allergen hybrid protein, and capable of expressing such nucleic acid molecule to produce the hybrid protein. The nucleic acids can be generated from allergens, e.g., as listed in Table 8 and in certain patents and patent applications disclosed herein.

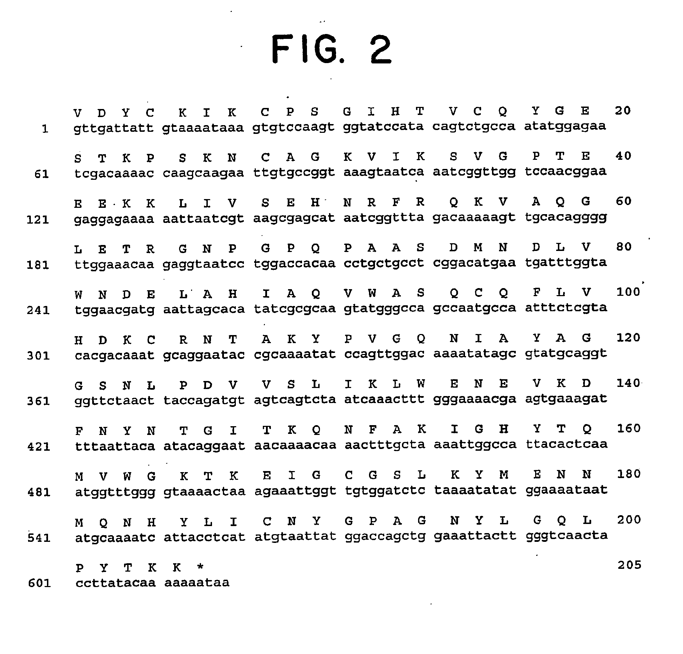

As a specific example, the present disclosure provides the complete nucleic acid sequence of a vespid venom protein. In particular, the present disclosure provides the nucleic acid sequence of a vespid Ag 5, in particular Ves v Ag 5 (SEQ ID NO: 14; see Fig. 1) and Pol a Ag 5 (SEQ ID NO: 15; see Fig. 2). Also provided are the amino acid sequences of Ves v Ag 5 (SEQ ID NO: 16; see Fig. 1) and Pol a Ag5 (SEQ ID NO: 17; see Fig. 2).

In a specific embodiment, to obtain a nucleic acid molecule of the invention, DNA fragments are amplified by polymerase chain reaction (PCR) to amplify a fragment encoding a sequence comprising the allergen peptide epitope sequence or a scaffold protein. Oligonucleotide primers representing an allergen protein or scaffold protein of the invention can be used as primers in PCR. Generally, such primers are prepared synthetically. PCR can be carried out, e.g., by use of a Perkin-Elmer Cetus thermal cycler and Taq polymerase (Gene Amp ).

Nucleic acids of the invention may also be obtained by cloning of restrictions fragments. Alternatively, nucleic acids of the invention may be obtained by recombination of nucleic acids in vivo or in vitro. In some instances recombination depends on sequence homology between the nucleic acids that participate in a recombination event, but in other instances the nucleic acids undergoing recombination need not contain significant homology, as is the case, for example, in "illegitimate" recombination events. One of ordinary will recognize recombination of nucleic acids may be an inter- or intramolecular event.

Alternatives to isolating the allergen proteins or scaffold DNA or cDNA include, but are not limited to, chemically synthesizing the gene sequence itself from the sequence provided herein.

The above methods are not meant to limit the methods by which DNA of the invention may be obtained.

The methods used to obtain a nucleic acid of the invention may lead to the insertion or deletion of nucleotides at junctions where nucleic acids are joined, by recombinant or other techniques. In one embodiment, nucleotides may be inserted or deleted at the junction of a nucleic acid encoding an antigenic peptide and the nucleic acid encoding a scaffold protein. Such nucleic acids are fully within the scope of the invention. Accordingly, the invention encompasses hybrid proteins wherein amino acids have been inserted or deleted at the junction of a peptide epitope sequence and a scaffold protein sequence.

Nucleic acid sequence of the cloned hybrid protein, or starting materials thereof, can be modified by any of numerous strategies known in the art (Maniatis, T., 1990, Molecular Cloning, A Laboratory Manual, 2d ed., Cold Spring Harbor Laboratory, Cold Spring Harbor, New York). The sequence can be cleaved at appropriate sites with restriction cndonuclease(s), followed by further enzymatic modification if desired, isolated, and ligated in vitro. In the production of the nucleic acid encoding a hybrid protein, care should be taken to ensure that the modified nucleic acid remains within the same translational reading frame as the scaffold protein, uninterrupted by translational stop signals.

Additionally, the nucleic encoding an allergen peptide epitope sequence or scaffold protein can be mutated *in vitro* or *in vivo,* to create and/or destroy translation, initiation, and/or termination sequences, or to create variations in coding regions and/or form new restriction endonuclease sites or destroy preexisting ones, to facilitate further *in vitro* modification. Any technique for mutagenesis known in the art can be used, including but not limited to, in vitro site-directed mutagenesis (Hutchinson et al., 1978, J. Biol. Chem. 253:6551; Zoller and Smith, 1984, DNA 3:479-488; Oliphant et al., 1986, Gene 44:177; Hutchinson et al., 1986, Proc. Natl. Acad. Sci. U.S.A. 83:710), use of TAB linkers (Pharmacia), etc. PCR techniques are preferred for site directed mutagenesis (see Higuchi, 1989, "Using PCR to Engineer DNA", in PCR Technology: Principles and Applications for DNA Amplification, H. Erlich, ed., Stockton Press, Chapter 6, pp. 61-70).

A large number of vector-host systems known in the art may be used to express a DNA of the invention. Possible vectors include, but are not limited to, plasmids or modified viruses, but the vector system must be compatible with the host cell used. Such vectors include, but are not limited to, bacteriophages such as lambda derivatives, or plasmids such as various pBR322 derivatives, for example, pUC, CR, pGEX vectors, pmal-c, pFLAG, etc. The insertion into a cloning vector can, for example, be accomplished by ligating the DNA fragment into a cloning vector which has complementary cohesive termini. In a preferred aspect of the invention, the PCR amplified nucleic acid molecules of the invention contain 3'-overhanging A-nucleotides, and can be used directly for cloning into a pCR vector with compatible T-nucleotide overhangs (Invitrogen Corp., San Diego, CA). However, if the complementary restriction sites used to fragment the DNA are not present in the cloning vector, the ends of the DNA molecules may be enzymatically modified. Alternatively, any site desired may be produced by ligating nucleotide sequences (linkers) onto the DNA termini; these ligated linkers may comprise specific chemically synthesized oligonucleotides encoding restriction endonuclease recognition sequences. In an alternative method, the cleaved vector and a DNA of the invention may be modified by homopolymeric tailing. Recombinant molecules can be introduced into host cells via transformation, transfection, infection, electroporation, etc., so that many copies of the gene sequence are generated.

In specific embodiments, transformation of host cells with recombinant DNA molecules that incorporate the DNA of the invention enables generation of multiple copies of the DNA. Thus, the DNA may be obtained in large quantities by growing transformants, isolating the recombinant DNA molecules from the transformants and, when necessary, retrieving the inserted sequences from the isolated recombinant DNA.

The nucleotide sequences encoding Ves v 5 polypeptide epitope sequences of SEQ ID NO: 1-13 and 93-95 are given respectively in SEQ ID NO: 18-30 and 96-98.

### Expression of an Allergen Hybrid Protein

The nucleotide sequence coding for a hybrid protein or an immunomodulatory fragment, derivative or analog thereof, can be inserted into an appropriate expression vector, *i.e.,* a vector that contains the necessary elements for the transcription and translation of the inserted protein-coding sequence. Such elements are termed herein a "promoter." Thus, the nucleic acid molecule encoding the hybrid protein is operationally associated with the promoter. An expression vector also preferably includes a replication origin. The necessary transcriptional and translational signals can also be supplied by the native gene encoding the allergen or scaffold protein and/or its flanking regions. Potential host-vector systems include but are not limited to mammalian cell systems, e.g., infected with virus (*e.g*., vaccinia virus, adenovirus, etc.); insect cell systems, e.g., infected with virus (*e.g*., baculovirus); microorganisms such as yeast containing yeast vectors; or bacteria transformed with bacteriophage, DNA, plasmid DNA, or cosmid DNA. The expression elements of vectors vary in their strengths and specificities. Depending on the host-vector system utilized, any one of a number of suitable transcription and translation elements may be used.

Alternatively a recombinant hybrid protein of the invention, or an immunomodulatory fragment, derivative or analog thereof, is expressed chromosomally, after integration of the hybrid protein coding sequence by recombination, In this regard, any of a number of amplification systems may be used to achieve high levels of stable gene expression (See Sambrook *et al.,* 1989, supra, at Section 16.28).

The cell into which the recombinant vector comprising the nucleic acid molecule encoding the hybrid protein is cultured in an appropriate cell culture medium under conditions that provide for expression of the hybrid protein by the cell. The expressed hybrid protein can then be recovered from the culture according to methods well known in the art. Such methods are described in detail, *infra.*

Alternatively, a hybrid protein can be expressed initially with amino acids that are subsequently cleaved from the hybrid protein. The sequences to be removed can be amino- or carboxyl-terminal to the hybrid protein sequences. The sequences may be removed either *in vivo* or *in vitro.* Preferably the sequences are removed by cleavage at a specific site by a protease, *e.g*., signal peptidase, Factor Xa, Kex2 or a dipeptidyl amino peptidase. A recombinant DNA molecule encoding such a hybrid protein that includes a polypeptide to be cleaved by a protease comprises a sequence encoding the peptide to be cleaved from the hybrid protein joined in-frame to the coding sequence for a allergen hybrid.

Alternatively, the hybrid proteins are expressed with an additional sequence comprising about six histidine residues, *e.g.,* using a pQE vector (QIAGEN, Chatsworth, CA). The presence of the histidine makes possible the selective isolation of recombinant proteins on a Ni-chelation column. Other such handles include, but are not limited to, FLAG, a myc tag, GST, etc.

Alternatively, a periplasmic form of the hybrid protein (containing a signal sequence) can be produced for export of the protein to a yeast periplasm or into a culture medium. Export to the periplasm or into the medium can promote proper folding of the expressed protein.

Any of the methods previously described for the insertion of DNA fragments into a vector may be used to construct expression vectors containing a gene consisting of appropriate transcriptional/translational control signals and the protein coding sequences. These methods may include in vitro recombinant DNA and synthetic techniques and in vivo recombinants (genetic recombination).

Expression of nucleic acid sequence encoding a hybrid protein, or an immunomodulatory fragment thereof, may be regulated by a second nucleic acid sequence so that the hybrid protein is expressed in a host transformed with the recombinant DNA molecule. For example, expression of a hybrid protein may be controlled by any promoter/enhancer element known in the art, but these regulatory elements must be functional in the host selected for expression. Promoters which may be used to control expression of the hybrid protein coding sequences include, but are not limited to, the CMV promoter, the SV40 early promoter region (Benoist and Chambon, 1981, Nature 290:304-310), the promoter contained in the 3' long terminal repeat of Rous sarcoma virus (Yamamoto et al., 1980, Cell 22:787-797), the herpes thymidine kinase promoter (Wagner et al., 1981, Proc. Natl. Acad. Sci. U.S.A. 78:1441-1445), the regulatory sequences of the metallothionein gene (Brinster et al., 1982, Nature 296:39-42); prokaryotic expression vectors such as the β-laetamase promoter (Villa-Kamaroff et al., 1978, Proc. Natl. Acad. Sci. U.S.A. 75:3727-3731), or the tac promoter (DeBoer et al., 1983, Proc. Natl. Acad. Sci. U.S.A. 80:21-25); see also "Useful proteins from recombinant bacteria" in Scientific American, 1980, 242:74-94; promoter elements from yeast or other fungi such as the Gal 4 promoter, the ADC (alcohol dehydrogenase) promoter, PGK (phosphoglycerol kinase) promoter, alkaline phosphatase promoter; and the animal transcriptional control regions, which exhibit tissue specificity and have been utilized in transgenic animals.

In addition, a host cell strain may be chosen which modulates the expression of the inserted sequences, or modifies and processes the gene product in the specific fashion desired. Different host cells have characteristic and specific mechanisms for the translational and post-translational processing and modification (e.g., glycosylation, cleavage [e.g., of a signal sequence]) of proteins. Appropriate cell lines or host systems can be chosen to ensure the desired modification and processing of the foreign protein expressed. For example, expression in a bacterial system can be used to produce an nonglycosylated core protein product. However, the enzyme protein expressed in bacteria may not be properly folded. Expression in yeast can produce a glycosylated product. Expression in insect cells can be used to increase the likelihood of native glycosylation and folding of a heterologous allergen hybrid protein. Furthermore, different vector/host expression systems may affect processing reactions, such as proteolytic cleavages, to a different extent.

Vectors are introduced into the desired host cells by methods known in the art, e.g., transfcction, electroporation, micro injection, transduction, cell hybrid, DEAE dextran, calcium phosphate precipitation, lipofection (lysosome fusion), use of a gene gun, or a DNA vector transporter (see, *e.g*., Wu et al., 1992, J. Biol. Chem. 267:963-967; Wu and Wu, 1988, J. Biol. Chcm. 263:14621-14624; Hartmut *et al.,* Canadian Patent Application No. 2,012,311, filed March 15, 1990).

Both cDNA and genomic sequences can be cloned and expressed.

It is further contemplated that the hybrid proteins of the present invention, or fragments, derivatives or analogs thereof, can be prepared synthetically, e.g., by solid phase peptide synthesis.

Once the recombinant hybrid protein is identified, it may be isolated and purified by standard methods including chromatography (*e.g*., ion exchange, affinity, size exclusion, and reverse phase chromatography), centrifugation, differential solubility, or by any other standard technique for the purification of proteins.

In a particular, a hybrid protein and fragments thereof can be engineered to include about six histidyl residues, which makes possible the selective isolation of the recombinant protein on a Ni-chelation column. In a preferred aspect, the proteins are further purified by reverse phase chromatography.

Alternatively, the recombinant hybrid protein may include additional sequences that allow the hybrid protein to be targeted for affinity purification such as FLAG, MYC, or GST (glutathione-S-transferase). For example, antibody specific for the additional sequences of the hybrid protein can be immobilized on a solid support, *e.g.*, cyanogen bromide-activated Sepharose, and used to purify the hybrid protein. In another embodiment, a binding partner of the additional sequences, such as a receptor or ligand, can be immobilized and used to affinity purify the hybrid protein.

For example, the hybrid protein, preferably purified, is used without further modification, *i.e.*, without cleaving or otherwise removing any sequences that may be present in addition to the peptide epitope sequence and the scaffold protein. In a preferred embodiment, the hybrid protein can be used therapeutically, *e.g.,* to modulate an immune response.

For example, the purified hybrid protein is treated to cleave and remove any sequences that may have been added to the scaffold protein. For example, where the hybrid protein has been prepared to include a protease sensitive cleavage site, the hybrid protein can be treated with the protease to cleave the protease specific site and release the hybrid protein. In a specific embodiment, the hybrid protein is cleaved by treatment with Factor Xa.

Hybrid proteins can contain altered epitope or scaffold, or both, sequences, in which functionally equivalent amino acid residues are substituted for residues within the sequence resulting in a conservative amino acid substitution. For example, one or more amino acid residues within the sequence can be substituted by another amino acid of a similar polarity which acts as a functional equivalent, resulting in a silent alteration. Substitutes for an amino acid within the sequence may be selected from other members of the class to which the amino acid belongs. For example, the nonpolar (hydrophobic) amino acids include alaninc, leucine, isoleucine, valine, prolinc, phenylalanine, tryptophan and methionine. The polar neutral amino acids include glycine, serine, threonine, cysteine, tyrosine, asparagine, and glutamine. The positively charged (basic) amino acids include arginine, lysine and histidine. The negatively charged (acidic) amino acids include aspartic acid and glutamic acid.

Manipulations of the recombinant hybrid protein may also be made at the protein level such as glycosylation, acetylation, phosphorylation, amidation, reduction and carboxymethylation, derivatization by known protecting/blocking groups, proteolytic cleavage, linkage to an antibody molecule or other cellular ligand, etc. Any of numerous chemical modifications may be carried out by known techniques, including but not limited to specific chemical cleavage by cyanogen bromide, trypsin, chymotrypsin, papain, V8 protease, NaBH4; acetylation, formylation, oxidation, reduction; metabolic synthesis in the presence of tunicamycin; etc.

In a particular, the hybrid protein is expressed in an insect cell expression system, *e.g.*, using a baculovirus expression vector. Preferably, the hybrid protein is expressed in yeast, *e.g*., without limitation, Picchia pastoris, using appropriate expression systems. As pointed out above, these expression systems should yield "native" glycosylation and structure, particularly secondary and tertiary structure, of the expressed polypeptide.

### Activity Assays With Hybrid Proteins of the Invention

Numerous assays are known in immunology for evaluating the immunomodulatory activity of an antigen. For example, the hybrid proteins can be tested for the ability to bind to antibodies specific for the allergen or the scaffold. Preferably, such antibodies that are detected in the diagnostic assay are of the IgG or IgE class. Hybrid proteins produced in eukaryotic expression systems, and particularly yeast cell expression systems, can have the correct structure for antibody binding. Hybrid proteins expressed in bacterial expression systems may not, and would thus require refolding prior to use in a diagnostic assay for antibody binding.

The hybrid proteins of the invention can be tested in a proliferation assay for T cell responses. For such T cell response assays, the expression system used to produce the protein does not appear to affect the immunomodulatory activity of the protein. Generally, lymphocytes from a sensitized host are obtained. The host can be a mouse that has been immunized with an allergen, scaffold or hybrid protein, such as a vespid venom Ag 5 that has been produced recombinantly.

Preferably, peripheral blood leukocytes are obtained from a human who is sensitive to the allergen. Using techniques that are well known in the art, T lymphocyte response to the protein can be measured in vitro. In a specific embodiment, infra, T cell responses are detected by measuring incorporation of ³H-thymidine, which increases with DNA synthesis associated with proliferation.

Cell proliferation can also be detected using an MTT assay (Mossman, 1983, J. Immunol. Methods 65:55; Niks and Otto, 1990, J. Immunol. Methods 130:140). Any method for detecting T cell proliferation known in the art can be used with the vespid protein produced according to the present invention.

Similarly, lymphokine production assays can be practiced according to the present invention. In one embodiment, lymphokine production can be assayed using immunological or co-stimulation assays (see, *e.g.,* Fehlner et al., 1991, J. Immunol. 146:799) or using the ELISPOT technique (Czerkinsky et al., 1988, J. Immunol. Methods 110:29). Alternatively, mRNA for lymphokines can be detected, *e.g.,* by amplification (see Brenner et al., 1989, BioTechniques 7:1096) or *in situ* hybridization (see, *e.g.,* Kasaian and Biron, 1989, J. Immunol. 142:1287). Of particular interest are those individuals whose T cells produce lymphokines associated with IgE isotype switch events, *e.g.,* IL-4 and IL-5 (Purkeson and Isakson, 1992, J. Exp. Med. 175:973).

Thus, in a preferred aspect, the hybrid proteins produced according to the present invention can be used in *in vitro* assays with peripheral blood lymphocytes or, more preferably, cell lines derived from peripheral blood lymphocytes, obtained from allergen sensitive individuals to detect secretion of lymphokincs ordinarily associated with allergic responses, *e.g*., IL-4. Such assays may indicate which component or components of the hybrid protein are responsible for the allergic condition.

### Therapeutic Uses of the Hybrid Protein and Nucleic Acid Vectors

The present invention provides a plentiful source of a hybrid protein, e.g., produced by recombinant techniques. Alternatively, a hybrid protein can be produced by peptide synthesis.

The invention contemplates hybrid proteins of the invention in therapeutic (pharmaceutical) composition, for the use in the therapy of allergen-specific allergic conditions, treating allergen-specific allergic conditions, immune system related conditions, and modulating immune response in a mammal against an immunogen.

The phrase "therapeutically effective amount" is used herein to mean an amount sufficient to treat, and preferably increase by at least about 30 percent, more preferably by at least 50 percent, most preferably by at least 90 percent, the ability of the immune system of a subject to combat effectively an immunogen. As further studies are conducted, information will emerge regarding appropriate dosage levels for modulation of immune system response towards an immunogen in various patients, and the ordinary skilled worker, considering the therapeutic context, age and general health of the recipient, will be able to ascertain proper dosing.

### Therapeutic Methods

Therapeutic compositions of the invention (see, *infra*) can be for use in immunotherapy, also referred to as hyposensitization therapy. Immunotherapy has proven effective in allergic diseases, particular insect allergy. Allergens are administered parenterally over a long period of time in gradually increasing doses. Such therapy may be particularly effective when the allergen or allergens to which the patient is sensitive have been specifically identified and the therapy is targeted to those allergen(s). However, this approach suffers the drawback of potentially precipitating an allergic reaction; especially anaphylaxis. Thus, the availability of hybrid proteins in large quantities is important for immunotherapy of allergy because they induce an effective IgG response against the allergen without an allergic reaction.

As discussed in the Background of the Invention, the presence of B cell epitopes on an allergen can cause an undesirable systemic reaction when the allergen is used for immunotherapy. Thus, a particular advantage of the invention is the capability to provide allergen polypeptides that do not cause undesirable systemic effects.

For example, one or more hybrid proteins can be injected subcutaneously to decrease the T cell response to the native molecule, *e.g.,* as described by Brine et al. (1993, Proc. Natl. Acad. Sci. U.S.A. 90:7608-12).

For example, one or more hybrid proteins can be administered intranasally to suppress allergen-specific responses in naive and sensitized subjects (see *e.g*., Hoyne et al., 1993, J. Exp. Med. 178:1783-88).

Administration of a hybrid protein of the invention is expected to induce a strong anti-allergen B cell (antibody), IgG response that will block IgE antibodies, and thus, have a therapeutic effect.

These results can also be achieved by administration of a vector that permits expression of the hybrid protein, *i.e.,* by gene therapy. Preferred vectors, particularly for cellular assays *in vitro* and *in vivo,* are viral vectors, such as lentiviruses, retroviruses, herpes viruses, adenoviruses, adeno-associated viruses, vaccinia virus, baculovirus, alphaviruses (especially Sindbis viruses and Semliki Forest viruses), and other recombinant viruses with desirable cellular tropism; and non-viral vectors. For gene therapy *in vivo* or *ex vivo,* a pharmaceutically acceptable vector is preferred, such as a replication incompetent viral vector. Pharmaceutically acceptable vectors containing the nucleic acids of this invention can be further modified for transient or stable expression. As used herein, the term "pharmaceutically acceptable vector" includes, but is not limited to, a vector or delivery vehicle having the ability to selectively target and introduce the nucleic acid into cells.

Thus, a gene encoding a functional or mutant protein or polypeptide domain fragment thereof can be introduced *in vivo*, *ex vivo,* or *in vitro* using a viral vector or through direct introduction of DNA. Expression in targeted tissues can be affected by targeting the transgenic vector to specific cells, such as with a viral vector or a receptor ligand, or by using a tissue-specific promoter, or both. Targeted gene delivery is described in PCT Publication No. WO 95/28494.

Viral vectors commonly used for *in vivo* or *ex vivo* targeting and therapy procedures arc DNA-bascd vectors and retroviral vectors. Methods for constructing and using viral vectors are known in the art (*see, e.g.,* Miller and Rosman, BioTechniques 1992, 7:980-990). Preferably, the viral vectors are replication-defective, that is, they are unable to replicate autonomously in the target cell. Preferably, the replication defective virus is a minimal virus, *i.e.,* it retains only the sequences of its genome that are necessary for encapsidating the genome to produce viral particles.

DNA viral vectors include an attenuated or defective DNA virus, such as but not limited to, herpes simplex virus (HSV), papillomavirus, Epstein Barr virus (EBV), adenovirus, adeno-associated virus (AAV), alphavirus (especially Sindbis virus), and the like. Defective viruses that entirely or almost entirely lack viral genes are preferred. Defective virus is not infective after introduction into a cell. Use of defective viral vectors allows for administration to cells in a specific, localized area, without concern that the vector can infect other cells. Thus, a specific tissue can be specifically targeted. Examples of particular vectors include, but are not limited to, a defective herpes virus 1 (HSV1) vector (Kaplitt et al., Molec. Cell. Neurosci. 1991, 2:320-330), defective herpes virus vector lacking a glyco-protein L gene, or other defective herpes virus vectors (PCT Publication Nos. WO 94/21807 and WO 92/05263); an attenuated adenovirus vector, such as the vector described by Stratford-Perricaudet et al. (J. Clin. Invest. 1992, 90:626-630; *see also* La Salle et al., Science 1993, 259:988-990); a defective adeno-associated virus vector (Samulski et al., J. Virol., 1987, 61:3096-3101; Samulski et al., J. Virol. 1989, 63:3822-3828; Lebkowski et al., Mol. Cell. Biol. 1988, 8:3988-3996); and Alphavirus vectors, including Sindbis virus and Semliki Forest virus-based vectors (U.S. Patent No. 5,091,309; PCT Publication No. WO 98/44132; Schlesinger and Dubensky, Curr. Opin. Biotechnol. 1999, 5:434-9; Zaks et al., Nat. Med. 1999, 7:823-7).

Various companies produce viral vectors commercially, including, but not limited to, Avigen, Inc. (Alameda, CA; AAV vectors), Cell Genesys (Foster City, CA; retroviral, adenoviral, AAV, and lentiviral vectors), Clontech (retroviral and baculoviral vectors), Genovo, Inc. (Sharon Hill, PA; adenoviral and AAV vectors), Genvec (France; adenoviral vectors), IntroGene (Leiden, Netherlands; adenoviral vectors), Molecular Medicine (retroviral, adenoviral, AAV, and herpes viral vectors), Norgen (adenoviral vectors), Oxford BioMedica (Oxford, United Kingdom; lentiviral vectors), and Transgene (Strasbourg, France; adenoviral, vaccinia, retroviral, and lentiviral vectors).

For example, the vector can be introduced *in vivo* by lipofection, as naked DNA, or with other transfection facilitating agents (peptides, polymers, etc.). Synthetic cationic lipids can be used to prepare liposomes for *in vivo* transfection of a gene encoding a marker (Felgner, et. al., Proc. Natl. Acad. Sci. USA 1987, 84:7413-7417; Felgner and Ringold, Science 1989, 337:387-388; see Mackey, et al., Proc. Natl. Acad. Sci. USA 1988, 85:8027-8031; Ulmer et al., Science 1993, 259:1745-1748). Useful lipid compounds and compositions for transfer of nucleic acids arc described in PCT Patent Publication Nos. WO 95/18863 and WO 96/17823, and in U.S. Patent No. 5,459,127. Lipids may be chemically coupled to other molecules for the purpose of targeting (see Mackey, *et. al., supra*). Targeted peptides, *e.g.,* hormones or neurotransmitters, and proteins such as antibodies, or non-peptide molecules could be coupled to liposomes chemically.

Other molecules are also useful for facilitating transfection of a nucleic acid *in vivo,* such as a cationic oligopeptide (*e.g.,* PCT Patent Publication No. WO 95/21931), peptides derived from DNA binding proteins (*e.g.*, PCT Patent Publication No. WO 96/25508), or a cationic polymer (*e.g*., PCT Patent Publication No. WO 95/21931).

It is also possible to introduce the vector *in vivo* as a naked DNA plasmid. Naked DNA vectors for gene therapy can be introduced into the desired host cells by methods known in the art, *e.g.,* electroporation, microinjection, cell fusion, DEAE dextran, calcium phosphate precipitation, use of a gene gun, or use of a DNA vector transporter (*see, e.g.,* Wu et al., J. Biol. Chem. 1992, 267:963-967; Wu and Wu, J. Biol. Chem. 1988, 263:14621-14624; Canadian Patent Application No. 2,012,311; Williams et al., Proc. Natl. Acad. Sci. USA 1991, 88:2726-2730). Receptor-mediated DNA delivery approaches can also be used (Curiel et al., Hum. Gene Ther. 1992, 3:147-154; Wu and Wu, J. Biol. Chem. 1987, 262:4429-4432). U.S. Patent Nos. 5,580,859 and 5,589,466 disclose delivery of exogenous DNA sequences, free oftransfection facilitating agents, in a mammal. Recently, a relatively low voltage, high efficiency *in vivo* DNA transfer technique, termed electrotransfer, has been described (Mir et al., C.P. Acad. Sci. 1988, 321:893; PCT Publication Nos. WO 99/01157, WO 99/01158, and WO 99/01175).

### Treatment of Immune System Related Diseases

As explained above, the present invention relates to hybrid proteins for treating immune system related diseases or disorders, or for modulating immune response in a mammal towards an immunogen. In particular, Applicant has discovered that the hybrid proteins have applications in modulating a subject's immune response to various immunogens, in a manner that elicits an immune response without eliciting an allergenic response. In a particular, hybrid proteins modulate a subject's immune system to have increased ability to combat pathogens and viruses including, but not limited to, HIV, Herpes Simplex virus, or papilloma virus. Such a method comprises administering to a subject a therapeutically effective amount of a pharmaceutical composition comprising a polypcptidc encoded by an isolated nucleic acid molecule comprising a DNA molecule of the invention.

Furthermore, it has been discovered that the hybrid proteins, nucleic acids and vectors of the invention also have applications in treating an immune system related disease or disorder, or a symptom related thereto. As used herein, the phrase "immune system related disease or disorder" refers to a disease or disorder which evokes an immune response in a subject, or effects the ability of the immune system to respond to an immunogen. Examples of immune system related diseases or disorders which can be treated with agents and pharmaceutical compositions of the invention include, but are not limited to, a pathogenic disease or disorder; a viral disease or disorder, *e.g.* HIV, Herpes Simplex virus, or papilloma virus; or an autoimmune disease, *e.g.* arthritis or Lupus.

Moreover, a method for treating an immune system related disease or disorder, or a symptom related thereto, comprising administering a therapeutically effective amount of a pharmaceutical composition for treating an immune system related disease or disorder to a subject is described. Hence, for example, should the immune system related disease or disorder involve HIV, a clinically significant change would, for example, involve an increase in white blood cell count in a subject to whom a pharmaceutical composition of the invention is administered relative to white blood cell count prior to administration. Other such examples of monitoring a clinically significant change in a subject will be readily apparent to one of ordinary skill in the art. Furthermore, as further studies are conducted, information will emerge regarding appropriate dosage levels for treating an immune system related disease or disorder, or a symptom related thereto in various patients, and the ordinary skilled worker, considering the therapeutic context, age and general health of the recipient, will be able to ascertain proper dosing. Examples of pharmaceutically acceptable compositions arc described infra.

### Pharmaceutically Acceptable Compositions

The *in vivo* therapeutic compositions of the invention may also contain appropriate pharmaceutically acceptable carriers, excipients, diluents and adjuvants. As used herein, the phrase "pharmaceutically acceptable" preferably means approved by a regulatory agency of a government, in particular the Federal government or a state government, or listed in the U.S. Pharmacopeia or another generally recognized pharmacopeia for use in animals, and more particularly in humans. Suitable pharmaceutical carriers arc described in "Remington's Pharmaceutical Sciences" by E.W. Martin.

Such pharmaceutically acceptable carriers can be sterile liquids, such as water and oils, including those of petroleum, animal, vegetable or synthetic origin, such as peanut oil, soybean oil, mineral oil, sesame oil and the like. Water is a preferred carrier when the pharmaceutical composition is administered intravenously. Saline solutions and aqueous dextrose and glycerol solutions can also be employed as liquid carriers, particularly for injectable solutions. Suitable pharmaceutical excipients include mannitol, human serum albumin (HSA), starch, glucose, lactose, sucrose, gelatin, malt, rice, flour, chalk, silica gel, magnesium carbonate, magnesium stearate, sodium stearate, glycerol monostearate, talc, sodium chloride, dried skim milk, glycerol, propylene, glycol, water, ethanol and the like. These compositions can take the form of solutions, suspensions, tablets, pills, capsules, powders, sustained-release formulations and the like.

Such compositions will contain an effective diagnostic or therapeutic amount of the active compound together with a suitable amount of carrier so as to provide the form for proper administration to the patient. While intravenous injection is a very effective form of administration, other modes can be employed, such as by injection, or by oral, nasal or parenteral administration.

The invention will be further clarified by the following examples, which are intended to be purely exemplary of the invention.

### Example 1 Construction of Ag5 hybrid cDNAs

Primers 1-24 used in the Examples are listed in Table 1.

Vcs v 5 EA and KR constructs were prepared by PCR amplification of Vcs v 5 cDNA template (Lu et al., 1993, J. Immunol. 150:2823) with the primers I (SEQ ID NO: 31) and 3 (SEQ ID NO: 33) or 2 (SEQ ID NO: 32) and 3 (SEQ ID NO: 33), respectively. Pol a 5 EA and KR constructs were prepared by PCR amplification of a Pol a cDNA template (Lu et al., 1993, J. Immunol. 150:2823) with the primers 4 (SEQ ID NO: 34) and 6 (SEQ ID NO: 24) or 5 (SEQ ID NO: 35) and 6 (SEQ ID NO: 36), respectively. Each cDNA construct contained an EcoRI or XhoI site at the 5'terminus and an XbaI site at the 3'-terminus. cDNAs were cloned in the plasmid vector pPICZαA (Invitrogen Corp, San Diego, CA) as either EcoRI -XbaI or XhoI-XbaI fragments. Positive clones were identified by PCR. The sequences of recombinant Ag5 and hybrid cDNAs in pPICZαA were confirmed by DNA sequencing of the inserts. Other constructs were prepared as described in King et al. (2001, J. Immunol. 166:6057-6065).
(i) *PV1-46.* The PV1-46 hybrid was constructed by joining amino-terminal sequences of Ves v 5 and carboxyl-terminal sequences of Pol a 5 at the peptide sequence EH, which is present at amino acids 47-48 and 49-50 of the respective proteins. The nucleotide sequence encoding the EH peptide in Ves v 5 is GAG CAC, which corresponds to a Bsi HKA I restriction enzyme cleavage site.
   To facilitate construction of the PV1-46 hybrid, the natural DNA sequence (GAG CAT) encoding the Pol a 5 EH peptide at amino acids 49-50 was mutated to a Bsi HKA I site by a PCR overlap extension method (Ho et al., 1989, Gene 77:51), as follows. A first step comprised two separate PCRs. In one PCR, primers 4 (SEQ ID NO: 34) and 8 (SEQ ID NO: 38) and were used to amplify DNA encoding residues 1-53 of Pol a 5 wherein the EH-encoding sequence was converted to a Bsi HKA I site. In a second PCR, primers 7 (SEQ ID NO: 37) and 6 (SEQ ID NO:36) were used to amplify DNA encoding residues 47-205 of Pol a 5 wherein the EH-encoding sequence was converted to a Bsi HKA I site. Both PCRs were performed with 1-40 ng Pol a cDNA as template and 50 pmole each of sense and anti-sense primers in 100 µl of PCR buffer containing 0.2 mM dNTPs and 5 units Taq polymerase. Cycling conditions were 0.5 min denaturation at 95°, 0.5 min annealing at 55° and 2 min extension at 72° for 35 cycles. The products of these two PCRs contained an overlap region. In the second step of the overlap extension procedure, the purified products of the first two reactions were mixed to served as the template for a third PCR with flanking primers 4 (SEQ ID NO: 34) and 6 (SEQ ID NO: 36), yielding a full length Pol a 5 with the EH-encoding sequence converted to a Bsi HKA I site.
   Hybrid PV 1-46 encoding cDNA was then prepared by ligation of the appropriate Bsi HKA I fragments from Vcs v 5 and the modified Pol a 5 cDNAs into pPICZαA, as described above for Ag5 encoding cDNAs.
*(ii) PV109-155.* The PV109-155 hybrid was constructed by joining amino-terminal sequences of Ves v 5 and carboxyl-terminal sequences of Pol a 5 at the peptide sequence KY, which is present at amino acids 106-107 and 109-110 of the respective proteins. The KY peptides of both Ag 5s are encoded by the nucleotide sequence AAA TAT. To construct PV109-155, KY-encoding sequences of appropriate Ag5 or hybrid cDNAs were mutated to an Apo I restriction enzyme cleavage site (AAA TTT) encoding a peptide sequence ofKF. These single base mutations were made using the PCR overlap extension method (Ho et al., 1989, Gene 77:51) described in Example 1. In one set of reactions, the KY-encoding nucleotide sequence of PV1-155 cDNA was converted by performing the PCR overlap procedure with mutagenic primers 9 (SEQ ID NO: 39) and 10 (SEQ ID NO: 40). In a second set of reactions, the KY-encoding nucleotide sequence of Pol a 5 cDNA was converted by performing the PCR overlap procedure with mutagenic primers 11 (SEQ ID NO: 41) and 12 (SEQ ID NO: 42). Hybrid PV109-155 encoding cDNA was prepared by ligation of the appropriate fragments from Apo 1 digestions of converted Pol a 5 and converted PV1-155 encoding cDNAs into pPICZαA.
*(iii) PVI-155 and PV156-204.* Ves v 5 and Pol a 5 cDNAs have a common Eae I restriction site encoding amino acid residues 154-156. Hybrid PV156-204 and PV1-155 encoding cDNAs were prepared by ligation of the appropriate Eae I fragments of their parent cDNAs into pPICZαA.
*(iv) PV1-8, PV1-18 and PV195-204.* These hybrids were prepared by PCR with cDNA of Pol a 5 as the template. PV1-8 was prepared using primers 2 (SEQ ID NO: 32) and 6 (SEQ ID NO: 36). PV1-18 was prepared using primers 6 (SEQ ID NO: 36) and 13 (SEQ ID NO: 43). PV195-204 was prepared using primers 4 (SEQ ID NO: 34) and 14 (SEQ ID NO: 44). The hybrids were cloned into pPICZαA.
*(v) PV1-24, PV1-32 , PV1-39, PV1-50, PV1-57 and PV1-70.* These hybrids were constructed using the PCR overlap extension method given in Example 1 (Ho et al., 1989, Gene 77:51). For PV1-24, first round PCRs were conducted using primers 1 (SEQ ID NO: 31) and 15 (SEQ ID NO: 45) with Ves v 5 cDNA as template and primers 6 (SEQ ID NO: 36) and 16 (SEQ ID NO: 46) with Pol a 5 cDNA as template. The two overlapping PCR products were then purified and used as template in a third PCR using flanking primers 1 (SEQ ID NO: 31) and 6 (SEQ ID NO: 36) to yield PV1-24. For PV1-32, first round PCRs were conducted using primers 1 (SEQ ID NO: 31) and 18 (SEQ ID NO: 48) with Ves v 5 cDNA as template and primers 6 (SEQ ID NO: 36) and 17 (SEQ ID NO: 47) with Pol a 5 cDNA as template. The two overlapping PCR products were then purified and used as template in a third PCR using flanking primers 1 (SEQ ID NO: 31) and 6 (SEQ ID NO: 36) to yield PV1-24. For PV1-39, first round PCRs were conducted using primers 2 (SEQ ID NO: 32) and 19 (SEQ ID NO: 49) with Ves v 5 cDNA as template and primers 6 (SEQ ID NO: 36) and 20 (SEQ ID NO: 50) with Pol a 5 cDNA as template. The two overlapping PCR products were then purified and used as template in a third PCR using flanking primers 2 (SEQ ID NO: 32) and 6 (SEQ ID NO: 36) to yield PV1-39. For PV1-50, first round PCRs were conducted using primers 2 (SEQ ID NO: 32) and 28 (SEQ ID NO: 58) with Ves v 5 cDNA as template and primers 6 (SEQ ID NO: 36) and 27 (SEQ ID NO: 57) with Pol a 5 cDNA as template. The two overlapping PCR products were then purified and used as template in a third PCR using flanking primers 2 (SEQ ID NO: 32) and 6 (SEQ ID NO: 36) to yield PV1-50. For PV1-57, first round PCRs were conducted using primers 2 (SEQ ID NO: 32) and 30 (SEQ ID NO: 60) with Ves v 5 cDNA as template and primers 6 (SEQ ID NO: 36) and 29 (SEQ ID NO: 59) with Pol a 5 cDNA as template. The two overlapping PCR products were then purified and used as template in a third PCR using flanking primers 2 (SEQ ID NO: 32) and 6 (SEQ ID NO: 36) to yield PV1-57. For PV1-76, first round PCRs were conducted using primers 2 (SEQ ID NO: 32) and 32 (SEQ ID NO: 62) with Ves v 5 cDNA as template and primers 6 (SEQ ID NO: 36) and 31 (SEQ ID NO: 61) with Pol a 5 cDNA as template. The two overlapping PCR products were then purified and used as template in a third PCR using flanking primers 2 (SEQ ID NO: 32) and 6 (SEQ ID NO: 36) to yield PV1-76. Hybrid cDNAs were cloned into pPICZαA.
*(vi) PV22-32, PV115-125, PV142-150 and PV176-182.* These constructs are hybrid Ag 5s wherein short Ves v 5 polypeptides replace homologous sequences in otherwise intact full length Pol a 5.

The Pol a 5 sequences were substituted with Ves v 5 sequences using the PCR overlap extension method given in Example 1 (Ho et al., 1989, Gene 77:51). The template DNA used for the first set of two PCRs was the Pol a cDNA of Lu et al. (1993, J. Immunol. 150:2823). The upstream and downstream Pol a primers used in the PCR extension protocols were primers 4 (SEQ ID NO: 22) and 6 (SEQ ID NO: 24), respectively. Final products were cloned into pPICZαA.

The overlapping primer pairs encoding the inserted Ves v 5 sequences were as follows: *(a)* PV22-32 primers 17 (SEQ ID NO: 47) and 18 (SEQ ID NO: 48) (b) PV115-125 primers 21 (SEQ ID NO: 51) and 22 (SEQ ID NO: 52) (c) PV142-150 primers 23 (SEQ ID NO: 53) and 24 (SEQ ID NO: 54) and *(d)* PV 176-182 primers 25 (SEQ ID NO: 55) and 26 (SEQ ID NO: 56). PCR reaction and cycling conditions were those described for PV1-46.

**Table 1. Primers for preparation of Ves v and Pol a 5s and their hybrids.**

| Primer | Sequence (5' to 3') |
|---|---|
| 1 | CGTGAATTCAACAATTATTGTAAAATAAAA (SEQ ID NO:31) |
| 2 | CGTCTCGAGAAAAGAAACAATTATTGTAAAATAAAA (SEQ ID NO: 32) |
| 3 | CGTTCTAGATTACTTTGTTTGATAAAGTTC (SEQ ID NO: 33) |
| 4 | CGTGAATTCGTTGATTATTGTAAAATAAAA (SEQ ID NO: 34) |
| 5 | CGTCTCGAGAAAAGAGTTGATTATTGTAAAATAAAA (SEQ ID NO: 35) |
| 6 | CGTTCTAGATTATTTTTTTGTATAAGGTAG (SEQ ID NO: 36) |
| 7 | GTAAGCGAGCACAATCGGTTT (SEQ ID NO: 37) |
| 8 | AAACCGATTGTGCTCGCTTAC (SEQ ID NO: 38) |
| 9 | GTAGCAAAATTTCAGGTTGGA (SEQ ID NO: 39) |
| 10 | TCCAACCTGAAATTTTGCTAC (SEQ ID NO: 40) |
| 11 | ACCGCAAAATTTCCAGTTGGA (SEQ ID NO: 41) |
| 12 | TCCAACTGGAAATTTTGCGGT (SEQ ID NO: 42) |
| | |
| | |
| 15 | GGCACAATTCTTGCTCGGTTTAAGACTTCCATA (SEQ ID NO: 45) |
| 16 | TATGGAAGTCTTAAACCGAGCAAGAATTGTGCC (SEQ ID NO: 46) |
| 17 | CTTAAACCGAATTGCGGTAATAAGGTAGTGGTATCGGTTGGTCCA (SEQ ID NO: 47) |
| 18 | TGGACCAACCGATACCACTACCTTATTACCGCAATTCGGTTTAAG (SEQ ID NO: 48) |
| 19 | TATGGTCTAACGAAACAAGAGAAAAAATTAATCGTA (SEC ID NO: 49) |
| 20 | TACGATTAATTTTTTCTCTTGTTTCGTTAGACCATA (SEC ID NO: 50) |
| 21 | TTAACAGGTAGCACGGCTGCTAAATACGATGATGTAGTCAGTCTA (SEQ ID NO: 51) |
| 22 | ATCATCGTATTTAGCAGCCGTGCTACCTGTTAACGCTATATTTTG (SEQ ID NO: 52) |
| 23 | CCTAAGAAAAAGTTTTCGGGAAACGACTTTGCTAAAATTGGC (SEQ ID NO: 53) |
| 24 | GTCGTTTCCCGAAAACTTTTTCTTAGGATTAAAATCTTTCAC (SEQ ID NO: 54) |
| 25 | ATTCAAGAGAAATGGCACAAACATTACCTCATA (SEQ ID NO: 55) |
| 26 | TTTGTGCCATTTCTCTTGAATATATTTTAGAGA (SEQ ID NO: 56) |
| 27 | GAGCACAATGACTTTAGACAAAAA (SEQ ID NO: 57) |
| 28 | TTTTTGTCTAAAGTCATTGTGCTC (SEQ ID NO: 58) |
| 29 | AAAATTGCACGAGGGTTGGAAACA (SEQ ID NO: 59) |
| 30 | TGTTTCCAACCCTCGTGCAATTTT (SEQ ID NO: 60) |
| 31 | AATATGAAAAATTTGGTATGGAAC (SEQ ID NO: 61) |
| 32 | GTTCCATACCAAATTTTTCATATT (SEQ ID NO: 62) |

Ag5- or hybrid-encoding cDNAs of the EA- or KR-series were digested, respectively, with restriction enzymes Eco RI or Xho I, and Xba I, then inserted into similarly cut pPICZα-A vector (Invitrogen, San Diego, CA). The recombinant plasmids were amplified in TOP10F' cells. The Ag 5-coding sequences of all recombinant plasmids were confirmed by DNA sequencing. The Ag 5 coding-sequences corresponded to the sequence data in Genbank (Accession number M98858 for Ves v Ag 5 and accession number M98857 for Pol a Ag 5), with the exceptions of two single-nucleotide differences observed for Ves v 5. These changes were at positions 579 and 587 and resulted, respectively, in a silent G to A mutation and a T to A substitution that resulted in a codon change of M to K at amino acid residue 196. The two nucleotide changes may represent insect polymorphism, rather than random mutations since the Ag 5 cDNAs used were prepared in the same manner as it was done previously (Lu et al., 1993, J. Immunol. 150:2823).

### Example 2 Expression and purification of Ag 5s and hybrids

Recombinant plasmids (1-2 µg) were linearized by cutting with the restriction enzyme Sac I then used to transform competent Pichia pastoris KM71 yeast cells (about 8 x 10⁹ cells in 40 µl of 1M sorbitol) by electroporation. Transformed cells were diluted to 2 ml with 1M sorbitol and allowed to recover at 30° for 1 hr without shaking and for an additional hour with shaking at 200 rpm. Aliquots of 50 µl or 100 µl aliquots were then spread on 100 mm plates of YPDS medium containing 1.5 mg/ml Zeocin for selection of multi-copy integrants (Invitrogen Manual). Selected clones were picked after 3-4 day incubation and screened by small scale expression to identify colonies producing hybrid protein. Small scale expression was carried out in 50 ml plastic tubes in the same manner as described below for large scale isolation but at 1/30 scale and the culture fluids were screened by SDS gel electrophoresis for secreted proteins.

Yeast cells from selected clones were grown in two 500 ml bottles, each containing 150 ml of pH 6.0 phosphate buffer containing yeast nitrogen base, biotin, glycerol and histidine at 30° with orbital shaking at 250 rpm to an A₆₀₀ₙₘ of 10-12. Cells were then collected by centrifugation and resuspended in 100 ml of similarly buffered medium containing methanol in place of glycerol. Incubation was continued at 30° with shaking at 250 rpm for 4-6 days with daily addition of 1 ml of 50% methanol.

Ag 5s or their hybrids were purified from the culture fluid concentrate by ionexchange chromatography on SE-cellulose (Sigma) using a previously reported procedure (Monsalve *et al.,* 1999, Protein Expr. Purif. 16:410). About 70% of the main peak was pooled, desalted by reversed phase chromatography on C18 silica and lyophilized. Recombinant Ag 5s or hybrids were dissolved in 0.01 M ammonium acetate buffer (pH 4.6) and stored at 4°. Recombinant protein concentrations were determined from absorbance at 280 nm, using molar extinctions calculated from tyrosine and tryptophan contents. The yields of Ag 5s or hybrids typically ranged from 1 to 7 mg per 100 ml of 4-day cultures.

Recombinant Ag 5s or hybrids were characterized by SDS gel electrophoresis, N-terminal sequence analysis and MALDI mass spectrometry. CD spectra at 0.2 mg/ml of recombinant proteins in 0.01 M acetate buffer ofpH 4.6 were taken in cells of 1 mm path length in an AVIV 62DS spectrometer.

### Example 3 Physico-chemical characterization of recombinant vespid Ag 5s and hybrids

The Ag5s and hybrid proteins expressed in yeast strain KM71contained a secretory signal peptide. The signal peptide was linked to the expressed protein via a peptide of KR or KREAEAEF sequence. These two types of proteins were designated as the KR- and EA-series, respectively. Upon secretion from the yeast cells, the signal peptide was cleaved from the secreted protein at the KR sequence (Kex 2 protease site) or the two EA sequences (Ste 13 dipeptidyl amino peptidase sites) (Invitrogen Manual).

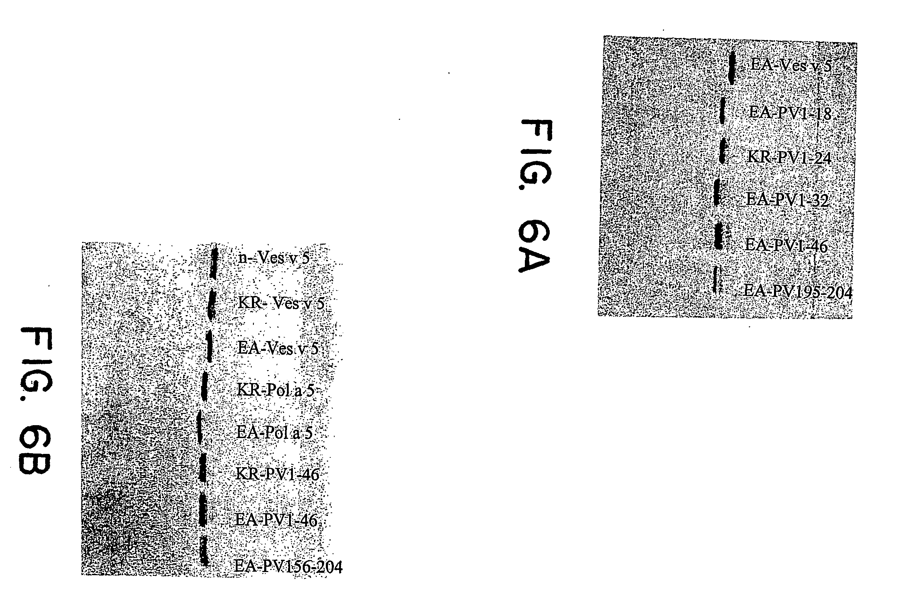

Recombinant proteins were isolated from culture fluid by ion exchange chromatography on SE-cellulose followed by reversed phase chromatography on C 18-silica and characterized by SDS gel electrophoresis. (Fig. 6). Several hybrids showed a closely-spaced doublet with mobilities similar to that of natural Ves v 5. The doublets are consistent with the varying extents of processing at their N-terminal ends, as indicated by N-terminal sequencing of hybrids PV1-155 and PV156-204 and mass spectrometry data (Table 2).

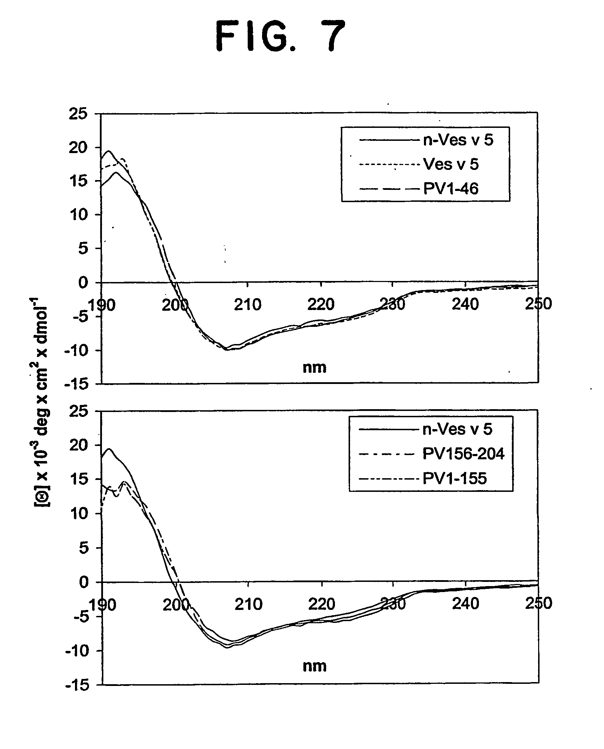

Recombinant Ag 5s and hybrids showed nearly identical CD spectra as those of the natural Ag 5s (Fig. 7). The spectra of the natural Ves v 5 and the EA-Ves v 5, and those of EA-PV1-46, EA-PV1-155 and EA-PV156-204 showed the presence of minima at about 208 nm with a shoulder at 225 nm (Fig. 7). These features are indicative of an ordered feature (Yang et al., 1986, Methods in Enzymology 130:208). Similar CD spectra were observed for the other hybrids listed in Table II (data are not shown). The CD spectrum of recombinant Ves v 5 from bacteria showed a minima at about 200 nm, which is indicative of a disordered structure (Monsalve *et al.,* 1999, Protein Expr. Purif. 16:410).

The recombinant Ag 5s and hybrids from yeast were freely soluble in acid or basic buffers, as were the natural Ag 5s. This is in contrast to recombinant vespid Ag 5s from bacteria, which were freely soluble only in acidic buffer.

Results of mass spectrometric analysis of Ag 5s and hybrids are given in Table 2. EA-series Ag 5s were cleaved efficiently at the Kex 2 site but showed variable cleavages at the two Ste 13 sites. Recombinant EA-scrics proteins, therefore, had amino-terminal sequences of EAEAEF and EAEF, where the EF sequence was encoded by the Eco R I site used to insert cDNA into the vector. These data were similar to results reported previously (Monsalve et al., 1999, Protein Expr. Purif. 16:410).

The EAEAEF sequence of recombinant Ves v 5 is known to function as a strong hapten (Monsalve et al., 1999, Protein Expr. Purif. 16:410). Therefore, Ag 5s were also expressed as KR-series hybrids. Cleavage of KR-series proteins at the Kex 2 site yielded recombinant proteins with the N-terminal sequence of the natural proteins. Mass spectrometry analysis of the KR-series proteins Ves v5, Pol a 5, and hybrids KR-PV1-24 and KR-PV1-46 showed that they were cleaved, with varied efficiencies, at the Kex2 site, and at residues 2, 7, and 9 upstream of the Kex2 site. (Table 2.) The recombinant proteins of the KR-series were usually of slightly lower yields than those of the EA-series.

**Table 2. Mass spectrometric data of recombinant vespid Ag 5s and hybrids.**

| | | | Mass units | |
|---|---|---|---|---|
| Protein | Assumed sequence | Abundance¹ | calc'd | found |
| EA-Ves v 5 | EAEAEF-Vv | 80% | 23,954 | 23,947 |
| | EAEF-Vv | 20% | 23,754 | 23,752 |
| | | | | |
| EA-Pol a 5 | EAEAEF-Pa | 100% | 23,611 | 23,613 |
| | | | | |
| EA-PV1-18 | EAEF-PV | 43% | 23,497 | 23,506 |
| | EAEAEF-PV | 36% | 23,697 | 23,698 |
| | REAEAEF-PV | 21% | 23,871 | 23,827 |
| | | | | |
| EA-PV1-18 | EAEAEF-PV | 100% | 23,697 | 23,701 |
| | | | | |
| EA-PV1-32 | EF-PV | 60% | 22,964 | 22,930 |
| | EAEF-PV | 40% | 23,151 | 23,134 |
| | | | | |
| EA-PV1-46 | EAEF-PV | 53% | 23,300 | 23,327 |
| | EAEAEF-PV | 47% | 23,500 | 23,515 |
| | | | | |
| EA-PV1-46 | EF-PV | 10% | 23,099 | 23,109 |
| | EAEF-PV | 50% | 23,300 | 23,327 |
| | EAEAEF-PV | 40% | 23,500 | 23,515 |
| | | | | |
| EA-PV1-155 | EF-PV | 53% | 23,375 | 23,334 |
| | EAEF-PV | 47% | 23,575 | 23,533 |
| | | | | |
| EA-PV22-32 | EAEF-PV | 55% | 23,135 | 23,203 |
| | EAEAEF-PV | 45% | 23,336 | 23,371 |
| | | | | |
| EA-PV115-125 | EAEAEF-PV | 100% | 23,873 | 23,887 |
| | | | | |
| EA-PV142-150 | EAEAEF-PV | 100% | 23,592 | 23,585 |
| | | | | |
| EA-PV156-204 | EAEF-PV | 59% | 23,776 | 23,775 |
| | EAEAEF-PV | 41% | 23,932 | 23,939 |
| | | | | |
| EA-PV195-204 | EAEAEF-PV | 70% | 23,700 | 23,688 |
| | REAEAEF-PV | 30% | 23,874 | 23,844 |
| | | | | |
| KR-Ves v 5 | Vv5 | 90% | 23,277 | 23,274 |
| | EEGVSLEKR-Vv | 10% | 24,305 | 24,298 |
| | | | | |
| KR-Ves v 5 | Vv | 95% | 23,277 | 23,284 |
| | EEGVSLEKR-Vv | 5% | 24,305 | 24,300 |
| | | | | |
| KR-Pol a 5 | Pa | 20% | 22,934 | 22,951 |
| | EEGVSLEKR-Pa | 80% | 23,962 | 23,992 |
| | | | | |
| KR-Pol a 5 | Pa | 10% | 22,934 | 22,935 |
| | EEGVSLEKR-Pa | 90% | 23,962 | 23,962 |
| | | | | |
| KR-PV1-24 | PV | 85% | 22,903 | 22,897 |
| | EEGVSLEKR-PV | 15% | 23,931 | 23,933 |
| | | | | |
| KR-PV1-46 | PV | 70% | 22,823 | 22,834 |
| | KR-PV | 30% | 23,107 | 23,157 |
| | | | | |
| KR-PV1-46 | PV | 60% | 22,823 | 22,834 |
| | KR-PV | 40% | 23,107 | 23,157 |

| | | | | |
|---|---|---|---|---|
| ¹Protein abundance was estimated from peak heights of samples in mass spectra. N-terminal sequences For of EA-Ves v 5, EA-Pol a 5, EA-PV3PV156-204 and EA- VP3PV1-155 samples of EA series, their assumed sequences were confirmed by Edman degradation. Results of two preparations are shown for each of EA-PV1-18, EA-PV1-46, KR-Ves v 5, KR-Pol a and KR-PV1-46. Amino terminal peptides have been assigned SEQ ID NO: as follows; EAEAEF [SEQ ID NO: 89]; EAEF [SEQ ID NO: 90]; REAEAEF [SEQ ID NO: 91] and EEGVSLEKR [SEQ ID NO: 92]. | | | | |

### Example 4 ELISA studies

ELISA was performed in 96-well plates in the wells coated with 4 µg/ml Ag 5 in 0.05 M Tris-HCI buffer of pH 8. Bound IgG₁ was detected with 2 µg/ml biotinylated goat anti-mouse IgG (γl specific) followed with 2 µg/ml avidin-peroxidase conjugate (King et al., 1995, J. Immunol 154:577). Antibody concentrations of sera samples were determined by comparison of their ELISA data with that of an immuno-affinity purified sample of Ves v 5-specific antibody.

### Example 5 Ves v 5-specific B cell epitopes of hybrids

Murine polyclonal antibodies specific for natural Ves v 5 were isolated from BALB/c sera by affinity chromatography on Ves v 5-specific immunosorbent and were depleted of Pol a 5-cross-reacting antibodies by passage through Pol a 5-specific immunosorbent. The immunosorbents were prepared with CNBr activated Sepharose 2B (Pharmacia). Murine monoclonal antibodies specific for Ves v 5 were obtained as described (King et al., 1987, Mol. Immunol 24:857).

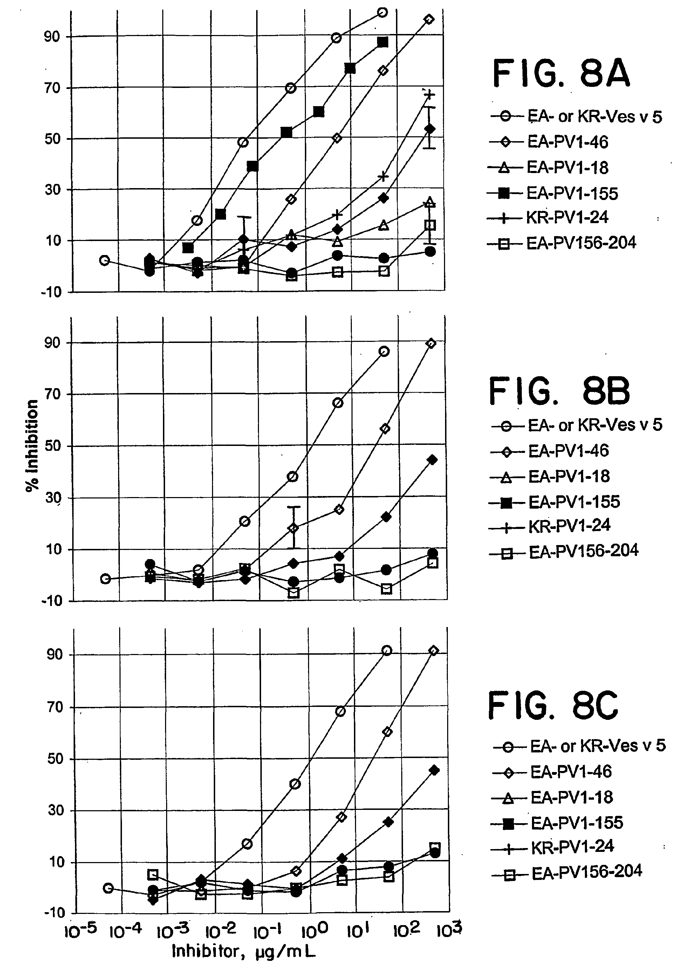

Ves v 5-specific B cell epitopes were detected by hybrid-inhibition of binding of mouse Ves v 5-specific antibodies to solid-phase Ves v 5. Both EA- and KR-Ves v 5 were tested as solid phase antigen with similar results. Five samples of mouse antisera were tested; three were from BALB/c strains and one each from ASW/sn and P/J strains. Results using one BALB/c serum sample are shown in Fig. 8A. At the highest concentration of 50 or 500µg/ml inhibitor tested, the two N-terminal hybrids EA-PV1-46 and Era-1-155 showed maximal inhibition approaching 100%, as did EA- or KR-Ves v 5. Two other N-terminal hybrids KR-PV1-24 and EA-PV1-32 had maximal inhibition of about 60% and the shortest N-terminal hybrid, EA-PV1-18, had maximal inhibition of about 20%. The C-terminal hybrid EA-PV 156-204 had maximal inhibition of about 15%. Similar results were obtained for results of inhibition ELISA using antisera from ASW/sn (Fig. 8B) and P/J (Fig. 8C) mice.

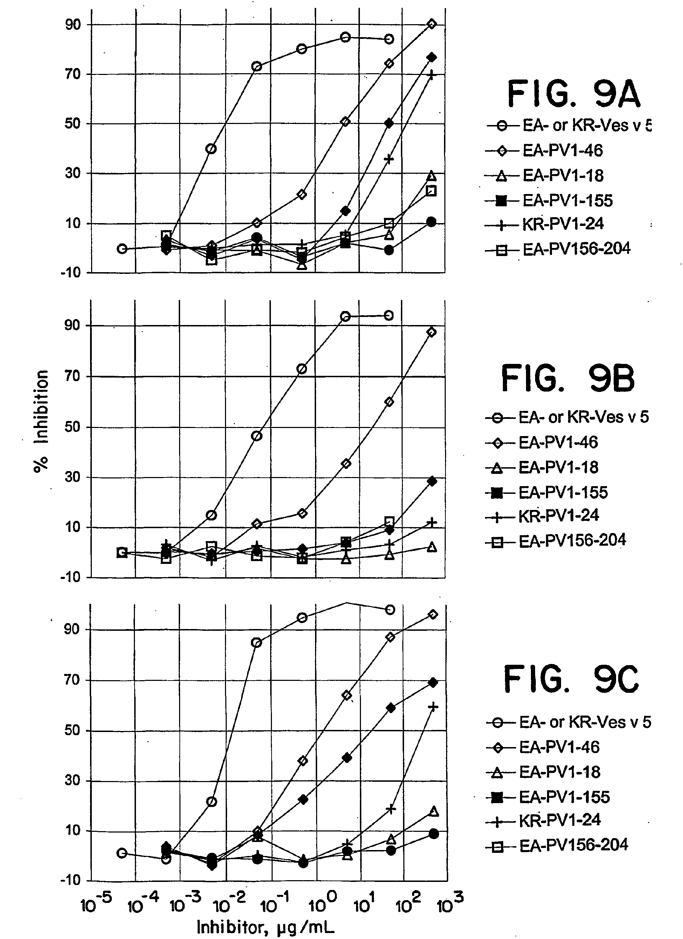

Ves v 5-specific B cell epitopes were also detected by inhibition analyses with sera from six yellow jacket sensitive patients. The data from three patients are shown in Fig.9A-C. The results were similar to those obtained with mouse IgGs.

The results of the ELISA inhibition studies using both mouse and human antisera indicated the immunodominance of the N-terminal region of Ves v 5.

The observed inhibition by the hybrids was not due to cross-reacting epitopes of the Pol a 5 portion of the molecule as the sample of Ves v 5-specific antibodies used for inhibition studies in BALB/c mice was depleted of Pol a 5-cross-reactive antibodies and no inhibition by Pol a 5 was detected (Fig. 8A). The high concentrations of hybrids required for half maximal inhibition relative to that of Ves v 5 did not reflect that the epitopes of the hybrids lacked the native structure of Ves v 5 as the recombinant Ves v 5 from bacteria that lacked the native structure did not show any inhibition (data not shown).

The difference in the inhibitory activities of Ves v 5 and hybrids was probably related to their epitope densities. Epitopc density is known to influence strongly the affinity constant of a multivalent antigen and a bivalent antibody (Hornick and Karush, 1972, Immunochemistry 9:325; Crothers and Metzger, 1972, Immunochemistry 9:341).

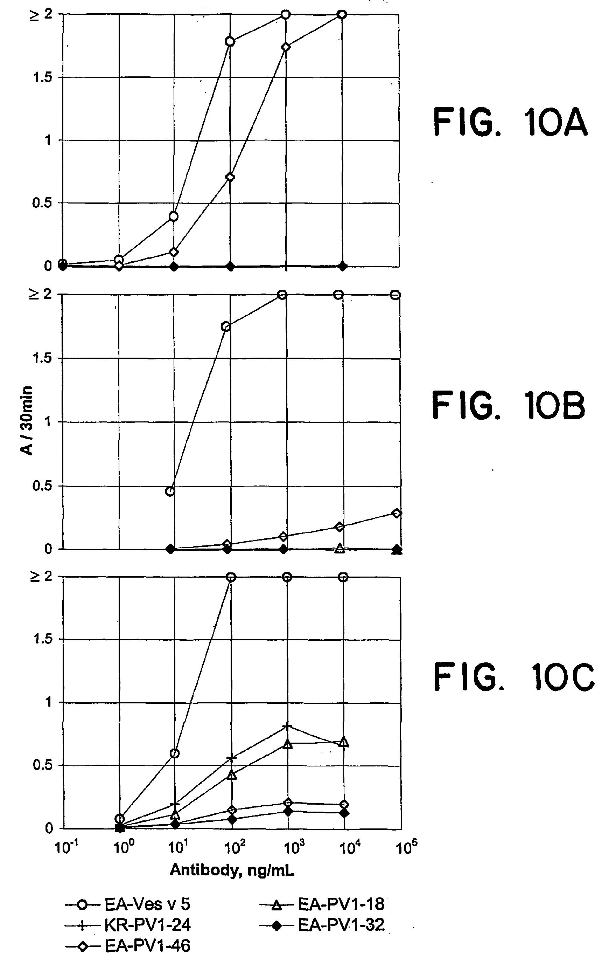

The data in Figs. 8 and 9 suggested that the amino terminal portion of Ves v 5 includes the immunodominant B cell epitopes of Ves v 5. This finding was confirmed by tests with a panel of 17 monoclonal antibodies specific for Ves v 5 (King et al., 1987, Mol. Immunol 24:857). These monoclonal antibodies were specific for the natural Ves v 5 and recombinant proteins from yeast, but they did not bind the denatured form of recombinant Ves v 5 from bacteria (data not shown). ELISA results showed that one monoclonal antibody bound EA-Ves v 5 and EA-PV 1-46 with similar affinity and maximal binding and it did not bind any of the other N- or C-terminal hybrids (Fig. 10A). Four other monoclonal antibodies showed greatly reduced maximal binding to EA-PV 1-46 but no binding to any of the shorter N-terminal hybrids; the data for one such antibody are given in Fig. 10B. Lastly, one monoclonal antibody showed greatly reduced binding to EA-PV1-32 and EA-PV 1-46 and moderate binding to EA-PV 1-18 and EA-PV 1-24 (Fig. 10C). These data show that six of the 17 monoclonal antibodies tested were specific for the N-terminal region of Ves v 5.

### Example 6 Immune responses to hybrids

Groups of 3 or 4 female BALB/c mice were given biweekly intraperitoneal injections of 2 µg immunogen and 1 µg alum in 0.2 ml of phosphate buffered saline. Ag 5 or hybrid specific sera were collected at week 5 or later. Similar antibody levels were observed for sera collected at weeks 5, 7, and 9.

Mice immunized with hybrids produced antibodies specific for the hybrid, Pol a 5 and Ves v 5. The antibody levels of sera samples were measured before and after absorption with Pol a 5 to determine their specificity for Ves v 5. These data are summarized in Table 3A. Mice immunized with natural, EA- or KR-Ves v 5 gave nearly the same antibody responses, and only those of the KR-Vcs v 5 arc given Table 3A. EA-PV1-46 gave a higher antibody response in set A mice than KR-PV1-46 did in set B mice. This difference may be due to the different sets of mice used. EA-PV1-18 was used in both sets of experiments, and it gave higher antibody response in set A mice than that in set B mice.

Comparison of antibody levels in the N-terminal hybrid-specific sera samples in Table 3, before and after Pol a 5 absorption, indicated that 30-80% of the antibodies were specific for Ves v 5 when tested on solid-phase Ves v 5, and these values were less when tested on solid-phase hybrid. The higher contents of Ves v 5-specific antibodies detected on solid-phase Ves v 5 than those on solid-phase hybrid suggest that the majority of hybrid-specific antibodies recognize overlapping regions of Ves v 5 and Pol a 5 in the hybrid. The data in set A of Table 3A indicated that of the three N-terminal hybrids, PV1-155 was as immunogenic as Ves v 5 was, PV1-46 was half as imunogenic as Ves v 5 and PV1-18 was about 1/9th as immunogenic as Ves v 5. The data in set B indicate that PV 1-46 and 1-32 were more immunogenic than PV1-24 and 1-18. The data from both sets suggest that the longer N-terminal hybrids PV 1-46 and 1-32 stimulate higher contents of Ves v 5-specific antibodies and lower contents of Pol a 5- specific antibodies than the two shorter hybrids PV1-24 and 1-18 did.

**Table 3A**

| **Murine antibody responses to vespid antigen 5s and hybrids** | | | | |
|---|---|---|---|---|
| **SET** | Immunogen¹ | **mg/ml specific IgG in sera** **by ELISA on solid-phase^{2,3}** | | |
| | | **EA-Ves v** 5 | **EA-Pol a 5** | **Hybrid** |
| **A** | KR-Ves v 5 | 8.9 (8.5) | 0.6 | - |
| | KR-Pol a 5 | 2.8(1.0) | 7.0 | - |
| | EA-PV1-155 | 12.0 | 0.7 | - |
| | EA-PV1-46 | 4.2 (3.5) | 1.9 | 7.6 (5.6) |
| | EA-PV1-18 | 1.0 (0.8) | 6.9 | 6.9 (0.7) |
| | EA-PV156-204 | 1.6 (0.6) | 10.0 | 2.6 (0.3) |
| | EA-PV195-204 | 1.3 (0.4) | 14.0 | 10.0 (0.3) |
| | | | | |
| **B** | KR-Ves v 5 | 15.0 (14.0) | 0.2 | - |
| | KR-PV1-46 | 0.6 (0.5) | 1.0 | 2.7 (3.0) |
| | EA-PV1-32 | 0.9 (0.7) | 4.3 | 8.0 (3.2) |
| | KR-PV1-24 | 0.4 (0.3) | 4.2 | 6.5 (0.9) |
| | EA-PV1-18 | 0.4 (0.3) | 4.5 | 5.3 (0.7) |

| | | | | |
|---|---|---|---|---|
| 1. Sera were collected on week 7, after 3 biweekly ip injections of immunogen. Sets A and B studies were made at separate occasions. 2. Antibody concentration was estimated from reciprocal sera concentration required to give an absorbance change of 1.0 in 30 minutes. Under the conditions used, this change corresponded to a 0.1µg/ml solution of purified Ves v 5 - specific antibody. The estimated antibody concentrations varied by about 40 % on repeat measurements. 3.Values in parenthesis were obtained after absorption of ¹/₅₀₀ diluted sera with 0.2 mg/ml EA-Pol a 5. | | | | |

The results shown in Table 3A indicate the B cell epitope of Ves v 5 is in its N-terminal region. Additional hybrids of Ves v 5 and Pol a 5 were prepared and tested for immunogenicity in mice as described above, to delineate the N and the C-terminal limits of the dominant B cell epitope region. Results are given in Table 3B, which lists the IgG1 content specific for Ves v, Pol a or hybrid, and percent of specific IgG1 remaining after absorption with Pa.

Hybrid PV1-8 with the lowest Ves v content did not induce Ves v-specific antibody response. All other hybrids induced 0.4-4.5 mg/ml of Ves v-specific Ab with the exception of PV22-32. Hybrids with Ves v contents <PV1-32 are moderately specific for Ves v response, as 34-81% of their Ves v-specific antbody and 15-27% of their hybrid-specific antibodies were not absorbed by Pol a 5. Hybrids with Ves v contents >PV1-39 are more specific, as 66-96% of their Ves v 5-specific antibody and 91-100% of their hybrid-specific antibody were not absorbed by Pol a 5. These results together suggest the C-terminal limit of the dominant epitope region is between residues 32-39.

Hybrids with Ves v contents of <PV1-32 show 2-4 mg/ml of Pol a-specific antibody, and hybrids with Ves v contents of >PV1-39 showed 0.04-1.34 mg/ml of Pol a-specific antibody. As the Ves v content of hybrids was increased from PV1-32 to 1-76, there was a progressive decrease of Pol a-specific response. These results together suggest the C-terminal limit of the dominant epitope extends beyond residues 39, as suggested by considerations of the Ves v-specific response to hybrids.

The lack of Ves v-specific antibody response of PV1-8 and 22-32 as compared to the response of PV1-32 suggests the N-terminal limit of the dominant epitope region to be within residues 9-21.

**Table 3B. Murine antibody responses to vespid antigen 5s and hybrids**

| **Construct** | **Groups of mice** | **Ves v 5 specific** **IgG1; % Ves v** | **Pol a 5 specific IgG1** | **Hybrid specific IgG1 ; % Ves v** |
|---|---|---|---|---|
| Pol a 5 | 1 | 1.80 mg/ml; 64% | 4.50 mg/ml | |
| Ves v 5 | 4 | 10.7 ∀ 3.2 mg/ml | 0.2 ∀ 0.1 mg/ml | |
| | | 104 ∀15% | | |
| PV1-8 | 1 | 0 | 8.2 mg/ml | |
| PV1-18 | 4 | 0.6 ∀0.44 mg/ml; | 4.1 ∀2.0 mg/ml | 7.5 ∀4.5 mg/ml; |
| | | 68 ∀14% | | 27 ∀26% |
| PV1-24 | 2 | 0.35 ∀0.06 mg/ml; | 2.26 ∀0.50 mg/ml | 5.86 ∀1.30 mg/ml; |
| | | 81 ∀17% | | 20 ∀12% |
| PV1-32 | 3 | 0.52 ∀0.39 mg/ml; | 3.77 ∀1.89 mg/ml | 6.82 ∀3.46 mg/ml; |
| | | 34 ∀25% | | 15 ∀6% |
| PV1-39 | 2 | 4.45 ∀0.70 mg/ml; | 1.72 ∀0.06 mg/ml | 8.25 ∀1/87 mg/ml; |
| | | 89 ∀27% | | 76 ∀23% |
| PV1-46 | 3 | 2.29 ∀3.41 mg/ml; | 1.18 ∀0.96 mg/ml | 7.57 ∀7.33 mg/ml; |
| | | 86 ∀14% | | 91 ∀9% |
| PV1-50 | 1 | 1.01 mg/ml; 94% | 0.44 mg/ml | 11.22 mg/ml; 90% |
| PV1-57 | 1 | 0.67 mg/ml; 96% | 0.22 mg/ml | 11.88 mg/ml; 85% |
| PV1-76 | 1 | 1.32 mg/ml; 92% | 0.04 mg/ml | 11.88 mg/ml; 92% |
| PV22-32 | 1 | 0.04 mg/ml; 0% | 4.88 mg/ml | 6.31 mg/ml; 6% |

| | | | | |
|---|---|---|---|---|
| Data are from averages of week 7 bleedings from 1-4 groups of 4 mice. % Vcs v refers to antibody content after absorption with Pol a 5 | | | | |

### Example 7 T cell response

Proliferation assays were performed with spleen cells from mice immunized with vespid antigen 5 or hybrid to study the specificity of T cell responses. Assays were performed in triplicate with spleen cells pooled from 2 to 3 mice, 10 days after 5 biweekly immunizations. Spleen cells (4 x 10⁵) were cultured with test antigen in 0.2 ml of culture medium at 37° and 5% CO₂. Tritiated thymidine (1 µCi) was added on day 3, and the thymidine uptake was determined on day 4. The results were expressed as stimulation index values.

Results showed that the hybrids EA-PV1-46, EA-PV1-155 and EA-PV156-204 induced hybrid-specific as well as vespid antigen 5-specific T cell responses (Table 4). The data indicated that the best proliferative responses were obtained when the stimulating antigen was the immunogen. This is apparent from comparing the maximal stimulation index values at the highest antigen concentration of 100 µg/ml tested, and from comparing the lowest antigen concentration required for a stimulation index value of 4.

**Table 4**

| **Vespid antigen 5 or hybrid stimulated proliferation of murine spleen cells** | | | |
|---|---|---|---|
| Spleen cells specific for | Stimulating Ag | | |
| | EA-Ves v5 | EA-Pol a 5 | EA-hybrid |
| | Stimulation Index at 100 µg/ml Ag | | |
| KR-V es v 5 | 8.2 | 1.5 | - |
| KR-Pol a 5 | 2.2 | 6.3 | - |
| EA-PV1-155 | 6.1 | 2.2 | 5.0 |
| EA-PV1-46 | 6.0 | 8.0 | 13.5 |
| EA-PV1-18 | 2.3 | 5.0 | 6.1 |
| EA-PV156-204 | 4.1 | 4.2 | 6.8 |
| EA-PV195-204 | 1.7 | 8.6 | 4.1 |

| | µg/ml Ag for stimulation index of 4 | | |
|---|---|---|---|
| KR-V es v 5 | 2.6 | >100 | - |
| KR-Pol a 5 | >100 | 16 | - |
| EA-PV1-155 | 11 | >100 | 0.54 |
| EA-PV1-46 | 20 | 2.2 | 0.26 |
| EA-PV1-18 | >100 | 47 | 19 |
| EA-PV156-204 | 60 | 70 | 2.3 |
| EA-PV195-204 | >100 | 8 | 82 |

| | | | |
|---|---|---|---|
| Background proliferation of spleen cells showed ³H-thymidine uptake of 400-900 cpm. | | | |

### Example 8 Allergenicity of recombinant vespid Ag 5s and hybrids in patients

Allergenicity was determined by histamine release assay from basophils of 10 yellow jacket sensitive patients, following challenge with Ag 5 or hybrids (Colombo *et al.,* 1995, J Allergy Clin. Imm. 95:565). The patients/results shown in Table 5 are divided into two groups. Group A patients (n=7) were about 1000 times more sensitive to Ves v 5 than to Pol a 5; Group B patients (n=3) were about equally sensitive to both antigen 5s.

**Table 5.**

| **Summary of histamine release data of hybrids** | | | | | | |
|---|---|---|---|---|---|---|
| | **Reciprocal Activity Relative to Ves v 5** | | | | | |
| | **Group A** | | | **Group B** | | |
| **Allergen** | **No. of patients** | **Mean** | **Range** | **No. of patients** | **Mean** | **Range** |
| Ves v 5 | 7 | 1 | 1 | 3 | 1 | 1 |
| Pol a 5 | 7 | 1154 | 330-5500 | 3 | 0.7 | 0.2-2 2 |
| PV1-155 | 3 | 1 | 1-2 | 2 | 1 | 1 |
| PV1-46 | 5 | 126 | 13-3300 | 2 | 0.7 | 0.1 - 5 |
| PV1-18 | 3 | 583 | 12-5000 | 2 | 24 | 3.0-200 |
| PV22-32 | 3 | 3207 | 2000-5000 | 2 | 6 | 6-20 |
| PV115-125 | 3 | 3207 | 2000-5000 | 2 | 5 | 2-15 |
| PV142-150 | 3 | 3000 | 2700-5000 | 2 | 5 | 2-15 |
| PV156-204 | 6 | 1139 | 1000-3000 | 3 | 3 | 0.4-70 |
| PV195-204 | 3 | 3207 | 50-5000 | 2 | 32 | 20.0-50 |

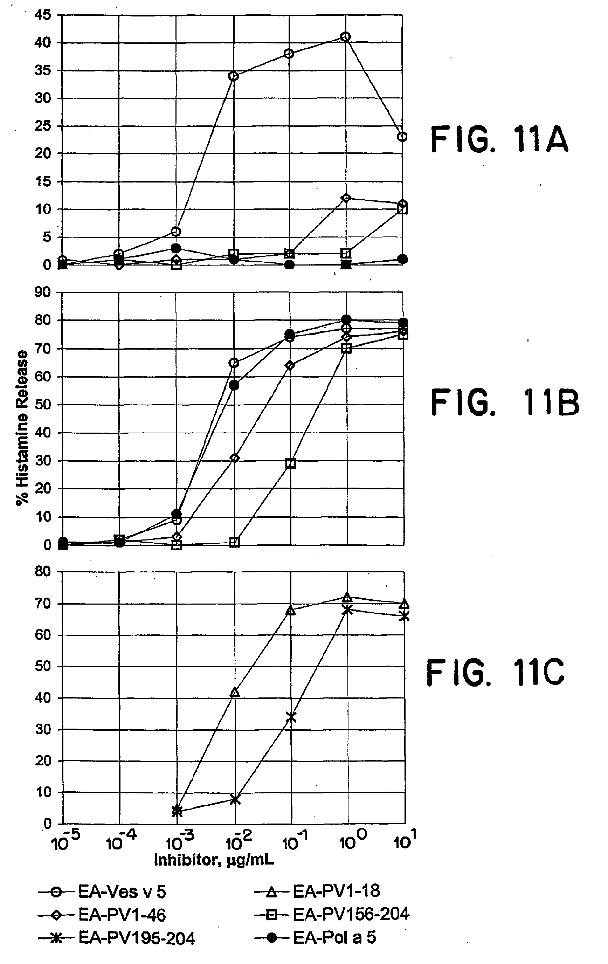

The complete data from one patient of each group are given in Figure 11.

Of the three N-terminal hybrids tested, EA-PV1-155 showed no decrease in allergnenicity. EA-PV1-46 and 1-18 showed geometric mean reductions of 126- and 583-fold respectively in group A patients, and 0.7- and 24-fold decreases respectively in group B patients. The two C-terminal hybrids EA-PV156-204 and 195-204 had reductions of 1139-and 3207-fold in group A patients respectively and 3- and 32-fold in group B patients respectively.

The different extents of reduction in allergenicity of the N- and C-terminal hybrids reflect both their IgE antibody concentration and their epitope density. The inhibition ELISA data in Figure 6 suggest a higher concentration of human IgG antibodies for the N-terminal region of Ves v 5 than those for the C-terminal region and this is likely also the case for IgE antibodies. Another contributing factor to the greater reduction in allergenicity of the C-terminal hybrid EA-PV 156-204 as compared to the N-terminal hybrid EA-PV1-46 is probably due to its decreased epitope density as the C-terminal hybrid has fewer surface accessible residues of Ves v 5 than the N-terminal hybrid does. Similarly, the greater reduction in allergenicity of the shorter N- or C-terminal hybrids, PV 1-18 or PV195-204, as compared to their respective longer ones also reflects the influence of epitope density.

The allergenicity of recombinant Ves v 5 from bacteria was compared with those of the natural Ves v and the recombinant Ves v 5 from yeast. In three patients tested, the recombinant protein from bacteria was about 103 times less potent than the natural protein or the recombinant protein from yeast (data not shown). These data confirm previous observations that the majority of B cell epitopes for allergens are dependent on the conformation of the native allergen (King *et al.,* 2000, Int Arch Allergy 123:99).

The decrease in allergenicity of the recombinant Ves v 5 from bacteria, was due to loss of the conformation dependent B cell epitopes as the CD spectrum of the recombinant protein from bacteria showed it to have a disordered structure. However, the decrease in allergenicity of the hybrid protein PV 1-46 or PV 156-204 was due to reduction of the number and density of Ves v 5-specific epitopes, as its CD spectrum indicated it had an ordered structure similar to that of Ves v 5. The reduction of the number and density epitopes of the hybrid PV1-46 and PV 156-204 is in agreement with the B cell epitope and immunogenicity data given in Examples 5-7.

### Example 9 Crystallization of recombinant Ves v 5

Crystals of Ves v 5 was grown by the vapor diffusion technique at 25°C. For crystallization, 5 µl of 5 mg/ml Ves v 5 was mixed with 5 µl of 18% PEG 6000, 0.1 M sodium citrate, pH 6.0 and equilibrated against 1 ml of 18% PEG 6000, 0.1 M sodium citrate, pH 6.0. X-ray diffraction data was collected at 100K from native Ves v 5 crystals and after incorporation of heavy-atom derivatives and used to solve the three-dimensional structure of Ves v 5. The atomic coordinates and structure factors of Ves v 5 have been deposited in the Protein Data Bank (PDB) with the accession number Q05110. The atomic coordinates of Ves v 5 are given in Table 6.

**Table 6. Ves v 5 crystal coordinates**

| REMARK FILENAME="brefinement.pdb" | | | | | | | | | | |
|---|---|---|---|---|---|---|---|---|---|---|
| REMARK r= 0.215955 free r= 0.298202 | | | | | | | | | | |
| REMARK DATE:28-Oct-98 | | | | 15:45:46 | created by user: anette | | | | | |
| ATOM | 1 | CB | GLU | 1 | 17.077 | 51.793 | 23.662 | 1.00 | 41.80 | APEP |
| ATOM | 2 | CG | GLU | 1 | 16.595 | 52.047 | 25.081 | 1.00 | 43.97 | APEP |
| ATOM | 3 | CD | GLU | 1 | 15.167 | 51.580 | 25.310 | 1.00 | 44.74 | APEP |
| ATOM | 4 | OE1 | GLU | 1 | 14.367 | 51.640 | 24.352 | 1.00 | 46.38 | APEP |
| ATOM | 5 | OE2 | GLU | 1 | 14.845 | 51.156 | 26.444 | 1.00 | 43.98 | APEP |
| ATOM | 6 | C | GLU | 1 | 19.169 | 50.429 | 23.664 | 1.00 | 39.72 | APEP |
| ATOM | 7 | O | GLU | 1 | 19.733 | 49.575 | 24.358 | 1.00 | 40.19 | APEP |
| ATOM | 8 | N | GLU | 1 | 17.005 | 49.431 | 24.404 | 1.00 | 41.50 | APEP |
| ATOM | 9 | CA | GLU | 1 | 17.655 | 50.391 | 23.458 | 1.00 | 40.85 | APEP |
| ATOM | 10 | N | ALA | 2 | 19.820 | 51.423 | 23.064 | 1.00 | 37.33 | APEP |
| ATOM | 11 | CA | ALA | 2 | 21.267 | 51.571 | 23.179 | 1.00 | 34.17 | APEP |
| ATOM | 12 | CB | ALA | 2 | 21.668 | 51.735 | 24.657 | 1.00 | 34.25 | APEP |
| ATOM | 13 | C | ALA | 2 | 21.935 | 50.341 | 22.585 | 1.00 | 32.32 | APEP |
| ATOM | 14 | O | ALA | 2 | 21.299 | 49.580 | 21.847 | 1.00 | 33.01 | APEP |
| ATOM | 15 | N | GLU | 3 | 23.215 | 50.148 | 22.899 | 1.00 | 29.81 | APEP |
| ATOM | 16 | CA | GLU | 3 | 23.956 | 48.991 | 22.402 | 1.00 | 26.33 | APEP |
| ATOM | 17 | CB | GLU | 3 | 24.948 | 49.413 | 21.325 | 1.00 | 30.89 | APEP |
| ATOM | 18 | CG | GLU | 3 | 25.246 | 48.320 | 20.303 | 1.00 | 35.96 | APEP |
| ATOM | 19 | CD | GLU | 3 | 24.029 | 47.468 | 19.973 | 1.00 | 38.25 | APEP |
| ATOM | 20 | OE1 | GLU | 3 | 23.428 | 47.678 | 18.891 | 1.00 | 39.27 | APEP |
| ATOM | 21 | OE2 | GLU | 3 | 23.681 | 46.586 | 20.793 | 1.00 | 37.45 | APEP |
| ATOM | 22 | C | GLU | 3 | 24.693 | 48.269 | 23.530 | 1.00 | 21.89 | APEP |
| ATOM | 23 | O | GLU | 3 | 25.780 | 48.679 | 23.959 | 1.00 | 20.16 | APEP |
| ATOM | 24 | N | ALA | 4 | 24.093 | 47.180 | 23.995 | 1.00 | 17.32 | APEP |
| ATOM | 25 | CA | ALA | 4 | 24.652 | 46.382 | 25.080 | 1.00 | 15.71 | APEP |
| ATOM | 26 | CB | ALA | 4 | 23.796 | 45.141 | 25.302 | 1.00 | 12.69 | APEP |
| ATOM | 27 | C | ALA | 4 | 26.103 | 45.970 | 24.862 | 1.00 | 14.17 | APEP |
| ATOM | 28 | O | ALA | 4 | 26.816 | 45.710 | 25.827 | 1.00 | 11.99 | APEP |
| ATOM | 29 | N | GLU | 5 | 26.542 | 45.908 | 23.603 | 1.00 | 12.66 | APEP |
| ATOM | 30 | CA | GLU | 5 | 27.917 | 45.503 | 23.319 | 1.00 | 13.51 | APEP |
| ATOM | 31 | CB | GLU | 5 | 28.222 | 45.583 | 21.817 | 1.00 | 15.08 | APEP |
| ATOM | 32 | CG | GLU | 5 | 29.647 | 45.127 | 21.479 | 1.00 | 20.99 | APEP |
| ATOM | 33 | CD | GLU | 5 | 30.068 | 45.447 | 20.049 | 1.00 | 22.60 | APEP |
| ATOM | 34 | OE1 | GLU | 5 | 29.224 | 45.948 | 19.278 | 1.00 | 29.69 | APEP |
| ATOM | 35 | OE2 | GLU | 5 | 31.245 | 45.199 | 19.699 | 1.00 | 23.87 | APEP |
| ATOM | 36 | C | GLU | 5 | 28.949 | 46.339 | 24.065 | 1.00 | 12.46 | APEP |
| ATOM | 37 | O | GLU | 5 | 30.025 | 45.847 | 24.394 | 1.00 | 12.28 | APEP |
| ATOM | 38 | N | PHE | 6 | 28.616 | 47.596 | 24.343 | 1.00 | 11.87 | APEP |
| ATOM | 39 | CA | PHE | 6 | 29.546 | 48.491 | 25.022 | 1.00 | 11.93 | APEP |
| ATOM | 40 | CB | PHE | 6 | 29.459 | 49.879 | 24.377 | 1.00 | 12.32 | APEP |
| ATOM | 41 | CG | PHE | 6 | 29.706 | 49.857 | 22.887 | 1.00 | 14.45 | APEP |
| ATOM | 42 | CD1 | PHE | 6 | 28.646 | 49.803 | 21.997 | 1.00 | 14.86 | APEP |
| ATOM | 43 | CD2 | PHE | 6 | 31.001 | 49.811 | 22.381 | 1.00 | 14.25 | APEP |
| ATOM | 44 | CE1 | PHE | 6 | 28.870 | 49.698 | 20.623 | 1.00 | 15.78 | APEP |
| ATOM | 45 | CE2 | PHE | 6 | 31.236 | 49.705 | 21.008 | 1.00 | 13.92 | APEP |
| ATOM | 46 | CZ | PHE | 6 | 30.166 | 49.648 | 20.131 | 1.00 | 13.36 | APEP |
| ATOM | 47 | C | PHE | 6 | 29.378 | 48.556 | 26.537 | 1.00 | 10.13 | APEP |
| ATOM | 48 | O | PHE | 6 | 29.892 | 49.463 | 27.201 | 1.00 | 9.26 | APEP |
| ATOM | 49 | N | ASN | 7 | 28.658 | 47.568 | 27.066 | 1.00 | 10.89 | APEP |
| ATOM | 50 | CA | ASN | 7 | 28.411 | 47.422 | 28.498 | 1.00 | 7.63 | APEP |
| ATOM | 51 | CB | ASN | 7 | 27.040 | 46.786 | 28.750 | 1.00 | 6.94 | APEP |
| ATOM | 52 | CG | ASN | 7 | 25.897 | 47.774 | 28.658 | 1.00 | 5.91 | APEP |
| ATOM | 53 | OD1 | ASN | 7 | 26.049 | 48.953 | 28.962 | 1.00 | 6.68 | APEP |
| ATOM | 54 | ND2 | ASN | 7 | 24.735 | 47.286 | 28.240 | 1.00 | 2.00 | APEP |
| ATOM | 55 | C | ASN | 7 | 29.477 | 46.428 | 28.929 | 1.00 | 8.03 | APEP |
| ATOM | 56 | O | ASN | 7 | 29.712 | 45.448 | 28.223 | 1.00 | 7.49 | APEP |
| ATOM | 57 | N | ASN | 8 | 30.126 | 46.663 | 30.066 | 1.00 | 7.97 | APEP |
| ATOM | 58 | CA | ASN | 8 | 31.155 | 45.735 | 30.536 | 1.00 | 9.65 | APEP |
| ATOM | 59 | CB | ASN | 8 | 32.193 | 46.469 | 31.384 | 1.00 | 11.85 | APEP |
| ATOM | 60 | CG | ASN | 8 | 33.241 | 45.531 | 31.961 | 1.00 | 13.69 | APEP |
| ATOM | 61 | OD1 | ASN | 8 | 33.493 | 44.459 | 31.415 | 1.00 | 12.11 | APEP |
| ATOM | 62 | ND2 | ASN | 8 | 33.858 | 45.935 | 33.071 | 1.00 | 12.79 | APEP |
| ATOM | 63 | C | ASN | 8 | 30.553 | 44.586 | 31.350 | 1.00 | 10.91 | APEP |
| ATOM | 64 | O | ASN | 8 | 30.397 | 44.690 | 32.564 | 1.00 | 11.39 | APEP |
| ATOM | 65 | N | TYR | 9 | 30.225 | 43.490 | 30.674 | 1.00 | 10.20 | APEP |
| ATOM | 66 | CA | TYR | 9 | 29.631 | 42.331 | 31.328 | 1.00 | 9.11 | APEP |
| ATOM | 67 | CB | TYR | 9 | 28.956 | 41.431 | 30.287 | 1.00 | 8.55 | APEP |
| ATOM | 68 | CG | TYR | 9 | 27.727 | 42.054 | 29.689 | 1.00 | 6.89 | APEP |
| ATOM | 69 | CD1 | TYR | 9 | 27.798 | 42.805 | 28.517 | 1.00 | 8.12 | APEP |
| ATOM | 70 | CE1 | TYR | 9 | 26.668 | 43.423 | 27.991 | 1.00 | 9.63 | APEP |
| ATOM | 71 | CD2 | TYR | 9 | 26.498 | 41.932 | 30.318 | 1.00 | 7.93 | APEP |
| ATOM | 72 | CE2 | TYR | 9 | 25.362 | 42.543 | 29.806 | 1.00 | 9.55 | APEP |
| ATOM | 73 | CZ | TYR | 9 | 25.452 | 43.286 | 28.646 | 1.00 | 10.64 | APEP |
| ATOM | 74 | OH | TYR | 9 | 24.325 | 43.893 | 28.149 | 1.00 | 11.41 | APEP |
| ATOM | 75 | C | TYR | 9 | 30.628 | 41.509 | 32.131 | 1.00 | 10.32 | APEP |
| ATOM | 76 | O | TYR | 9 | 30.237 | 40.584 | 32.840 | 1.00 | 8.46 | APEP |
| ATOM | 77 | N | CYS | 10 | 31.912 | 41.834 | 32.017 | 1.00 | 11.72 | APEP |
| ATOM | 78 | CA | CYS | 10 | 32.934 | 41.098 | 32.750 | 1.00 | 13.13 | APEP |
| ATOM | 79 | C | CYS | 10 | 32.832 | 41.404 | 34.240 | 1.00 | 14.57 | APEP |
| ATOM | 80 | O | CYS | 10 | 33.565 | 40.835 | 35.051 | 1.00 | 14.20 | APEP |
| ATOM | 81 | CB | CYS | 10 | 34.329 | 41.471 | 32.242 | 1.00 | 14.59 | APEP |
| ATOM | 82 | SG | CYS | 10 | 34.747 | 40.862 | 30.569 | 1.00 | 13.90 | APEP |
| ATOM | 83 | N | LYS | 11 | 31.913 | 42.300 | 34.593 | 1.00 | 15.58 | APEP |
| ATOM | 84 | CA | LYS | 11 | 31.706 | 42.695 | 35.982 | 1.00 | 17.16 | APEP |
| ATOM | 85 | CB | LYS | 11 | 31.514 | 44.213 | 36.073 | 1.00 | 17.37 | APEP |
| ATOM | 86 | CG | LYS | 11 | 32.805 | 45.020 | 35.908 | 1.00 | 19.88 | APEP |
| ATOM | 87 | CD | LYS | 11 | 33.879 | 44.549 | 36.872 | 1.00 | 19.32 | APEP |
| ATOM | 88 | CE | LYS | 11 | 35.252 | 44.994 | 36.442 | 1.00 | 22.07 | APEP |
| ATOM | 89 | NZ | LYS | 11 | 36.148 | 43.824 | 36.212 | 1.00 | 26.09 | APEP |
| ATOM | 90 | C | LYS | 11 | 30.503 | 41.987 | 36.600 | 1.00 | 18.39 | APEP |
| ATOM | 91 | O | LYS | 11 | 30.330 | 41.990 | 37.822 | 1.00 | 18.93 | APEP |
| ATOM | 92 | N | ILE | 12 | 29.676 | 41.382 | 35.748 | 1.00 | 17.37 | APEP |
| ATOM | 93 | CA | ILE | 12 | 28.488 | 40.662 | 36.197 | 1.00 | 17.54 | APEP |
| ATOM | 94 | CB | ILE | 12 | 27.522 | 40.348 | 35.011 | 1.00 | 15.92 | APEP |
| ATOM | 95 | CG2 | ILE | 12 | 26.347 | 39.507 | 35.497 | 1.00 | 14.62 | APEP |
| ATOM | 96 | CG1 | ILE | 12 | 27.033 | 41.645 | 34.353 | 1.00 | 14.71 | APEP |
| ATOM | 97 | CD1 | ILE | 12 | 26.197 | 42.543 | 35.246 | 1.00 | 14.44 | APEP |
| ATOM | 98 | C | ILE | 12 | 28.902 | 39.331 | 36.817 | 1.00 | 18.50 | APEP |
| ATOM | 99 | O | ILE | 12 | 29.884 | 38.728 | 36.401 | 1.00 | 19.73 | APEP |
| ATOM | 100 | N | LYS | 13 | 28.144 | 38.884 | 37.813 | 1.00 | 19.79 | APEP |
| ATOM | 101 | CA | LYS | 13 | 28.391 | 37.605 | 38.468 | 1.00 | 21.47 | APEP |
| ATOM | 102 | CB | LYS | 13 | 28.978 | 37.811 | 39.871 | 1.00 | 29.55 | APEP |
| ATOM | 103 | CG | LYS | 13 | 28.349 | 38.959 | 40.664 | 1.00 | 29.46 | APEP |
| ATOM | 104 | CD | LYS | 13 | 29.139 | 39.272 | 41.934 | 1.00 | 32.01 | APEP |
| ATOM | 105 | CE | LYS | 13 | 29.966 | 40.546 | 41.786 | 1.00 | 34.07 | APEP |
| ATOM | 106 | NZ | LYS | 13 | 30.867 | 40.516 | 40.591 | 1.00 | 34.69 | APEP |
| ATOM | 107 | C | LYS | 13 | 27.051 | 36.867 | 38.555 | 1.00 | 20.70 | APEP |
| ATOM | 108 | O | LYS | 13 | 26.050 | 37.433 | 38.976 | 1.00 | 19.96 | APEP |
| ATOM | 109 | N | CYS | 14 | 27.029 | 35.611 | 38.132 | 1.00 | 20.06 | APEP |
| ATOM | 110 | CA | CYS | 14 | 25.808 | 34.831 | 38.176 | 1.00 | 20.78 | APEP |
| ATOM | 111 | C | CYS | 14 | 25.741 | 34.062 | 39.482 | 1.00 | 22.69 | APEP |
| ATOM | 112 | O | CYS | 14 | 26.724 | 33.994 | 40.218 | 1.00 | 22.31 | APEP |
| ATOM | 113 | CB | CYS | 14 | 25.752 | 33.875 | 36.987 | 1.00 | 19.10 | APEP |
| ATOM | 114 | SG | CYS | 14 | 25.352 | 34.724 | 35.422 | 1.00 | 16.84 | APEP |
| ATOM | 115 | N | LEU | 15 | 24.577 | 33.492 | 39.775 | 1.00 | 29.99 | APEP |
| ATOM | 116 | CA | LEU | 15 | 24.400 | 32.746 | 41.015 | 1.00 | 27.03 | APEP |
| ATOM | 117 | CB | LEU | 15 | 22.953 | 32.251 | 41.138 | 1.00 | 27.78 | APEP |
| ATOM | 118 | CG | LEU | 15 | 22.054 | 32.963 | 42.152 | 1.00 | 28.08 | APEP |
| ATOM | 119 | CD1 | LEU | 15 | 20.699 | 32.269 | 42.194 | 1.00 | 28.30 | APEP |
| ATOM | 120 | CD2 | LEU | 15 | 22.699 | 32.953 | 43.535 | 1.00 | 27.17 | APEP |
| ATOM | 121 | C | LEU | 15 | 25.365 | 31.574 | 41.090 | 1.00 | 27.24 | APEP |
| ATOM | 122 | O | LEU | 15 | 26.065 | 31.402 | 42.088 | 1.00 | 28.76 | APEP |
| ATOM | 123 | N | LYS | 16 | 25.410 | 30.774 | 40.033 | 1.00 | 28.73 | APEP |
| ATOM | 124 | CA | LYS | 16 | 26.300 | 29.621 | 40.005 | 1.00 | 30.09 | APEP |
| ATOM | 125 | CB | LYS | 16 | 25.679 | 28.478 | 39.201 | 1.00 | 31.71 | APEP |
| ATOM | 126 | CG | LYS | 16 | 24.162 | 28.401 | 39.271 | 1.00 | 32.24 | APEP |
| ATOM | 127 | CD | LYS | 16 | 23.562 | 27.757 | 38.009 | 1.00 | 33.96 | APEP |
| ATOM | 128 | CE | LYS | 16 | 24.536 | 27.738 | 36.820 | 1.00 | 33.82 | APEP |
| ATOM | 129 | NZ | LYS | 16 | 23.828 | 27.604 | 35.515 | 1.00 | 33.08 | APEP |
| ATOM | 130 | C | LYS | 16 | 27.659 | 29.966 | 39.417 | 1.00 | 30.04 | APEP |
| ATOM | 131 | O | LYS | 16 | 28.442 | 29.071 | 39.092 | 1.00 | 31.31 | APEP |
| ATOM | 132 | N | GLY | 17 | 27.933 | 31.261 | 39.273 | 1.00 | 29.07 | APEP |
| ATOM | 133 | CA | GLY | 17 | 29.214 | 31.698 | 38.744 | 1.00 | 27.07 | APEP |
| ATOM | 134 | C | GLY | 17 | 29.410 | 31.553 | 37.243 | 1.00 | 26.38 | APEP |
| ATOM | 135 | O | GLY | 17 | 28.448 | 31.552 | 36.472 | 1.00 | 25.25 | APEP |
| ATOM | 136 | N | GLY | 18 | 30.670 | 31.428 | 36.831 | 1.00 | 25.19 | APEP |
| ATOM | 137 | CA | GLY | 18 | 30.983 | 31.294 | 35.420 | 1.00 | 22.24 | APEP |
| ATOM | 138 | C | GLY | 18 | 31.139 | 32.655 | 34.771 | 1.00 | 20.24 | APEP |
| ATOM | 139 | O | GLY | 18 | 30.510 | 33.622 | 35.195 | 1.00 | 21.83 | APEP |
| ATOM | 140 | N | VAL | 19 | 31.974 | 32.735 | 33.743 | 1.00 | 16.65 | APEP |
| ATOM | 141 | CA | VAL | 19 | 32.212 | 33.989 | 33.040 | 1.00 | 15.58 | APEP |
| ATOM | 142 | CB | VAL | 19 | 33.516 | 33.896 | 32.222 | 1.00 | 15.68 | APEP |
| ATOM | 143 | CG1 | VAL | 19 | 33.884 | 35.254 | 31.649 | 1.00 | 13.84 | APEP |
| ATOM | 144 | CG2 | VAL | 19 | 34.633 | 33.364 | 33.108 | 1.00 | 15.09 | APEP |
| ATOM | 145 | C | VAL | 19 | 31.045 | 34.361 | 32.115 | 1.00 | 14.11 | APEP |
| ATOM | 146 | O | VAL | 19 | 30.622 | 33.562 | 31.278 | 1.00 | 14.03 | APEP |
| ATOM | 147 | N | HIS | 20 | 30.528 | 35.577 | 32.265 | 1.00 | 11.37 | APEP |
| ATOM | 148 | CA | HIS | 20 | 29.410 | 36.020 | 31.444 | 1.00 | 11.65 | APEP |
| ATOM | 149 | CB | HIS | 20 | 29.094 | 37.493 | 31.704 | 1.00 | 12.93 | APEP |
| ATOM | 150 | CG | HIS | 20 | 27.721 | 37.900 | 31.264 | 1.00 | 13.85 | APEP |
| ATOM | 151 | CD2 | HIS | 20 | 26.597 | 38.156 | 31.974 | 1.00 | 15.96 | APEP |
| ATOM | 152 | ND1 | HIS | 20 | 27.392 | 38.102 | 29.941 | 1.00 | 15.59 | APEP |
| ATOM | 153 | CE1 | HIS | 20 | 26.126 | 38.466 | 29.853 | 1.00 | 15.25 | APEP |
| ATOM | 154 | NE2 | HIS | 20 | 25.620 | 38.506 | 31.072 | 1.00 | 17.34 | APEP |
| ATOM | 155 | C | HIS | 20 | 29.679 | 35.811 | 29.961 | 1.00 | 11.56 | APEP |
| ATOM | 156 | O | HIS | 20 | 30.783 | 36.054 | 29.467 | 1.00 | 9.12 | APEP |
| ATOM | 157 | N | THR | 21 | 28.650 | 35.355 | 29.260 | 1.00 | 12.15 | APEP |
| ATOM | 158 | CA | THR | 21 | 28.739 | 35.090 | 27.828 | 1.00 | 12.76 | APEP |
| ATOM | 159 | CB | THR | 21 | 27.349 | 34.686 | 27.287 | 1.00 | 13.90 | APEP |
| ATOM | 160 | OG1 | THR | 21 | 27.016 | 33.387 | 27.792 | 1.00 | 14.96 | APEP |
| ATOM | 161 | CG2 | THR | 21 | 27.336 | 34.658 | 25.756 | 1.00 | 13.84 | APEP |
| ATOM | 162 | C | THR | 21 | 29.294 | 36.278 | 27.025 | 1.00 | 12.07 | APEP |
| ATOM | 163 | O | THR | 21 | 30.102 | 36.090 | 26.111 | 1.00 | 8.89 | APEP |
| ATOM | 164 | N | ALA | 22 | 28.873 | 37.490 | 27.380 | 1.00 | 10.72 | APEP |
| ATOM | 165 | CA | ALA | 22 | 29.312 | 38.698 | 26.693 | 1.00 | 11.63 | APEP |
| ATOM | 166 | CB | ALA | 22 | 28.311 | 39.816 | 26.925 | 1.00 | 12.20 | APEP |
| ATOM | 167 | C | ALA | 22 | 30.706 | 39.156 | 27.102 | 1.00 | 13.47 | APEP |
| ATOM | 168 | O | ALA | 22 | 31.200 | 40.178 | 26.621 | 1.00 | 13.74 | APEP |
| ATOM | 169 | N | CYS | 23 | 31.332 | 38.410 | 28.006 | 1.00 | 14.12 | APEP |
| ATOM | 170 | CA | CYS | 23 | 32.683 | 38.715 | 28.460 | 1.00 | 14.19 | APEP |
| ATOM | 171 | C | CYS | 23 | 33.564 | 37.670 | 27.793 | 1.00 | 12.16 | APEP |
| ATOM | 172 | O | CYS | 23 | 34.725 | 37.909 | 27.497 | 1.00 | 13.84 | APEP |
| ATOM | 173 | CB | CYS | 23 | 32.782 | 38.599 | 29.995 | 1.00 | 12.96 | APEP |
| ATOM | 174 | SG | CYS | 23 | 34.454 | 38.855 | 30.695 | 1.00 | 14.19 | APEP |
| ATOM | 175 | N | LYS | 24 | 32.987 | 36.501 | 27.561 | 1.00 | 13.18 | APEP |
| ATOM | 176 | CA | LYS | 24 | 33.697 | 35.405 | 26.917 | 1.00 | 14.00 | APEP |
| ATOM | 177 | CB | LYS | 24 | 32.894 | 34.109 | 27.048 | 1.00 | 13.62 | APEP |
| ATOM | 178 | CG | LYS | 24 | 33.111 | 33.347 | 28.334 | 1.00 | 13.30 | APEP |
| ATOM | 179 | CD | LYS | 24 | 32.593 | 31.929 | 28.193 | 1.00 | 14.90 | APEP |
| ATOM | 180 | CE | LYS | 24 | 31.656 | 31.540 | 29.311 | 1.00 | 15.48 | APEP |
| ATOM | 181 | NZ | LYS | 24 | 32.009 | 30.188 | 29.830 | 1.00 | 21.39 | APEP |
| ATOM | 182 | C | LYS | 24 | 33.853 | 35.742 | 25.446 | 1.00 | 13.93 | APEP |
| ATOM | 183 | O | LYS | 24 | 34.917 | 35.578 | 24.861 | 1.00 | 14.28 | APEP |
| ATOM | 184 | N | TYR | 25 | 32.767 | 36.219 | 24.857 | 1.00 | 16.64 | APEP |
| ATOM | 185 | CA | TYR | 25 | 32.737 | 36.585 | 23.448 | 1.00 | 17.22 | APEP |
| ATOM | 186 | CB | TYR | 25 | 31.736 | 35.684 | 22.719 | 1.00 | 18.12 | ,APEP |
| ATOM | 187 | CG | TYR | 25 | 31.716 | 34.245 | 23.217 | 1.00 | 16.13 | APEP |
| ATOM | 188 | CD1 | TYR | 25 | 30.600 | 33.727 | 23.879 | 1.00 | 18.60 | APEP |
| ATOM | 189 | CE1 | TYR | 25 | 30.574 | 32.404 | 24.332 | 1.00 | 15.87 | APEP |
| ATOM | 190 | CD2 | TYR | 25 | 32.810 | 33.403 | 23.021 | 1.00 | 16.98 | APEP |
| ATOM | 191 | CE2 | TYR | 25 | 32.794 | 32.081 | 23.469 | 1.00 | 14.73 | APEP |
| ATOM | 192 | CZ | TYR | 25 | 31.677 | 31.590 | 24.120 | 1.00 | 16.69 | APEP |
| ATOM | 193 | OH | TYR | 25 | 31.661 | 30.283 | 24.566 | 1.00 | 19.74 | APEP |
| ATOM | 194 | C | TYR | 25 | 32.339 | 38.060 | 23.336 | 1.00 | 18.24 | APEP |
| ATOM | 195 | O | TYR | 25 | 31.155 | 38.404 | 23.332 | 1.00 | 17.58 | APEP |
| ATOM | 196 | N | GLY | 26 | 33.340 | 38.929 | 23.250 | 1.00 | 19.90 | APEP |
| ATOM | 197 | CA | GLY | 26 | 33.086 | 40.358 | 23.182 | 1.00 | 22.78 | APEP |
| ATOM | 198 | C | GLY | 26 | 32.536 | 40.927 | 21.886 | 1.00 | 25.12 | APEP |
| ATOM | 199 | O | GLY | 26 | 32.260 | 42.125 | 21.815 | 1.00 | 26.30 | APEP |
| ATOM | 200 | N | SER | 27 | 32.362 | 40.092 | 20.867 | 1.00 | 26.19 | APEP |
| ATOM | 201 | CA | SER | 27 | 31.855 | 40.570 | 19.583 | 1.00 | 26.72 | APEP |
| ATOM | 202 | CB | SER | 27 | 32.960 | 40.435 | 18.522 | 1.00 | 25.95 | APEP |
| ATOM | 203 | OG | SER | 27 | 32.457 | 40.041 | 17.259 | 1.00 | 29.78 | APEP |
| ATOM | 204 | C | SER | 27 | 30.586 | 39.839 | 19.139 | 1.00 | 26.86 | APEP |
| ATOM | 205 | O | SER | 27 | 30.159 | 38.878 | 19.774 | 1.00 | 25.87 | APEP |
| ATOM | 206 | N | LEU | 28 | 29.979 | 40.312 | 18.053 | 1.00 | 29.54 | APEP |
| ATOM | 207 | CA | LEU | 28 | 28.766 | 39.695 | 17.518 | 1.00 | 30.96 | APEP |
| ATOM | 208 | CB | LEU | 28 | 27.793 | 40.769 | 17.021 | 1.00 | 33.13 | APEP |
| ATOM | 209 | CG | LEU | 28 | 28.127 | 42.217 | 17.391 | 1.00 | 34.56 | APEP |
| ATOM | 210 | CD1 | LEU | 28 | 29.022 | 42.812 | 16.319 | 1.00 | 34.22 | APEP |
| ATOM | 211 | CD2 | LEU | 28 | 26.843 | 43.030 | 17.551 | 1.00 | 34.12 | APEP |
| ATOM | 212 | C | LEU | 28 | 29.142 | 38.769 | 16.365 | 1.00 | 30.72 | APEP |
| ATOM | 213 | O | LEU | 28 | 28.277 | 38.224 | 15.673 | 1.00 | 31.18 | APEP |
| ATOM | 214 | N | LYS | 29 | 30.448 | 38.602 | 16.176 | 1.00 | 30.29 | APEP |
| ATOM | 215 | CA | LYS | 29 | 31.008 | 37.759 | 15.124 | 1.00 | 29.17 | APEP |
| ATOM | 216 | CB | LYS | 29 | 32.490 | 38.102 | 14.937 | 1.00 | 31.20 | APEP |
| ATOM | 217 | CG | LYS | 29 | 33.016 | 37.866 | 13.534 | 1.00 | 32.99 | APEP |
| ATOM | 218 | CD | LYS | 29 | 34.528 | 37.785 | 13.521 | 1.00 | 34.25 | APEP |
| ATOM | 219 | CE | LYS | 29 | 35.150 | 39.121 | 13.885 | 1.00 | 35.23 | APEP |
| ATOM | 220 | NZ | LYS | 29 | 35.686 | 39.098 | 15.273 | 1.00 | 37.84 | APEP |
| ATOM | 221 | C | LYS | 29 | 30.867 | 36.269 | 15.444 | 1.00 | 27.53 | APEP |
| ATOM | 222 | O | LYS | 29 | 31.446 | 35.772 | 16.413 | 1.00 | 25.87 | APEP |
| ATOM | 223 | N | PRO | 30 | 30.104 | 35.530 | 14.621 | 1.00 | 27.59 | APEP |
| ATOM | 224 | CD | PRO | 30 | 29.362 | 36.011 | 13.442 | 1.00 | 26.03 | APEP |
| ATOM | 225 | CA | PRO | 30 | 29.905 | 34.091 | 14.840 | 1.00 | 25.87 | APEP |
| ATOM | 226 | CB | PRO | 30 | 28.982 | 33.675 | 13.694 | 1.00 | 25.48 | APEP |
| ATOM | 227 | CG | PRO | 30 | 28.330 | 34.949 | 13.245 | 1.00 | 24.87 | APEP |
| ATOM | 228 | C | PRO | 30 | 31.182 | 33.253 | 14.871 | 1.00 | 25.41 | APEP |
| ATOM | 229 | O | PRO | 30 | 32.061 | 33.404 | 14.018 | 1.00 | 26.47 | APEP |
| ATOM | 230 | N | ASN | 31 | 31.273 | 32.376 | 15.866 | 1.00 | 22.04 | APEP |
| ATOM | 231 | CA | ASN | 31 | 32.407 | 31.469 | 16.030 | 1.00 | 21.43 | APEP |
| ATOM | 232 | CB | ASN | 31 | 33.061 | 31.623 | 17.413 | 1.00 | 21.58 | APEP |
| ATOM | 233 | CG | ASN | 31 | 33.840 | 32.911 | 17.564 | 1.00 | 23.13 | APEP |
| ATOM | 234 | OD1 | ASN | 31 | 34.581 | 33.319 | 16.672 | 1.00 | 23.71 | APEP |
| ATOM | 235 | ND2 | ASN | 31 | 33.680 | 33.558 | 18.713 | 1.00 | 25.97 | APEP |
| ATOM | 236 | C | ASN | 31 | 31.817 | 30.071 | 15.944 | 1.00 | 19.60 | APEP |
| ATOM | 237 | O | ASN | 31 | 31.743 | 29.365 | 16.948 | 1.00 | 18.51 | APEP |
| ATOM | 238 | N | CYS | 32 | 31.384 | 29.667 | 14.756 | 1.00 | 18.76 | APEP |
| ATOM | 239 | CA | CYS | 32 | 30.779 | 28.348 | 14.605 | 1.00 | 18.03 | APEP |
| ATOM | 240 | C | CYS | 32 | 31.690 | 27.310 | 13.975 | 1.00 | 17.09 | APEP |
| ATOM | 241 | O | CYS | 32 | 31.234 | 26.464 | 13.207 | 1.00 | 13.04 | APEP |
| ATOM | 242 | CB | CYS | 32 | 29.493 | 28.456 | 13.792 | 1.00 | 17.35 | APEP |
| ATOM | 243 | SG | CYS | 32 | 28.253 | 29.528 | 14.570 | 1.00 | 16.28 | APEP |
| ATOM | 244 | N | GLY | 33 | 32.974 | 27.379 | 14.311 | 1.00 | 19.59 | APEP |
| ATOM | 245 | CA | GLY | 33 | 33.942 | 26.433 | 13.786 | 1.00 | 21.31 | APEP |
| ATOM | 246 | C | GLY | 33 | 33.914 | 26.269 | 12.278 | 1.00 | 22.56 | APEP |
| ATOM | 247 | O | GLY | 33 | 33.985 | 27.250 | 11.532 | 1.00 | 22.89 | APEP |
| ATOM | 248 | N | ASN | 34 | 33.812 | 25.021 | 11.830 | 1.00 | 22.35 | APEP |
| ATOM | 249 | CA | ASN | 34 | 33.787 | 24.724 | 10.409 | 1.00 | 23.03 | APEP |
| ATOM | 250 | CB | ASN | 34 | 34.531 | 23.410 | 10.136 | 1.00 | 26.79 | APEP |
| ATOM | 251 | CG | ASN | 34 | 33.754 | 22.187 | 10.581 | 1.00 | 31.53 | APEP |
| ATOM | 252 | OD1 | ASN | 34 | 33.028 | 22.221 | 11.579 | 1.00 | 35.39 | APEP |
| ATOM | 253 | ND2 | ASN | 34 | 33.908 | 21.088 | 9.840 | 1.00 | 32.88 | APEP |
| ATOM | 254 | C | ASN | 34 | 32.377 | 24.682 | 9.821 | 1.00 | 22.38 | APEP |
| ATOM | 255 | O | ASN | 34 | 32.193 | 24.351 | 8.647 | 1.00 | 21.38 | APEP |
| ATOM | 256 | N | LYS | 35 | 31.377 | 25.029 | 10.629 | 1.00 | 19.97 | APEP |
| ATOM | 257 | CA | LYS | 35 | 30.007 | 25.053 | 10.133 | 1.00 | 17.88 | APEP |
| ATOM | 258 | CB | LYS | 35 | 29.011 | 25.166 | 11.289 | 1.00 | 17.85 | APEP |
| ATOM | 259 | CG | LYS | 35 | 29.323 | 24.277 | 12.482 | 1.00 | 19.14 | APEP |
| ATOM | 260 | CD | LYS | 35 | 28.050 | 23.847 | 13.179 | 1.00 | 18.82 | APEP |
| ATOM | 261 | CE | LYS | 35 | 28.196 | 23.884 | 14.689 | 1.00 | 18.39 | APEP |
| ATOM | 262 | NZ | LYS | 35 | 29.499 | 23.329 | 15.115 | 1.00 | 18.61 | APEP |
| ATOM | 263 | C | LYS | 35 | 29.879 | 26.281 | 9.235 | 1.00 | 16.90 | APEP |
| ATOM | 264 | O | LYS | 35 | 30.557 | 27.284 | 9.453 | 1.00 | 16.79 | APEP |
| ATOM | 265 | N | VAL | 36 | 29.029 | 26.202 | 8.218 | 1.00 | 16.21 | APEP |
| ATOM | 266 | CA | VAL | 36 | 28.831 | 27.342 | 7.330 | 1.00 | 15.62 | APEP |
| ATOM | 267 | CB | VAL | 36 | 28.560 | 26.916 | 5.872 | 1.00 | 15.89 | APEP |
| ATOM | 268 | CG1 | VAL | 36 | 28.474 | 28.150 | 4.990 | 1.00 | 14.85 | APEP |
| ATOM | 269 | CG2 | VAL | 36 | 29.663 | 26.000 | 5.374 | 1.00 | 17.84 | APEP |
| ATOM | 270 | C | VAL | 36 | 27.636 | 28.149 | 7.820 | 1.00 | 13.37 | APEP |
| ATOM | 271 | O | VAL | 36 | 26.530 | 27.631 | 7.949 | 1.00 | 11.50 | APEP |
| ATOM | 272 | N | VAL | 37 | 27.882 | 29.422 | 8.095 | 1.00 | 13.37 | APEP |
| ATOM | 273 | CA | VAL | 37 | 26.857 | 30.337 | 8.573 | 1.00 | 15.79 | APEP |
| ATOM | 274 | CB | VAL | 37 | 27.506 | 31.450 | 9.424 | 1.00 | 16.40 | APEP |
| ATOM | 275 | CG1 | VAL | 37 | 26.487 | 32.521 | 9.765 | 1.00 | 16.09 | APEP |
| ATOM | 276 | CG2 | VAL | 37 | 28.096 | 30.847 | 10.681 | 1.00 | 13.21 | APEP |
| ATOM | 277 | C | VAL | 37 | 26.067 | 30.971 | 7.422 | 1.00 | 16.67 | APEP |
| ATOM | 278 | O | VAL | 37 | 26.557 | 31.873 | 6.738 | 1.00 | 18.09 | APEP |
| ATOM | 279 | N | VAL | 38 | 24.843 | 30.492 | 7.211 | 1.00 | 16.92 | APEP |
| ATOM | 280 | CA | VAL | 38 | 23.991 | 31.020 | 6.149 | 1.00 | 17.73 | APEP |
| ATOM | 281 | CB | VAL | 38 | 22.662 | 30.229 | 6.051 | 1.00 | 15.03 | APEP |
| ATOM | 282 | CG1 | VAL | 38 | 21.770 | 30.820 | 4.976 | 1.00 | 15.83 | APEP |
| ATOM | 283 | CG2 | VAL | 38 | 22.953 | 28.778 | 5.740 | 1.00 | 17.06 | APEP |
| ATOM | 284 | C | VAL. | 38 | 23.704 | 32.480 | 6.486 | 1.00 | 17.90 | APEP |
| ATOM | 285 | O | VAL | 38 | 23.852 | 33.372 | 5.645 | 1.00 | 18.01 | APEP |
| ATOM | 286 | N | SER | 39 | 23.305 | 32.713 | 7.731 | 1.00 | 15.41 | APEP |
| ATOM | 287 | CA | SER | 39 | 23.019 | 34.052 | 8.214 | 1.00 | 14.21 | APEP |
| ATOM | 288 | CB | SER | 39 | 21.857 | 34.674 | 7.438 | 1.00 | 14.70 | APEP |
| ATOM | 289 | OG | SER | 39 | 20.721 | 33.837 | 7.467 | 1.00 | 14.28 | APEP |
| ATOM | 290 | C | SER | 39 | 22.679 | 34.006 | 9.700 | 1.00 | 14.75 | APEP |
| ATOM | 291 | O | SER | 39 | 22.636 | 32.936 | 10.308 | 1.00 | 12.05 | APEP |
| ATOM | 292 | N | TYR | 40 | 22.444 | 35.179 | 10.278 | 1.00 | 14.22 | APEP |
| ATOM | 293 | CA | TYR | 40 | 22.111 | 35.272 | 11.686 | 1.00 | 14.10 | APEP |
| ATOM | 294 | CB | TYR | 40 | 23.397 | 35.179 | 12.530 | 1.00 | 15.42 | APEP |
| ATOM | 295 | CG | TYR | 40 | 24.239 | 36.438 | 12.583 | 1.00 | 14.41 | APEP |
| ATOM | 296 | CD1 | TYR | 40 | 23.921 | 37.472 | 13.464 | 1.00 | 15.34 | APEP |
| ATOM | 297 | CE1 | TYR | 40 | 24.711 | 38.605 | 13.563 | 1.00 | 16.91 | APEP |
| ATOM | 298 | CD2 | TYR | 40 | 25.375 | 36.575 | 11.790 | 1.00 | 14.36 | APEP |
| ATOM | 299 | CE2 | TYR | 40 | 26.179 | 37.712 | 11.879 | 1.00 | 17.60 | APEP |
| ATOM | 300 | CZ | TYR | 40 | 25.842 | 38.723 | 12.771 | 1.00 | 18.38 | APEP |
| ATOM | 301 | OH | TYR | 40 | 26.639 | 39.841 | 12.896 | 1.00 | 19.23 | APEP |
| ATOM | 302 | C | TYR | 40 | 21.360 | 36.569 | 11.969 | 1.00 | 13.71 | APEP |
| ATOM | 303 | O | TYR | 40 | 21.456 | 37.526 | 11.201 | 1.00 | 13.53 | APEP |
| ATOM | 304 | N | GLY | 41 | 20.602 | 36.590 | 13.061 | 1.00 | 12.13 | APEP |
| ATOM | 305 | CA | GLY | 41 | 19.857 | 37.783 | 13.418 | 1.00 | 13.27 | APEP |
| ATOM | 306 | C | GLY | 41 | 18.381 | 37.656 | 13.102 | 1.00 | 13.46 | APEP |
| ATOM | 307 | O | GLY | 41 | 17.968 | 36.726 | 12.419 | 1.00 | 14.55 | APEP |
| ATOM | 308 | N | LEU | 42 | 17.586 | 38.601 | 13.590 | 1.00 | 12.94 | APEP |
| ATOM | 309 | CA | LEU | 42 | 16.150 | 38.581 | 13.365 | 1.00 | 12.38 | APEP |
| ATOM | 310 | CB | LEU | 42 | 15.421 | 38.302 | 14.676 | 1.00 | 11.85 | APEP |
| ATOM | 311 | CG | LEU | 42 | 15.962 | 36.858 | 15.170 | 1.00 | 9.57 | APEP |
| ATOM | 312 | CD1 | LEU | 42 | 15.279 | 36.828 | 16.682 | 1.00 | 10.07 | APEP |
| ATOM | 313 | CD2 | LEU | 42 | 14.374 | 36.063 | 14.475 | 1.00 | 9.98 | APEP |
| ATOM | 314 | C | LEU | 42 | 15.651 | 39.895 | 12.791 | 1.00 | 12.90 | APEP |
| ATOM | 315 | O | LEU | 42 | 16.066 | 40.968 | 13.223 | 1.00 | 13.81 | APEP |
| ATOM | 316 | N | THR | 43 | 14.758 | 39.808 | 11.816 | 1.00 | 12.41 | APEP |
| ATOM | 317 | CA | THR | 43 | 14.200 | 41.006 | 11.210 | 1.00 | 13.32 | APEP |
| ATOM | 318 | CB | THR | 43 | 13.412 | 40.693 | 9.919 | 1.00 | 11.63 | APEP |
| ATOM | 319 | OG1 | THR | 43 | 12.195 | 40.028 | 10.254 | 1.00 | 12.20 | APEP |
| ATOM | 320 | CG2 | THR | 43 | 14.222 | 39.804 | 8.994 | 1.00 | 11.85 | APEP |
| ATOM | 321 | C | THR | 43 | 13.249 | 41.637 | 12.208 | 1.00 | 13.30 | APEP |
| ATOM | 322 | O | THR | 43 | 12.801 | 40.990 | 13.161 | 1.00 | 12.67 | APEP |
| ATOM | 323 | N | LYS | 44 | 12.939 | 42.904 | 11.977 | 1.00 | 14.11 | APEP |
| ATOM | 324 | CA | LYS | 44 | 12.050 | 43.640 | 12.851 | 1.00 | 14.99 | APEP |
| ATOM | 325 | CB | LYS | 44 | 11.975 | 45.100 | 12.379 | 1.00 | 16.22 | APEP |
| ATOM | 326 | CG | LYS | 44 | 10.594 | 45.667 | 12.152 | 1.00 | 18.80 | APEP |
| ATOM | 327 | CD | LYS | 44 | 10.567 | 47.157 | 12.489 | 1.00 | 19.36 | APEP |
| ATOM | 328 | CE | LYS | 44 | 9.655 | 47.915 | 11.552 | 1.00 | 21.90 | APEP |
| ATOM | 329 | NZ | LYS | 44 | 10.430 | 48.714 | 10.570 | 1.00 | 20.87 | APEP |
| ATOM | 330 | C | LYS | 44 | 10.672 | 42.985 | 12.923 | 1.00 | 13.76 | APEP |
| ATOM | 331 | O | LYS | 44 | 10.083 | 42.910 | 13.999 | 1.00 | 14.04 | APEP |
| ATOM | 332 | N | GLN | 45 | 10.162 | 42.487 | 11.798 | 1.00 | 12.41 | APEP |
| ATOM | 333 | CA | GLN | 45 | 8.849 | 41.839 | 11.806 | 1.00 | 11.51 | APEP |
| ATOM | 334 | CB | GLN | 45 | 8.334 | 41.646 | 10.370 | 1.00 | 10.79 | APEP |
| ATOM | 335 | CG | GLN | 45 | 7.063 | 40.816 | 10.246 | 1.00 | 10.70 | APEP |
| ATOM | 336 | CD | GLN | 45 | 5.812 | 41.538 | 10.743 | 1.00 | 12.43 | APEP |
| ATOM | 337 | OE1 | GLN | 45 | 5.696 | 42.763 | 10.650 | 1.00 | 12.72 | APEP |
| ATOM | 338 | NE2 | GLN | 45 | 4.869 | 40.772 | 11.274 | 1.00 | 11.44 | APEP |
| ATOM | 339 | C | GLN | 45 | 8.917 | 40.496 | 12.548 | 1.00 | 10.87 | APEP |
| ATOM | 340 | O | GLN | 45 | 7.987 | 40.123 | 13.267 | 1.00 | 9.48 | APEP |
| ATOM | 341 | N | GLU | 46 | 10.024 | 39.779 | 12.382 | 1.00 | 9.10 | APEP |
| ATOM | 342 | CA | GLU | 46 | 10.207 | 38.496 | 13.059 | 1.00 | 9.69 | APEP |
| ATOM | 343 | CB | GLU | 46 | 11.511 | 37.845 | 12.610 | 1.00 | 8.84 | APEP |
| ATOM | 344 | CG | GLU | 46 | 11.366 | 36.916 | 11.407 | 1.00 | 9.10 | APEP |
| ATOM | 345 | CD | GLU | 46 | 12.710 | 36.534 | 10.806 | 1.00 | 9.37 | APEP |
| ATOM | 346 | OE1 | GLU | 46 | 13.723 | 37.158 | 11.173 | 1.00 | 7.41 | APEP |
| ATOM | 347 | OE2 | GLU | 46 | 12.755 | 35.607 | 9.966 | 1.00 | 10.21 | APEP |
| ATOM | 348 | C | GLU | 46 | 10.217 | 38.666 | 14.582 | 1.00 | 10.19 | APEP |
| ATOM | 349 | O | GLU | 46 | 9.708 | 37.817 | 15.310 | 1.00 | 10.51 | APEP |
| ATOM | 350 | N | LYS | 47 | 10.807 | 39.761 | 15.057 | 1.00 | 10.08 | APEP |
| ATOM | 351 | CA | LYS | 47 | 10.865 | 40.042 | 16.486 | 1.00 | 9.91 | APEP |
| ATOM | 352 | CB | LYS | 47 | 11.675 | 41.318 | 16.749 | 1.00 | 9.24 | APEP |
| ATOM | 353 | CG | LYS | 47 | 13.167 | 41.191 | 16.459 | 1.00 | 7.94 | APEP |
| ATOM | 354 | CD | LYS | 47 | 13.906 | 42.509 | 16.710 | 1.00 | 9.13 | APEP |
| ATOM | 355 | CE | LYS | 47 | 15.411 | 42.361 | 16.498 | 1.00 | 11.45 | APEP |
| ATOM | 356 | NZ | LYS | 47 | 16.127 | 43.675 | 16.431 | 1.00 | 11.96 | APEP |
| ATOM | 357 | C | LYS | 47 | 9.438 | 40.229 | 16.984 | 1.00 | 10.20 | APEP |
| ATOM | 358 | O | LYS | 47 | 9.027 | 39.626 | 17.969 | 1.00 | 10.91 | APEP |
| ATOM | 359 | N | GLN | 48 | 8.689 | 41.065 | 16.275 | 1.00 | 11.41 | APEP |
| ATOM | 360 | CA | GLN | 48 | 7.299 | 41.366 | 16.602 | 1.00 | 11.75 | APEP |
| ATOM | 361 | CB | GLN | 48 | 6.759 | 42.409 | 15.624 | 1.00 | 11.16 | APEP |
| ATOM | 362 | CG | GLN | 48 | 5.254 | 42.607 | 15.669 | 1.00 | 12.11 | APEP |
| ATOM | 363 | CD | GLN | 48 | 4.767 | 43.515 | 14.556 | 1.00 | 12.60 | APEP |
| ATOM | 364 | OE1 | GLN | 48 | 5.301 | 44.606 | 14.359 | 1.00 | 10.09 | APEP |
| ATOM | 365 | NE2 | GLN | 48 | 3.758 | 43.065 | 13.816 | 1.00 | 11.92 | APEP |
| ATOM | 366 | C | GLN | 48 | 6.420 | 40.123 | 16.563 | 1.00 | 12.69 | APEP |
| ATOM | 367 | O | GLN | 48 | 5.488 | 39.993 | 17.353 | 1.00 | 13.53 | APEP |
| ATOM | 368 | N | ASP | 49 | 6.716 | 39.219 | 15.633 | 1.00 | 12.63 | APEP |
| ATOM | 369 | CA | ASP | 49 | 5.964 | 37.977 | 15.487 | 1.00 | 11.04 | APEP |
| ATOM | 370 | CB | ASP | 49 | 6.290 | 37.322 | 14.144 | 1.00 | 14.99 | APEP |
| ATOM | 371 | CG | ASP | 49 | 5.578 | 37.990 | 12.981 | 1.00 | 17.72 | APEP |
| ATOM | 372 | OD1 | ASP | 49 | 4.518 | 38.620 | 13.200 | 1.00 | 18.74 | APEP |
| ATOM | 373 | OD2 | ASP | 49 | 6.082 | 37.878 | 11.844 | 1.00 | 19.80 | APEP |
| ATOM | 374 | C | ASP | 49 | 6.285 | 36.998 | 16.615 | 1.00 | 9.65 | APEP |
| ATOM | 375 | O | ASP | 49 | 5.433 | 36.211 | 17.020 | 1.00 | 9.33 | APEP |
| ATOM | 376 | N | ILE | 50 | 7.519 | 37.034 | 17.107 | 1.00 | 8.25 | APEP |
| ATOM | 377 | CA | ILE | 50 | 7.916 | 36.152 | 18.203 | 1.00 | 8.01 | APEP |
| ATOM | 378 | CB | ILE | 50 | 9.454 | 36.132 | 18.387 | 1.00 | 7.72 | APEP |
| ATOM | 379 | CG2 | ILE | 50 | 9.823 | 35.416 | 19.693 | 1.00 | 7.19 | APEP |
| ATOM | 380 | CG1 | ILE | 50 | 10.103 | 35.410 | 17.203 | 1.00 | 6.44 | APEP |
| ATOM | 381 | CD1 | ILE | 50 | 11.582 | 35.687 | 17.041 | 1.00 | 4.97 | APEP |
| ATOM | 382 | C | ILE | 50 | 7.256 | 36.621 | 19.499 | 1.00 | 8.14 | APEP |
| ATOM | 383 | O | ILE | 50 | 6.805 | 35.808 | 20.303 | 1.00 | 7.29 | APEP |
| ATOM | 384 | N | LEU | 51 | 7.191 | 37.938 | 19.679 | 1.00 | 8.57 | APEP |
| ATOM | 385 | CA | LEU | 51 | 6.571 | 38.529 | 20.854 | 1.00 | 9.76 | APEP |
| ATOM | 386 | CB | LEU | 51 | 6.733 | 40.055 | 20.836 | 1.00 | 9.57 | APEP |
| ATOM | 387 | CG | LEU | 51 | 6.509 | 40.844 | 22.139 | 1.00 | 12.08 | APEP |
| ATOM | 388 | CD1 | LEU | 51 | 7.509 | 40.401 | 23.216 | 1.00 | 9.69 | APEP |
| ATOM | 389 | CD2 | LEU | 51 | 6.659 | 42.333 | 21.861 | 1.00 | 10.85 | APEP |
| ATOM | 390 | C | LEU | 51 | 5.091 | 38.172 | 20.863 | 1.00 | 10.95 | APEP |
| ATOM | 391 | O | LEU | 51 | 4.571 | 37.664 | 21.861 | 1.00 | 12.16 | APEP |
| ATOM | 392 | N | LYS | 52 | 4.423 | 38.427 | 19.739 | 1.00 | 10.41 | APEP |
| ATOM | 393 | CA | LYS | 52 | 2.994 | 38.156 | 19.601 | 1.00 | 10.29 | APEP |
| ATOM | 394 | CB | LYS | 52 | 2.520 | 38.535 | 18.196 | 1.00 | 10.94 | APEP |
| ATOM | 395 | CG | LYS | 52 | 1.066 | 38.956 | 18.132 | 1.00 | 14.35 | APEP |
| ATOM | 396 | CD | LYS | 52 | 0.258 | 38.029 | 17.236 | 1.00 | 16.34 | APEP |
| ATOM | 397 | CE | LYS | 52 | -0.870 | 38.780 | 16.543 | 1.00 | 17.57 | APEP |
| ATOM | 398 | NZ | LYS | 52 | -2.107 | 38.817 | 17.374 | 1.00 | 17.77 | APEP |
| ATOM | 399 | C | LYS | 52 | 2.627 | 36.709 | 19.893 | 1.00 | 9.70 | APEP |
| ATOM | 400 | O | LYS | 52 | 1.553 | 36.432 | 20.419 | 1.00 | 9.63 | APEP |
| ATOM | 401 | N | GLU | 53 | 3.508 | 35.780 | 19.540 | 1.00 | 10.85 | APEP |
| ATOM | 402 | CA | GLU | 53 | 3.249 | 34.366 | 19.799 | 1.00 | 11.95 | APEP |
| ATOM | 403 | CB | GLU | 53 | 4.261 | 33.491 | 19.057 | 1.00 | 13.57 | APEP |
| ATOM | 404 | CG | GLU | 53 | 3.957 | 31.996 | 19.089 | 1.00 | 15.51 | APEP |
| ATOM | 405 | CD | GLU | 53 | 2.525 | 31.651 | 18.695 | 1.00 | 20.29 | APEP |
| ATOM | 406 | OE1 | GLU | 53 | 1.876 | 32.439 | 17.971 | 1.00 | 21.72 | APEP |
| ATOM | 407 | OE2 | GLU | 53 | 2.044 | 30.577 | 19.111 | 1.00 | 21.86 | APEP |
| ATOM | 408 | C | GLU | 53 | 3.362 | 34.120 | 21.294 | 1.00 | 12.09 | APEP |
| ATOM | 409 | O | GLU | 53 | 2.568 | 33.382 | 21.878 | 1.00 | 11.99 | APEP |
| ATOM | 410 | N | HIS | 54 | 4.357 | 34.750 | 21.910 | 1.00 | 12.11 | APEP |
| ATOM | 411 | CA | HIS | 54 | 4.580 | 34.610 | 23.340 | 1.00 | 11.34 | APEP |
| ATOM | 412 | CB | HIS | 54 | 5.829 | 35.399 | 23.769 | 1.00 | 9.29 | APEP |
| ATOM | 413 | CG | HIS | 54 | 7.089 | 34.584 | 23.817 | 1.00 | 7.76 | APEP |
| ATOM | 414 | CD2 | HIS | 54 | 7.695 | 33.933 | 24.840 | 1.00 | 9.61 | APEP |
| ATOM | 415 | ND1 | HIS | 54 | 7.895 | 34.394 | 22.716 | 1.00 | 6.60 | APEP |
| ATOM | 416 | CE1 | HIS | 54 | 8.941 | 33.663 | 23.056 | 1.00 | 5.37 | APEP |
| ATOM | 417 | NE2 | HIS | 54 | 8.844 | 33.370 | 24.340 | 1.00 | 7.30 | APEP |
| ATOM | 418 | C | HIS | 54 | 3.365 | 35.143 | 24.092 | 1.00 | 10.89 | APEP |
| ATOM | 419 | O | HIS | 54 | 2.844 | 34.492 | 24.983 | 1.00 | 10.22 | APEP |
| ATOM | 420 | N | ASN | 55 | 2.913 | 36.331 | 23.703 | 1.00 | 12.43 | APEP |
| ATOM | 421 | CA | ASN | 55 | 1.784 | 36.982 | 24.356 | 1.00 | 13.65 | APEP |
| ATOM | 422 | CB | ASN | 55 | 1.791 | 38.473 | 23.991 | 1.00 | 12.77 | APEP |
| ATOM | 423 | CG | ASN | 55 | 2.950 | 39.232 | 24.655 | 1.00 | 12.31 | APEP |
| ATOM | 424 | OD1 | ASN | 55 | 3.396 | 38.871 | 25.747 | 1.00 | 7.53 | APEP |
| ATOM | 425 | ND2 | ASN | 55 | 3.436 | 40.280 | 23.993 | 1.00 | 9.40 | APEP |
| ATOM | 426 | C | ASN | 55 | 0.413 | 36.347 | 24.097 | 1.00 | 13.82 | APEP |
| ATOM | 427 | O | ASN | 55 | -0.457 | 36.355 | 24.973 | 1.00 | 13.31 | APEP |
| ATOM | 428 | N | ASP | 56 | 0.221 | 35.795 | 22.907 | 1.00 | 12.69 | APEP |
| ATOM | 429 | CA | ASP | 56 | -1.036 | 35.134 | 22.572 | 1.00 | 12.59 | APEP |
| ATOM | 430 | CB | ASP | 56 | -1.049 | 34.675 | 21.111 | 1.00 | 12.80 | APEP |
| ATOM | 431 | CG | ASP | 56 | -1.359 | 35.787 | 20.143 | 1.00 | 13.37 | APEP |
| ATOM | 432 | OD1 | ASP | 56 | -1.902 | 36.825 | 20.565 | 1.00 | 13.48 | APEP |
| ATOM | 433 | OD2 | ASP | 56 | -1.059 | 35.615 | 18.945 | 1.00 | 15.39 | APEP |
| ATOM | 434 | C | ASP | 56 | -1.153 | 33.899 | 23.450 | 1.00 | 10.48 | APEP |
| ATOM | 435 | O | ASP | 56 | -2.224 | 33.577 | 23.953 | 1.00 | 10.14 | APEP |
| ATOM | 436 | N | PHE | 57 | -0.046 | 33.191 | 23.604 | 1.00 | 9.15 | APEP |
| ATOM | 437 | CA | PHE | 57 | -0.047 | 31.989 | 24.418 | 1.00 | 11.09 | APEP |
| ATOM | 438 | CB | PHE | 57 | 1.272 | 31.227 | 24.252 | 1.00 | 12.68 | APEP |
| ATOM | 439 | CG | PHE | 57 | 1.261 | 29.863 | 24.884 | 1.00 | 11.85 | APEP |
| ATOM | 440 | CD1 | PHE | 57 | 0.346 | 28.899 | 24.471 | 1.00 | 12.69 | APEP |
| ATOM | 441 | CD2 | PHE | 57 | 2.150 | 29.549 | 25.903 | 1.00 | 12.28 | APEP |
| ATOM | 442 | CE1 | PHE | 57 | 0.316 | 27.642 | 25.067 | 1.00 | 11.80 | APEP |
| ATOM | 443 | CE2 | PHE | 57 | 2.132 | 28.296 | 26.508 | 1.00 | 12.07 | APEP |
| ATOM | 444 | CZ | PHE | 57 | 1.216 | 27.342 | 26.092 | 1.00 | 12.36 | APEP |
| ATOM | 445 | C | PHE | 57 | -0.267 | 32.346 | 25.890 | 1.00 | 10.66 | APEP |
| ATOM | 446 | O | PHE | 57 | -1.012 | 31.664 | 26.597 | 1.00 | 11.28 | APEP |
| ATOM | 447 | N | ARG | 58 | 0.360 | 33.423 | 26.349 | 1.00 | 8.83 | APEP |
| ATOM | 448 | CA | ARG | 58 | 0.204 | 33.830 | 27.738 | 1.00 | 10.25 | APEP |
| ATOM | 449 | CB | ARG | 58 | 1.107 | 35.024 | 28.057 | 1.00 | 7.50 | APEP |
| ATOM | 450 | CG | ARG | 58 | 2.483 | 34.615 | 28.530 | 1.00 | 7.56 | APEP |
| ATOM | 451 | CD | ARG | 58 | 3.478 | 35.755 | 28.446 | 1.00 | 7.29 | APEP |
| ATOM | 452 | NE | ARG | 58 | 3.391 | 36.649 | 29.601 | 1.00 | 8.58 | APEP |
| ATOM | 453 | CZ | ARG | 58 | 4.025 | 36.450 | 30.750 | 1.00 | 7.65 | APEP |
| ATOM | 454 | NH1 | ARG | 58 | 4.797 | 35.388 | 30.908 | 1.00 | 8.61 | APEP |
| ATOM | 455 | NH2 | ARG | 58 | 3.892 | 37.318 | 31.738 | 1.00 | 9.84 | APEP |
| ATOM | 456 | C | ARG | 58 | -1.246 | 34.170 | 28.032 | 1.00 | 9.76 | APEP |
| ATOM | 457 | O | ARG | 58 | -1.793 | 33.733 | 29.042 | 1.00 | 11.57 | APEP |
| ATOM | 458 | N | GLN | 59 | -1.874 | 34.932 | 27.141 | 1.00 | 10.78 | APEP |
| ATOM | 459 | CA | GLN | 59 | -3.270 | 35.321 | 27.314 | 1.00 | 9.25 | APEP |
| ATOM | 460 | CB | GLN | 59 | -3.621 | 36.464 | 26.368 | 1.00 | 11.30 | APEP |
| ATOM | 461 | CG | GLN | 59 | -3.388 | 37.870 | 26.937 | 1.00 | 14.86 | APEP |
| ATOM | 462 | CD | GLN | 59 | -3.254 | 37.924 | 28.457 | 1.00 | 15.40 | APEP |
| ATOM | 463 | OE1 | GLN | 59 | -2.306 | 38.508 | 28.976 | 1.00 | 20.39 | APEP |
| ATOM | 464 | NE2 | GLN | 59 | -4.203 | 37.328 | 29.171 | 1.00 | 16.19 | APEP |
| ATOM | 465 | C | GLN | 59 | -4.233 | 34.156 | 27.107 | 1.00 | 8.85 | APEP |
| ATOM | 466 | O | GLN | 59 | -5.275 | 34.084 | 27.753 | 1.00 | 8.65 | APEP |
| ATOM | 467 | N | LYS | 60 | -3.900 | 33.240 | 26.209 | 1.00 | 9.28 | APEP |
| ATOM | 468 | CA | LYS | 60 | -4.765 | 32.084 | 25.999 | 1.00 | 9.29 | APEP |
| ATOM | 469 | CB | LYS | 60 | -4.190 | 31.170 | 24.919 | 1.00 | 11.77 | APEP |
| ATOM | 470 | CG | LYS | 60 | -5.097 | 29.999 | 24.555 | 1.00 | 12.61 | APEP |
| ATOM | 471 | CD | LYS | 60 | -4.357 | 28.678 | 24.660 | 1.00 | 12.86 | APEP |
| ATOM | 472 | CE | LYS | 60 | -3.849 | 28.205 | 23.310 | 1.00 | 10.33 | APEP |
| ATOM | 473 | NZ | LYS | 60 | -4.535 | 26.970 | 22.830 | 1.00 | 12.74 | APEP |
| ATOM | 474 | C | LYS | 60 | -4.840 | 31.329 | 27.320 | 1.00 | 9.51 | APEP |
| ATOM | 475 | O | LYS | 60 | -5.922 | 31.017 | 27.816 | 1.00 | 7.72 | APEP |
| ATOM | 476 | N | ILE | 61 | -3.671 | 31.049 | 27.887 | 1.00 | 9.04 | APEP |
| ATOM | 477 | CA | ILE | 61 | -3.570 | 30.348 | 29.161 | 1.00 | 9.87 | APEP |
| ATOM | 478 | CB | ILE | 61 | -2.075 | 30.101 | 29.536 | 1.00 | 10.78 | APEP |
| ATOM | 479 | CG2 | ILE | 61 | -1.970 | 29.598 | 30.973 | 1.00 | 10.13 | APEP |
| ATOM | 480 | CG1 | ILE | 61 | -1.428 | 29.152 | 28.507 | 1.00 | 9.94 | APEP |
| ATOM | 481 | CD1 | ILE | 61 | -1.212 | 27.720 | 28.980 | 1.00 | 10.64 | APEP |
| ATOM | 482 | C | ILE | 61 | -4.254 | 31.141 | 30.283 | 1.00 | 9.04 | APEP |
| ATOM | 483 | O | ILE | 61 | -4.980 | 30.571 | 31.092 | 1.00 | 9.20 | APEP |
| ATOM | 484 | N | ALA | 62 | -4.041 | 32.454 | 30.314 | 1.00 | 8.91 | APEP |
| ATOM | 485 | CA | ALA | 62 | -4.628 | 33.308 | 31.350 | 1.00 | 9.06 | APEP |
| ATOM | 486 | CB | ALA | 62 | -4.090 | 34.729 | 31.209 | 1.00 | 5.84 | APEP |
| ATOM | 487 | C | ALA | 62 | -6.165 | 33.327 | 31.363 | 1.00 | 11.19 | APEP |
| ATOM | 488 | O | ALA | 62 | -6.794 | 33.581 | 32.397 | 1.00 | 12.79 | APEP |
| ATOM | 489 | N | ARG | 63 | -6.769 | 33.053 | 30.214 | 1.00 | 12.40 | APEP |
| ATOM | 490 | CA | ARG | 63 | -8.219 | 33.050 | 30.096 | 1.00 | 10.93 | APEP |
| ATOM | 491 | CB | ARG | 63 | -8.618 | 33.627 | 28.736 | 1.00 | 10.77 | APEP |
| ATOM | 492 | CG | ARG | 63 | -8.043 | 35.012 | 28.505 | 1.00 | 12.79 | APEP |
| ATOM | 493 | CD | ARG | 63 | -8.608 | 35.684 | 27.278 | 1.00 | 15.66 | APEP |
| ATOM | 494 | NE | ARG | 63 | -7.868 | 36.904 | 26.968 | 1.00 | 17.96 | APEP |
| ATOM | 495 | CZ | ARG | 63 | -7.346 | 37.179 | 25.777 | 1.00 | 20.00 | APEP |
| ATOM | 496 | NH1 | ARG | 63 | -7.483 | 36.321 | 24.772 | 1.00 | 19.36 | APEP |
| ATOM | 497 | NH2 | NH2 | 63 | -6.679 | 38.313 | 25.590 | 1.00 | 22.72 | APEP |
| ATOM | 498 | C | ARG | 63 | -8.827 | 31.661 | 30.285 | 1.00 | 10.92 | APEP |
| ATOM | 499 | O | ARG | 63 | -10.036 | 31.489 | 30.179 | 1.00 | 12.37 | APEP |
| ATOM | 500 | N | GLY | 64 | -7.986 | 30.677 | 30.575 | 1.00 | 12.22 | APEP |
| ATOM | 501 | CA | GLY | 64 | -8.475 | 29.325 | 30.780 | 1.00 | 11.71 | APEP |
| ATOM | 502 | C | GLY | 64 | -8.985 | 28.685 | 29.509 | 1.00 | 13.26 | APEP |
| ATOM | 503 | O | GLY | 64 | -9.950 | 27.911 | 29.540 | 1.00 | 14.03 | APEP |
| ATOM | 504 | N | LEU | 65 | -8.331 | 28.998 | 28.391 | 1.00 | 11.24 | APEP |
| ATOM | 505 | CA | LEU | 65 | -8.711 | 28.463 | 27.095 | 1.00 | 10.84 | APEP |
| ATOM | 506 | CB | LEU | 65 | -8.747 | 29.581 | 26.044 | 1.00 | 10.48 | APEP |
| ATOM | 507 | CG | LEU | 65 | -9.602 | 30.803 | 26.396 | 1.00 | 8.01 | APEP |
| ATOM | 508 | CD1 | LEU | 65 | -9.278 | 31.946 | 25.470 | 1.00 | 13.03 | APEP |
| ATOM | 509 | CD2 | LEU | 65 | -11.074 | 30.450 | 26.291 | 1.00 | 10.77 | APEP |
| ATOM | 510 | C | LEU | 65 | -7.764 | 27.361 | 26.644 | 1.00 | 12.39 | APEP |
| ATOM | 511 | O | LEU | 65 | -7.998 | 26.719 | 25.625 | 1.00 | 12.90 | APEP |
| ATOM | 512 | N | GLU | 66 | -6.686 | 27.147 | 27.387 | 1.00 | 11.27 | APEP |
| ATOM | 513 | CA | GLU | 66 | -5.754 | 26.094 | 27.023 | 1.00 | 12.09 | APEP |
| ATOM | 514 | CB | GLU | 66 | -4.365 | 26.361 | 27.610 | 1.00 | 11.39 | APEP |
| ATOM | 515 | CG | GLU | 66 | -3.327 | 25.308 | 27.245 | 1.00 | 12.91 | APEP |
| ATOM | 516 | CD | GLU | 66 | -3.362 | 24.941 | 25.774 | 1.00 | 13.85 | APEP |
| ATOM | 517 | OE1 | GLU | 66 | -2.689 | 25.629 | 24.988 | 1.00 | 18.33 | APEP |
| ATOM | 518 | OE2 | GLU | 66 | -4.054 | 23.971 | 25.401 | 1.00 | 12.27 | APEP |
| ATOM | 519 | C | GLU | 66 | -6.323 | 24.799 | 27.575 | 1.00 | 12.38 | APEP |
| ATOM | 520 | O | GLU | 66 | -6.214 | 24.512 | 28.764 | 1.00 | 12.85 | APEP |
| ATOM | 521 | N | THR | 67 | -6.943 | 24.022 | 26.696 | 1.00 | 13.75 | APEP |
| ATOM | 522 | CA | THR | 67 | -7.553 | 22.769 | 27.091 | 1.00 | 13.16 | APEP |
| ATOM | 523 | CB | THR | 67 | -8.562 | 22.295 | 26.018 | 1.00 | 13.92 | APEP |
| ATOM | 524 | OG1 | THR | 67 | -7.858 | 21.894 | 24.830 | 1.00 | 14.81 | APEP |
| ATOM | 525 | CG2 | THR | 67 | -9.524 | 23.413 | 25.671 | 1.00 | 11.42 | APEP |
| ATOM | 526 | C | THR | 67 | -6.548 | 21.652 | 27.368 | 1.00 | 13.63 | APEP |
| ATOM | 527 | O | THR | 67 | -6.875 | 20.682 | 28.049 | 1.00 | 14.84 | APEP |
| ATOM | 528 | N | ARG | 68 | -5.326 | 21.793 | 26.861 | 1.00 | 13.12 | APEP |
| ATOM | 529 | CA | ARG | 68 | -4.301 | 20.770 | 27.042 | 1.00 | 11.47 | APEP |
| ATOM | 530 | CB | ARG | 68 | -3.222 | 20.914 | 25.967 | 1.00 | 13.83 | APEP |
| ATOM | 531 | CG | ARG | 68 | -3.715 | 20.741 | 24.538 | 1.00 | 13.10 | APEP |
| ATOM | 532 | CD | ARG | 68 | -2.626 | 21.128 | 23.542 | 1.00 | 13.89 | APEP |
| ATOM | 533 | NE | ARG | 68 | -2.317 | 22.556 | 23.590 | 1.00 | 12.99 | APEP |
| ATOM | 534 | CZ | ARG | 68 | -1.244 | 23.111 | 23.033 | 1.00 | 11.20 | APEP |
| ATOM | 535 | NH1 | ARG | 68 | -0.372 | 22.351 | 22.383 | 1.00 | 10.56 | APEP |
| ATOM | 536 | NH2 | ARG | 68 | -1.042 | 24.420 | 23.135 | 1.00 | 5.87 | APEP |
| ATOM | 537 | C | ARG | 68 | -3.631 | 20.746 | 28.415 | 1.00 | 11.74 | APEP |
| ATOM | 538 | O | ARG | 68 | -3.420 | 21.789 | 29.032 | 1.00 | 10.99 | APEP |
| ATOM | 539 | N | GLY | 69 | -3.295 | 19.536 | 28.867 | 1.00 | 11.61 | APEP |
| ATOM | 540 | CA | GLY | 69 | -2.641 | 19.334 | 30.147 | 1.00 | 14.23 | APEP |
| ATOM | 541 | C | GLY | 69 | -2.565 | 17.857 | 30.518 | 1.00 | 16.11 | APEP |
| ATOM | 542 | O | GLY | 69 | -2.998 | 17.001 | 29.747 | 1.00 | 16.74 | APEP |
| ATOM | 543 | N | ASN | 70 | -2.006 | 17.551 | 31.687 | 1.00 | 16.09 | APEP |
| ATOM | 544 | CA | ASN | 70 | -1.896 | 16.172 | 32.156 | 1.00 | 17.23 | APEP |
| ATOM | 545 | CB | ASN | 70 | -0.439 | 15.704 | 32.132 | 1.00 | 18.05 | APEP |
| ATOM | 546 | CG | ASN | 70 | -0.310 | 14.203 | 32.291 | 1.00 | 20.68 | APEP |
| ATOM | 547 | OD1 | ASN | 70 | -1.204 | 13.452 | 31.894 | 1.00 | 20.26 | APEP |
| ATOM | 548 | ND2 | ASN | 70 | 0.806 | 13.752 | 32.874 | 1.00 | 20.67 | APEP |
| ATOM | 549 | C | ASN | 70 | -2.452 | 16.025 | 33.578 | 1.00 | 16.86 | APEP |
| ATOM | 550 | O | ASN | 70 | -1.717 | 15.739 | 34.523 | 1.00 | 15.70 | APEP |
| ATOM | 551 | N | PRO | 71 | -3.770 | 16.204 | 33.738 | 1.00 | 16.37 | APEP |
| ATOM | 552 | CD | PRO | 71 | -4.467 | 16.046 | 35.026 | 1.00 | 16.71 | APEP |
| ATOM | 553 | CA | PRO | 71 | -4.713 | 16.522 | 32.663 | 1.00 | 16.24 | APEP |
| ATOM | 554 | CB | PRO | 71 | -5.962 | 15.777 | 33.086 | 1.00 | 16.30 | APEP |
| ATOM | 555 | CG | PRO | 71 | -5.928 | 15.906 | 34.614 | 1.00 | 16.81 | APEP |
| ATOM | 556 | C | PRO | 71 | -4.999 | 18.012 | 32.491 | 1.00 | 16.23 | APEP |
| ATOM | 557 | O | PRO | 71 | -4.638 | 18.837 | 33.338 | 1.00 | 16.06 | APEP |
| ATOM | 558 | N | GLY | 72 | -5.666 | 18.342 | 31.392 | 1.00 | 13.86 | APEP |
| ATOM | 559 | CA | GLY | 72 | -6.042 | 19.720 | 31.143 | 1.00 | 14.67 | APEP |
| ATOM | 560 | C | GLY | 72 | -7.437 | 19.902 | 31.716 | 1.00 | 14.79 | APEP |
| ATOM | 561 | O | GLY | 72 | -8.030 | 18.935 | 32.192 | 1.00 | 16.06 | APEP |
| ATOM | 562 | N | PRO | 73 | -8.000 | 21.115 | 31.695 | 1.00 | 13.41 | APEP |
| ATOM | 563 | CD | PRO | 73 | -9.343 | 21.354 | 32.253 | 1.00 | 13.38 | APEP |
| ATOM | 564 | CA | PRO | 73 | -7.412 | 22.347 | 31.164 | 1.00 | 14.44 | APEP |
| ATOM | 565 | CB | PRO | 73 | -8.621 | 23.250 | 30.976 | 1.00 | 13.74 | APEP |
| ATOM | 566 | CG | PRO | 73 | -9.519 | 22.850 | 32.113 | 1.00 | 12.91 | APEP |
| ATOM | 567 | C | PRO | 73 | -6.412 | 22.977 | 32.129 | 1.00 | 13.52 | APEP |
| ATOM | 568 | O | PRO | 73 | -6.271 | 22.537 | 33.268 | 1.00 | 13.30 | APEP |
| ATOM | 569 | N | GLN | 74 | -5.713 | 24.004 | 31.658 | 1.00 | 12.54 | APEP |
| ATOM | 570 | CA | GLN | 74 | -4.782 | 24.723 | 32.506 | 1.00 | 11.28 | APEP |
| ATOM | 571 | CB | GLN | 74 | -3.708 | 25.433 | 31.672 | 1.00 | 10.31 | APEP |
| ATOM | 572 | CG | GLN | 74 | -2.658 | 24.505 | 31.043 | 1.00 | 9.27 | APEP |
| ATOM | 573 | CD | GLN | 74 | -2.070 | 23.484 | 32.024 | 1.00 | 12.07 | APEP |
| ATOM | 574 | OE1 | GLN | 74 | -1.558 | 23.838 | 33.087 | 1.00 | 10.98 | APEP |
| ATOM | 575 | NE2 | GLN | 74 | -2.137 | 22.210 | 31.654 | 1.00 | 12.44 | APEP |
| ATOM | 576 | C | GLN | 74 | -5.707 | 25.736 | 33.170 | 1.00 | 11.59 | APEP |
| ATOM | 577 | O | GLN | 74 | -6.710 | 26.139 | 32.579 | 1.00 | 12.75 | APEP |
| ATOM | 578 | N | PRO | 75 | -5.393 | 26.158 | 34.401 | 1.00 | 10.76 | APEP |
| ATOM | 579 | CD | PRO | 75 | -9.230 | 25.770 | 35.221 | 1.00 | 10.45 | APEP |
| ATOM | 580 | CA | PRO | 75 | -6.254 | 27.128 | 35.092 | 1.00 | 10.89 | APEP |
| ATOM | 581 | CB | PRO | 75 | -5.876 | 26.948 | 36.561 | 1.00 | 10.78 | APEP |
| ATOM | 582 | CG | PRO | 75 | -4.430 | 26.540 | 36.515 | 1.00 | 9.92 | APEP |
| ATOM | 583 | C | PRO | 75 | -6.077 | 28.571 | 34.642 | 1.00 | 10.22 | APEP |
| ATOM | 584 | O | PRO | 75 | -5.017 | 28.955 | 34.170 | 1.00 | 11.44 | APEP |
| ATOM | 585 | N | PRO | 76 | -7.123 | 29.394 | 34.782 | 1.00 | 11.88 | APEP |
| ATOM | 586 | CD | PRO | 76 | -8.461 | 29.107 | 35.327 | 1.00 | 13.85 | APEP |
| ATOM | 587 | CA | PRO | 76 | -6.968 | 30.791 | 34.365 | 1.00 | 13.97 | APEP |
| ATOM | 588 | CB | PRO | 76 | -8.383 | 31.373 | 34.440 | 1.00 | 12.73 | APEP |
| ATOM | 589 | CG | PRO | 76 | -9.284 | 30.245 | 34.830 | 1.00 | 14.35 | APEP |
| ATOM | 590 | C | PRO | 76 | -6.000 | 31.507 | 35.315 | 1.00 | 14.80 | APEP |
| ATOM | 591 | O | PRO | 76 | -5.672 | 30.987 | 36.382 | 1.00 | 14.45 | APEP |
| ATOM | 592 | N | ALA | 77 | -5.553 | 32.697 | 34.930 | 1.00 | 15.36 | APEP |
| ATOM | 593 | CA | ALA | 77 | -4.617 | 33.458 | 35.745 | 1.00 | 16.81 | APEP |
| ATOM | 594 | CB | ALA | 77 | -3.264 | 33.531 | 35.050 | 1.00 | 15.29 | APEP |
| ATOM | 595 | C | ALA | 77 | -5.111 | 34.864 | 36.034 | 1.00 | 18.21 | APEP |
| ATOM | 596 | O | ALA | 77 | -5.946 | 35.414 | 35.315 | 1.00 | 18.77 | APEP |
| ATOM | 597 | N | LYS | 78 | -4.578 | 35.447 | 37.095 | 1.00 | 19.69 | APEP |
| ATOM | 598 | CA | LYS | 78 | -4.944 | 36.799 | 37.487 | 1.00 | 21.45 | APEP |
| ATOM | 599 | CB | LYS | 78 | -5.453 | 36.779 | 38.934 | 1.00 | 19.28 | APEP |
| ATOM | 600 | CG | LYS | 78 | -5.408 | 38.109 | 39.658 | 1.00 | 21.07 | APEP |
| ATOM | 601 | CD | LYS | 78 | -5.939 | 37.969 | 41.078 | 1.00 | 22.93 | APEP |
| ATOM | 602 | CE | LYS | 78 | -7.039 | 38.993 | 41.380 | 1.00 | 22.07 | APEP |
| ATOM | 603 | NZ | LYS | 78 | -8.416 | 38.442 | 41.192 | 1.00 | 18.83 | APEP |
| ATOM | 604 | C | LYS | 78 | -3.681 | 37.655 | 37.351 | 1.00 | 20.82 | APEP |
| ATOM | 605 | O | LYS | 78 | -3.735 | 38.825 | 36.973 | 1.00 | 22.49 | APEP |
| ATOM | 606 | N | ASN | 79 | -2.546 | 37.024 | 37.632 | 1.00 | 20.62 | APEP |
| ATOM | 607 | CA | ASN | 79 | -1.225 | 37.650 | 37.596 | 1.00 | 20.96 | APEP |
| ATOM | 608 | CB | ASN | 79 | -0.322 | 36.893 | 38.591 | 1.00 | 21.73 | APEP |
| ATOM | 609 | CG | ASN | 79 | 0.895 | 37.696 | 39.041 | 1.00 | 26.41 | APEP |
| ATOM | 610 | OD1 | ASN | 79 | 1.739 | 37.194 | 39.794 | 1.00 | 27.31 | APEP |
| ATOM | 611 | ND2 | ASN | 79 | 0.995 | 38.941 | 38.586 | 1.00 | 30.92 | APEP |
| ATOM | 612 | C | ASN | 79 | -0.570 | 37.649 | 36.199 | 1.00 | 20.23 | APEP |
| ATOM | 613 | O | ASN | 79 | 0.658 | 37.679 | 36.109 | 1.00 | 20.51 | APEP |
| ATOM | 614 | N | MET | 80 | -1.354 | 37.648 | 35.117 | 1.00 | 17.31 | APEP |
| ATOM | 615 | CA | MET | 80 | -0.745 | 37.581 | 33.783 | 1.00 | 16.95 | APEP |
| ATOM | 616 | CB | MET | 80 | -1.334 | 36.398 | 33.014 | 1.00 | 14.01 | APEP |
| ATOM | 617 | CG | MET | 80 | -0.475 | 35.941 | 31.848 | 1.00 | 10.95 | APEP |
| ATOM | 618 | SD | MET | 80 | 1.001 | 35.032 | 32.360 | 1.00 | 10.66 | APEP |
| ATOM | 619 | CE | MET | 80 | 0.309 | 33.392 | 32.631 | 1.00 | 9.65 | APEP |
| ATOM | 620 | C | MET | 80 | -0.743 | 38.809 | 32.863 | 1.00 | 17.13 | APEP |
| ATOM | 621 | O | MET | 80 | -1.785 | 39.236 | 32.377 | 1.00 | 17.76 | APEP |
| ATOM | 622 | N | LYS | 81 | 0.450 | 39.343 | 32.602 | 1.00 | 16.19 | APEP |
| ATOM | 623 | CA | LYS | 81 | 0.621 | 40.509 | 31.734 | 1.00 | 15.79 | APEP |
| ATOM | 624 | CB | LYS | 81 | 1.360 | 41.626 | 32.480 | 1.00 | 19.28 | APEP |
| ATOM | 625 | CG | LYS | 81 | 0.485 | 42.479 | 33.376 | 1.00 | 23.69 | APEP |
| ATOM | 626 | CD | LYS | 81 | 1.283 | 42.976 | 34.581 | 1.00 | 29.37 | APEP |
| ATOM | 627 | CE | LYS | 81 | 0.652 | 42.551 | 35.914 | 1.00 | 30.79 | APEP |
| ATOM | 628 | NZ | LYS | 81 | 1.639 | 41.850 | 36.794 | 1.00 | 31.43 | APEP |
| ATOM | 629 | C | LYS | 81 | 1.428 | 40.135 | 30.489 | 1.00 | 15.20 | APEP |
| ATOM | 630 | O | LYS | 81 | 2.144 | 39.133 | 30.478 | 1.00 | 13.27 | APEP |
| ATOM | 631 | N | ASN | 82 | 1.317 | 40.947 | 29.445 | 1.00 | 14.40 | APEP |
| ATOM | 632 | CA | ASN | 82 | 2.047 | 40.695 | 28.214 | 1.00 | 14.06 | APEP |
| ATOM | 633 | CB | ASN | 82 | 1.442 | 41.492 | 27.059 | 1.00 | 15.84 | APEP |
| ATOM | 634 | CG | ASN | 82 | 0.081 | 40.970 | 26.636 | 1.00 | 19.06 | APEP |
| ATOM | 635 | OD1 | ASN | 82 | -0.837 | 41.746 | 26.366 | 1.00 | 20.37 | APEP |
| ATOM | 636 | ND2 | ASN | 82 | -0.058 | 39.649 | 26.579 | 1.00 | 20.55 | APEP |
| ATOM | 637 | C | ASN | 82 | 3.496 | 41.107 | 28.400 | 1.00 | 11.87 | APEP |
| ATOM | 638 | O | ASN | 82 | 3.800 | 41.968 | 29.226 | 1.00 | 11.67 | APEP |
| ATOM | 639 | N | LEU | 83 | 4.384 | 40.483 | 27.633 | 1.00 | 10.14 | APEP |
| ATOM | 640 | CA | LEU | 83 | 5.809 | 40.789 | 27.684 | 1.00 | 9.10 | APEP |
| ATOM | 641 | CB | LEU | 83 | 6.648 | 39.577 | 27.272 | 1.00 | 9.30 | APEP |
| ATOM | 642 | CG | LEU | 83 | 6.373 | 38.230 | 27.935 | 1.00 | 9.01 | APEP |
| ATOM | 643 | CD1 | LEU | 83 | 7.077 | 37.119 | 27.178 | 1.00 | 10.20 | APEP |
| ATOM | 644 | CD2 | LEU | 83 | 6.843 | 38.283 | 29.378 | 1.00 | 10.62 | APEP |
| ATOM | 645 | C | LEU | 83 | 6.104 | 41.919 | 26.718 | 1.00 | 7.69 | APEP |
| ATOM | 646 | O | LEU | 83 | 5.285 | 42.254 | 25.866 | 1.00 | 7.01 | APEP |
| ATOM | 647 | N | VAL | 84 | 7.277 | 42.516 | 26.878 | 1.00 | 7.90 | APEP |
| ATOM | 648 | CA | VAL | 84 | 7.736 | 43.585 | 26.004 | 1.00 | 9.09 | APEP |
| ATOM | 649 | CB | VAL | 84 | 7.888 | 44.947 | 26.765 | 1.00 | 9.60 | APEP |
| ATOM | 650 | CGL | VAL | 84 | 6.511 | 45.476 | 27.152 | 1.00 | 11.40 | APEP |
| ATOM | 651 | CG2 | VAL | 84 | 8.753 | 44.788 | 28.003 | 1.00 | 8.85 | APEP |
| ATOM | 652 | C | VAL | 84 | 9.088 | 43.110 | 25.479 | 1.00 | 8.16 | APEP |
| ATOM | 653 | O | VAL | 84 | 9.720 | 42.245 | 26.086 | 1.00 | 7.18 | APEP |
| ATOM | 654 | N | TRP | 85 | 9.524 | 43.637 | 24.343 | 1.00 | 9.08 | APEP |
| ATOM | 655 | CA | TRP | 85 | 10.807 | 43.222 | 23.801 | 1.00 | 8.24 | APEP |
| ATOM | 656 | CB | TRP | 85 | 10.844 | 43.412 | 22.283 | 1.00 | 7.78 | APEP |
| ATOM | 657 | CG | TRP | 85 | 12.054 | 42.789 | 21.623 | 1.00 | 7.96 | APEP |
| ATOM | 658 | CD2 | TRP | 85 | 12.162 | 41.460 | 21.092 | 1.00 | 6.06 | APEP |
| ATOM | 659 | CE2 | TRP | 85 | 13.459 | 41.330 | 20.544 | 1.00 | 5.27 | APEP |
| ATOM | 660 | CE3 | TRP | 85 | 11.290 | 40.366 | 21.023 | 1.00 | 5.63 | APEP |
| ATOM | 661 | CD1 | TRP | 85 | 13.260 | 43.392 | 21.384 | 1.00 | 6.75 | APEP |
| ATOM | 662 | NE1 | TRP | 85 | 14.104 | 42.522 | 20.737 | 1.00 | 5.69 | APEP |
| ATOM | 663 | CZ2 | TRP | 85 | 13.905 | 40.153 | 19.935 | 1.00 | 5.15 | APEP |
| ATOM | 664 | CZ3 | TRP | 85 | 11.736 | 39.192 | 20.414 | 1.00 | 3.85 | APEP |
| ATOM | 665 | CH2 | TRP | 85 | 13.035 | 39.099 | 19.879 | 1.00 | 3.87 | APEP |
| ATOM | 666 | C | TRP | 85 | 11.928 | 44.018 | 24.451 | 1.00 | 9.70 | APEP |
| ATOM | 667 | O | TRP | 85 | 11.790 | 45.214 | 24.713 | 1.00 | 12.63 | APEP |
| ATOM | 668 | N | ASN | 86 | 13.036 | 43.340 | 24.722 | 1.00 | 9.17 | APEP |
| ATOM | 669 | CA | ASN | 86 | 14.191 | 43.980 | 25.340 | 1.00 | 8.03 | APEP |
| ATOM | 670 | CB | ASN | 86 | 14.399 | 43.407 | 26.748 | 1.00 | 4.83 | APEP |
| ATOM | 671 | CG | ASN | 86 | 15.484 | 44.121 | 27.505 | 1.00 | 5.77 | APEP |
| ATOM | 672 | OD1 | ASN | 86 | 16.657 | 43.826 | 27.332 | 1.00 | 5.23 | APEP |
| ATOM | 673 | ND2 | ASN | 86 | 15.100 | 45.070 | 28.349 | 1.00 | 6.46 | APEP |
| ATOM | 674 | C | ASN | 86 | 15.450 | 43.789 | 24.474 | 1.00 | 7.66 | APEP |
| ATOM | 675 | O | ASN | 86 | 15.885 | 42.667 | 24.215 | 1.00 | 6.17 | APEP |
| ATOM | 676 | N | ASP | 87 | 16.028 | 44.899 | 24.030 | 1.00 | 8.98 | APEP |
| ATOM | 677 | CA | ASP | 87 | 17.213 | 44.866 | 23.179 | 1.00 | 9.94 | APEP |
| ATOM | 678 | CB | ASP | 87 | 17.548 | 46.278 | 22.695 | 1.00 | 10.21 | APEP |
| ATOM | 679 | CG | ASP | 87 | 16.602 | 46.757 | 21.622 | 1.00 | 9.93 | APEP |
| ATOM | 680 | OD1 | ASP | 87 | 16.065 | 45.902 | 20.901 | 1.00 | 10.59 | APEP |
| ATOM | 681 | OD2 | ASP | 87 | 16.392 | 47.980 | 21.498 | 1.00 | 11.15 | APEP |
| ATOM | 682 | C | ASP | 87 | 18.445 | 44.249 | 23.827 | 1.00 | 11.13 | APEP |
| ATOM | 683 | O | ASP | 87 | 19.271 | 43.651 | 23.141 | 1.00 | 11.97 | APEP |
| ATOM | 684 | N | GLU | 88 | 18.576 | 44.395 | 25.142 | 1.00 | 9.78 | APEP |
| ATOM | 685 | CA | GLU | 88 | 19.728 | 43.836 | 25.838 | 1.00 | 10.33 | APEP |
| ATOM | 686 | CB | GLU | 88 | 19.841 | 44.422 | 27.255 | 1.00 | 12.21 | APEP |
| ATOM | 687 | CG | GLU | 88 | 21.210 | 44.213 | 27.888 | 1.00 | 9.98 | APEP |
| ATOM | 688 | CD | GLU | 88 | 21.204 | 44.400 | 29.390 | 1.00 | 9.88 | APEP |
| ATOM | 689 | OE1 | GLU | 88 | 20.125 | 44.660 | 29.957 | 1.00 | 13.29 | APEP |
| ATOM | 690 | OE2 | GLU | 88 | 22.282 | 44.289 | 30.010 | 1.00 | 9.90 | APEP |
| ATOM | 691 | C | GLU | 88 | 19.660 | 42.314 | 25.912 | 1.00 | 9.04 | APEP |
| ATOM | 692 | O | GLU | 88 | 20.651 | 41.629 | 25.658 | 1.00 | 8.42 | APEP |
| ATOM | 693 | N | LEU | 89 | 18.491 | 41.789 | 26.269 | 1.00 | 8.21 | APEP |
| ATOM | 694 | CA | LEU | 89 | 18.305 | 40.343 | 26.367 | 1.00 | 7.65 | APEP |
| ATOM | 695 | CB | LEU | 89 | 16.881 | 40.016 | 26.824 | 1.00 | 7.64 | APEP |
| ATOM | 696 | CG | LEU | 89 | 16.499 | 40.394 | 28.254 | 1.00 | 6.63 | APEP |
| ATOM | 697 | CD1 | LEU | 89 | 15.111 | 39.904 | 28.549 | 1.00 | 5.53 | APEP |
| ATOM | 698 | CD2 | LEU | 89 | 17.487 | 39.785 | 29.237 | 1.00 | 7.49 | APEP |
| ATOM | 699 | C | LEU | 89 | 18.554 | 39.719 | 24.997 | 1.00 | 8.92 | APEP |
| ATOM | 700 | O | LEU | 89 | 19.214 | 38.689 | 24.885 | 1.00 | 7.56 | APEP |
| ATOM | 701 | N | ALA | 90 | 18.010 | 40.357 | 23.964 | 1.00 | 8.77 | APEP |
| ATOM | 702 | CA | ALA | 90 | 18.162 | 39.902 | 22.588 | 1.00 | 9.89 | APEP |
| ATOM | 703 | CB | ALA | 90 | 17.406 | 40.830 | 21.654 | 1.00 | 6.21 | APEP |
| ATOM | 704 | C | ALA | 90 | 19.640 | 39.849 | 22.197 | 1.00 | 9.83 | APEP |
| ATOM | 705 | O | ALA | 90 | 20.064 | 38.940 | 21.491 | 1.00 | 10.34 | APEP |
| ATOM | 706 | N | TYR | 91 | 20.415 | 40.821 | 22.672 | 1.00 | 10.22 | APEP |
| ATOM | 707 | CA | TYR | 91 | 21.846 | 40.894 | 22.380 | 1.00 | 10.21 | APEP |
| ATOM | 708 | CB | TYR | 91 | 22.426 | 42.203 | 22.921 | 1.00 | 11.27 | APEP |
| ATOM | 709 | CG | TYR | 91 | 23.921 | 42.329 | 22.730 | 1.00 | 13.72 | APEP |
| ATOM | 710 | CD1 | TYR | 91 | 24.458 | 42.653 | 21.487 | 1.00 | 14.77 | APEP |
| ATOM | 711 | CE1 | TYR | 91 | 25.837 | 42.747 | 21.301 | 1.00 | 16.90 | APEP |
| ATOM | 712 | CD2 | TYR | 91 | 24.802 | 42.104 | 23.788 | 1.00 | 14.30 | APEP |
| ATOM | 713 | CE2 | TYR | 91 | 26.178 | 42.195 | 23.614 | 1.00 | 14.06 | APEP |
| ATOM | 714 | CZ | TYR | 91 | 26.688 | 42.516 | 22.370 | 1.00 | 18.00 | APEP |
| ATOM | 715 | OH | TYR | 91 | 28.052 | 42.608 | 22.191 | 1.00 | 18.78 | APEP |
| ATOM | 716 | C | TYR | 91 | 22.620 | 39.714 | 22.967 | 1.00 | 11.02 | APEP |
| ATOM | 717 | O | TYR | 91 | 23.411 | 39.077 | 22.279 | 1.00 | 11.79 | APEP |
| ATOM | 718 | N | VAL | 92 | 22.397 | 39.432 | 24.244 | 1.00 | 10.38 | APEP |
| ATOM | 719 | CA | VAL | 92 | 23.075 | 38.325 | 24.903 | 1.00 | 8.66 | APEP |
| ATOM | 720 | CB | VAL | 92 | 22.785 | 38.319 | 26.427 | 1.00 | 8.13 | APEP |
| ATOM | 721 | CG1 | VAL | 92 | 23.488 | 37.142 | 2T.095 | 1.00 | 5.04 | APEP |
| ATOM | 722 | CG2 | VAL | 92 | 23.267 | 39.622 | 27.046 | 1.00 | 6.97 | APEP |
| ATOM | 723 | C | VAL | 92 | 22.634 | 37.002 | 24.286 | 1.00 | 9.57 | APEP |
| ATOM | 724 | O | VAL | 92 | 23.418 | 36.063 | 24.194 | 1.00 | 10.64 | APEP |
| ATOM | 725 | N | ALA | 93 | 21.376 | 36.933 | 23.858 | 1.00 | 9.31 | APEP |
| ATOM | 726 | CA | ALA | 93 | 20.854 | 35.722 | 23.238 | 1.00 | 9.67 | APEP |
| ATOM | 727 | CB | ALA | 93 | 19.349 | 35.848 | 23.030 | 1.00 | 8.26 | APEP |
| ATOM | 728 | C | ALA | 93 | 21.561 | 35.489 | 21.898 | 1.00 | 9.72 | APEP |
| ATOM | 729 | O | ALA | 93 | 21.954 | 34.366 | 21.581 | 1.00 | 10.89 | APEP |
| ATOM | 730 | N | GLN | 94 | 21.730 | 36.565 | 21.130 | 1.00 | 8.57 | APEP |
| ATOM | 731 | CA | GLN | 94 | 22.386 | 36.515 | 19.828 | 1.00 | 6.19 | APEP |
| ATOM | 732 | CB | GLN | 94 | 22.316 | 37.892 | 19.162 | 1.00 | 7.13 | APEP |
| ATOM | 733 | CG | GLN | 94 | 22.606 | 37.891 | 17.668 | 1.00 | 6.55 | APEP |
| ATOM | 734 | CD | GLN | 94 | 21.778 | 36.875 | 16.911 | 1.00 | 6.86 | APEP |
| ATOM | 735 | OE1 | GLN | 94 | 20.551 | 37.018 | 16.775 | 1.00 | 7.69 | APEP |
| ATOM | 736 | NE2 | GLN | 94 | 22.441 | 35.836 | 16.412 | 1.00 | 4.45 | APEP |
| ATOM | 737 | C | GLN | 94 | 23.843 | 36.082 | 19.946 | 1.00 | 7.41 | APEP |
| ATOM | 738 | O | GLN | 94 | 24.302 | 35.217 | 19.203 | 1.00 | 8.55 | APEP |
| ATOM | 739 | N | VAL | 95 | 24.574 | 36.699 | 20.868 | 1.00 | 7.81 | APEP |
| ATOM | 740 | CA | VAL | 95 | 25.971 | 36.357 | 21.089 | 1.00 | 6.18 | APEP |
| ATOM | 741 | CB | VAL | 95 | 26.551 | 37.112 | 22.327 | 1.00 | 8.06 | APEP |
| ATOM | 742 | CG1 | VAL | 95 | 27.899 | 36.523 | 22.728 | 1.00 | 8.13 | APEP |
| ATOM | 743 | CG2 | VAL | 95 | 26.716 | 38.583 | 22.011 | 1.00 | 7.34 | APEP |
| ATOM | 744 | C | VAL | 95 | 26.091 | 34.855 | 21.324 | 1.00 | 7.07 | APEP |
| ATOM | 745 | O | VAL | 95 | 26.949 | 34.199 | 20.737 | 1.00 | 3.91 | PEP |
| ATOM | 746 | N | TRP | 96 | 25.224 | 34.312 | 22.180 | 1.00 | 8.26 | APEP |
| ATOM | 747 | CA | TRP | 96 | 25.244 | 32.879 | 22.494 | 1.00 | 8.88 | APEP |
| ATOM | 748 | CB | TRP | 96 | 24.284 | 32.555 | 23.650 | 1.00 | 6.54 | APEP |
| ATOM | 749 | CG | TRP | 96 | 24.258 | 31.089 | 24.030 | 1.00 | 7.96 | APEP |
| ATOM | 750 | CD2 | TRP | 96 | 25.390 | 30.232 | 24.240 | 1.00 | 7.64 | APEP |
| ATOM | 751 | CE2 | TRP | 96 | 24.892 | 28.946 | 24.549 | 1.00 | 7.17 | APEP |
| ATOM | 752 | CE3 | TRP | 96 | 26.778 | 30.426 | 24.197 | 1.00 | 8.39 | APEP |
| ATOM | 753 | CD1 | TRP | 96 | 23.150 | 30.305 | 24.217 | 1.00 | 8.48 | APEP |
| ATOM | 754 | NE1 | TRP | 96 | 23.524 | 29.016 | 24.527 | 1.00 | 5.50 | APEP |
| ATOM | 755 | CZ2 | TRP | 96 | 25.734 | 27.859 | 24.812 | 1.00 | 6.91 | APEP |
| ATOM | 756 | CZ3 | TRP | 96 | 27.614 | 29.341 | 24.459 | 1.00 | 8.97 | APEP |
| ATOM | 757 | CH2 | TRP | 96 | 27.087 | 28.076 | 24.761 | 1.00 | 8.90 | APEP |
| ATOM | 758 | C | TRP | 96 | 24.867 | 32.033 | 21.281 | 1.00 | 8.85 | APEP |
| ATOM | 759 | O | TRP | 96 | 25.500 | 31.011 | 21.007 | 1.00 | 8.27 | APEP |
| ATOM | 760 | N | ALA | 97 | 23.827 | 32.453 | 20.566 | 1.00 | 7.57 | APEP |
| ATOM | 761 | CA | ALA | 97 | 23.390 | 31.721 | 19.381 | 1.00 | 9.88 | APEP |
| ATOM | 762 | CB | ALA | 97 | 22.182 | 32.415 | 18.742 | 1.00 | 4.36 | APEP |
| ATOM | 763 | C | ALA | 97 | 24.547 | 31.665 | 18.387 | 1.00 | 8.40 | APEP |
| ATOM | 764 | O | ALA | 97 | 24.777 | 30.647 | 17.734 | 1.00 | 8.69 | APEP |
| ATOM | 765 | N | ASN | 98 | 25.282 | 32.767 | 18.300 | 1.00 | 9.32 | APEP |
| ATOM | 766 | CA | ASN | 98 | 26.402 | 32.883 | 17.375 | 1.00 | 9.40 | APEP |
| ATOM | 767 | CB | ASN | 98 | 26.898 | 34.336 | 17.347 | 1.00 | 8.07 | APEP |
| ATOM | 768 | CG | ASN | 98 | 26.084 | 35.217 | 16.402 | 1.00 | 8.00 | APEP |
| ATOM | 769 | OD1 | ASN | 98 | 25.093 | 34.776 | 15.821 | 1.00 | 11.11 | APEP |
| ATOM | 770 | ND2 | ASN | 98 | 26.500 | 36.464 | 16.250 | 1.00 | 9.71 | APEP |
| ATOM | 771 | C | ASN | 98 | 27.568 | 31.926 | 17.647 | 1.00 | 9.78 | APEP |
| ATOM | 772 | O | ASN | 98 | 28.524 | 31.874 | 16.869 | 1.00 | 8.97 | APEP |
| ATOM | 773 | N | GLN | 99 | 27.492 | 31.160 | 18.733 | 1.00 | 8.27 | APEP |
| ATOM | 774 | CA | GLN | 99 | 28.556 | 30.212 | 19.051 | 1.00 | 9.27 | APEP |
| ATOM | 775 | CB | GLN | 99 | 28.774 | 30.120 | 20.572 | 1.00 | 10.68 | APEP |
| ATOM | 776 | CG | GLN | 99 | 29.117 | 31.452 | 21.241 | 1.00 | 9.08 | APEP |
| ATOM | 777 | CD | GLN | 99 | 30.119 | 32.266 | 20.444 | 1.00 | 10.60 | APEP |
| ATOM | 778 | OE1 | GLN | 99 | 31.195 | 31.780 | 20.107 | 1.00 | 11.69 | APEP |
| ATOM | 779 | NE2 | GLN | 99 | 29.772 | 33.511 | 20.146 | 1.00 | 12.17 | APEP |
| ATOM | 780 | C | GLN | 99 | 28.205 | 28.839 | 18.484 | 1.00 | 10.52 | APEP |
| ATOM | 781 | O | GLN | 99 | 29.049 | 27.942 | 18.426 | 1.00 | 11.67 | APEP |
| ATOM | 782 | N | CYS | 100 | 26.959 | 28.690 | 18.047 | 1.00 | 11.03 | APEP |
| ATOM | 783 | CA | CYS | 100 | 26.474 | 27.439 | 17.470 | 1.00 | 12.62 | APEP |
| ATOM | 784 | C | CYS | 100 | 26.711 | 26.234 | 18.373 | 1.00 | 14.10 | APEP |
| ATOM | 785 | O | CYS | 100 | 27.113 | 25.166 | 17.906 | 1.00 | 13.71 | APEP |
| ATOM | 786 | CB | CYS | 100 | 27.126 | 27.182 | 16.108 | 1.00 | 12.51 | APEP |
| ATOM | 787 | SG | CYS | 100 | 26.639 | 28.321 | 14.766 | 1.00 | 13.92 | APEP |
| ATOM | 788 | N | GLN | 101 | 26.457 | 26.411 | 19.667 | 1.00 | 13.78 | APEP |
| ATOM | 789 | CA | GLN | 101 | 26.615 | 25.337 | 20.640 | 1.00 | 14.58 | APEP |
| ATOM | 790 | CB | GLN | 101 | 27.656 | 25.723 | 21.696 | 1.00 | 16.78 | APEP |
| ATOM | 791 | CG | GLN | 101 | 29.106 | 25.506 | 21.269 | 1.00 | 19.68 | APEP |
| ATOM | 792 | CD | GLN | 101 | 30.097 | 26.125 | 22.239 | 1.00 | 20.61 | APEP |
| ATOM | 793 | OE1 | GLN | 101 | 31.113 | 26.690 | 21.833 | 1.00 | 23.08 | APEP |
| ATOM | 794 | NE2 | GLN | 101 | 29.802 | 26.023 | 23.530 | 1.00 | 24.57 | APEP |
| ATOM | 795 | C | GLN | 101 | 25.272 | 25.098 | 21.323 | 1.00 | 14.56 | APEP |
| ATOM | 796 | O | GLN | 101 | 24.987 | 25.716 | 22.347 | 1.00 | 14.71 | APEP |
| ATOM | 797 | N | TYR | 102 | 24.457 | 24.201 | 20.767 | 1.00 | 12.66 | APEP |
| ATOM | 798 | CA | TYR | 102 | 23.131 | 23.911 | 21.326 | 1.00 | 14.54 | APEP |
| ATOM | 799 | CB | TYR | 102 | 22.469 | 22.721 | 20.610 | 1.00 | 13.93 | APEP |
| ATOM | 800 | CG | TYR | 102 | 21.015 | 22.531 | 21.012 | 1.00 | 13.05 | APEP |
| ATOM | 801 | CD1 | TYR | 102 | 20.033 | 23.418 | 20.574 | 1.00 | 11.54 | APEP |
| ATOM | 802 | CE1 | TYR | 102 | 18.710 | 23.295 | 20.990 | 1.00 | 10.81 | APEP |
| ATOM | 803 | CD2 | TYR | 102 | 20.632 | 21.505 | 21.881 | 1.00 | 13.43 | APEP |
| ATOM | 804 | CE2 | TYR | 102 | 19.298 | 21.373 | 22.307 | 1.00 | 13.52 | APEP |
| ATOM. | 805 | CZ | TYR | 102 | 18.348 | 22.276 | 21.853 | 1.00 | 11.37 | APEP |
| ATOM | 806 | OH | TYR | 102 | 17.031 | 22.154 | 22.242 | 1.00 | 12.72 | APEP |
| ATOM | 807 | C | TYR | 102 | 23.123 | 23.636 | 22.824 | 1.00 | 14.75 | APEP |
| ATOM | 808 | O | TYR | 102 | 23.825 | 22.747 | 23.305 | 1.00 | 14.15 | APEP |
| ATOM | 809 | N | GLY | 103 | 22.303 | 24.399 | 23.548 | 1.00 | 15.34 | APEP |
| ATOM | 810 | CA | GLY | 103 | 22.194 | 24.241 | 24.988 | 1.00 | 13.83 | APEP |
| ATOM | 811 | C | GLY | 103 | 22.174 | 25.586 | 25.698 | 1.00 | 14.82 | APEP |
| ATOM | 812 | O | GLY | 103 | 22.051 | 26.627 | 25.050 | 1.00 | 13.51 | APEP |
| ATOM | 813 | N | HIS | 104 | 22.309 | 25.576 | 27.022 | 1.00 | 13.28 | APEP |
| ATOM | 814 | CA | HIS | 104 | 22.293 | 26.821 | 27.792 | 1.00 | 13.00 | APEP |
| ATOM | 815 | CB | HIS | 104 | 21.535 | 26.627 | 29.111 | 1.00 | 14.84 | APEP |
| ATOM | 816 | CG | HIS | 104 | 20.085 | 26.309 | 28.938 | 1.00 | 17.77 | APEP |
| ATOM | 817 | CD2 | HIS | 104 | 19.345 | 25.263 | 29.370 | 1.00 | 18.90 | APEP |
| ATOM | 818 | ND1 | HIS | 104 | 19.224 | 27.125 | 28.236 | 1.00 | 19.74 | APEP |
| ATOM | 819 | CE1 | HIS | 104 | 18.014 | 26.594 | 28.245 | 1.00 | 19.42 | APEP |
| ATOM | 820 | NE2 | HIS | 104 | 18.060 | 25.465 | 28.925 | 1.00 | 19.61 | APEP |
| ATOM | 821 | C | HIS | 104 | 23.689 | 27.322 | 28.116 | 1.00 | 10.17 | APEP |
| ATOM | 822 | O | HIS | 104 | 24.573 | 26.532 | 28.403 | 1.00 | 8.70 | APEP |
| ATOM | 823 | N | ASP | 105 | 23.890 | 28.635 | 28.058 | 1.00 | 10.86 | APEP |
| ATOM | 824 | CA | ASP | 105 | 25.188 | 29.197 | 28.417 | 1.00 | 12.92 | APEP |
| ATOM | 825 | CB | ASP | 105 | 25.400 | 30.590 | 27.794 | 1.00 | 10.99 | APEP |
| ATOM | 826 | CG | ASP | 105 | 24.172 | 31.463 | 27.875 | 1.00 | 11.97 | APEP |
| ATOM | 827 | OD1 | ASP | 105 | 23.054 | 30.914 | 27.966 | 1.00 | 14.08 | APEP |
| ATOM | 828 | OD2 | ASP | 105 | 24.324 | 32.705 | 27.844 | 1.00 | 11.83 | APEP |
| ATOM | 829 | C | ASP | 105 | 25.200 | 29.274 | 29.949 | 1.00 | 13.37 | APEP |
| ATOM | 830 | O | ASP | 105 | 24.145 | 29.250 | 30.592 | 1.00 | 13.12 | APEP |
| ATOM | 831 | N | THR | 106 | 26.395 | 29.361 | 30.522 | 1.00 | 14.55 | APEP |
| ATOM | 832 | CA | THR | 106 | 26.573 | 29.385 | 31.971 | 1.00 | 15.70 | APEP |
| ATOM | 833 | CB | THR | 106 | 28.032 | 29.051 | 32.322 | 1.00 | 17.02 | APEP |
| ATOM | 834 | OG1 | THR | 106 | 28.349 | 27.739 | 31.837 | 1.00 | 19.67 | APEP |
| ATOM | 835 | CG2 | THR | 106 | 28.244 | 29.101 | 33.815 | 1.00 | 19.92 | APEP |
| ATOM | 836 | C | THR | 106 | 26.181 | 30.661 | 32.712 | 1.00 | 14.86 | APEP |
| ATOM | 837 | O | THR | 106 | 25.648 | 30.598 | 33.826 | 1.00 | 14.67 | APEP |
| ATOM | 838 | N | CYS | 107 | 26.444 | 31.813 | 32.107 | 1.00 | 12.86 | APEP |
| ATOM | 839 | CA | CYS | 107 | 26.131 | 33.086 | 32.748 | 1.00 | 11.94 | APEP |
| ATOM | 840 | C | CYS | 107 | 25.608 | 34.112 | 31.741 | 1.00 | 11.28 | APEP |
| ATOM | 841 | O | CYS | 107 | 26.354 | 34.594 | 30.886 | 1.00 | 8.75 | APEP |
| ATOM | 842 | CB | CYS | 107 | 27.389 | 33.618 | 33.451 | 1.00 | 11.80 | APEP |
| ATOM | 843 | SG | CYS | 107 | 27.155 | 35.045 | 34.567 | 1.00 | 14.81 | APEP |
| ATOM | 844 | N | ARG | 108 | 24.324 | 34.448 | 31.857 | 1.00 | 10.42 | APEP |
| ATOM | 845 | CA | ARG | 108 | 23.694 | 35.408 | 30.956 | 1.00 | 10.25 | APEP |
| ATOM | 846 | CB | ARG | 108 | 22.656 | 34.703 | 30.080 | 1.00 | 7.60 | APEP |
| ATOM | 847 | CG | ARG | 108 | 21.299 | 34.525 | 30.746 | 1.00 | 6.23 | APEP |
| ATOM | 848 | CD | ARG | 108 | 20.458 | 33.460 | 30.047 | 1.00 | 4.46 | APEP |
| ATOM | 849 | NE | ARG | 108 | 21.066 | 32.136 | 30.118 | 1.00 | 8.96 | APEP |
| ATOM | 850 | CZ | ARG | 108 | 20.688 | 31.192 | 30.971 | 1.00 | 9.87 | APEP |
| ATOM | 851 | NH1 | ARG | 108 | 19.703 | 31.427 | 31.825 | 1.00 | 9.20 | APEP |
| ATOM | 852 | NH2 | ARG | 108 | 21.284 | 30.013 | 30.968 | 1.00 | 9.69 | APEP |
| ATOM | 853 | C | ARG | 108 | 23.015 | 36.575 | 31.667 | 1.00 | 10.62 | APEP |
| ATOM | 854 | O | ARG | 108 | 22.465 | 37.454 | 31.011 | 1.00 | 11.81 | APEP |
| ATOM | 855 | N | ASP | 109 | 23.051 | 36.583 | 32.998 | 1.00 | 10.19 | APEP |
| ATOM | 856 | CA | ASP | 109 | 22.421 | 37.644 | 33.784 | 1.00 | 9.53 | APEP |
| ATOM | 857 | CB | ASP | 109 | 22.737 | 37.457 | 35.267 | 1.00 | 11.00 | APEP |
| ATOM | 858 | CG | ASP | 109 | 22.049 | 36.248 | 35.864 | 1.00 | 10.30 | APEP |
| ATOM | 859 | OD1 | ASP | 109 | 21.137 | 35.704 | 35.213 | 1.00 | 8.82 | APEP |
| ATOM | 860 | OD2 | ASP | 109 | 22.420 | 35.839 | 36.984 | 1.00 | 12.03 | APEP |
| ATOM | 861 | C | ASP | 109 | 22.827 | 39.051 | 33.368 | 1.00 | 10.45 | APEP |
| ATOM | 862 | O | ASP | 109 | 23.931 | 39.274 | 32.878 | 1.00 | 11.18 | APEP |
| ATOM | 863 | N | VAL | 110 | 21.919 | 40.001 | 33.565 | 1.00 | 10.47 | APEP |
| ATOM | 864 | CA | VAL | 110 | 22.192 | 41.400 | 33.240 | 1.00 | 11.15 | APEP |
| ATOM | 865 | CB | VAL | 110 | 21.083 | 42.025 | 32.346 | 1.00 | 8.57 | APEP |
| ATOM | 866 | CG1 | VAL | 110 | 21.282 | 41.607 | 30.884 | 1.00 | 8.96 | APEP |
| ATOM | 867 | CG2 | VAL | 110 | 19.711 | 41.600 | 32.840 | 1.00 | 8.45 | APEP |
| ATOM | 868 | C | VAL | 110 | 22.263 | 42.168 | 34.564 | 1.00 | 12.39 | APEP |
| ATOM | 869 | O | VAL | 110 | 22.044 | 41.591 | 35.631 | 1.00 | 11.18 | APEP |
| ATOM | 870 | N | ALA | 111 | 22.567 | 43.460 | 34.493 | 1.00 | 12.60 | APEP |
| ATOM | 871 | CA | ALA | 111 | 22.670 | 44.283 | 35.691 | 1.00 | 14.41 | APEP |
| ATOM | 872 | CB | ALA | 111 | 23.192 | 45.665 | 35.329 | 1.00 | 14.98 | APEP |
| ATOM | 873 | C | ALA | 111 | 21.351 | 44.412 | 36.445 | 1.00 | 15.05 | APEP |
| ATOM | 874 | O | ALA | 111 | 21.348 | 44.491 | 37.665 | 1.00 | 17.05 | APEP |
| ATOM | 875 | N | LYS | 112 | 20.233 | 44.425 | 35.723 | 1.00 | 15.22 | APEP |
| ATOM | 876 | CA | LYS | 112 | 18.919 | 44.565 | 36.346 | 1.00 | 14.82 | APEP |
| ATOM | 877 | CB | LYS | 112 | 17.889 | 44.979 | 35.295 | 1.00 | 17.33 | APEP |
| ATOM | 878 | CG | LYS | 112 | 16.518 | 45.301 | 35.854 | 1.00 | 18.63 | APEP |
| ATOM | 879 | CD | LYS | 112 | 15.722 | 46.156 | 34.885 | 1.00 | 20.78 | APEP |
| ATOM | 880 | CE | LYS | 112 | 14.275 | 46.298 | 35.331 | 1.00 | 22.90 | APEP |
| ATOM | 881 | NZ | LYS | 112 | 13.378 | 46.775 | 34.230 | 1.00 | 24.78 | APEP |
| ATOM | 882 | C | LYS | 112 | 18.395 | 43.334 | 37.092 | 1.00 | 14.63 | APEP |
| ATOM | 883 | O | LYS | 112 | 17.763 | 43.462 | 38.138 | 1.00 | 15.19 | APEP |
| ATOM | 884 | N | TYR | 113 | 18.652 | 42.145 | 36.565 | 1.00 | 14.03 | APEP |
| ATOM | 885 | CA | TYR | 113 | 18.155 | 40.941 | 37.211 | 1.00 | 12.90 | APEP |
| ATOM | 886 | CB | TYR | 113 | 16.627 | 40.866 | 37.062 | 1.00 | 14.34 | APEP |
| ATOM | 887 | CG | TYR | 113 | 16.094 | 41.275 | 35.701 | 1.00 | 13.96 | APEP |
| ATOM | 888 | CD1 | TYR | 113 | 16.725 | 40.867 | 34.529 | 1.00 | 14.86 | APEP |
| ATOM | 889 | CE1 | TYR | 113 | 16.236 | 41.234 | 33.279 | 1.00 | 15.41 | APEP |
| ATOM | 890 | CD2 | TYR | 113 | 14.950 | 42.064 | 35.590 | 1.00 | 15.52 | APEP |
| ATOM | 891 | CE2 | TYR | 113 | 14.447 | 42.439 | 34.345 | 1.00 | 17.15 | APEP |
| ATOM | 892 | CZ | TYR | 113 | 15.098 | 42.021 | 33.192 | 1.00 | 18.32 | APEP |
| ATOM | 893 | OH | TYR | 113 | 14.619 | 42.406 | 31.958 | 1.00 | 20.11 | APEP |
| ATOM | 894 | C | TYR | 113 | 18.761 | 39.659 | 36.658 | 1.00 | 12.75 | APEP |
| ATOM | 895 | O | TYR | 113 | 19.592 | 39.685 | 35.742 | 1.00 | 9.39 | APEP |
| ATOM | 896 | N | GLN | 114 | 18.334 | 38.542 | 37.241 | 1.00 | 11.10 | APEP |
| ATOM | 897 | CA | GLN | 114 | 18.762 | 37.223 | 36.820 | 1.00 | 11.13 | APEP |
| ATOM | 898 | CB | GLN | 114 | 18.397 | 36.182 | 37.872 | 1.00 | 13.13 | APEP |
| ATOM | 899 | CG | GLN | 114 | 19.492 | 35.921 | 38.881 | 1.00 | 17.82 | APEP |
| ATOM | 900 | CD | GLN | 114 | 19.049 | 34.969 | 39.971 | 1.00 | 21.01 | APEP |
| ATOM | 901 | OE1 | GLN | 114 | 18.984 | 33.754 | 39.767 | 1.00 | 24.17 | APEP |
| ATOM | 902 | NE2 | GLN | 114 | 18.735 | 35.517 | 41.140 | 1.00 | 21.13 | APEP |
| ATOM | 903 | C | GLN | 114 | 17.969 | 36.977 | 35.549 | 1.00 | 10.99 | APEP |
| ATOM | 904 | O | GLN | 114 | 16.851 | 37.489 | 35.418 | 1.00 | 10.27 | APEP |
| ATOM | 905 | N | VAL | 115 | 18.529 | 36.195 | 34.626 | 1.00 | 8.78 | APEP |
| ATOM | 906 | CA | VAL | 115 | 17.879 | 35.936 | 33.339 | 1.00 | 6.98 | APEP |
| ATOM | 907 | CB | VAL | 115 | 18.679 | 36.628 | 32.204 | 1.00 | 8.14 | APEP |
| ATOM | 908 | CG1 | VAL | 115 | 18.037 | 36.358 | 30.868 | 1.00 | 9.82 | APEP |
| ATOM | 909 | CG2 | VAL | 115 | 18.750 | 38.125 | 32.461 | 1.00 | 6.98 | APEP |
| ATOM | 910 | C | VAL | 115 | 17.669 | 34.457 | 32.975 | 1.00 | 6.17 | APEP |
| ATOM | 911 | O | VAL | 115 | 18.581 | 33.634 | 33.093 | 1.00 | 4.78 | APEP |
| ATOM | 912 | N | GLY | 116 | 16.449 | 34.142 | 32.540 | 1.00 | 5.97 | APEP |
| ATOM | 913 | CA | GLY | 116 | 16.105 | 32.788 | 32.139 | 1.00 | 7.14 | APEP |
| ATOM | 914 | C | GLY | 116 | 16.364 | 32.568 | 30.654 | 1.00 | 7.72 | APEP |
| ATOM | 915 | O | GLY | 116 | 16.706 | 33.504 | 29.930 | 1.00 | 6.29 | APEP |
| ATOM | 916 | N | GLN | 117 | 16.195 | 31.337 | 30.186 | 1.00 | 8.62 | APEP |
| ATOM | 917 | CA | GLN | 117 | 16.456 | 31.058 | 28.780 | 1.00 | 9.69 | APEP |
| ATOM | 918 | CB | GLN | 117 | 17.980 | 31.001 | 28.550 | 1.00 | 8.96 | APEP |
| ATOM | 919 | CG | GLN | 117 | 18.419 | 30.465 | 27.179 | 1.00 | 8.07 | APEP |
| ATOM | 920 | CD | GLN | 117 | 19.935 | 30.331 | 27.046 | 1.00 | 7.90 | APEP |
| ATOM | 921 | OE1 | GLN | 117 | 20.507 | 29.288 | 27.360 | 1.00 | 10.49 | APEP |
| ATOM | 922 | NE2 | GLN | 117 | 20.586 | 31.386 | 26.575 | 1.00 | 7.02 | APEP |
| ATOM | 923 | C | GLN | 117 | 15.813 | 29.790 | 28.230 | 1.00 | 9.46 | APEP |
| ATOM | 924 | O | GLN | 117 | 15.713 | 28.775 | 28.920 | 1.00 | 8.69 | APEP |
| ATOM | 925 | N | ASN | 118 | 15.372 | 29.876 | 26.978 | 1.00 | 10.04 | APEP |
| ATOM | 926 | CA | ASN | 118 | 14.762 | 28.759 | 26.253 | 1.00 | 9.76 | APEP |
| ATOM | 927 | CB | ASN | 118 | 13.280 | 29.037 | 25.950 | 1.00 | 9.04 | APEP |
| ATOM | 928 | CG | ASN | 118 | 12.357 | 28.735 | 27.127 | 1.00 | 9.64 | APEP |
| ATOM | 929 | OD1 | ASN | 118 | 12.696 | 27.976 | 28.035 | 1.00 | 9.37 | APEP |
| ATOM | 930 | ND2 | ASN | 118 | 11.178 | 29.337 | 27.108 | 1.00 | 8.63 | APEP |
| ATOM | 931 | C | ASN | 118 | 15.526 | 28.674 | 24.926 | 1.00 | 9.88 | APEP |
| ATOM | 932 | O | ASN | 118 | 15.847 | 29.707 | 24.342 | 1.00 | 9.07 | APEP |
| ATOM | 933 | N | VAL | 119 | 15.836 | 27.464 | 24.465 | 1.00 | 10.24 | APEP |
| ATOM | 934 | CA | VAL | 119 | 16.533 | 27.288 | 23.188 | 1.00 | 9.82 | APEP |
| ATOM | 935 | CB | VAL | 119 | 18.021 | 26.811 | 23.340 | 1.00 | 9.70 | APEP |
| ATOM | 936 | CG1 | VAL | 119 | 18.764 | 27.684 | 24.349 | 1.00 | 11.24 | APEP |
| ATOM | 937 | CG2 | VAL | 119 | 18.072 | 25.344 | 23.749 | 1.00 | 11.10 | APEP |
| ATOM | 938 | C | VAL | 119 | 15.784 | 26.247 | 22.379 | 1.00 | 10.42 | APEP |
| ATOM | 939 | O | VAL | 119 | 15.116 | 25.380 | 22.939 | 1.00 | 7.84 | APEP |
| ATOM | 940 | N | ALA | 120 | 15.894 | 26.345 | 21.057 | 1.00 | 11.69 | APEP |
| ATOM | 941 | CA | ALA | 120 | 15.224 | 25.416 | 20.164 | 1.00 | 10.58 | APEP |
| ATOM | 942 | CB | ALA | 120 | 13.853 | 25.960 | 19.783 | 1.00 | 9.38 | APEP |
| ATOM | 943 | C | ALA | 120 | 16.065 | 25.203 | 18.913 | 1.00 | 11.91 | APEP |
| ATOM | 944 | O | ALA | 120 | 16.749 | 26.114 | 18.447 | 1.00 | 11.05 | APEP |
| ATOM | 945 | N | LEU | 121 | 16.005 | 23.999 | 18.363 | 1.00 | 11.80 | APEP |
| ATOM | 946 | CA | LEU | 121 | 16.762 | 23.707 | 17.164 | 1.00 | 10.62 | APEP |
| ATOM | 947 | CB | LEU | 121 | 18.219 | 23.423 | 17.534 | 1.00 | 12.00 | APEP |
| ATOM | 948 | CG | LEU | 121 | 19.162 | 23.065 | 16.383 | 1.00 | 14.58 | APEP |
| ATOM | 949 | CD1 | LEU | 121 | 19.914 | 24.310 | 15.937 | 1.00 | 14.77 | APEP |
| ATOM | 950 | CD2 | LEU | 121 | 20.124 | 21.975 | 16.830 | 1.00 | 16.44 | APEP |
| ATOM | 951 | C | LEU | 121 | 16.190 | 22.521 | 16.395 | 1.00 | 11.28 | APEP |
| ATOM | 952 | O | LEU | 121 | 15.744 | 21.540 | 16.989 | 1.00 | 8.33 | APEP |
| ATOM | 953 | N | THR | 122 | 16.183 | 22.633 | 15.069 | 1.00 | 9.47 | APEP |
| ATOM | 954 | CA | THR | 122 | 15.723 | 21.551 | 14.203 | 1.00 | 9.80 | APEP |
| ATOM | 955 | CB | THR | 122 | 14.282 | 21.766 | 13.691 | 1.00 | 8.73 | APEP |
| ATOM | 956 | OG1 | THR | 122 | 14.272 | 22.801 | 12.704 | 1.00 | 8.66 | APEP |
| ATOM | 957 | CG2 | THR | 122 | 13.357 | 22.133 | 14.838 | 1.00 | 11.47 | APEP |
| ATOM | 958 | C | THR | 122 | 16.666 | 21.502 | 13.009 | 1.00 | 9.64 | APEP |
| ATOM | 959 | O | THR | 122 | 17.232 | 22.524 | 12.616 | 1.00 | 9.15 | APEP |
| ATOM | 960 | N | GLY | 123 | 16.847 | 20.308 | 12.451 | 1.00 | 9.74 | APEP |
| ATOM | 961 | CA | GLY | 123 | 17.728 | 20.137 | 11.313 | 1.00 | 8.54 | APEP |
| ATOM | 962 | C | GLY | 123 | 17.048 | 19.326 | 10.228 | 1.00 | 8.95 | APEP |
| ATOM | 963 | O | GLY | 123 | 16.199 | 18.482 | 10.514 | 1.00 | 9.03 | APEP |
| ATOM | 964 | N | SER | 124 | 17.420 | 19.580 | 8.979 | 1.00 | 7.43 | APEP |
| ATOM | 965 | CA | SER | 124 | 16.824 | 18.874 | 7.857 | 1.00 | 9.14 | APEP |
| ATOM | 966 | CB | SER | 124 | 15.584 | 19.642 | 7.393 | 1.00 | 10.09 | APEP |
| ATOM | 967 | OG | SER | 124 | 15.333 | 19.459 | 6.016 | 1.00 | 11.96 | APEP |
| ATOM | 968 | C | SER | 124 | 17.827 | 18.718 | 6.709 | 1.00 | 9.54 | APEP |
| ATOM | 969 | O | SER | 124 | 18.716 | 19.551 | 6.537 | 1.00 | 10.56 | APEP |
| ATOM | 970 | N | THR | 125 | 17.693 | 17.641 | 5.936 | 1.00 | 10.06 | APEP |
| ATOM | 971 | CA | THR | 125 | 18.591 | 17.415 | 4.812 | 1.00 | 10.19 | APEP |
| ATOM | 972 | CB | THR | 125 | 18.513 | 15.974 | 4.257 | 1.00 | 11.20 | APEP |
| ATOM | 973 | OG1 | THR | 125 | 17.142 | 15.593 | 4.086 | 1.00 | 12.88 | APEP |
| ATOM | 974 | CG2 | THR | 125 | 19.218 | 15.001 | 5.191 | 1.00 | 8.70 | APEP |
| ATOM | 975 | C | THR | 125 | 18.274 | 18.369 | 3.676 | 1.00 | 9.96 | APEP |
| ATOM | 976 | O | THR | 125 | 19.081 | 18.532 | 2.772 | 1.00 | 10.12 | APEP |
| ATOM | 977 | N | ALA | 126 | 17.103 | 18.999 | 3.731 | 1.00 | 10.40 | APEP |
| ATOM | 978 | CA | ALA | 126 | 16.678 | 19.955 | 2.705 | 1.00 | 11.31 | APEP |
| ATOM | 979 | CB | ALA | 126 | 15.169 | 19.863 | 2.492 | 1.00 | 11.00 | APEP |
| ATOM | 980 | C | ALA | 126 | 17.060 | 21.383 | 3.086 | 1.00 | 13.19 | APEP |
| ATOM | 981 | O | ALA | 126 | 17.116 | 21.735 | 4.271 | 1.00 | 12.55 | APEP |
| ATOM | 982 | N | ALA | 127 | 17.314 | 22.207 | 2.078 | 1.00 | 12.78 | APEP |
| ATOM | 983 | CA | ALA | 127 | 17.700 | 23.590 | 2.315 | 1.00 | 15.72 | APEP |
| ATOM | 984 | CB | ALA | 127 | 18.471 | 24.135 | 1.106 | 1.00 | 15.36 | APEP |
| ATOM | 985 | C | ALA | 127 | 16.496 | 24.474 | 2.610 | 1.00 | 17.23 | APEP |
| ATOM | 986 | O | ALA | 127 | 16.080 | 25.271 | 1.773 | 1.00 | 17.44 | APEP |
| ATOM | 987 | N | LYS | 128 | 15.941 | 24.324 | 3.810 | 1.00 | 19.74 | APEP |
| ATOM | 988 | CA | LYS | 128 | 14.790 | 25.110 | 4.251 | 1.00 | 19.25 | APEP |
| ATOM | 989 | CB | LYS | 128 | 13.481 | 24.387 | 3.917 | 1.00 | 21.28 | APEP |
| ATOM | 990 | CG | LYS | 128 | 12.930 | 24.721 | 2.527 | 1.00 | 26.82 | APEP |
| ATOM | 991 | CD | LYS | 128 | 12.083 | 25.993 | 2.549 | 1.00 | 27.74 | APEP |
| ATOM | 992 | CE | LYS | 128 | 11.582 | 26.365 | 1.152 | 1.00 | 27.56 | APEP |
| ATOM | 993 | NZ | LYS | 128 | 10.376 | 27.258 | 1.191 | 1.00 | 24.53 | APEP |
| ATOM | 994 | C | LYS | 128 | 14.918 | 25.311 | 5.760 | 1.00 | 20.56 | APEP |
| ATOM | 995 | O | LYS | 128 | 15.299 | 24.384 | 6.488 | 1.00 | 18.95 | APEP |
| ATOM | 996 | N | TYR | 129 | 14.599 | 26.517 | 6.224 | 1.00 | 19.02 | APEP |
| ATOM | 997 | CA | TYR | 129 | 14.712 | 26.853 | 7.644 | 1.00 | 18.90 | APEP |
| ATOM | 998 | CB | TYR | 129 | 15.728 | 27.985 | 7.812 | 1.00 | 17.17 | APEP |
| ATOM | 999 | CG | TYR | 129 | 17.060 | 27.645 | 7.188 | 1.00 | 15.78 | APEP |
| ATOM | 1000 | CD1 | TYR | 129 | 17.319 | 27.934 | 5.847 | 1.00 | 15.45 | APEP |
| ATOM | 1001 | CE1 | TYR | 129 | 18.519 | 27.564 | 5.250 | 1.00 | 13.12 | APEP |
| ATOM | 1002 | CD2 | TYR | 129 | 18.043 | 26.984 | 7.918 | 1.00 | 16.01 | APEP |
| ATOM | 1003 | CE2 | TYR | 129 | 19.250 | 26.610 | 7.330 | 1.00 | 16.66 | APEP |
| ATOM | 1004 | CZ | TYR | 129 | 19.479 | 26.900 | 5.994 | 1.00 | 15.93 | APEP |
| ATOM | 1005 | OH | TYR | 129 | 20.652 | 26.495 | 5.404 | 1.00 | 11.93 | APEP |
| ATOM | 1006 | C | TYR | 129 | 13.384 | 27.213 | 8.312 | 1.00 | 18.94 | APEP |
| ATOM | 1007 | O | TYR | 129 | 12.574 | 27.980 | 7.775 | 1.00 | 19.85 | APEP |
| ATOM | 1008 | N | ASP | 130 | 13.178 | 26.645 | 9.496 | 1.00 | 17.23 | APEP |
| ATOM | 1009 | CA | ASP | 130 | 11.953 | 26.844 | 10.259 | 1.00 | 16.64 | APEP |
| ATOM | 1010 | CB | ASP | 130 | 12.012 | 26.050 | 11.568 | 1.00 | 18.70 | APEP |
| ATOM | 1011 | CG | ASP | 130 | 11.399 | 24.677 | 11.446 | 1.00 | 18.76 | APEP |
| ATOM | 1012 | OD1 | ASP | 130 | 11.067 | 24.267 | 10.319 | 1.00 | 17.38 | APEP |
| ATOM | 1013 | OD2 | ASP | 130 | 11.253 | 24.005 | 12.489 | 1.00 | 20.96 | APEP |
| ATOM | 1014 | C | ASP | 130 | 11.615 | 28.285 | 10.588 | 1.00 | 14.91 | APEP |
| ATOM | 1015 | O | ASP | 130 | 12.489 | 29.111 | 10.831 | 1.00 | 14.08 | APEP |
| ATOM | 1016 | N | ASP | 131 | 10.317 | 28.557 | 10.584 | 1.00 | 16.25 | APEP |
| ATOM | 1017 | CA | ASP | 131 | 9.759 | 29.858 | 10.911 | 1.00 | 16.60 | APEP |
| ATOM | 1018 | CB | ASP | 131 | 8.255 | 29.834 | 10.571 | 1.00 | 19.29 | APEP |
| ATOM | 1019 | CG | ASP | 131 | 7.558 | 31.166 | 10.807 | 1.00 | 29.36 | APEP |
| ATOM | 1020 | OD1 | ASP | 131 | 8.036 | 31.978 | 11.630 | 1.00 | 27.58 | APEP |
| ATOM | 1021 | OD2 | ASP | 131 | 6.506 | 31.396 | 10.168 | 1.00 | 28.19 | APEP |
| ATOM | 1022 | C | ASP | 131 | 9.993 | 29.961 | 12.428 | 1.00 | 15.04 | APEP |
| ATOM | 1023 | O | ASP | 131 | 9.708 | 29.012 | 13.159 | 1.00 | 14.34 | APEP |
| ATOM | 1024 | N | PRO | 132 | 10.534 | 31.092 | 12.910 | 1.00 | 12.77 | APEP |
| ATOM | 1025 | CD | PRO | 132 | 10.950 | 32.276 | 12.139 | 1.00 | 12.42 | APEP |
| ATOM | 1026 | CA | PRO | 132 | 10.788 | 31.253 | 14.354 | 1.00 | 13.80 | APEP |
| ATOM | 1027 | CB | PRO | 132 | 11.197 | 32.722 | 14.492 | 1.00 | 13.81 | APEP |
| ATOM | 1028 | CG | PRO | 132 | 11.711 | 33.104 | 13.149 | 1.00 | 14.43 | APEP |
| ATOM | 1029 | C | PRO | 132 | 9.592 | 30.895 | 15.251 | 1.00 | 13.34 | APEP |
| ATOM | 1030 | O | PRO | 132 | 9.758 | 30.239 | 16.278 | 1.00 | 12.55 | APEP |
| ATOM | 1031 | N | VAL | 133 | 8.396 | 31.325 | 14.850 | 1.00 | 12.80 | APEP |
| ATOM | 1032 | CA | VAL | 133 | 7.170 | 31.054 | 15.591 | 1.00 | 12.13 | APEP |
| ATOM | 1033 | CB | VAL | 133 | 5.944 | 31.661 | 14.862 | 1.00 | 11.80 | APEP |
| ATOM | 1034 | CG1 | VAL | 133 | 4.653 | 31.032 | 15.364 | 1.00 | 10.96 | APEP |
| ATOM | 1035 | CG2 | VAL | 133 | 5.923 | 33.159 | 15.064 | 1.00 | 13.44 | APEP |
| ATOM | 1036 | C | VAL | 133 | 6.964 | 29.549 | 15.744 | 1.00 | 13.10 | APEP |
| ATOM | 1037 | O | VAL | 133 | 6.452 | 29.083 | 16.763 | 1.00 | 11.95 | APEP |
| ATOM | 1038 | N | LYS | 134 | 7.361 | 28.796 | 14.721 | 1.00 | 13.08 | APEP |
| ATOM | 1039 | CA | LYS | 134 | 7.227 | 27.341 | 14.738 | 1.00 | 14.09 | APEP |
| ATOM | 1040 | CB | LYS | 134 | 7.594 | 26.756 | 13.374 | 1.00 | 14.78 | APEP |
| ATOM | 1041 | CG | LYS | 134 | 7.716 | 25.238 | 13.367 | 1.00 | 17.92 | APEP |
| ATOM | 1042 | CD | LYS | 134 | 7.273 | 24.661 | 12.024 | 1.00 | 20.75 | APEP |
| ATOM | 1043 | CE | LYS | 134 | 7.454 | 23.147 | 11.974 | 1.00 | 21.88 | APEP |
| ATOM | 1044 | NZ | LYS | 134 | 7.979 | 22.704 | 10.646 | 1.00 | 22.20 | APEP |
| ATOM | 1045 | C | LYS | 134 | 8.125 | 26.734 | 15.805 | 1.00 | 13.26 | APEP |
| ATOM | 1046 | O | LYS | 134 | 7.775 | 25.732 | 16.437 | 1.00 | 12.31 | APEP |
| ATOM | 1047 | N | LEU | 135 | 9.289 | 27.343 | 15.990 | 1.00 | 12.25 | APEP |
| ATOM | 1048 | CA | LEU | 135 | 10.245 | 26.883 | 16.987 | 1.00 | 12.29 | APEP |
| ATOM | 1049 | CB | LEU | 135 | 11.604 | 27.551 | 16.755 | 1.00 | 11.92 | APEP |
| ATOM | 1050 | CG | LEU | 135 | 12.371 | 26.968 | 15.563 | 1.00 | 12.40 | APEP |
| ATOM | 1051 | CD1 | LEU | 135 | 13.673 | 27.703 | 15.354 | 1.00 | 10.11 | APEP |
| ATOM | 1052 | CD2 | LEU | 135 | 12.633 | 25.492 | 15.816 | 1.00 | 13.69 | APEP |
| ATOM | 1053 | C | LEU | 135 | 9.711 | 27.222 | 18.371 | 1.00 | 11.79 | APEP |
| ATOM | 1054 | O | LEU | 135 | 9.862 | 26.443 | 19.311 | 1.00 | 12.52 | APEP |
| ATOM | 1055 | N | VAL | 136 | 9.070 | 28.378 | 18.492 | 1.00 | 11.76 | APEP |
| ATOM | 1056 | CA | VAL | 136 | 8.507 | 28.805 | 19.773 | 1.00 | 11.55 | APEP |
| ATOM | 1057 | CB | VAL | 136 | 7.926 | 30.236 | 19.674 | 1.00 | 9.15 | APEP |
| ATOM | 1058 | CG1 | VAL | 136 | 7.043 | 30.531 | 20.874 | 1.00 | 9.61 | APEP |
| ATOM | 1059 | CG2 | VAL | 136 | 9.053 | 31.247 | 19.587 | 1.00 | 5.53 | APEP |
| ATOM | 1060 | C | VAL | 136 | 7.405 | 27.836 | 20.227 | 1.00 | 12.73 | APEP |
| ATOM | 1061 | O | VAL | 136 | 7.370 | 27.421 | 21.385 | 1.00 | 11.68 | APEP |
| ATOM | 1062 | N | LYS | 137 | 6.521 | 27.477 | 19.298 | 1.00 | 13.14 | APEP |
| ATOM | 1063 | CA | LYS | 137 | 5.422 | 26.554 | 19.563 | 1.00 | 12.46 | APEP |
| ATOM | 1064 | CB | LYS | 137 | 4.562 | 26.376 | 18.307 | 1.00 | 12.12 | APEP |
| ATOM | 1065 | CG | LYS | 137 | 3.866 | 27.659 | 17.847 | 1.00 | 16.24 | APEP |
| ATOM | 1066 | CD | LYS | 137 | 2.763 | 27.391 | 16.836 | 1.00 | 13.34 | APEP |
| ATOM | 1067 | CE | LYS | 137 | 1.560 | 28.297 | 17.064 | 1.00 | 16.29 | APEP |
| ATOM | 1068 | NZ | LYS | 137 | 0.433 | 27.565 | 17.706 | 1.00 | 12.20 | APEP |
| ATOM | 1069 | C | LYS | 137 | 5.939 | 25.198 | 20.031 | 1.00 | 12.60 | APEP |
| ATOM | 1070 | O | LYS | 137 | 5.183 | 24.406 | 20.589 | 1.00 | 13.30 | APEP |
| ATOM | 1071 | N | MET | 138 | 7.220 | 24.924 | 19.797 | 1.00 | 12.70 | APEP |
| ATOM | 1072 | CA | MET | 138 | 7.807 | 23.662 | 20.240 | 1.00 | 15.80 | APEP |
| ATOM | 1073 | CB | MET | 138 | 9.266 | 23.545 | 19.779 | 1.00 | 17.09 | APEP |
| ATOM | 1074 | CG | MET | 138 | 9.478 | 22.767 | 18.482 | 1.00 | 21.36 | APEP |
| ATOM | 1075 | SD | MET | 138 | 11.111 | 23.089 | 17.711 | 1.00 | 26.01 | APEP |
| ATOM | 1076 | CE | MET | 138 | 12.066 | 21.673 | 18.272 | 1.00 | 23.00 | APEP |
| ATOM | 1077 | C | MET | 138 | 7.755 | 23.665 | 21.768 | 1.00 | 15.35 | APEP |
| ATOM | 1078 | O | MET | 138 | 7.447 | 22.650 | 22.395 | 1.00 | 15.78 | APEP |
| ATOM | 1079 | N | TRP | 139 | 8.069 | 24.824 | 22.346 | 1.00 | 13.56 | APEP |
| ATOM | 1080 | CA | TRP | 139 | 8.069 | 25.035 | 23.791 | 1.00 | 10.21 | APEP |
| ATOM | 1081 | CB | TRP | 139 | 8.700 | 26.395 | 24.122 | 1.00 | 6.88 | APEP |
| ATOM | 1082 | CG | TRP | 139 | 10.112 | 26.582 | 23.589 | 1.00 | 7.62 | APEP |
| ATOM | 1083 | CD2 | TRP | 139 | 10.746 | 27.821 | 23.220 | 1.00 | 4.45 | APEP |
| ATOM | 1084 | CE2 | TRP | 139 | 12.051 | 27.507 | 22.784 | 1.00 | 4.04 | APEP |
| ATOM | 1085 | CE3 | TRP | 139 | 10.335 | 29.160 | 23.214 | 1.00 | 4.82 | APEP |
| ATOM | 1086 | CD1 | TRP | 139 | 11.037 | 25.606 | 23.367 | 1.00 | 6.61 | APEP |
| ATOM | 1087 | NE1 | TRP | 139 | 12.203 | 26.151 | 22.886 | 1.00 | 5.06 | APEP |
| ATOM | 1088 | CZ2 | TRP | 139 | 12.955 | 28.490 | 22.347 | 1.00 | 2.98 | APEP |
| ATOM | 1089 | CZ3 | TRP | 139 | 11.229 | 30.137 | 22.778 | 1.00 | 2.00 | APEP |
| ATOM | 1090 | CH2 | TRP | 139 | 12.525 | 29.795 | 22.351 | 1.00 | 4.18 | APEP |
| ATOM | 1091 | C | TRP | 139 | 6.628 | 24.997 | 24.312 | 1.00 | 11.53 | APEP |
| ATOM | 1092 | O | TRP | 139 | 6.350 | 24.419 | 25.365 | 1.00 | 11.91 | APEP |
| ATOM | 1093 | N | GLU | 140 | 5.723 | 25.622 | 23.557 | 1.00 | 11.23 | APEP |
| ATOM | 1094 | CA | GLU | 140 | 4.306 | 25.690 | 23.890 | 1.00 | 10.95 | APEP |
| ATOM | 1095 | CB | GLU | 140 | 3.538 | 26.427 | 22.798 | 1.00 | 9.60 | APEP |
| ATOM | 1096 | CG | GLU | 140 | 3.622 | 27.919 | 22.834 | 1.00 | 7.49 | APEP |
| ATOM | 1097 | CD | GLU | 140 | 2.893 | 28.544 | 21.666 | 1.00 | 8.80 | APEP |
| ATOM | 1098 | OE1 | GLU | 140 | 1.937 | 27.921 | 21.150 | 1.00 | 11.85 | APEP |
| ATOM | 1099 | OE2 | GLU | 140 | 3.277 | 29.654 | 21.259 | 1.00 | 12.42 | APEP |
| ATOM | 1100 | C | GLU | 140 | 3.672 | 24.321 | 24.038 | 1.00 | 11.65 | APEP |
| ATOM | 1101 | O | GLU | 140 | 2.891 | 24.089 | 24.960 | 1.00 | 13.93 | APEP |
| ATOM | 1102 | N | ASP | 141 | 3.993 | 23.423 | 23.112 | 1.00 | 12.05 | APEP |
| ATOM | 1103 | CA | ASP | 141 | 3.433 | 22.078 | 23.106 | 1.00 | 13.22 | APEP |
| ATOM | 1104 | CB | ASP | 141 | 3.850 | 21.346 | 21.833 | 1.00 | 12.72 | APEP |
| ATOM | 1105 | CG | ASP | 141 | 3.200 | 21.923 | 20.601 | 1.00 | 13.07 | APEP |
| ATOM | 1106 | OD1 | ASP | 141 | 2.240 | 22.706 | 20.747 | 1.00 | 12.10 | APEP |
| ATOM | 1107 | OD2 | ASP | 141 | 3.646 | 21.599 | 19.484 | 1.00 | 16.74 | APEP |
| ATOM | 1108 | C | ASP | 141 | 3.782 | 21.235 | 24.320 | 1.00 | 13.83 | APEP |
| ATOM | 1109 | O | ASP | 141 | 3.199 | 20.172 | 24.530 | 1.00 | 13.83 | APEP |
| ATOM | 1110 | N | GLU | 142 | 4.726 | 21.705 | 25.124 | 1.00 | 14.37 | APEP |
| ATOM | 1111 | CA | GLU | 142 | 5.110 | 20.974 | 26.323 | 1.00 | 13.86 | APEP |
| ATOM | 1112 | CB | GLU | 142 | 6.335 | 21.626 | 26.974 | 1.00 | 13.22 | APEP |
| ATOM | 1113 | CG | GLU | 142 | 7.619 | 21.449 | 26.158 | 1.00 | 13.31 | APEP |
| ATOM | 1114 | CD | GLU | 142 | 8.866 | 21.896 | 26.889 | 1.00 | 11.68 | APEP |
| ATOM | 1115 | OE1 | GLU | 142 | 8.749 | 22.706 | 27.829 | 1.00 | 14.70 | APEP |
| ATOM | 1116 | OE2 | GLU | 142 | 9.968 | 21.439 | 26.523 | 1.00 | 10.37 | APEP |
| ATOM | 1117 | C | GLU | 142 | 3.937 | 20.957 | 27.301 | 1.00 | 14.65 | APEP |
| ATOM | 1118 | O | GLU | 142 | 3.819 | 20.049 | 28.120 | 1.00 | 16.37 | APEP |
| ATOM | 1119 | N | VAL | 143 | 3.063 | 21.954 | 27.197 | 1.00 | 14.52 | APEP |
| ATOM | 1120 | CA | VAL | 143 | 1.904 | 22.071 | 28.084 | 1.00 | 14.56 | APEP |
| ATOM | 1121 | CB | VAL | 143 | 0.970 | 23.221 | 27.644 | 1.00 | 14.44 | APEP |
| ATOM | 1122 | CG1 | VAL | 143 | 0.210 | 22.834 | 26.376 | 1.00 | 12.33 | APEP |
| ATOM | 1123 | CG2 | VAL | 143 | -0.005 | 23.548 | 28.769 | 1.00 | 10.75 | APEP |
| ATOM | 1124 | C | VAL | 143 | 1.057 | 20.809 | 28.237 | 1.00 | 16.78 | APEP |
| ATOM | 1125 | O | VAL | 143 | 0.399 | 20.631 | 29.258 | 1.00 | 16.28 | APEP |
| ATOM | 1126 | N | LYS | 144 | 1.059 | 19.942 | 27.228 | 1.00 | 17.87 | APEP |
| ATOM | 1127 | CA | LYS | 144 | 0.281 | 18.706 | 27.293 | 1.00 | 19.21 | APEP |
| ATOM | 1128 | CB | LYS | 144 | 0.257 | 18.025 | 25.913 | 1.00 | 21.23 | APEP |
| ATOM | 1129 | CG | LYS | 144 | 1.602 | 17.471 | 25.452 | 1.00 | 23.20 | APEP |
| ATOM | 1130 | CD | LYS | 144 | 1.578 | 15.949 | 25.346 | 1.00 | 25.43 | APEP |
| ATOM | 1131 | CE | LYS | 144 | 2.739 | 15.423 | 24.506 | 1.00 | 25.49 | APEP |
| ATOM | 1132 | NZ | LYS | 144 | 2.960 | 16.244 | 23.282 | 1.00 | 29.96 | APEP |
| ATOM | 1133 | C | LYS | 144 | 0.852 | 17.746 | 28.350 | 1.00 | 18.76 | APEP |
| ATOM | 1134 | O | LYS | 144 | 0.188 | 16.794 | 28.776 | 1.00 | 18.94 | APEP |
| ATOM | 1135 | N | ASP | 145 | 2.080 | 18.008 | 28.774 | 1.00 | 17.39 | APEP |
| ATOM | 1136 | CA | ASP | 145 | 2.743 | 17.180 | 29.778 | 1.00 | 18.35 | APEP |
| ATOM | 1137 | CB | ASP | 145 | 4.199 | 16.916 | 29.364 | 1.00 | 20.30 | APEP |
| ATOM | 1138 | CG | ASP | 145 | 4.316 | 15.942 | 28.195 | 1.00 | 20.35 | APEP |
| ATOM | 1139 | OD1 | ASP | 145 | 3.374 | 15.153 | 27.959 | 1.00 | 22.15 | APEP |
| ATOM | 1140 | OD2 | ASP | 145 | 5.359 | 15.966 | 27.510 | 1.00 | 21.64 | APEP |
| ATOM | 1141 | C | ASP | 145 | 2.714 | 17.829 | 31.173 | 1.00 | 17.37 | APEP |
| ATOM | 1142 | O | ASP | 145 | 3.069 | 17.192 | 32.164 | 1.00 | 15.03 | APEP |
| ATOM | 1143 | N | TYR | 146 | 2.284 | 19.090 | 31.240 | 1.00 | 17.14 | APEP |
| ATOM | 1144 | CA | TYR | 146 | 2.200 | 19.821 | 32.506 | 1.00 | 15.39 | APEP |
| ATOM | 1145 | CB | TYR | 146 | 2.368 | 21.320 | 32.264 | 1.00 | 14.76 | APEP |
| ATOM | 1146 | CG | TYR | 146 | 2.696 | 22.071 | 33.533 | 1.00 | 15.28 | APEP |
| ATOM | 1147 | CD1 | TYR | 146 | 3.992 | 22.053 | 34.065 | 1.00 | 14.62 | APEP |
| ATOM | 1148 | CE1 | TYR | 146 | 4.286 | 22.682 | 35.277 | 1.00 | 13.76 | APEP |
| ATOM | 1149 | CD2 | TYR | 146 | 1.705 | 22.743 | 34.242 | 1.00 | 14.99 | APEP |
| ATOM | 1150 | CE2 | TYR | 146 | 1.991 | 23.377 | 35.457 | 1.00 | 13.01 | APEP |
| ATOM | 1151 | CZ | TYR | 146 | 3.281 | 23.340 | 35.964 | 1.00 | 12.87 | APEP |
| ATOM | 1152 | OH | TYR | 146 | 3.563 | 23.958 | 37.162 | 1.00 | 12.82 | APEP |
| ATOM | 1153 | C | TYR | 146 | 0.906 | 19.580 | 33.294 | 1.00 | 16.31 | APEP |
| ATOM | 1154 | O | TYR | 146 | -0.202 | 19.837 | 32.804 | 1.00 | 15.92 | APEP |
| ATOM | 1155 | N | ASN | 147 | 1.062 | 19.097 | 34.525 | 1.00 | 15.90 | APEP |
| ATOM | 1156 | CA | ASN | 147 | -0.066 | 18.792 | 35.415 | 1.00 | 17.53 | APEP |
| ATOM | 1157 | CB | ASN | 147 | 0.265 | 17.551 | 36.248 | 1.00 | 17.07 | APEP |
| ATOM | 1158 | CG | ASN | 147 | -0.851 | 17.172 | 37.193 | 1.00 | 18.14 | APEP |
| ATOM | 1159 | OD1 | ASN | 147 | -1.885 | 17.835 | 37.242 | 1.00 | 19.58 | APEP |
| ATOM | 1160 | ND2 | ASN | 147 | -0.651 | 16.096 | 37.949 | 1.00 | 15.35 | APEP |
| ATOM | 1161 | C | ASN | 147 | -0.405 | 19.957 | 36.355 | 1.00 | 17.15 | APEP |
| ATOM | 1162 | O | ASN | 147 | 0.289 | 20.196 | 37.334 | 1.00 | 16.44 | APEP |
| ATOM | 1163 | N | PRO | 148 | -1.499 | 20.677 | 36.082 | 1.00 | 17.84 | APEP |
| ATOM | 1164 | CD | PRO | 148 | -2.462 | 20.508 | 34.981 | 1.00 | 16.74 | APEP |
| ATOM | 1165 | CA | PRO | 148 | -1.854 | 21.805 | 36.951 | 1.00 | 19.69 | APEP |
| ATOM | 1166 | CB | PRO | 148 | -2.982 | 22.504 | 36.189 | 1.00 | 17.29 | APEP |
| ATOM | 1167 | CG | PRO | 148 | -3.588 | 21.436 | 35.367 | 1.00 | 17.85 | APEP |
| ATOM | 1168 | C | PRO | 148 | -2.246 | 21.467 | 38.395 | 1.00 | 21.26 | APEP |
| ATOM | 1169 | O | PRO | 148 | -2.040 | 22.285 | 39.289 | 1.00 | 22.85 | APEP |
| ATOM | 1170 | N | LYS | 149 | -2.802 | 20.275 | 38.624 | 1.00 | 23.35 | APEP |
| ATOM | 1171 | CA | LYS | 149 | -3.219 | 19.862 | 39.970 | 1.00 | 25.52 | APEP |
| ATOM | 1172 | CB | LYS | 149 | -4.006 | 18.546 | 39.917 | 1.00 | 25.99 | APEP |
| ATOM | 1173 | CG | LYS | 149 | -5.036 | 18.477 | 38.800 | 1.00 | 29.64 | APEP |
| ATOM | 1174 | CD | LYS | 149 | -6.438 | 18.800 | 39.307 | 1.00 | 30.49 | APEP |
| ATOM | 1175 | CE | LYS | 149 | -7.162 | 19.775 | 38.382 | 1.00 | 30.51 | APEP |
| ATOM | 1176 | NZ | LYS | 149 | -8.401 | 20.351 | 39.004 | 1.00 | 30.17 | APEP |
| ATOM | 1177 | C | LYS | 149 | -2.041 | 19.704 | 40.929 | 1.00 | 26.61 | APEP |
| ATOM | 1178 | O | LYS | 149 | -2.153 | 19.082 | 41.993 | 1.00 | 27.31 | APEP |
| ATOM | 1179 | N | LYS | 150 | -0.905 | 20.271 | 40.559 | 1.00 | 26.69 | APEP |
| ATOM | 1180 | CA | LYS | 150 | 0.262 | 20.186 | 41.408 | 1.00 | 27.83 | APEP |
| ATOM | 1181 | CB | LYS | 150 | 0.904 | 18.795 | 41.260 | 1.00 | 26.22 | APEP |
| ATOM | 1182 | CG | LYS | 150 | 2.205 | 18.718 | 40.495 | 1.00 | 25.69 | APEP |
| ATOM | 1183 | CD | LYS | 150 | 2.416 | 17.320 | 39.908 | 1.00 | 25.52 | APEP |
| ATOM | 1184 | CE | LYS | 150 | 2.140 | 16.214 | 40.922 | 1.00 | 24.65 | APEP |
| ATOM | 1185 | NZ | LYS | 150 | 0.695 | 15.847 | 40.971 | 1.00 | 23.30 | APEP |
| ATOM | 1186 | C | LYS | 150 | 1.218 | 21.320 | 41.062 | 1.00 | 29.22 | APEP |
| ATOM | 1187 | O | LYS | 150 | 1.504 | 21.577 | 39.895 | 1.00 | 31.30 | APEP |
| ATOM | 1188 | N | LYS | 151 | 1.681 | 22.023 | 42.088 | 1.00 | 30.63 | APEP |
| ATOM | 1189 | CA | LYS | 151 | 2.581 | 23.148 | 41.896 | 1.00 | 29.98 | APEP |
| ATOM | 1190 | CB | LYS | 151 | 3.134 | 23.622 | 43.244 | 1.00 | 30.75 | APEP |
| ATOM | 1191 | CG | LYS | 151 | 2.308 | 24.738 | 43.888 | 1.00 | 32.85 | APEP |
| ATOM | 1192 | CD | LYS | 151 | 2.605 | 26.093 | 43.246 | 1.00 | 32.11 | APEP |
| ATOM | 1193 | CE | LYS | 151 | 1.512 | 27.104 | 43.562 | 1.00 | 30.61 | APEP |
| ATOM | 1194 | NZ | LYS | 151 | 2.061 | 28.331 | 44.196 | 1.00 | 27.07 | APEP |
| ATOM | 1195 | C | LYS | 151 | 3.720 | 22.801 | 40.956 | 1.00 | 28.92 | APEP |
| ATOM | 1196 | O | LYS | 151 | 3.984 | 21.633 | 40.685 | 1.00 | 28.09 | APEP |
| ATOM | 1197 | N | PHE | 152 | 4.377 | 23.842 | 40.460 | 1.00 | 28.09 | APEP |
| ATOM | 1198 | CA | PHE | 152 | 5.494 | 23.719 | 39.539 | 1.00 | 27.23 | APEP |
| ATOM | 1199 | CB | PHE | 152 | 6.138 | 25.098 | 39.349 | 1.00 | 23.04 | APEP |
| ATOM | 1200 | CG | PHE | 152 | 7.486 | 25.064 | 38.687 | 1.00 | 21.73 | APEP |
| ATOM | 1201 | CD1 | PHE | 152 | 7.595 | 24.951 | 37.307 | 1.00 | 19.77 | APEP |
| ATOM | 1202 | CD2 | PHE | 152 | 8.646 | 25.171 | 39.442 | 1.00 | 21.08 | APEP |
| ATOM | 1203 | CE1 | PHE | 152 | 8.833 | 24.948 | 36.688 | 1.00 | 18.18 | APEP |
| ATOM | 1204 | CE2 | PHE | 152 | 9.894 | 25.169 | 38.832 | 1.00 | 20.33 | APEP |
| ATOM | 1205 | CZ | PHE | 152 | 9.986 | 25.057 | 37.447 | 1.00 | 20.45 | APEP |
| ATOM | 1206 | C | PHE | 152 | 6.543 | 22.708 | 39.996 | 1.00 | 28.98 | APEP |
| ATOM | 1207 | O | PHE | 152 | 6.821 | 21.736 | 39.293 | 1.00 | 29.79 | APEP |
| ATOM | 1208 | N | SER | 153 | 7.112 | 22.940 | 41.176 | 1.00 | 30.67 | APEP |
| ATOM | 1209 | CA | SER | 153 | 8.162 | 22.084 | 41.735 | 1.00 | 32.34 | APEP |
| ATOM | 1210 | CB | SER | 153 | 8.313 | 22.357 | 43.234 | 1.00 | 33.66 | APEP |
| ATOM | 1211 | OG | SER | 153 | 9.539 | 21.834 | 43.713 | 1.00 | 35.63 | APEP |
| ATOM | 1212 | C | SER | 153 | 7.990 | 20.581 | 41.522 | 1.00 | 31.88 | APEP |
| ATOM | 1213 | O | SER | 153 | 8.977 | 19.859 | 41.342 | 1.00 | 29.52 | APEP |
| ATOM | 1214 | N | GLY | 154 | 6.744 | 20.113 | 41.547 | 1.00 | 32.09 | APEP |
| ATOM | 1215 | CA | GLY | 154 | 6.488 | 18.694 | 41.373 | 1.00 | 31.42 | APEP |
| ATOM | 1216 | C | GLY | 154 | 6.124 | 18.282 | 39.962 | 1.00 | 31.52 | APEP |
| ATOM | 1217 | O | GLY | 154 | 5.317 | 17.371 | 39.771 | 1.00 | 31.82 | APEP |
| ATOM | 1218 | N | ASN | 155 | 6.719 | 18.941 | 38.973 | 1.00 | 30.65 | APEP |
| ATOM | 1219 | CA | ASN | 155 | 6.448 | 18.635 | 37.573 | 1.00 | 28.76 | APEP |
| ATOM | 1220 | CB | ASN | 155 | 5.796 | 19.842 | 36.893 | 1.00 | 27.69 | APEP |
| ATOM | 1221 | CG | ASN | 155 | 4.332 | 19.614 | 36.579 | 1.00 | 26.54 | APEP |
| ATOM | 1222 | OD1 | ASN | 155 | 3.991 | 18.873 | 35.652 | 1.00 | 25.37 | APEP |
| ATOM | 1223 | ND2 | ASN | 155 | 3.455 | 20.248 | 37.354 | 1.00 | 23.70 | APEP |
| ATOM | 1224 | C | ASN | 155 | 7.729 | 18.257 | 36.833 | 1.00 | 29.00 | APEP |
| ATOM | 1225 | O | ASN | 155 | 8.828 | 18.636 | 37.242 | 1.00 | 28.36 | APEP |
| ATOM | 1226 | N | ASP | 156 | 7.580 | 17.507 | 35.744 | 1.00 | 28.55 | APEP |
| ATOM | 1227 | CA | ASP | 156 | 8.714 | 17.071 | 34.933 | 1.00 | 28.23 | APEP |
| ATOM | 1228 | CB | ASP | 156 | 8.219 | 16.108 | 33.848 | 1.00 | 29.34 | APEP |
| ATOM | 1229 | CG | ASP | 156 | 9.251 | 15.058 | 33.474 | 1.00 | 30.85 | APEP |
| ATOM | 1230 | OD1 | ASP | 156 | 8.915 | 13.855 | 33.515 | 1.00 | 30.64 | APEP |
| ATOM | 1231 | OD2 | ASP | 156 | 10.396 | 15.434 | 33.133 | 1.00 | 32.06 | APEP |
| ATOM | 1232 | C | ASP | 156 | 9.399 | 18.281 | 34.284 | 1.00 | 27.76 | APEP |
| ATOM | 1233 | O | ASP | 156 | 8.971 | 18.746 | 33.230 | 1.00 | 27.49 | APEP |
| ATOM | 1234 | N | PHE | 157 | 10.464 | 18.789 | 34.897 | 1.00 | 28.19 | APEP |
| ATOM | 1235 | CA | PHE | 157 | 11.129 | 19.951 | 34.326 | 1.00 | 29.10 | APEP |
| ATOM | 1236 | CB | PHE | 157 | 11.963 | 20.705 | 35.387 | 1.00 | 32.07 | APEP |
| ATOM | 1237 | CG | PHE | 157 | 13.062 | 19.893 | 36.038 | 1.00 | 35.87 | APEP |
| ATOM | 1238 | CD1 | PHE | 157 | 14.226 | 19.555 | 35.330 | 1.00 | 36.99 | APEP |
| ATOM | 1239 | CD2 | PHE | 157 | 12.982 | 19.555 | 37.397 | 1.00 | 35.92 | APEP |
| ATOM | 1240 | CE1 | PHE | 157 | 15.297 | 18.901 | 35.966 | 1.00 | 36.12 | APEP |
| ATOM | 1241 | CE2 | PHE | 157 | 14.047 | 18.902 | 38.044 | 1.00 | 36.27 | APEP |
| ATOM | 1242 | CZ | PHE | 157 | 15.208 | 18.577 | 37.323 | 1.00 | 36.25 | APEP |
| ATOM | 1243 | C | PHE | 157 | 11.967 | 19.639 | 33.100 | 1.00 | 28.86 | APEP |
| ATOM | 1244 | O | PHE | 157 | 12.423 | 20.543 | 32.400 | 1.00 | 28.11 | APEP |
| ATOM | 1245 | N | LEU | 158 | 12.158 | 18.357 | 32.824 | 1.00 | 28.54 | APEP |
| ATOM | 1246 | CA | LEU | 158 | 12.928 | 17.962 | 31.653 | 1.00 | 28.65 | APEP |
| ATOM | 1247 | CB | LEU | 158 | 13.685 | 16.657 | 31.922 | 1.00 | 29.80 | APEP |
| ATOM | 1248 | CG | LEU | 158 | 15.217 | 16.660 | 31.842 | 1.00 | 28.78 | APEP |
| ATOM | 1249 | CD1 | LEU | 158 | 15.688 | 15.223 | 31.692 | 1.00 | 29.48 | APEP |
| ATOM | 1250 | CD2 | LEU | 158 | 15.706 | 17.507 | 30.669 | 1.00 | 26.31 | APEP |
| ATOM | 1251 | C | LEU | 158 | 11.962 | 17.773 | 30.488 | 1.00 | 27.95 | APEP |
| ATOM | 1252 | O | LEU | 158 | 12.375 | 17.501 | 29.366 | 1.00 | 30.04 | APEP |
| ATOM | 1253 | N | LYS | 159 | 10.671 | 17.932 | 30.763 | 1.00 | 26.29 | APEP |
| ATOM | 1254 | CA | LYS | 159 | 9.654 | 17.774 | 29.734 | 1.00 | 24.09 | APEP |
| ATOM | 1255 | CB | LYS | 159 | 8.801 | 16.542 | 30.039 | 1.00 | 23.00 | APEP |
| ATOM | 1256 | CG | LYS | 159 | 9.619 | 15.265 | 30.203 | 1.00 | 24.44 | APEP |
| ATOM | 1257 | CD | LYS | 159 | 8.749 | 14.035 | 30.403 | 1.00 | 23.01 | APEP |
| ATOM | 1258 | CE | LYS | 159 | 7.414 | 14.154 | 29.691 | 1.00 | 22.17 | APEP |
| ATOM | 1259 | NZ | LYS | 159 | 6.363 | 13.362 | 30.384 | 1.00 | 21.65 | APEP |
| ATOM | 1260 | C | LYS | 159 | 8.756 | 19.000 | 29.595 | 1.00 | 22.82 | APEP |
| ATOM | 1261 | O | LYS | 159 | 8.099 | 19.182 | 28.566 | 1.00 | 20.81 | APEP |
| ATOM | 1262 | N | THR | 160 | 8.731 | 19.845 | 30.623 | 1.00 | 21.93 | APEP |
| ATOM | 1263 | CA | THR | 160 | 7.889 | 21.041 | 30.590 | 1.00 | 19.90 | APEP |
| ATOM | 1264 | CB | THR | 160 | 6.684 | 20.884 | 31.554 | 1.00 | 17.71 | APEP |
| ATOM | 1265 | OG1 | THR | 160 | 7.163 | 20.721 | 32.894 | 1.00 | 17.45 | APEP |
| ATOM | 1266 | CG2 | THR | 160 | 5.856 | 19.670 | 31.182 | 1.00 | 14.19 | APEP |
| ATOM | 1267 | C | THR | 160 | 8.619 | 22.352 | 30.921 | 1.00 | 19.16 | APEP |
| ATOM | 1268 | O | THR | 160 | 8.005 | 23.419 | 30.937 | 1.00 | 20.80 | APEP |
| ATOM | 1269 | N | GLY | 161 | 9.925 | 22.270 | 31.160 | 1.00 | 17.07 | APEP |
| ATOM | 1270 | CA | GLY | 161 | 10.707 | 23.446 | 31.506 | 1.00 | 15.84 | APEP |
| ATOM | 1271 | C | GLY | 161 | 10.629 | 24.679 | 30.616 | 1.00 | 15.32 | APEP |
| ATOM | 1272 | O | GLY | 161 | 10.825 | 25.796 | 31.093 | 1.00 | 15.24 | APEP |
| ATOM | 1273 | N | HIS | 162 | 10.356 | 24.498 | 29.329 | 1.00 | 15.82 | APEP |
| ATOM | 1274 | CA | HIS | 162 | 10.274 | 25.637 | 28.421 | 1.00 | 14.73 | APEP |
| ATOM | 1275 | CB | HIS | 162 | 10.587 | 25.193 | 26.995 | 1.00 | 17.02 | APEP |
| ATOM | 1276 | CG | HIS | 162 | 11.979 | 24.675 | 26.823 | 1.00 | 20.85 | APEP |
| ATOM | 1277 | CD2 | HIS | 162 | 13.162 | 25.120 | 27.308 | 1.00 | 21.78 | APEP |
| ATOM | 1278 | ND1 | HIS | 162 | 12.268 | 23.554 | 26.076 | 1.00 | 23.37 | APEP |
| ATOM | 1279 | CE1 | HIS | 162 | 13.572 | 23.333 | 26.107 | 1.00 | 24.65 | APEP |
| ATOM | 1280 | NE2 | HIS | 162 | 14.136 | 24.269 | 26.848 | 1.00 | 24.25 | APEP |
| ATOM | 1281 | C | HIS | 162 | 8.893 | 26.277 | 28.486 | 1.00 | 13.27 | APEP |
| ATOM | 1282 | O | HIS | 162 | 8.753 | 27.497 | 28.413 | 1.00 | 11.88 | APEP |
| ATOM | 1283 | N | TYR | 163 | 7.875 | 25.442 | 28.628 | 1.00 | 11.72 | APEP |
| ATOM | 1284 | CA | TYR | 163 | 6.509 | 25.926 | 28.733 | 1.00 | 11.23 | APEP |
| ATOM | 1285 | CB | TYR | 163 | 5.550 | 24.750 | 28.924 | 1.00 | 10.40 | APEP |
| ATOM | 1286 | CG | TYR | 163 | 4.271 | 25.122 | 29.653 | 1.00 | 10.24 | APEP |
| ATOM | 1287 | CD1 | TYR | 163 | 3.369 | 26.028 | 29.095 | 1.00 | 10.55 | APEP |
| ATOM | 1288 | CE1 | TYR | 163 | 2.196 | 26.378 | 29.759 | 1.00 | 9.30 | APEP |
| ATOM | 1289 | CD2 | TYR | 163 | 3.970 | 24.576 | 30.898 | 1.00 | 6.58 | APEP |
| ATOM | 1290 | CE2 | TYR | 163 | 2.802 | 24.920 | 31.571 | 1.00 | 8.87 | APEP |
| ATOM | 1291 | CZ | TYR | 163 | 1.918 | 25.818 | 30.995 | 1.00 | 10.63 | APEP |
| ATOM | 1292 | OH | TYR | 163 | 0.745 | 26.143 | 31.635 | 1.00 | 10.74 | APEP |
| ATOM | 1293 | C | TYR | 163 | 6.387 | 26.863 | 29.937 | 1.00 | 10.06 | APEP |
| ATOM | 1294 | O | TYR | 163 | 5.919 | 27.994 | 29.820 | 1.00 | 10.02 | APEP |
| ATOM | 1295 | N | THR | 164 | 6.830 | 26.382 | 31.093 | 1.00 | 9.35 | APEP |
| ATOM | 1296 | CA | THR | 164 | 6.740 | 27.143 | 32.335 | 1.00 | 8.02 | APEP |
| ATOM | 1297 | CB | THR | 164 | 7.259 | 26.301 | 33.511 | 1.00 | 6.41 | APEP |
| ATOM | 1298 | OG1 | THR | 164 | 8.571 | 25.811 | 33.209 | 1.00 | 5.81 | APEP |
| ATOM | 1299 | CG2 | THR | 164 | 6.312 | 25.111 | 33.750 | 1.00 | 2.00 | APEP |
| ATOM | 1300 | C | THR | 164 | 7.422 | 28.513 | 32.327 | 1.00 | 8.62 | APEP |
| ATOM | 1301 | O | THR | 164 | 6.962 | 29.433 | 33.010 | 1.00 | 8.07 | APEP |
| ATOM | 1302 | N | GLN | 165 | 8.508 | 28.663 | 31.570 | 1.00 | 8.53 | APEP |
| ATOM | 1303 | CA | GLN | 165 | 9.183 | 29.964 | 31.499 | 1.00 | 7.81 | APEP |
| ATOM | 1304 | CB | GLN | 165 | 10.598 | 29.837 | 30.930 | 1.00 | 7.00 | APEP |
| ATOM | 1305 | CG | GLN | 165 | 11.241 | 31.179 | 30.604 | 1.00 | 7.27 | APEP |
| ATOM | 1306 | CD | GLN | 165 | 11.537 | 32.023 | 31.840 | 1.00 | 7.94 | APEP |
| ATOM | 1307 | OE1 | GLN | 165 | 12.631 | 31.963 | 32.407 | 1.00 | 7.98 | APEP |
| ATOM | 1308 | NE2 | GLN | 165 | 10.566 | 32.815 | 32.257 | 1.00 | 5.43 | APEP |
| ATOM | 1309 | C | GLN | 165 | 8.370 | 30.902 | 30.609 | 1.00 | 7.95 | APEP |
| ATOM | 1310 | O | GLN | 165 | 8.363 | 32.113 | 30.811 | 1.00 | 8.38 | APEP |
| ATOM | 1311 | N | MET | 166 | 7.683 | 30.330 | 29.625 | 1.00 | 7.26 | APEP |
| ATOM | 1312 | CA | MET | 166 | 6.859 | 31.118 | 28.715 | 1.00 | 8.35 | APEP |
| ATOM | 1313 | CB | MET | 166 | 6.377 | 30.253 | 27.553 | 1.00 | 7.91 | APEP |
| ATOM | 1314 | CG | MET | 166 | 7.245 | 30.356 | 26.309 | 1.00 | 7.80 | APEP |
| ATOM | 1315 | SD | MET | 166 | 6.486 | 29.500 | 24.931 | 1.00 | 12.38 | APEP |
| ATOM | 1316 | CE | MET | 166 | 5.508 | 30.823 | 24.207 | 1.00 | 11.75 | APEP |
| ATOM | 1317 | C | MET | 166 | 5.654 | 31.746 | 29.409 | 1.00 | 6.47 | APEP |
| ATOM | 1318 | O | MET | 166 | 5.313 | 32.890 | 29.128 | 1.00 | 7.34 | APEP |
| ATOM | 1319 | N | VAL | 167 | 5.011 | 31.009 | 30.314 | 1.00 | 7.21 | APEP |
| ATOM | 1320 | CA | VAL | 167 | 3.847 | 31.550 | 31.018 | 1.00 | 7.06 | APEP |
| ATOM | 1321 | CB | VAL | 167 | 2.676 | 30.526 | 31.065 | 1.00 | 7.32 | APEP |
| ATOM | 1322 | CG1 | VAL | 167 | 2.295 | 30.107 | 29.654 | 1.00 | 3.76 | APEP |
| ATOM | 1323 | CG2 | VAL | 167 | 3.048 | 29.321 | 31.901 | 1.00 | 5.77 | APEP |
| ATOM | 1324 | C | VAL | 167 | 4.125 | 32.046 | 32.444 | 1.00 | 8.39 | APEP |
| ATOM | 1325 | O | VAL | 167 | 3.200 | 32.211 | 33.231 | 1.00 | 8.06 | APEP |
| ATOM | 1326 | N | TRP | 168 | 5.396 | 32.290 | 32.767 | 1.00 | 9.11 | APEP |
| ATOM | 1327 | CA | TRP | 168 | 5.793 | 32.784 | 34.089 | 1.00 | 8.50 | APEP |
| ATOM | 1328 | CB | TRP | 168 | 7.320 | 32.745 | 34.232 | 1.00 | 7.79 | APEP |
| ATOM | 1329 | CG | TRP | 168 | 7.806 | 32.690 | 35.657 | 1.00 | 9.67 | APEP |
| ATOM | 1330 | CD2 | TRP | 168 | 7.960 | 31.519 | 36.474 | 1.00 | 8.72 | APEP |
| ATOM | 1331 | CE2 | TRP | 168 | 8.452 | 31.946 | 37.725 | 1.00 | 10.18 | APEP |
| ATOM | 1332 | CE3 | TRP | 168 | 7.730 | 30.153 | 36.268 | 1.00 | 10.13 | APEP |
| ATOM | 1333 | CD1 | TRP | 168 | 8.200 | 33.747 | 36.430 | 1.00 | 8.39 | APEP |
| ATOM | 1334 | NE1 | TRP | 168 | 8.589 | 33.309 | 37.670 | 1.00 | 8.61 | APEP |
| ATOM | 1335 | CZ2 | TRP | 168 | 8.722 | 31.054 | 38.769 | 1.00 | 8.53 | APEP |
| ATOM | 1336 | CZ3 | TRP | 168 | 7.998 | 29.265 | 37.305 | 1.00 | 6.40 | APEP |
| ATOM | 1337 | CH2 | TRP | 168 | 8.488 | 29.721 | 38.539 | 1.00 | 9.27 | APEP |
| ATOM | 1338 | C | TRP | 168 | 5.294 | 34.213 | 34.275 | 1.00 | 8.27 | APEP |
| ATOM | 1339 | O | TRP | 168 | 5.782 | 35.136 | 33.627 | 1.00 | 8.24 | APEP |
| ATOM | 1340 | N | ALA | 169 | 4.324 | 34.392 | 35.167 | 1.00 | 7.85 | APEP |
| ATOM | 1341 | CA | ALA | 169 | 3.734 | 35.704 | 35.415 | 1.00 | 7.42 | APEP |
| ATOM | 1342 | CB | ALA | 169 | 2.668 | 35.585 | 36.476 | 1.00 | 6.86 | APEP |
| ATOM | 1343 | C | ALA | 169 | 4.715 | 36.805 | 35.797 | 1.00 | 9.10 | APEP |
| ATOM | 1344 | O | ALA | 169 | 4.525 | 37.968 | 35.433 | 1.00 | 10.62 | APEP |
| ATOM | 1345 | N | ASN | 170 | 5.758 | 36.436 | 36.531 | 1.00 | 9.55 | APEP |
| ATOM | 1346 | CA | ASN | 170 | 6.760 | 37.392 | 36.990 | 1.00 | 10.81 | APEP |
| ATOM | 1347 | CB | ASN | 170 | 7.557 | 36.792 | 38.158 | 1.00 | 10.24 | APEP |
| ATOM | 1348 | CG | ASN | 170 | 6.907 | 37.057 | 39.513 | 1.00 | 11.09 | APEP |
| ATOM | 1349 | OD1 | ASN | 170 | 5.758 | 37.497 | 39.598 | 1.00 | 11.64 | APEP |
| ATOM | 1350 | ND2 | ASN | 170 | 7.643 | 36.783 | 40.578 | 1.00 | 14.04 | APEP |
| ATOM | 1351 | C | ASN | 170 | 7.716 | 37.859 | 35.887 | 1.00 | 10.96 | APEP |
| ATOM | 1352 | O | ASN | 170 | 8.317 | 38.918 | 35.999 | 1.00 | 10.88 | APEP |
| ATOM | 1353 | N | THR | 171 | 7.866 | 37.071 | 34.830 | 1.00 | 10.69 | APEP |
| ATOM | 1354 | CA | THR | 171 | 8.741 | 37.472 | 33.733 | 1.00 | 9.90 | APEP |
| ATOM | 1355 | CB | THR | 171 | 9.002 | 36.308 | 32.757 | 1.00 | 8.82 | APEP |
| ATOM | 1356 | OG1 | THR | 171 | 9.738 | 35.278 | 33.430 | 1.00 | 10.00 | APEP |
| ATOM | 1357 | CG2 | THR | 171 | 9.793 | 36.790 | 31.541 | 1.00 | 6.32 | APEP |
| ATOM | 1358 | C | THR | 171 | 8.026 | 38.592 | 32.992 | 1.00 | 10.43 | APEP |
| ATOM | 1359 | O | THR | 171 | 6.842 | 38.468 | 32.669 | 1.00 | 11.58 | APEP |
| ATOM | 1360 | N | LYS | 172 | 8.736 | 39.680 | 32.715 | 1.00 | 11.90 | APEP |
| ATOM | 1361 | CA | LYS | 172 | 8.131 | 40.818 | 32.027 | 1.00 | 12.07 | APEP |
| ATOM | 1362 | CB | LYS | 172 | 8.176 | 42.054 | 32.934 | 1.00 | 13.23 | APEP |
| ATOM | 1363 | CG | LYS | 172 | 7.424 | 41.893 | 34.261 | 1.00 | 16.65 | APEP |
| ATOM | 1364 | CD | LYS | 172 | 5.905 | 41.830 | 34.074 | 1.00 | 18.48 | APEP |
| ATOM | 1365 | CE | LYS | 172 | 5.354 | 43.044 | 33.312 | 1.00 | 21.38 | APEP |
| ATOM | 1366 | NZ | LYS | 172 | 4.386 | 43.852 | 34.117 | 1.00 | 19.33 | APEP |
| ATOM | 1367 | C | LYS | 172 | 8.751 | 41.166 | 30.677 | 1.00 | 9.74 | APEP |
| ATOM | 1368 | O | LYS | 172 | 8.122 | 41.834 | 29.856 | 1.00 | 9.87 | APEP |
| ATOM | 1369 | N | GLU | 173 | 9.982 | 40.719 | 30.450 | 1.00 | 11.94 | APEP |
| ATOM | 1370 | CA | GLU | 173 | 10.684 | 41.005 | 29.198 | 1.00 | 13.45 | APEP |
| ATOM | 1371 | CB | GLU | 173 | 11.812 | 42.014 | 29.438 | 1.00 | 16.52 | APEP |
| ATOM | 1372 | CG | GLU | 173 | 11.752 | 42.721 | 30.778 | 1.00 | 21.67 | APEP |
| ATOM | 1373 | CD | GLU | 173 | 11.695 | 44.220 | 30.618 | 1.00 | 23.96 | APEP |
| ATOM | 1374 | OE1 | GLU | 173 | 11.727 | 44.679 | 29.455 | 1.00 | 24.88 | APEP |
| ATOM | 1375 | OE2 | GLU | 173 | 11.621 | 44.935 | 31.643 | 1.00 | 28.83 | APEP |
| ATOM | 1376 | C | GLU | 173 | 11.280 | 39.765 | 28.531 | 1.00 | 10.86 | APEP |
| ATOM | 1377 | O | GLU | 173 | 11.622 | 38.790 | 29.199 | 1.00 | 10.81 | APEP |
| ATOM | 1378 | N | VAL | 174 | 11.404 | 39.830 | 27.209 | 1.00 | 10.16 | APEP |
| ATOM | 1379 | CA | VAL | 174 | 11.968 | 38.741 | 26.416 | 1.00 | 9.96 | APEP |
| ATOM | 1380 | CB | VAL | 174 | 10.856 | 37.811 | 25.846 | 1.00 | 9.93 | APEP |
| ATOM | 1381 | CG1 | VAL | 174 | 10.099 | 38.519 | 24.740 | 1.00 | 7.96 | APEP |
| ATOM | 1382 | CG2 | VAL | 174 | 11.460 | 36.508 | 25.323 | 1.00 | 7.52 | APEP |
| ATOM | 1383 | C | VAL | 174 | 12.790 | 39.316 | 25.258 | 1.00 | 11.17 | APEP |
| ATOM | 1384 | O | VAL | 174 | 12.485 | 40.383 | 24.728 | 1.00 | 11.23 | APEP |
| ATOM | 1385 | N | GLY | 175 | 13.845 | 38.605 | 24.886 | 1.00 | 10.40 | APEP |
| ATOM | 1386 | CA | GLY | 175 | 14.692 | 39.045 | 23.797 | 1.00 | 8.75 | APEP |
| ATOM | 1387 | C | GLY | 175 | 15.337 | 37.813 | 23.211 | 1.00 | 9.59 | APEP |
| ATOM | 1388 | O | GLY | 175 | 15.882 | 36.991 | 23.949 | 1.00 | 6.65 | APEP |
| ATOM | 1389 | N | CYS | 176 | 15.291 | 37.685 | 21.885 | 1.00 | 8.02 | APEP |
| ATOM | 1390 | CA | CYS | 176 | 15.853 | 36.511 | 21.226 | 1.00 | 7.90 | APEP |
| ATOM | 1391 | C | CYS | 176 | 16.940 | 36.771 | 20.186 | 1.00 | 7.85 | APEP |
| ATOM | 1392 | O | CYS | 176 | 17.114 | 37.893 | 19.693 | 1.00 | 6.33 | APEP |
| ATOM | 1393 | CB | CYS | 176 | 14.721 | 35.701 | 20.582 | 1.00 | 6.21 | APEP |
| ATOM | 1394 | SG | CYS | 176 | 13.249 | 35.553 | 21.641 | 1.00 | 9.41 | APEP |
| ATOM | 1395 | N | GLY | 177 | 17.672 | 35.703 | 19.880 | 1.00 | 8.22 | APEP |
| ATOM | 1396 | CA | GLY | 177 | 18.737 | 35.744 | 18.895 | 1.00 | 10.14 | APEP |
| ATOM | 1397 | C | GLY | 177 | 18.618 | 34.466 | 18.085 | 1.00 | 10.70 | APEP |
| ATOM | 1398 | O | GLY | 177 | 18.182 | 33.446 | 18.623 | 1.00 | 8.40 | APEP |
| ATOM | 1399 | N | SER | 178 | 18.983 | 34.509 | 16.806 | 1.00 | 9.75 | APEP |
| ATOM | 1400 | CA | SER | 178 | 18.881 | 33.325 | 15.959 | 1.00 | 9.62 | APEP |
| ATOM | 1401 | CB | SER | 178 | 17.523 | 33.305 | 15.247 | 1.00 | 12.13 | APEP |
| ATOM | 1402 | OG | SER | 178 | 17.614 | 33.902 | 13.964 | 1.00 | 15.65 | APEP |
| ATOM | 1403 | C | SER | 178 | 19.999 | 33.227 | 14.921 | 1.00 | 8.56 | APEP |
| ATOM | 1404 | O | SER | 178 | 20.597 | 34.231 | 14.532 | 1.00 | 5.09 | APEP |
| ATOM | 1405 | N | ILE | 179 | 20.270 | 32.001 | 14.482 | 1.00 | 9.37 | APEP |
| ATOM | 1406 | CA | ILE | 179 | 21.310 | 31.744 | 13.498 | 1.00 | 9.01 | APEP |
| ATOM | 1407 | CB | ILE | 179 | 22.673 | 31.531 | 14.181 | 1.00 | 8.43 | APEP |
| ATOM | 1408 | CG2 | ILE | 179 | 22.625 | 30.296 | 15.054 | 1.00 | 6.63 | APEP |
| ATOM | 1409 | CG1 | ILE | 179 | 23.774 | 31.415 | 13.122 | 1.00 | 7.53 | APEP |
| ATOM | 1410 | CD1 | ILE | 179 | 25.093 | 32.035 | 13.535 | 1.00 | 8.62 | APEP |
| ATOM | 1411 | C | ILE | 179 | 20.980 | 30.517 | 12.650 | 1.00 | 10.87 | APEP |
| ATOM | 1412 | O | ILE | 179 | 20.506 | 29.497 | 13.158 | 1.00 | 10.47 | APEP |
| ATOM | 1413 | N | LYS | 180 | 21.216 | 30.632 | 11.347 | 1.00 | 10.60 | APEP |
| ATOM | 1414 | CA | LYS | 180 | 20.952 | 29.536 | 10.430 | 1.00 | 11.05 | APEP |
| ATOM | 1415 | CB | LYS | 180 | 20.077 | 30.018 | 9.269 | 1.00 | 11.05 | APEP |
| ATOM | 1416 | CG | LYS | 180 | 18.745 | 30.596 | 9.724 | 1.00 | 13.07 | APEP |
| ATOM | 1417 | CD | LYS | 180 | 17.902 | 31.048 | 8.543 | 1.00 | 15.79 | APEP |
| ATOM | 1418 | CE | LYS | 180 | 16.580 | 31.655 | 8.996 | 1.00 | 14.81 | APEP |
| ATOM | 1419 | NZ | LYS | 180 | 15.620 | 31.802 | 7.862 | 1.00 | 16.66 | APEP |
| ATOM | 1420 | C | LYS | 180 | 22.291 | 29.029 | 9.920 | 1.00 | 10.53 | APEP |
| ATOM | 1421 | O | LYS | 180 | 23.137 | 29.817 | 9.505 | 1.00 | 12.20 | APEP |
| ATOM | 1422 | N | TYR | 181 | 22.490 | 27.716 | 9.955 | 1.00 | 9.11 | APEP |
| ATOM | 1423 | CA | TYR | 181 | 23.756 | 27.166 | 9.510 | 1.00 | 8.14 | APEP |
| ATOM | 1424 | CB | TYR | 181 | 24.786 | 27.300 | 10.633 | 1.00 | 6.13 | APEP |
| ATOM | 1425 | CG | TYR | 181 | 24.460 | 26.483 | 11.863 | 1.00 | 8.18 | APEP |
| ATOM | 1426 | CD1 | TYR | 181 | 24.984 | 25.201 | 12.027 | 1.00 | 9.63 | APEP |
| ATOM | 1427 | CE1 | TYR | 181 | 24.706 | 24.450 | 13.172 | 1.00 | 8.66 | APEP |
| ATOM | 1428 | CD2 | TYR | 181 | 23.643 | 26.999 | 12.876 | 1.00 | 8.34 | APEP |
| ATOM | 1429 | CE2 | TYR | 181 | 23.360 | 26.257 | 14.020 | 1.00 | 7.46 | APEP |
| ATOM | 1430 | CZ | TYR | 181 | 23.896 | 24.986 | 14.161 | 1.00 | 8.38 | APEP |
| ATOM | 1431 | OH | TYR | 181 | 23.634 | 24.244 | 15.293 | 1.00 | 8.05 | APEP |
| ATOM | 1432 | C | TYR | 181 | 23.695 | 25.719 | 9.022 | 1.00 | 7.08 | APEP |
| ATOM | 1433 | O | TYR | 181 | 22.728 | 25.001 | 9.262 | 1.00 | 7.97 | APEP |
| ATOM | 1434 | N | ILE | 182 | 24.743 | 25.303 | 8.323 | 1.00 | 8.25 | APEP |
| ATOM | 1435 | CA | ILE | 182 | 24.814 | 23.948 | 7.804 | 1.00 | 7.94 | APEP |
| ATOM | 1436 | CB | ILE | 182 | 25.011 | 23.959 | 6.293 | 1.00 | 9.43 | APEP |
| ATOM | 1437 | CG2 | ILE | 182 | 24.641 | 22.599 | 5.713 | 1.00 | 9.49 | APEP |
| ATOM | 1438 | CG1 | ILE | 182 | 24.147 | 25.065 | 5.680 | 1.00 | 9.61 | APEP |
| ATOM | 1439 | CD1 | ILE | 182 | 24.502 | 25.429 | 4.278 | 1.00 | 7.05 | APEP |
| ATOM | 1440 | C | ILE | 182 | 25.961 | 23.184 | 8.445 | 1.00 | 8.67 | APEP |
| ATOM | 1441 | O | ILE | 182 | 27.112 | 23.588 | 8.333 | 1.00 | 8.62 | APEP |
| ATOM | 1442 | N | GLN | 183 | 25.642 | 22.084 | 9.122 | 1.00 | 6.39 | APEP |
| ATOM | 1443 | CA | GLN | 183 | 26.657 | 21.271 | 9.776 | 1.00 | 7.60 | APEP |
| ATOM | 1444 | CB | GLN | 183 | 26.485 | 21.313 | 11.304 | 1.00 | 8.26 | APEP |
| ATOM | 1445 | CG | GLN | 183 | 27.184 | 20.160 | 12.059 | 1.00 | 11.17 | APEP |
| ATOM | 1446 | CD | GLN | 183 | 26.842 | 20.105 | 13.560 | 1.00 | 10.23 | APEP |
| ATOM | 1447 | OE1 | GLN | 183 | 25.927 | 20.779 | 14.029 | 1.00 | 13.96 | APEP |
| ATOM | 1448 | NE2 | GLN | 183 | 27.578 | 19.293 | 14.304 | 1.00 | 9.47 | APEP |
| ATOM | 1449 | C | GLN | 183 | 26.603 | 19.824 | 9.308 | 1.00 | 6.95 | APEP |
| ATOM | 1450 | O | GLN | 183 | 25.653 | 19.096 | 9.602 | 1.00 | 4.41 | APEP |
| ATOM | 1451 | N | GLU | 184 | 27.624 | 19.404 | 8.576 | 1.00 | 6.92 | APEP |
| ATOM | 1452 | CA | GLU | 184 | 27.685 | 18.025 | 8.123 | 1.00 | 7.66 | APEP |
| ATOM | 1453 | CB | GLU | 184 | 27.885 | 17.120 | 9.349 | 1.00 | 9.14 | APEP |
| ATOM | 1454 | CG | GLU | 184 | 29.110 | 17.551 | 10.172 | 1.00 | 8.94 | APEP |
| ATOM | 1455 | CD | GLU | 184 | 29.207 | 16.912 | 11.558 | 1.00 | 12.48 | APEP |
| ATOM | 1456 | OE1 | GLU | 184 | 28.235 | 16.963 | 12.340 | 1.00 | 13.16 | APEP |
| ATOM | 1457 | OE2 | GLU | 184 | 30.278 | 16.361 | 11.869 | 1.00 | 15.28 | APEP |
| ATOM | 1458 | C | GLU | 184 | 26.447 | 17.645 | 7.316 | 1.00 | 6.36 | APEP |
| ATOM | 1459 | O | GLU | 184 | 25.858 | 16.581 | 7.493 | 1.00 | 4.35 | APEP |
| ATOM | 1460 | N | LYS | 185 | 26.089 | 18.560 | 6.417 | 1.00 | 8.43 | APEP |
| ATOM | 1461 | CA | LYS | 185 | 24.956 | 18.451 | 5.504 | 1.00 | 10.57 | APEP |
| ATOM | 1462 | CB | LYS | 185 | 25.054 | 17.165 | 4.685 | 1.00 | 12.55 | APEP |
| ATOM | 1463 | CG | LYS | 185 | 25.705 | 17.371 | 3.331 | 1.00 | 16.19 | APEP |
| ATOM | 1464 | CD | LYS | 185 | 26.930 | 16.498 | 3.173 | 1.00 | 20.18 | APEP |
| ATOM | 1465 | CE | LYS | 185 | 26.783 | 15.553 | 1.990 | 1.00 | 22.27 | APEP |
| ATOM | 1466 | NZ | LYS | 185 | 25.360 | 15.182 | 1.744 | 1.00 | 25.09 | APEP |
| ATOM | 1467 | C | LYS | 185 | 23.571 | 18.567 | 6.131 | 1.00 | 10.92 | APEP |
| ATOM | 1468 | O | LYS | 185 | 22.566 | 18.219 | 5.509 | 1.00 | 10.67 | APEP |
| ATOM | 1469 | N | TRP | 186 | 23.519 | 19.062 | 7.362 | 1.00 | 10.21 | APEP |
| ATOM | 1470 | CA | TRP | 186 | 22.250 | 19.252 | 8.039 | 1.00 | 9.13 | APEP |
| ATOM | 1471 | CB | TRP | 186 | 22.297 | 18.677 | 9.461 | 1.00 | 8.92 | APEP |
| ATOM | 1472 | CG | TRP | 186 | 22.105 | 17.173 | 9.563 | 1.00 | 7.23 | APEP |
| ATOM | 1473 | CD2 | TRP | 186 | 20.883 | 16.433 | 9.380 | 1.00 | 7.44 | APEP |
| ATOM | 1474 | CE2 | TRP | 186 | 21.179 | 15.070 | 9.624 | 1.00 | 7.10 | APEP |
| ATOM | 1475 | CE3 | TRP | 186 | 19.569 | 16.787 | 9.036 | 1.00 | 7.24 | APEP |
| ATOM | 1476 | CD1 | TRP | 186 | 23.057 | 16.253 | 9.895 | 1.00 | 7.55 | APEP |
| ATOM | 1477 | NE1 | TRP | 186 | 22.510 | 14.991 | 9.934 | 1.00 | 7.21 | APEP |
| ATOM | 1478 | CZ2 | TRP | 186 | 20.209 | 14.061 | 9.536 | 1.00 | 4.65 | APEP |
| ATOM | 1479 | CZ3 | TRP | 186 | 18.602 | 15.776 | 8.950 | 1.00 | 5.07 | APEP |
| ATOM | 1480 | CH2 | TRP | 186 | 18.934 | 14.430 | 9.198 | 1.00 | 4.34 | APEP |
| ATOM | 1481 | C | TRP | 186 | 22.029 | 20.757 | 8.097 | 1.00 | 9.22 | APEP |
| ATOM | 1482 | O | TRP | 186 | 22.895 | 21.495 | 8.547 | 1.00 | 10.78 | APEP |
| ATOM | 1483 | N | HIS | 187 | 20.876 | 21.214 | 7.622 | 1.00 | 11.53 | APEP |
| ATOM | 1484 | CA | HIS | 187 | 20.555 | 22.637 | 7.642 | 1.00 | 10.59 | APEP |
| ATOM | 1485 | CB | HIS | 187 | 19.773 | 23.005 | 6.375 | 1.00 | 11.28 | APEP |
| ATOM | 1486 | CG | HIS | 187 | 20.381 | 22.455 | 5.119 | 1.00 | 12.80 | APEP |
| ATOM | 1487 | CD2 | HIS | 187 | 20.511 | 21.183 | 4.674 | 1.00 | 13.63 | APEP |
| ATOM | 1488 | ND1 | HIS | 187 | 20.984 | 23.254 | 4.170 | 1.00 | 14.63 | APEP |
| ATOM | 1489 | CE1 | HIS | 187 | 21.463 | 22.497 | 3.198 | 1.00 | 14.97 | APEP |
| ATOM | 1490 | NE2 | HIS | 187 | 21.189 | 21.236 | 3.480 | 1.00 | 14.98 | APEP |
| ATOM | 1491 | C | HIS | 187 | 19.738 | 22.910 | 8.904 | 1.00 | 8.99 | APEP |
| ATOM | 1492 | O | HIS | 187 | 18.636 | 22.408 | 9.058 | 1.00 | 10.40 | APEP |
| ATOM | 1493 | N | LYS | 188 | 20.287 | 23.700 | 9.817 | 1.00 | 10.12 | APEP |
| ATOM | 1494 | CA | LYS | 188 | 19.593 | 23.970 | 11.068 | 1.00 | 9.30 | APEP |
| ATOM | 1495 | CB | LYS | 188 | 20.403 | 23.413 | 12.251 | 1.00 | 10.66 | APEP |
| ATOM | 1496 | CG | LYS | 188 | 21.395 | 22.314 | 11.909 | 1.00 | 7.22 | APEP |
| ATOM | 1497 | CD | LYS | 188 | 21.627 | 21.422 | 13.118 | 1.00 | 8.34 | APEP |
| ATOM | 1498 | CE | LYS | 188 | 22.696 | 20.369 | 12.871 | 1.00 | 9.03 | APEP |
| ATOM | 1499 | NZ | LYS | 188 | 23.268 | 19.865 | 14.162 | 1.00 | 13.31 | APEP |
| ATOM | 1500 | C | LYS | 188 | 19.289 | 25.428 | 11.349 | 1.00 | 7.81 | APEP |
| ATOM | 1501 | O | LYS | 188 | 19.988 | 26.328 | 10.890 | 1.00 | 9.61 | APEP |
| ATOM | 1502 | N | HIS | 189 | 18.216 | 25.646 | 12.097 | 1.00 | 9.34 | APEP |
| ATOM | 1503 | CA | HIS | 189 | 17.823 | 26.977 | 12.532 | 1.00 | 9.56 | APEP |
| ATOM | 1504 | CB | HIS | 189 | 16.391 | 27.315 | 12.119 | 1.00 | 9.45 | APEP |
| ATOM | 1505 | CG | HIS | 189 | 16.033 | 28.756 | 12.331 | 1.00 | 11.25 | APEP |
| ATOM | 1506 | CD2 | HIS | 189 | 16.739 | 29.777 | 12.870 | 1.00 | 11.06 | APEP |
| ATOM | 1507 | ND1 | HIS | 189 | 14.822 | 29.291 | 11.946 | 1.00 | 13.10 | APEP |
| ATOM | 1508 | CE1 | HIS | 189 | 14.800 | 30.579 | 12.237 | 1.00 | 11.47 | APEP |
| ATOM | 1509 | NE2 | HIS | 189 | 15.950 | 30.900 | 12.799 | 1.00 | 12.33 | APEP |
| ATOM | 1510 | C | HIS | 189 | 17.911 | 26.904 | 14.049 | 1.00 | 9.30 | APEP |
| ATOM | 1511 | O | HIS | 189 | 17.265 | 26.060 | 14.671 | 1.00 | 9.01 | APEP |
| ATOM | 1512 | N | TYR | 190 | 18.717 | 27.781 | 14.635 | 1.00 | 10.00 | APEP |
| ATOM | 1513 | CA | TYR | 190 | 18.928 | 27.816 | 16.080 | 1.00 | 9.41 | APEP |
| ATOM | 1514 | CB | TYR | 190 | 20.433 | 27.745 | 16.343 | 1.00 | 11.08 | APEP |
| ATOM | 1515 | CG | TYR | 190 | 20.872 | 27.618 | 17.788 | 1.00 | 11.54 | APEP |
| ATOM | 1516 | CD1 | TYR | 190 | 20.017 | 27.124 | 18.777 | 1.00 | 11.05 | APEP |
| ATOM | 1517 | CE1 | TYR | 190 | 20.458 | 26.983 | 20.108 | 1.00 | 10.69 | APEP |
| ATOM | 1518 | CD2 | TYR | 190 | 22.173 | 27.971 | 18.157 | 1.00 | 11.80 | APEP |
| ATOM | 1519 | CE2 | TYR | 190 | 22.618 | 27.835 | 19.467 | 1.00 | 12.63 | APEP |
| ATOM | 1520 | CZ | TYR | 190 | 21.767 | 27.342 | 20.435 | 1.00 | 11.58 | APEP |
| ATOM | 1521 | OH | TYR | 190 | 22.257 | 27.190 | 21.713 | 1.00 | 12.58 | APEP |
| ATOM | 1522 | C | TYR | 190 | 18.337 | 29.076 | 16.716 | 1.00 | 8.19 | APEP |
| ATOM | 1523 | O | TYR | 190 | 18.803 | 30.184 | 16.456 | 1.00 | 6.34 | APEP |
| ATOM | 1524 | N | LEU | 191 | 17.313 | 28.895 | 17.549 | 1.00 | 7.47 | APEP |
| ATOM | 1525 | CA | LEU | 191 | 16.656 | 30.011 | 18.226 | 1.00 | 8.92 | APEP |
| ATOM | 1526 | CB | LEU | 191 | 15.151 | 30.001 | 17.926 | 1.00 | 8.75 | APEP |
| ATOM | 1527 | CG | LEU | 191 | 14.308 | 31.101 | 18.599 | 1.00 | 11.51 | APEP |
| ATOM | 1528 | CD1 | LEU | 191 | 14.540 | 32.444 | 17.916 | 1.00 | 9.71 | APEP |
| ATOM | 1529 | CD2 | LEU | 191 | 12.831 | 30.724 | 18.541 | 1.00 | 9.30 | APEP |
| ATOM | 1530 | C | LEU | 191 | 16.890 | 29.996 | 19.743 | 1.00 | 9.50 | APEP |
| ATOM | 1531 | O | LEU | 191 | 16.667 | 28.988 | 20.416 | 1.00 | 11.01 | APEP |
| ATOM | 1532 | N | VAL | 192 | 17.347 | 31.128 | 20.266 | 1.00 | 9.30 | APEP |
| ATOM | 1533 | CA | VAL | 192 | 17.629 | 31.291 | 21.690 | 1.00 | 10.13 | APEP |
| ATOM | 1534 | CB | VAL | 192 | 19.145 | 31.536 | 21.923 | 1.00 | 10.96 | APEP |
| ATOM | 1535 | CG1 | VAL | 192 | 19.387 | 32.044 | 23.344 | 1.00 | 11.81 | APEP |
| ATOM | 1536 | CG2 | VAL | 192 | 19.934 | 30.267 | 21.659 | 1.00 | 11.43 | APEP |
| ATOM | 1537 | C | VAL | 192 | 16.875 | 32.510 | 22.231 | 1.00 | 10.34 | APEP |
| ATOM | 1538 | O | VAL | 192 | 17.078 | 33.621 | 21.745 | 1.00 | 10.10 | APEP |
| ATOM | 1539 | N | CYS | 193 | 16.009 | 32.315 | 23.226 | 1.00 | 8.18 | APEP |
| ATOM | 1540 | CA | CYS | 193 | 15.276 | 33.442 | 23.810 | 1.00 | 8.37 | APEP |
| ATOM | 1541 | C | CYS | 193 | 15.600 | 33.609 | 25.296 | 1.00 | 8.44 | APEP |
| ATOM | 1542 | O | CYS | 193 | 15.514 | 32.650 | 26.061 | 1.00 | 6.27 | APEP |
| ATOM | 1543 | CB | CYS | 193 | 13.762 | 33.258 | 23.649 | 1.00 | 9.08 | APEP |
| ATOM | 1544 | SG | CYS | 193 | 13.062 | 33.556 | 21.985 | 1.00 | 8.61 | APEP |
| ATOM | 1545 | N | ASN | 194 | 15.978 | 34.826 | 25.694 | 1.00 | 8.32 | APEP |
| ATOM | 1546 | CA | ASN | 194 | 16.310 | 35.133 | 27.086 | 1.00 | 7.48 | APEP |
| ATOM | 1547 | CB | ASN | 194 | 17.582 | 35.991 | 27.149 | 1.00 | 8.69 | APEP |
| ATOM | 1548 | CG | ASN | 194 | 18.840 | 35.190 | 26.868 | 1.00 | 6.67 | APEP |
| ATOM | 1549 | OD1 | ASN | 194 | 18.772 | 33.995 | 26.596 | 1.00 | 9.58 | APEP |
| ATOM | 1550 | ND2 | ASN | 194 | 19.989 | 35.843 | 26.932 | 1.00 | 4.71 | APEP |
| ATOM | 1551 | C | ASN | 194 | 15.140 | 35.859 | 27.766 | 1.00 | 7.75 | APEP |
| ATOM | 1552 | O | ASN | 194 | 14.540 | 36.760 | 27.175 | 1.00 | 5.25 | APEP |
| ATOM | 1553 | N | TYR | 195 | 14.834 | 35.475 | 29.009 | 1.00 | 7.83 | APEP |
| ATOM | 1554 | CA | TYR | 195 | 13.699 | 36.047 | 29.749 | 1.00 | 7.00 | APEP |
| ATOM | 1555 | CB | TYR | 195 | 12.736 | 34.924 | 30.138 | 1.00 | 6.30 | APEP |
| ATOM | 1556 | CG | TYR | 195 | 12.180 | 34.180 | 28.949 | 1.00 | 8.42 | APEP |
| ATOM | 1557 | CD1 | TYR | 195 | 12.918 | 33.175 | 28.329 | 1.00 | 7.89 | APEP |
| ATOM | 1558 | CE1 | TYR | 195 | 12.436 | 32.510 | 27.219 | 1.00 | 8.54 | APEP |
| ATOM | 1559 | CD2 | TYR | 195 | 10.934 | 34.501 | 28.422 | 1.00 | 6.50 | APEP |
| ATOM | 1560 | CE2 | TYR | 195 | 10.438 | 33.835 | 27.300 | 1.00 | 8.99 | APEP |
| ATOM | 1561 | CZ | TYR | 195 | 11.199 | 32.837 | 26.707 | 1.00 | 8.08 | APEP |
| ATOM | 1562 | OH | TYR | 195 | 10.724 | 32.142 | 25.617 | 1.00 | 8.91 | APEP |
| ATOM | 1563 | C | TYR | 195 | 14.047 | 36.860 | 30.999 | 1.00 | 6.48 | APEP |
| ATOM | 1564 | O | TYR | 195 | 14.840 | 36.422 | 31.822 | 1.00 | 7.26 | APEP |
| ATOM | 1565 | N | GLY | 196 | 13.418 | 38.025 | 31.154 | 1.00 | 6.42 | APEP |
| ATOM | 1566 | CA | GLY | 196 | 13.709 | 38.867 | 32.303 | 1.00 | 6.88 | APEP |
| ATOM | 1567 | C | GLY | 196 | 12.558 | 39.431 | 33.131 | 1.00 | 8.03 | APEP |
| ATOM | 1568 | O | GLY | 196 | 11.649 | 40.075 | 32.596 | 1.00 | 8.29 | APEP |
| ATOM | 1569 | N | PRO | 197 | 12.541 | 39.157 | 34.445 | 1.00 | 6.50 | APEP |
| ATOM | 1570 | CD | PRO | 197 | 11.525 | 39.698 | 35.363 | 1.00 | 7.41 | APEP |
| ATOM | 1571 | CA | PRO | 197 | 13.536 | 38.343 | 35.153 | 1.00 | 6.68 | APEP |
| ATOM | 1572 | CB | PRO | 197 | 13.300 | 38.688 | 36.610 | 1.00 | 6.91 | APEP |
| ATOM | 1573 | CG | PRO | 197 | 11.856 | 39.041 | 36.672 | 1.00 | 6.39 | APEP |
| ATOM | 1574 | C | PRO | 197 | 13.266 | 36.868 | 34.854 | 1.00 | 6.69 | APEP |
| ATOM | 1575 | O | PRO | 197 | 12.255 | 36.537 | 34.238 | 1.00 | 6.02 | APEP |
| ATOM | 1576 | N | SER | 198 | 14.153 | 35.975 | 35.286 | 1.00 | 7.01 | APEP |
| ATOM | 1577 | CA | SER | 198 | 13.953 | 34.557 | 35.001 | 1.00 | 7.41 | APEP |
| ATOM | 1578 | CB | SER | 198 | 15.233 | 33.755 | 35.303 | 1.00 | 4.71 | APEP |
| ATOM | 1579 | OG | SER | 198 | 15.558 | 33.752 | 36.682 | 1.00 | 11.52 | APEP |
| ATOM | 1580 | C | SER | 198 | 12.765 | 33.927 | 35.717 | 1.00 | 7.39 | APEP |
| ATOM | 1581 | O | SER | 198 | 12.161 | 34.528 | 36.605 | 1.00 | 6.19 | APEP |
| ATOM | 1582 | N | GLY | 199 | 12.423 | 32.716 | 35.289 | 1.00 | 7.86 | APEP |
| ATOM | 1583 | CA | GLY | 199 | 11.332 | 31.977 | 35.893 | 1.00 | 8.49 | APEP |
| ATOM | 1584 | C | GLY | 199 | 11.894 | 30.622 | 36.274 | 1.00 | 8.12 | APEP |
| ATOM | 1585 | O | GLY | 199 | 13.110 | 30.450 | 36.306 | 1.00 | 8.44 | APEP |
| ATOM | 1586 | N | ASN | 200 | 11.022 | 29.670 | 36.570 | 1.00 | 9.40 | APEP |
| ATOM | 1587 | CA | ASN | 200 | 11.433 | 28.317 | 36.929 | 1.00 | 11.14 | APEP |
| ATOM | 1588 | CB | ASN | 200 | 12.344 | 27.742 | 35.844 | 1.00 | 11.38 | APEP |
| ATOM | 1589 | CG | ASN | 200 | 11.581 | 27.365 | 34.591 | 1.00 | 12.36 | APEP |
| ATOM | 1590 | OD1 | ASN | 200 | 10.360 | 27.478 | 34.550 | 1.00 | 13.35 | APEP |
| ATOM | 1591 | ND2 | ASN | 200 | 12.293 | 26.919 | 33.566 | 1.00 | 10.02 | APEP |
| ATOM | 1592 | C | ASN | 200 | 12.102 | 28.177 | 38.296 | 1.00 | 12.89 | APEP |
| ATOM | 1593 | O | ASN | 200 | 13.019 | 27.373 | 38.477 | 1.00 | 12.94 | APEP |
| ATOM | 1594 | N | PHE | 201 | 11.632 | 28.957 | 39.262 | 1.00 | 14.46 | APEP |
| ATOM | 1595 | CA | PHE | 201 | 12.157 | 28.890 | 40.622 | 1.00 | 16.00 | APEP |
| ATOM | 1596 | CB | PHE | 201 | 11.947 | 30.224 | 41.339 | 1.00 | 14.75 | APEP |
| ATOM | 1597 | CG | PHE | 201 | 12.805 | 31.338 | 40.811 | 1.00 | 13.67 | APEP |
| ATOM | 1598 | CD1 | PHE | 201 | 12.267 | 32.311 | 39.982 | 1.00 | 13.83 | APEP |
| ATOM | 1599 | CD2 | PHE | 201 | 14.151 | 31.421 | 41.157 | 1.00 | 17.70 | APEP |
| ATOM | 1600 | CE1 | PHE | 201 | 13.047 | 33.350 | 39.505 | 1.00 | 14.74 | APEP |
| ATOM | 1601 | CE2 | PHE | 201 | 14.948 | 32.460 | 40.685 | 1.00 | 17.45 | APEP |
| ATOM | 1602 | CZ | PHE | 201 | 14.394 | 33.427 | 39.857 | 1.00 | 17.09 | APEP |
| ATOM | 1603 | C | PHE | 201 | 11.347 | 27.786 | 41.304 | 1.00 | 16.58 | APEP |
| ATOM | 1604 | O | PHE | 201 | 10.124 | 27.876 | 41.385 | 1.00 | 16.44 | APEP |
| ATOM | 1605 | N | LYS | 202 | 12.026 | 26.755 | 41.797 | 1.00 | 18.81 | APEP |
| ATOM | 1606 | CA | LYS | 202 | 11.351 | 25.617 | 42.421 | 1.00 | 21.27 | APEP |
| ATOM | 1607 | CB | LYS | 202 | 12.386 | 24.588 | 42.883 | 1.00 | 25.00 | APEP |
| ATOM | 1608 | CG | LYS | 202 | 12.314 | 23.274 | 42.101 | 1.00 | 29.78 | APEP |
| ATOM | 1609 | CD | LYS | 202 | 13.215 | 22.197 | 42.698 | 1.00 | 31.92 | APEP |
| ATOM | 1610 | CE | LYS | 202 | 12.574 | 20.816 | 42.609 | 1.00 | 32.51 | APEP |
| ATOM | 1611 | NZ | LYS | 202 | 12.138 | 20.304 | 43.944 | 1.00 | 30.68 | APEP |
| ATOM | 1612 | C | LYS | 202 | 10.365 | 25.899 | 43.555 | 1.00 | 21.29 | APEP |
| ATOM | 1613 | O | LYS | 202 | 9.347 | 25.218 | 43.676 | 1.00 | 22.50 | APEP |
| ATOM | 1614 | N | ASN | 203 | 10.642 | 26.894 | 44.385 | 1.00 | 20.90 | APEP |
| ATOM | 1615 | CA | ASN | 203 | 9.726 | 27.190 | 45.485 | 1.00 | 22.00 | APEP |
| ATOM | 1616 | CB | ASN | 203 | 10.520 | 27.657 | 46.711 | 1.00 | 23.02 | APEP |
| ATOM | 1617 | CG | ASN | 203 | 11.274 | 28.953 | 46.466 | 1.00 | 23.60 | APEP |
| ATOM | 1618 | OD1 | ASN | 203 | 11.559 | 29.698 | 47.401 | 1.00 | 24.95 | APEP |
| ATOM | 1619 | ND2 | ASN | 203 | 11.605 | 29.223 | 45.209 | 1.00 | 25.38 | APEP |
| ATOM | 1620 | C | ASN | 203 | 8.656 | 28.232 | 45.134 | 1.00 | 20.82 | APEP |
| ATOM | 1621 | O | ASN | 203 | 8.096 | 28.877 | 46.025 | 1.00 | 20.77 | APEP |
| ATOM | 1622 | N | GLU | 204 | 8.363 | 28.384 | 43.845 | 1.00 | 16.60 | APEP |
| ATOM | 1623 | CA | GLU | 204 | 7.382 | 29.372 | 43.414 | 1.00 | 17.29 | APEP |
| ATOM | 1624 | CB | GLU | 204 | 8.093 | 30.550 | 42.737 | 1.00 | 17.84 | APEP |
| ATOM | 1625 | CG | GLU | 204 | 9.303 | 31.084 | 43.489 | 1.00 | 17.21 | APEP |
| ATOM | 1626 | CD | GLU | 204 | 9.801 | 32.408 | 42.937 | 1.00 | 17.35 | APEP |
| ATOM | 1627 | OE1 | GLU | 204 | 9.157 | 32.963 | 42.023 | 1.00 | 15.88 | APEP |
| ATOM | 1628 | OE2 | GLU | 204 | 10.842 | 32.895 | 43.422 | 1.00 | 17.79 | APEP |
| ATOM | 1629 | C | GLU | 204 | 6.298 | 28.842 | 42.475 | 1.00 | 17.89 | APEP |
| ATOM | 1630 | O | GLU | 204 | 6.383 | 27.727 | 41.963 | 1.00 | 16.49 | APEP |
| ATOM | 1631 | N | GLU | 205 | 5.283 | 29.672 | 42.251 | 1.00 | 19.78 | APEP |
| ATOM | 1632 | CA | GLU | 205 | 4.159 | 29.335 | 41.383 | 1.00 | 20.21 | APEP |
| ATOM | 1633 | CB | GLU | 205 | 2.851 | 29.854 | 41.992 | 1.00 | 23.42 | APEP |
| ATOM | 1634 | CG | GLU | 205 | 2.609 | 31.347 | 41.772 | 1.00 | 28.77 | APEP |
| ATOM | 1635 | CD | GLU | 205 | 2.874 | 32.176 | 43.022 | 1.00 | 32.85 | APEP |
| ATOM | 1636 | OE1 | GLU | 205 | 3.942 | 31.984 | 43.660 | 1.00 | 31.69 | APEP |
| ATOM | 1637 | OE2 | GLU | 205 | 2.008 | 33.019 | 43.364 | 1.00 | 32.48 | APEP |
| ATOM | 1638 | C | GLU | 205 | 4.348 | 29.956 | 40.009 | 1.00 | 18.17 | APEP |
| ATOM | 1639 | O | GLU | 205 | 4.918 | 31.039 | 39.891 | 1.00 | 15.97 | APEP |
| ATOM | 1640 | N | LEU | 206 | 3.863 | 29.275 | 38.974 | 1.00 | 16.44 | APEP |
| ATOM | 1641 | CA | LEU | 206 | 3.983 | 29.785 | 37.617 | 1.00 | 15.66 | APEP |
| ATOM | 1642 | CB | LEU | 206 | 3.275 | 28.860 | 36.626 | 1.00 | 15.15 | APEP |
| ATOM | 1643 | CG | LEU | 206 | 3.869 | 27.478 | 36.368 | 1.00 | 12.15 | APEP |
| ATOM | 1644 | CD1 | LEU | 206 | 3.189 | 26.871 | 35.173 | 1.00 | 9.94 | APEP |
| ATOM | 1645 | CD2 | LEU | 206 | 5.370 | 27.579 | 36.148 | 1.00 | 10.37 | APEP |
| ATOM | 1646 | C | LEU | 206 | 3.333 | 31.155 | 37.561 | 1.00 | 15.68 | APEP |
| ATOM | 1647 | O | LEU | 206 | 3.928 | 32.125 | 37.076 | 1.00 | 16.18 | APEP |
| ATOM | 1648 | N | TYR | 207 | 2.105 | 31.217 | 38.065 | 1.00 | 14.52 | APEP |
| ATOM | 1649 | CA | TYR | 207 | 1.332 | 32.451 | 38.090 | 1.00 | 15.05 | APEP |
| ATOM | 1650 | CB | TYR | 207 | 0.742 | 32.746 | 36.705 | 1.00 | 12.49 | APEP |
| ATOM | 1651 | CG | TYR | 207 | -0.046 | 31.604 | 36.083 | 1.00 | 12.80 | APEP |
| ATOM | 1652 | CD1 | TYR | 207 | -1.379 | 31.365 | 36.441 | 1.00 | 13.16 | APEP |
| ATOM | 1653 | CE1 | TYR | 207 | -2.113 | 30.327 | 35.856 | 1.00 | 12.28 | APEP |
| ATOM | 1654 | CD2 | TYR | 207 | 0.533 | 30.774 | 35.120 | 1.00 | 14.06 | APEP |
| ATOM | 1655 | CE2 | TYR | 207 | -0.195 | 29.731 | 34.528 | 1.00 | 14.10 | APEP |
| ATOM | 1656 | CZ | TYR | 207 | -1.513 | 29.515 | 34.903 | 1.00 | 12.49 | APEP |
| ATOM | 1657 | OH | TYR | 207 | -2.220 | 28.487 | 34.332 | 1.00 | 12.47 | APEP |
| ATOM | 1658 | C | TYR | 207 | 0.206 | 32.331 | 39.113 | 1.00 | 14.67 | APEP |
| ATOM | 1659 | O | TYR | 207 | -0.088 | 31.244 | 39.595 | 1.00 | 15.57 | APEP |
| ATOM | 1660 | N | GLN | 208 | -0.425 | 33.453 | 39.432 | 1.00 | 15.61 | APEP |
| ATOM | 1661 | CA | GLN | 208 | -1.523 | 33.466 | 40.393 | 1.00 | 15.81 | APEP |
| ATOM | 1662 | CB | GLN | 208 | -1.733 | 34.892 | 40.896 | 1.00 | 14.83 | APEP |
| ATOM | 1663 | CG | GLN | 208 | -2.440 | 34.994 | 42.231 | 1.00 | 16.28 | APEP |
| ATOM | 1664 | CD | GLN | 208 | -2.843 | 36.418 | 42.564 | 1.00 | 16.96 | APEP |
| ATOM | 1665 | OE1 | GLN | 208 | -2.074 | 37.364 | 42.352 | 1.00 | 16.63 | APEP |
| ATOM | 1666 | NE2 | GLN | 208 | -4.051 | 36.581 | 43.086 | 1.00 | 16.21 | APEP |
| ATOM | 1667 | C | GLN | 208 | -2.809 | 32.947 | 39.739 | 1.00 | 17.01 | APEP |
| ATOM | 1668 | O | GLN | 208 | -3.243 | 33.469 | 38.717 | 1.00 | 14.76 | APEP |
| ATOM | 1669 | N | THR | 209 | -3.422 | 31.921 | 40.316 | 1.00 | 19.39 | APEP |
| ATOM | 1670 | CA | THR | 209 | -4.649 | 31.392 | 39.736 | 1.00 | 23.16 | APEP |
| ATOM | 1671 | CB | THR | 209 | -4.792 | 29.877 | 39.953 | 1.00 | 23.21 | APEP |
| ATOM | 1672 | OG1 | THR | 209 | -5.010 | 29.617 | 41.343 | 1.00 | 27.25 | APEP |
| ATOM | 1673 | CG2 | THR | 209 | -3.559 | 29.151 | 39.496 | 1.00 | 23.39 | APEP |
| ATOM | 1674 | C | THR | 209 | -5.873 | 32.063 | 40.340 | 1.00 | 25.33 | APEP |
| ATOM | 1675 | O | THR | 209 | -5.868 | 32.455 | 41.505 | 1.00 | 25.28 | APEP |
| ATOM | 1676 | N | LYS | 210 | -6.922 | 32.188 | 39.536 | 1.00 | 27.53 | APEP |
| ATOM | 1677 | CA | LYS | 210 | -8.160 | 32.801 | 39.986 | 1.00 | 29.31 | APEP |
| ATOM | 1678 | CB | LYS | 210 | -8.491 | 34.019 | 39.122 | 1.00 | 30.03 | APEP |
| ATOM | 1679 | CG | LYS | 210 | -8.643 | 33.696 | 37.647 | 1.00 | 28.82 | APEP |
| ATOM | 1680 | CD | LYS | 210 | -9.575 | 34.675 | 36.963 | 1.00 | 29.76 | APEP |
| ATOM | 1681 | CE | LYS | 210 | -8.897 | 35.345 | 35.771 | 1.00 | 28.88 | APEP |
| ATOM | 1682 | NZ | LYS | 210 | -9.500 | 36.669 | 35.437 | 1.00 | 28.04 | APEP |
| ATOM | 1683 | C | LYS | 210 | -9.272 | 31.775 | 39.873 | 1.00 | 31.66 | APEP |
| ATOM | 1684 | OT1 | LYS | 210 | -10.171 | 31.775 | 40.744 | 1.00 | 33.57 | APEP |
| ATOM | 1685 | OT2 | LYS | 210 | -9.224 | 30.981 | 38.906 | 1.00 | 33.91 | APEP |
| ATOM | 1686 | OH2 | WAT | 1001 | 28.321 | 31.884 | 30.023 | 1.00 | 4.99 | AWAT |
| ATOM | 1687 | OH2 | WAT | 1002 | 0.070 | 28.637 | 38.280 | 1.00 | 5.19 | AWAT |
| ATOM | 1688 | OH2 | WAT | 1003 | 9.574 | 34.984 | 40.199 | 1.00 | 6.03 | AWAT |
| ATOM | 1689 | OH2 | WAT | 1004 | 13.423 | 28.241 | 4.674 | 1.00 | 6.60 | AWAT |
| ATOM | 1690 | OH2 | WAT | 1005 | 25.593 | 14.211 | 8.905 | 1.00 | 9.08 | AWAT |
| ATOM | 1691 | OH2 | WAT | 1006 | -5.948 | 28.133 | 30.378 | 1.00 | 7.55 | AWAT |
| ATOM | 1692 | OH2 | WAT | 1007 | 13.729 | 27.746 | 30.599 | 1.00 | 8.15 | AWAT |
| ATOM | 1693 | OH2 | WAT | 1008 | 22.453 | 33.974 | 26.365 | 1.00 | 6.87 | AWAT |
| ATOM | 1694 | OH2 | WAT | 1009 | 11.644 | 46.107 | 27.594 | 1.00 | 4.61 | AWAT |
| ATOM | 1695 | OH2 | WAT | 1010 | -0.650 | 26.162 | 33.901 | 1.00 | 8.02 | AWAT |
| ATOM | 1696 | OH2 | WAT | 1011 | 8.755 | 23.060 | 34.455 | 1.00 | 10.12 | AWAT |
| ATOM | 1697 | OH2 | WAT | 1012 | 10.789 | 39.348 | 8.288 | 1.00 | 3.59 | AWAT |
| ATOM | 1698 | OH2 | WAT | 1013 | 28.091 | 15.737 | 14.912 | 1.00 | 8.00 | AWAT |
| ATOM | 1699 | OH2 | WAT | 1015 | 16.397 | 43.678 | 19.387 | 1.00 | 2.04 | AWAT |
| ATOM | 1700 | OH2 | WAT | 1016 | 14.311 | 29.731 | 32.127 | 1.00 | 5.53 | AWAT |
| ATOM | 1701 | OH2 | WAT | 1017 | 2.570 | 41.167 | 21.545 | 1.00 | 5.42 | AWAT |
| ATOM | 1702 | OH2 | WAT | 1018 | 25.364 | 28.506 | 21.332 | 1.00 | 9.41 | AWAT |
| ATOM | 1703 | OH2 | WAT | 1019 | 26.107 | 50.461 | 26.214 | 1.00 | 5.64 | AWAT |
| ATOM | 1704 | OH2 | WAT | 1020 | 30.469 | 46.598 | 34.207 | 1.00 | 7.23 | AWAT |
| ATOM | 1705 | OH2 | WAT | 1021 | 30.251 | 20.969 | 8.904 | 1.00 | 13.93 | AWAT |
| ATOM | 1706 | OH2 | WAT | 1022 | -4.476 | 37.486 | 34.043 | 1.00 | 7.76 | AWAT |
| ATOM | 1707 | OH2 | WAT | 1023 | 31.770 | 27.794 | 19.020 | 1.00 | 13.29 | AWAT |
| ATOM | 1708 | OH2 | WAT | 1024 | 17.644 | 44.091 | 30.228 | 1.00 | 8.53 | AWAT |
| ATOM | 1709 | OH2 | WAT | 1025 | -6.207 | 19.852 | 35.253 | 1.00 | 17.83 | AWAT |
| ATOM | 1710 | OH2 | WAT | 1026 | 14.737 | 24.657 | 10.954 | 1.00 | 12.71 | AWAT |
| ATOM | 1711 | OH2 | WAT | 1027 | 3.824 | 43.790 | 24.674 | 1.00 | 11.15 | AWAT |
| ATOM | 1712 | OH2 | WAT | 1028 | 7.499 | 17.209 | 26.860 | 1.00 | 18.25 | AWAT |
| ATOM | 1713 | OH2 | WAT | 1029 | 0.968 | 25.199 | 21.221 | 1.00 | 13.34 | AWAT |
| ATOM | 1714 | OH2 | WAT | 1030 | 11.738 | 36.462 | 38.807 | 1.00 | 14.39 | AWAT |
| ATOM | 1715 | OH2 | WAT | 1031 | 5.648 | 34.014 | 38.427 | 1.00 | 9.11 | AWAT |
| ATOM | 1716 | OH2 | WAT | 1032 | 1.664 | 14.320 | 37.328 | 1.00 | 15.77 | AWAT |
| ATOM | 1717 | OH2 | WAT | 1033 | 31.940 | 28.802 | 10.755 | 1.00 | 8.72 | AWAT |
| ATOM | 1718 | OH2 | WAT | 1034 | 5.832 | 22.098 | 18.171 | 1.00 | 6.17 | AWAT |
| ATOM | 1719 | OH2 | WAT | 1035 | 33.701 | 30.509 | 31.974 | 1.00 | 18.85 | AWAT |
| ATOM | 1720 | OH2 | WAT | 1036 | 29.165 | 34.668 | 37.418 | 1.00 | 10.68 | AWAT |
| ATOM | 1721 | OH2 | WAT | 1037 | -0.407 | 43.489 | 29.418 | 1.00 | 8.15 | AWAT |
| ATOM | 1722 | OH2 | WAT | 1038 | 30.861 | 44.589 | 26.320 | 1.00 | 13.36 | AWAT |
| ATOM | 1723 | OH2 | WAT | 1039 | 8.345 | 41.081 | 37.778 | 1.00 | 13.31 | AWAT |
| ATOM | 1724 | OH2 | WAT | 1040 | 10.895 | 22.815 | 23.399 | 1.00 | 20.54 | AWAT |
| ATOM | 1725 | OH2 | WAT | 1041 | 31.503 | 42.501 | 27.942 | 1.00 | 12.75 | AWAT |
| ATOM | 1726 | OH2 | WAT | 1042 | -4.123 | 17.556 | 26.927 | 1.00 | 6.26 | AWAT |
| ATOM | 1727 | OH2 | WAT | 1043 | 23.631 | 25.350 | 17.618 | 1.00 | 16.68 | AWAT |
| ATOM | 1728 | OH2 | WAT | 1044 | -9.263 | 19.789 | 28.769 | 1.00 | 17.07 | AWAT |
| ATOM | 1729 | OH2 | WAT | 1045 | 2.681 | 26.188 | 40.094 | 1.00 | 10.27 | AWAT |
| ATOM | 1730 | OH2 | WAT | 1046 | 6.157 | 33.281 | 40.876 | 1.00 | 10.99 | AWAT |
| ATOM | 1731 | OH2 | WAT | 1047 | 1.411 | 42.305 | 11.357 | 1.00 | 12.65 | AWAT |
| ATOM | 1732 | OH2 | WAT | 1048 | 11.027 | 43.128 | 8.836 | 1.00 | 13.35 | AWAT |
| ATOM | 1733 | OH2 | WAT | 1049 | 8.163 | 26.637 | 9.371 | 1.00 | 9.12 | AWAT |
| ATOM | 1734 | OH2 | WAT | 1050 | 30.812 | 52.897 | 21.367 | 1.00 | 5.26 | AWAT |
| ATOM | 1735 | OH2 | WAT | 1051 | -1.056 | 38.906 | 21.594 | 1.00 | 21.26 | AWAT |
| ATOM | 1736 | OH2 | WAT | 1052 | 23.484 | 37.806 | 38.523 | 1.00 | 5.01 | AWAT |
| ATOM | 1737 | OH2 | WAT | 1053 | 16.091 | 23.219 | 9.132 | 1.00 | 9.59 | AWAT |
| ATOM | 1738 | OH2 | WAT | 1054 | 10.515 | 44.724 | 16.202 | 1.00 | 21.22 | AWAT |
| ATOM | 1739 | OH2 | WAT | 1055 | 3.858 | 42.457 | 19.188 | 1.00 | 18.71 | AWAT |
| ATOM | 1740 | OH2 | WAT | 1056 | 20.767 | 38.301 | 29.092 | 1.00 | 7.32 | AWAT |
| ATOM | 1741 | OH2 | WAT | 1057 | 31.450 | 37.717 | 33.751 | 1.00 | 12.78 | AWAT |
| ATOM | 1742 | OH2 | WAT | 1058 | -6.469 | 15.556 | 29.885 | 1.00 | 18.83 | AWAT |
| ATOM | 1743 | OH2 | WAT | 1059 | 19.569 | 32.500 | 35.567 | 1.00 | 13.66 | AWAT |
| ATOM | 1744 | OH2 | WAT | 1060 | 12.883 | 32.203 | 45.018 | 1.00 | 19.55 | AWAT |
| ATOM | 1745 | OH2 | WAT | 1061 | 16.666 | 38.811 | 39.230 | 1.00 | 12.36 | AWAT |
| ATOM | 1746 | OH2 | WAT | 1062 | 1.627 | 24.661 | 38.597 | 1.00 | 11.62 | AWAT |
| ATOM | 1747 | OH2 | WAT | 1063 | -3.797 | 23.480 | 20.462 | 1.00 | 13.70 | AWAT |
| ATOM | 1748 | OH2 | WAT | 1064 | 19.662 | 43.909 | 20.583 | 1.00 | 17.87 | AWAT |
| ATOM | 1749 | OH2 | WAT | 1065 | 28.959 | 36.788 | 18.981 | 1.00 | 20.15 | AWAT |
| ATOM | 1750 | OH2 | WAT | 1066 | 15.034 | 47.186 | 24.909 | 1.00 | 7.01 | AWAT |
| ATOM | 1751 | OH2 | WAT | 1067 | 1.479 | 45.140 | 16.462 | 1.00 | 15.19 | AWAT |
| ATOM | 1752 | OH2 | WAT | 1068 | -9.159 | 26.374 | 32.728 | 1.00 | 10.66 | AWAT |
| ATOM | 1753 | OH2 | WAT | 1069 | 18.343 | 40.026 | 15.719 | 1.00 | 11.74 | AWAT |
| ATOM | 1754 | OH2 | WAT | 1070 | -4.926 | 34.883 | 22.765 | 1.00 | 5.57 | AWAT |
| ATOM | 1755 | OH2 | WAT | 1071 | 11.439 | 44.250 | 34.445 | 1.00 | 17.87 | AWAT |
| ATOM | 1756 | OH2 | WAT | 1072 | 22.346 | 33.157 | 38.515 | 1.00 | 15.02 | AWAT |
| ATOM | 1757 | OH2 | WAT | 1073 | 16.431 | 21.026 | -0.735 | 1.00 | 17.18 | AWAT |
| ATOM | 1758 | OH2 | WAT | 1074 | 17.273 | 13.097 | 2.769 | 1.00 | 16.44 | AWAT |
| ATOM | 1759 | OH2 | WAT | 1075 | 20.717 | 41.158 | 18.875 | 1.00 | 11.16 | AWAT |
| ATOM | 1760 | OH2 | WAT | 1076 | 13.429 | 19.165 | 10.121 | 1.00 | 9.99 | AWAT |
| ATOM | 1761 | OH2 | WAT | 1077 | 22.253 | 23.110 | 28.097 | 1.00 | 14.11 | AWAT |
| ATOM | 1762 | OH2 | WAT | 1078 | -1.729 | 14.634 | 27.851 | 1.00 | 9.23 | AWAT |
| ATOM | 1763 | OH2 | WAT | 1079 | 16.196 | 36.453 | 9.936 | 1.00 | 18.57 | AWAT |
| ATOM | 1764 | OH2 | WAT | 1080 | 26.774 | 40.940 | 39.176 | 1.00 | 15.71 | AWAT |
| ATOM | 1765 | OH2 | WAT | 1081 | 27.996 | 20.266 | 5.357 | 1.00 | 3.12 | AWAT |
| ATOM | 1766 | OH2 | WAT | 1082 | 14.345 | 44.903 | 9.828 | 1.00 | 16.53 | AWAT |
| ATOM | 1767 | OH2 | WAT | 1083 | -6.956 | 19.549 | 24.667 | 1.00 | 13.41 | AWAT |
| ATOM | 1768 | OH2 | WAT | 1084 | 6.677 | 22.676 | 16.370 | 1.00 | 9.78 | AWAT |
| ATOM | 1769 | OH2 | WAT | 1085 | 24.055 | 17.307 | 14.279 | 1.00 | 9.70 | AWAT |
| ATOM | 1770 | OH2 | WAT | 1086 | 32.348 | 29.000 | 20.500 | 1.00 | 15.00 | AWAT |
| ATOM | 1771 | OH2 | WAT | 1087 | -6.421 | 34.306 | 42.856 | 1.00 | 13.87 | AWAT |
| ATOM | 1772 | OH2 | WAT | 1088 | 28.806 | 26.184 | 27.568 | 1.00 | 16.70 | AWAT |
| ATOM | 1773 | OH2 | WAT | 1089 | 9.354 | 17.475 | 43.914 | 1.00 | 16.99 | AWAT |
| ATOM | 1774 | OH2 | WAT | 1090 | 30.672 | 20.876 | 12.987 | 1.00 | 21.44 | AWAT |
| ATOM | 1775 | OH2 | WAT | 1091 | -7.795 | 23.654 | 35.198 | 1.00 | 12.77 | AWAT |
| ATOM | 1776 | OH2 | WAT | 1092 | 6.675 | 42.663 | 7.635 | 1.00 | 17.23 | AWAT |
| ATOM | 1777 | OH2 | WAT | 1093 | 14.348 | 49.247 | 34.229 | 1.00 | 10.41 | AWAT |
| ATOM | 1778 | OH2 | WAT | 1094 | -2.481 | 40.065 | 24.802 | 1.00 | 16.54 | AWAT |
| ATOM | 1779 | OH2 | WAT | 1095 | -5.184 | 39.229 | 18.838 | 1.00 | 28.58 | AWAT |
| ATOM | 1780 | OH2 | WAT | 1096 | -6.282 | 29.165 | 17.208 | 1.00 | 27.91 | AWAT |
| ATOM | 1781 | OH2 | WAT | 1097 | 3.526 | 19.041 | 19.713 | 1.00 | 16.33 | AWAT |
| ATOM | 1782 | OH2 | WAT | 1098 | -5.490 | 40.336 | 27.666 | 1.00 | 20.30 | AWAT |
| ATOM | 1783 | OH2 | WAT | 1099 | 5.791 | 43.554 | 30.434 | 1.00 | 14.00 | AWAT |
| ATOM | 1784 | OH2 | WAT | 1100 | 10.085 | 34.242 | 9.352 | 1.00 | 19.88 | AWAT |
| ATOM | 1785 | OH2 | WAT | 1101 | 22.752 | 37.487 | 8.325 | 1.00 | 16.96 | AWAT |
| ATOM | 1786 | OH2 | WAT | 1102 | 22.364 | 40.020 | 38.129 | 1.00 | 15.64 | AWAT |
| ATOM | 1787 | OH2 | WAT | 1103 | 33.666 | 37.537 | 19.756 | 1.00 | 23.15 | AWAT |
| ATOM | 1788 | OH2 | WAT | 1104 | 36.579 | 34.638 | 23.256 | 1.00 | 16.03 | AWAT |
| ATOM | 1789 | OH2 | WAT | 1105 | 31.645 | 31.136 | 11.971 | 1.00 | 18.60 | AWAT |
| ATOM | 1790 | OH2 | WAT | 1106 | 14.823 | 26.519 | 41.694 | 1.00 | 15.23 | AWAT |
| ATOM | 1791 | OH2 | WAT | 1107 | 13.638 | 21.317 | 9.375 | 1.00 | 12.16 | AWAT |
| ATOM | 1792 | OH2 | WAT | 1108 | 33.913 | 48.939 | 32.690 | 1.00 | 11.76 | AWAT |
| ATOM | 1793 | OH2 | WAT | 1109 | 33.415 | 48.326 | 34.490 | 1.00 | 20.71 | AWAT |
| ATOM | 1794 | OH2 | WAT | 1110 | -8.560 | 41.287 | 43.725 | 1.00 | 12.63 | AWAT |
| ATOM | 1795 | OH2 | WAT | 1111 | 22.656 | 24.209 | 0.884 | 1.00 | 17.46 | AWAT |
| ATOM | 1796 | OH2 | WAT | 1112 | 2.716 | 41.252 | 15.063 | 1.00 | 19.18 | AWAT |
| ATOM | 1797 | OH2 | WAT | 1113 | 30.635 | 31.007 | 7.714 | 1.00 | 10.21 | AWAT |
| ATOM | 1798 | OH2 | WAT | 1114 | 14.815 | 22.010 | 39.023 | 1.00 | 27.49 | AWAT |
| ATOM | 1799 | OH2 | WAT | 1115 | 33.286 | 47.303 | 24.007 | 1.00 | 12.66 | AWAT |
| ATOM | 1800 | OH2 | WAT | 1116 | 14.042 | 33.412 | 10.622 | 1.00 | 10.91 | AWAT |
| ATOM | 1801 | OH2 | WAT | 1117 | 20.195 | 27.499 | 32.658 | 1.00 | 18.38 | AWAT |
| ATOM | 1802 | OH2 | WAT | 1118 | 31.215 | 17.825 | 13.678 | 1.00 | 17.20 | AWAT |
| ATOM | 1803 | OH2 | WAT | 1119 | 30.831 | 21.030 | 11.086 | 1.00 | 17.97 | AWAT |
| ATOM | 1804 | OH2 | WAT | 1120 | 30.910 | 27.311 | 25.284 | 1.00 | 17.48 | AWAT |
| ATOM | 1805 | OH2 | WAT | 1121 | 6.259 | 13.355 | 26.082 | 1.00 | 21.34 | AWAT |
| ATOM | 1806 | OH2 | WAT | 1122 | 34.780 | 29.659 | 15.089 | 1.00 | 23.24 | AWAT |
| ATOM | 1807 | OH2 | WAT | 1123 | 33.170 | 28.242 | 23.488 | 1.00 | 16.58 | AWAT |
| ATOM | 1808 | OH2 | WAT | 1124 | 0.913 | 40.672 | 41.455 | 1.00 | 17.75 | AWAT |
| ATOM | 1809 | OH2 | WAT | 1125 | 25.393 | 36.689 | 42.266 | 1.00 | 22.18 | AWAT |
| ATOM | 1810 | OH2 | WAT | 1126 | 21.923 | 40.748 | 15.035 | 1.00 | 20.08 | AWAT |
| ATOM | 1811 | OH2 | WAT | 1127 | -1.339 | 28.433 | 21.094 | 1.00 | 17.33 | AWAT |
| ATOM | 1812 | OH2 | WAT | 1128 | 22.058 | 28.769 | 33.380 | 1.00 | 22.93 | AWAT |
| ATOM | 1813 | OH2 | WAT | 1129 | 2.232 | 23.035 | 17.663 | 1.00 | 13.73 | AWAT |
| ATOM | 1814 | OH2 | WAT | 1130 | 4.834 | 40.228 | 40.117 | 1.00 | 39.84 | AWAT |
| ATOM | 1815 | OH2 | WAT | 1131 | 16.182 | 27.937 | 1.692 | 1.00 | 9.10 | AWAT |
| ATOM | 1816 | OH2 | WAT | 1132 | 36.696 | 43.322 | 33.662 | 1.00 | 14.41 | AWAT |
| ATOM | 1817 | NA | NAT | 500 | -4.312 | 15.332 | 28.374 | 1.00 | 11.67 | ANAT |
| END | | | | | | | | | | |

The solvent accessibilities of Ves v 5 amino acid residues are given in Table 7.

**Table 7 Surface Exposure of Ves v 5 amino acids**

| NO | AA | Solv exp |
|---|---|---|
| 3 | E | 0.802 |
| 4 | A | 0.060 |
| 5 | E | 0.390 |
| 6 | F | 0.868 |
| 7 | N | 0.484 |
| 8 | N | 0.555 |
| 9 | Y | 0.033 |
| 10 | C | 0.412 |
| 11 | K | 0.978 |
| 12 | I | 0.225 |
| 13 | K | 0.951 |
| 14 | C | 0.038 |
| 15 | L | 0.714 |
| 16 | K | 1.000 |
| 17 | G | 0.143 |
| 18 | G | 0.275 |
| 19 | V | 0.445 |
| 20 | H | 0.016 |
| 21 | T | 0.000 |
| 22 | A | 0.049 |
| 23 | C | 0.209 |
| 24 | K | 0.489 |
| 25 | Y | 0.280 |
| 26 | G | 0.352 |
| 27 | S | 0.159 |
| 28 | L | 0.423 |
| 29 | K | 0.797 |
| 30 | P | 0.231 |
| 31 | N | 0.396 |
| 32 | C | 0.055 |
| 33 | G | 0.429 |
| 34 | N | 0.775 |
| 35 | K | 0.297 |
| 36 | V | 0.489 |
| 37 | V | 0.280 |
| 38 | V | 0.379 |
| 39 | S | 0.291 |
| 40 | Y | 0.593 |
| 41 | G | 0.165 |
| 42 | L | 0.121 |
| 43 | T | 0.423 |
| 44 | K | 0.978 |
| 45 | Q | 0.538 |
| 46 | E | 0.264 |
| 47 | K | 0.396 |
| 48 | Q | 0.593 |
| 49 | D | 0.302 |
| 50 | I | 0.000 |
| 51 | L | 0.198 |
| 52 | K | 0.615 |
| 53 | E | 0.170 |
| 54 | H | 0.000 |
| 55 | N | 0.115 |
| 56 | D | 0.445 |
| 57 | F | 0.027 |
| 58 | R | 0.000 |
| 59 | Q | 0.198 |
| 60 | K | 0.407 |
| 61 | I | 0.000 |
| 62 | A | 0.033 |
| 63 | R | 0.956 |
| 64 | G | 0.148 |
| 65 | L | 0.593 |
| 66 | E | 0.005 |
| 67 | T | 0.610 |
| 68 | R | 0.335 |
| 69 | G | 0.110 |
| 70 | N | 0.549 |
| 71 | P | 0.363 |
| 72 | G | 0.170 |
| 73 | P | 0.440 |
| 74 | Q | 0.005 |
| 75 | P | 0.209 |
| 76 | P | 0.236 |
| 77 | A | 0.022 |
| 78 | K | 0.775 |
| 79 | N | 0.236 |
| 80 | M | 0.066 |
| 81 | K | 0.588 |
| 82 | N | 0.500 |
| 83 | L | 0.016 |
| 84 | V | 0.462 |
| 85 | W | 0.275 |
| 86 | N | 0.165 |
| 87 | D | 0.621 |
| 88 | E | 0.247 |
| 89 | L | 0.005 |
| 90 | A | 0.055 |
| 91 | Y | 0.115 |
| 92 | V | 0.005 |
| 93 | A | 0.000 |
| 94 | Q | 0.159 |
| 95 | V | 0.011 |
| 96 | W | 0.077 |
| 97 | A | 0.000 |
| 98 | N | 0.005 |
| 99 | Q | 0.027 |
| 100 | C | 0.027 |
| 101 | Q | 0.577 |
| 102 | Y | 0.687 |
| 103 | G | 0.110 |
| 104 | H | 0.549 |
| 105 | D | 0.022 |
| 106 | T | 0.429 |
| 107 | C | 0.000 |
| 108 | R | 0.203 |
| 109 | D | 0.093 |
| 110 | V | 0.044 |
| 111 | A | 0.500 |
| 112 | K | 0.824 |
| 113 | Y | 0.209 |
| 114 | Q | 0.423 |
| 115 | V | 0.011 |
| 116 | G | 0.011 |
| 117 | Q | 0.066 |
| 118 | N | 0.005 |
| 119 | V | 0.022 |
| 120 | A | 0.016 |
| 121 | L | 0.198 |
| 122 | T | 0.198 |
| 123 | G | 0.231 |
| 124 | S | 0.236 |
| 125 | T | 0.610 |
| 126 | A | 0.253 |
| 127 | A | 0.379 |
| 128 | K | 0.857 |
| 129 | Y | 0.352 |
| 130 | D | 0.220 |
| 131 | D | 0.495 |
| 132 | P | 0.033 |
| 133 | V | 0.137 |
| 134 | K | 0.654 |
| 135 | L | 0.000 |
| 136 | V | 0.000 |
| 137 | K | 0.538 |
| 138 | M | 0.473 |
| 139 | W | 0.016 |
| 140 | E | 0.071 |
| 141 | D | 0.341 |
| 142 | E | 0.154 |
| 143 | V | 0.000 |
| 144 | K | 0.560 |
| 145 | D | 0.390 |
| 146 | Y | 0.044 |
| 147 | N | 0.165 |
| 148 | P | 0.214 |
| 149 | K | 0.868 |
| 150 | K | 0.604 |
| 151 | K | 0.753 |
| 152 | F | 0.071 |
| 153 | S | 0.302 |
| 154 | G | 0.192 |
| 155 | N | 0.121 |
| 156 | D | 0.379 |
| 157 | F | 0.819 |
| 158 | L | 0.714 |
| 159 | K | 0.533 |
| 160 | T | 0.000 |
| 161 | G | 0.077 |
| 162 | H | 0.231 |
| 163 | Y | 0.000 |
| 164 | T | 0.000 |
| 165 | Q | 0.011 |
| 166 | M | 0.000 |
| 167 | V | 0.000 |
| 168 | W | 0.005 |
| 169 | A | 0.011 |
| 170 | N | 0.429 |
| 171 | T | 0.000 |
| 172 | K | 0.451 |
| 173 | E | 0.165 |
| 174 | V | 0.000 |
| 175 | G | 0.000 |
| 176 | C | 0.016 |
| 177 | G | 0.000 |
| 178 | S | 0.016 |
| 179 | I | 0.000 |
| 180 | K | 0.214 |
| 181 | Y | 0.016 |
| 182 | I | 0.275 |
| 183 | Q | 0.231 |
| 184 | E | 0.841 |
| 185 | K | 0.989 |
| 186 | W | 0.665 |
| 187 | H | 0.159 |
| 188 | K | 0.203 |
| 189 | H | 0.011 |
| 190 | Y | 0.000 |
| 191 | L | 0.000 |
| 192 | V | 0.000 |
| 193 | C | 0.000 |
| 194 | N | 0.000 |
| 195 | Y | 0.000 |
| 196 | G | 0.000 |
| 197 | P | 0.225 |
| 198 | S | 0.110 |
| 199 | G | 0.027 |
| 200 | N | 0.308 |
| 201 | F | 0.341 |
| 202 | K | 0.824 |
| 203 | N | 0.797 |
| 204 | E | 0.374 |
| 205 | E | 0.511 |
| 206 | L | 0.055 |
| 207 | Y | 0.082 |
| 208 | Q | 0.566 |
| 209 | T | 0.473 |
| 210 | K | 0.962 |

### Example 10 Alignment of Ag 5s

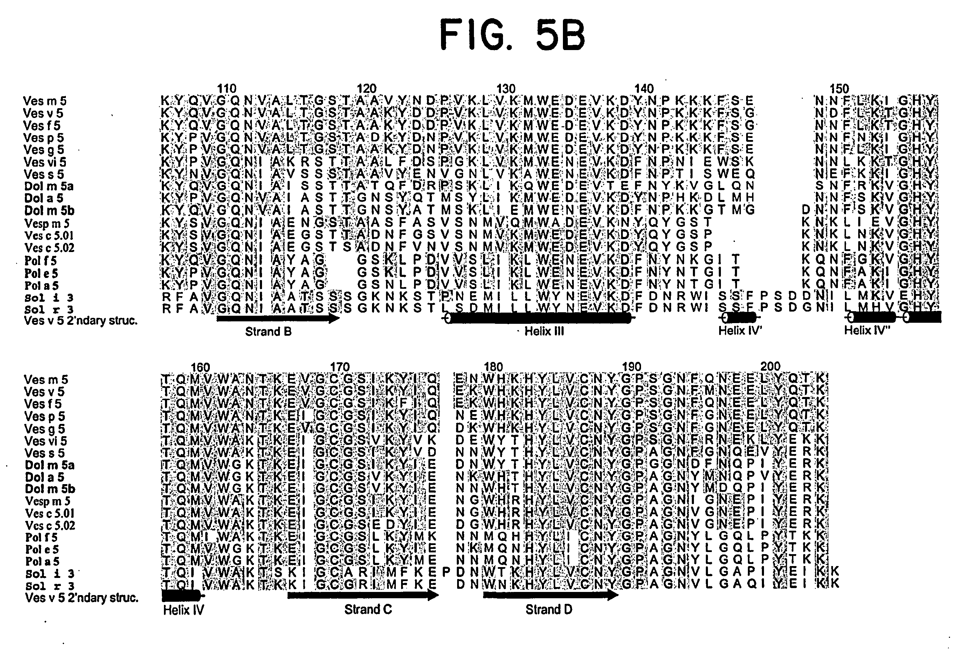

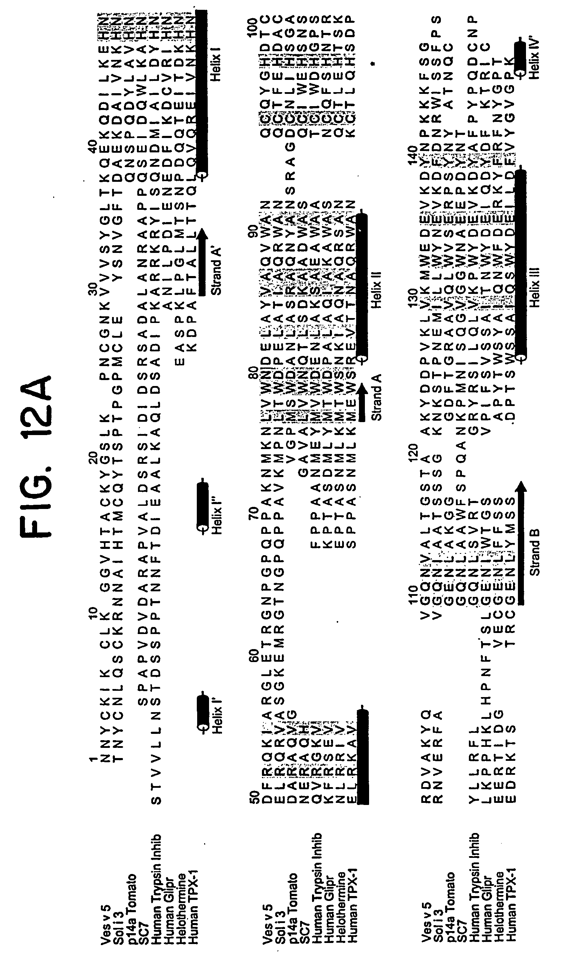

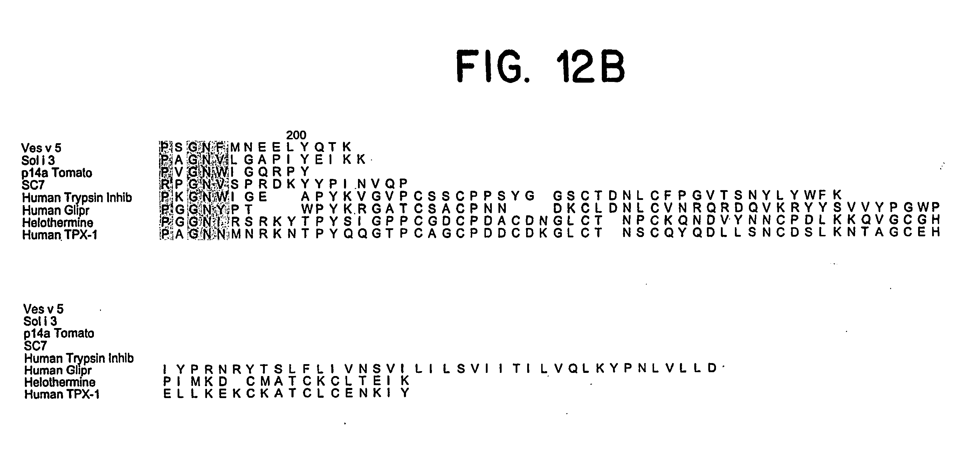

An alignment of selected antigen 5 sequences from Vespula, Dolichovespula , Vespa, Polistes and Solenopsis (fire ants) is shown in Fig. 12. Vespula, Dolichovespula, Vespa and Polistes all belong to the Vespidae family. The figure also includes the secondary structural elements of Ves v 5. When considering only the Vespula antigen 5s a very high degree of surface conservation is observed (Fig. 5), the conservation of residues being almost evenly distributed with only a few non-conserved residues scattered over the molecule.

In contrast, the surfaces conserved, when comparing sequences from the Vespula and Polistes genera, are restricted to 5 regions with solvent accessible areas of 392 Å², 585 Å², 589 Å², 673 Å² and 1053 Å², respectively. Solvent accessibility was calculated using the NACCESS program (S. J. Hubbard and J. M. Thornton, 1992, NACCESS. (v2.1.1) Department of Biochemistry and Molecular Biology, University College London) with a probe radius of 1.4 Å. Similarly, five surface patches corresponding to the 5 surface patches conserved between Vespula and Polistes, were conserved between Vespula and Vespa/Dolichovespula. In the latter case the areas are 280 Å², 496 Å², 730 Å², 803 Å² and 1043 Å², respectively. The residues contributing to one surface patch are primarily from the beginning of the B strand and from helix IV, the residues contributing to a second surface patch are primarily from the A strand and the loop between helix II and strand B, the residues contributing to a third surface patch is primarily from helix I and its surroundings and from the end of helix 11, the residues contributing to a fourth surface patch is mainly of N-terminal origin while a fifth surface patch is dominated by residues from the end of helix I and the loop between helix I and the A strand.

### DISCUSSION

Crystallographic studies of protein antigen-antibody complexes have shown that the contact residues of an epitope may contain as many as 17 residues on the surface of an antigen, and that these residues may, or may not, be contiguous to each other in the peptide chain (Davies et al., 1996, Proc. Natl Acad. Sci USA, 93:7). Epitopc mapping of lysozyme with monoclonal antibodies have shown that the entire surface of a protein is potentially antigenic (Newmann et al., 1992, J. Immunol. 149:3260). Thus the hybrids with 1/10 to 3/4 of yellow jacket antigen 5, will have fewer epitopes than the parent molecule.

The CD spectral data in Figure 7 suggest that the hybrids have secondary structures closely similar, if not identical, with those of vespid antigen 5s. The inhibition data in Figures 8 and 9 with Ves v 5-specific human and mouse antibodies and the antibody binding data in Table 3 with hybrid-specific antibodies suggest that the hybrids have tertiary structures closely similar or identical with that of Ves v 5, as these antibodies do not bind the denatured Ves v 5. Additional evidence came from screening with 17 monoclonal mouse IgG1 antibodies specific for the natural Ves v 5, six of which bound the N-terminal hybrid PV1-46. Therefore these data indicate that the hybrids contain the discontinuous B cell epitopes of Ves v 5.

The inhibition data with polyclonal antibodies and the binding data with monoclonal antibodies indicate that the dominant B cell epitopes of Ves v 5 are in its N-terminal region. Inspection of the structure of Ves v 5 in shows that nearly all residues in the N-terminal hybrid PV1-46 are surface accessible. (See Table 7) This is in contrast to the C-terminal hybrid PV156-204, in which only segments of Ves v 5 are surface accessible. (See Table 7) This difference in surface accessibility may explain the immunodominance of the N-terminal region of antigen 5. Others have shown that the entire surface of a protein is potentially antigenic but the regions with high surface accessibility and surface protrusion are dominant (Newmann et al., 1992, J. Immunol 149:3260 and Novotny et al., 1996, Adv Prot Chem 49:149).

At present the only known way to map discontinuous epitopes is by X-ray crystallography of Ag-Ab complexes (Davies et al., 1996, Proc. Natl Acad. Sci USA, 93:7) and this requires having specific monoclonal antibodies. The discontinuous epitopes of CD39 was mapped with a series of mouse-human hybrids, mouse and human CD39 molecules have 75% sequence identity and they share limited antigenic cross-reactivity (Maliszewski et al., 1994, J. Immunol 153:3574). These findings with CD39 and antigen 5 indicate that hybrids of two homologous proteins represent a useful approach to mapping their discontinuous B cell epitopes.

Our results with hybrid Ag 5s demonstrate that hybrid allergens can have a hundred to a thousand-fold reduction in allergenicity yet retain the immunogenicity of the natural allergens. This reduction in allergenicity of hybrids is believed to be mainly due to a decrease of B cell epitope density. Each hybrid of the Examples has only a portion of the B and T cell epitopes of Ves v 5. In principle, however, a mixture of hybrids can reconstitute the complete epitope library of Ves v 5. Thus, all epitopes can be reconstituted to prepare modified allergens for use as vaccines. Our results suggest that a PV hybrid with 20-30 residues of Ves v5 will have maximal reduction in allergenicity yet retaining immunogenicity for Ves v 5.

Many allergens have sequence homology with proteins from diverse sources (Larsen et al., 1996, J Allergy Clin Immunol 97:577). For example, vespid Ag 5s have varying degrees of sequence homology with a variety of extracellular proteins from different organisms, ranging from fungi to humans (see Fig. 12). It is known that homologous proteins of 30% sequence identity may have the same or closely similar structures (Chothia et al., 1990, Annual Review Biochem 59:1007 and Russell et al., 1994, J. Mol. Biol. 244:332). Thus, hybrids may be prepared with a variety of homologous host proteins to function as scaffolds for the guest allergen fragment of interest.

**Table 8. Allergens**

| **ORGANISM** | **ALLERGEN** | **PROTEIN** | **SIZE** **(kD)** | **C/P^{a}** | **REFERENCE/ ACCESSION NO** |
|---|---|---|---|---|---|
| **Weed pollens** | | | | | |
| Asterales | | | | | |
| Ambrosia artemisiifolia | | | | | |
| short ragweed | Amb a 1 | antigen E | 38 | C | 8,20 |
| | Amb a 2 | antigen K | 38 | C | 8, 21 |
| | Amb a 3 | Ra3 | 11 | C | 22 |
| | Amb a 5 | Ra5 | 5 | C | 11,23 |
| | Amb a 6 | Ra6 | 10 | P | 24,25 |
| | Amb a 7 | Ra7 | 12 | C | 26 |
| | Amb a ? | | 11 | C | 27 |
| Ambrosia trifida | | | | | |
| giant ragweed | Amb t 5 | Ra5G | 4.4 | C | 9, 10, 28 |
| Artemisia vulgaris | | | | | |
| mugwort | Art v 1 | | 27-29 | C | 28A |
| | Art v 2 | | 35 | P | 29 |
| | Art v 3 | | 12 | P | 53 |
| | Art v 4 | | 14 | C | |
| Helianthus annuus | | | | | |
| sunflower | Hel a 1 | | 34 | | 29A |
| | Hel a 2 | profilin | 15.7 | C | Y15210 |
| Mercurialis annua | | | | | |
| | Mer a 1 | profilin | 14-15 | C | Y13271 |
| | | | | | |
| Caryophyllales | | | | | |
| Salsola kali | | | | | |
| Russian thistle | Salk 1 | 43 | 43 | P | 29B |
| **Grass pollens** | | | | | |
| Poales | | | | | |
| Cynodon dactylon | | | | | |
| Bermuda grass | Cyn d 1 | | 32 | C | 30, S83343 |
| | Cyn d 7 | | | C | 31, X91256 |
| | Cyn d 12 | profilin | 14 | C | 31a, Y08390 |
| Dactylis glomerata | | | | | |
| orchard grass | Dac g1 | AgDgl | 32 | P | 32 |
| | Dac g2 | | 11 | C | 33, S45354 |
| | Dac g3 | | | C | 33A, U25343 |
| | Dac g5 | | 31 | P | 34 |
| Holcus lanatus | | | | | |
| velvet grass | Hol 11 | | | C | Z27084 |
| Lolium perenne | | | | | |
| rye grass | Lol p 1 | group I | 27 | C | 35, 36 |
| | Lol p 2 | group II | 11 | P | 37, 37A, X73363 |
| | Lol p 3 | group III | 11 | P | 38 |
| | Lol p 5 | Lol pIX, Lol p Ib | 31/35 | C | 34, 39 |
| | Lol p 11 | hom: trypsin inhibitor | 16 | | 39A |
| Phalaris aquatica | | | | | |
| canary grass | Pha a 1 | | | C | 40, S80654 |
| Phleum pratense | | | | | |
| timothy | Phl p 1 | | 27 | C | X78813 |
| | Phl p 2 | | | C | 41, X75925 |
| | Phl p 4 | | | P | 41A |
| | Phl p 5 | Ag25 | 32 | C | 42 |
| | Phl p 6 | | | C | 43, Z27082 |
| | Phl p 12 | profilin | | C | 44, X77583 |
| | Phl p 13 | polygalacturonase | 55-60 | C | AJ238848 |
| Poa pratensis | | | | | |
| Kentucky blue grass | Poa p 1 | group I | 33 | P | 46 |
| | Poa p 5 | | 31/34 | C | 34,47 |
| Sorghum halepense | | | | | |
| Johnson grass | Sor h 1 | | | C | 48 |
| Tree pollens | | | | | |
| Fagales | | | | | |
| Alnus glutinosa | | | | | |
| alder | Aln g 1 | | 17 | C | S50892 |
| Betula verrucosa | | | | | |
| birch | Bet v 1 | | 17 | C | 49,50, Z80098 |
| | Bet v 2 | profilin | 15 | C | M65179 |
| | Bet v 3 | | | C | X79267 |
| | Bet v 4 | | 8 | C | X87153, S54819 |
| | Bet v 6 | h: isoflavone reductase | 33.5 | C | AF135127 |
| | Bet v 7 | cyclophilin | 18 | P | P81531 |
| Carpinus betulus | | | | | |
| hornbeam | Car b 1 | | 17 | C | 51, X66932, |
| | | | | | X66918 |
| Castanea sativa | | | | | |
| chestnut | Cas s 1 | | 22 | P | 52 |
| | Cas s 5 | chitinase | | | |
| | Cas s 8 | lipid transfer protein | 9.7 | p | 53 |
| Corylus avellana | | | | | |
| hazel | Cor a 1 | | 17 | C | 54A, X70999 |
| | Cor a 2 | profilin | 14 | C | AF327622 |
| Quercus alba | | | | | |
| White oak | Que a 1 | | 17 | P | 54 |
| Lamiales | | | | | |
| Oleaceae | | | | | |
| Fraxinus excelsior | | | | | |
| ash | Fra e 1 | | 20 | P | 58A |
| Ligustrum vulgare | | | | | |
| privet | Lig v 1 | | 20 | P | 58A |
| Olea europea | | | | | |
| olive | Ole e 1 | | 16 | C | 59,60 |
| | Ole e 2 | profilin | 15-18 | C | 60A |
| | Ole e 3 | | 9.2 | | 60B |
| | Ole e 4 | | 32 | P | P80741 |
| | Ole e 5 | superoxide dismutase | 16 | P | P80740 |
| | Ole e 6 | | 10 | C | 60C, U86342 |
| | Ole e 7 | | | P | 60D, P81430 |
| Syringa vulgaris | | | | | |
| lilac | Syr v 1 | | 20 | P | 58A |
| Plantaginaceae | | | | | |
| Plantago lanceolata | | | | | |
| English plantain | Pla l1 | | 18 | P | P842242 |
| Pinales | | | | | |
| Cryptomeria japonica | | | | | |
| sugi | Cry j 1 | | 41-45 | C | 55,56 |
| | Cry j 2 | | | C | 57, D29772 |
| Cupressus arizonica | | | | | |
| cypress | Cup a 1 | | 43 | C | A1243570 |
| Juniperus ashei | | | | | |
| mountain cedar | Jun a 1 | | 43 | P | P81294 |
| | Jun a 2 | | | C | 57A, AJ404653 |
| | Jun a 3 | | 30 | P | 57B, P81295 |
| Juniperus oxycedrus | | | | | |
| prickly juniper | Jun o 4 | horn: calmodulin | 29 | C | 57C, AF031471 |
| Juniperus sabinoides | | | | | |
| mountain cedar | Jun s | | 50 | P | 58 |
| Juniperus virginiana | | | | | |
| eastern red cedar | Jun v 1 | | 43 | P | P81825 |
| Mites | | | | | |
| Acarus siro | | | | | |
| mite | Aca s 13 | fatty acid binding prot | 14* | C | AJ006774 |
| Blomia tropicalis | | | | | |
| mite | Blo t 5 | | | C | U59102 |
| | Blo t 12 | Bt11a | | C | U27479 |
| | Blo t 13 | Bt6, fatty acid bind prot. | | C | U58106 |
| Dermatophagoides | | | | | |
| pteronyssinus | | | | | |
| mite | Der p 1 | antigen P1 | 25 | C | 61 |
| | Der p 2 | | 14 | C | 62 |
| | Der p 3 | trypsin | 28/30 | C | 63 |
| | Der p 4 | amylase | 60 | P | 64 |
| | Der p 5 | | 14 | C | 65 |
| | Der p 6 | chymotrypsin | 25 | P | 66 |
| | Der p 7 | | 22/28 | C | 67 |
| | Der p 8 | glutathione transferase | | C | 67A |
| | Der p 9 | collagenolytic serine pro. | | P | 67B |
| | Der p 10 | tropomyosin | 36 | C | Y14906 |
| | Der p 14 | apolipophorin like prot. | | C | |
| Dermatophagoides | | | | | |
| microceras | Der m 1 | | 25 | P | 68 |
| mite | | | | | |
| Dermatophagoides farinae | | | | | |
| mite | Der f 1 | | 25 | C | 69 |
| | Der f 2 | | 14 | C | 70, 71 |
| | Der f 3 | | 30 | C | 63 |
| | Der f 10 | tropomyosin | | C | 72 |
| | Der f 11 | paramyosin | 98 | C | 72A |
| | Der f 14 | mag3, apolipophorin | | C | D17686 |
| | Derf f 15 | 98k chitinase | 98 | C | AF178772 |
| | Derf f 16 | gelsolin/villin | 53 | C | 71A |
| | Derf f 17 | Ca binding EF protein | 53 | C | 71A |
| Euroglyphus maynei | | | | | |
| mite | Eur m 14 | apolipophorin | 177 | C | AF149827 |
| Lepidoglyphus destructor | | | | | |
| storage mite | Lep d 2 | | 15 | C | 73, 74, 75 |
| | Lep d 5 | | | C | 75A, AJ 250278 |
| | Lep d 7 | | | C | 75A, AJ271058 |
| | Lep d 10 | tropomyosin | | C | AJ25096 |
| | Lep d 13 | | | C | 75A, AJ250279 |
| Animals | | | | | |
| Bos domesticus | | | | | |
| domestic cattle | Bos d 2 | Ag3, lipocalin | 20 | C | 76, L42867 |
| (see also foods) | Bos d 3 | Ca-binding S100 hom | 11 | C | L39834 |
| | Bos d 4 | alpha-lactalbumin | 14.2 | C | M18780 |
| | Bos d 5 | beta-lactoglobulin | 18.3 | C | X14712 |
| | Bos d 6 | serum albumin | 67 | C | M73993 |
| | Bos d 7 | immunoglobulin | 160 | | 77 |
| | Bos d 8 | caseins | 20-30 | | 77 |
| Canis familiaris | | | | | |
| (Canis domesticus) | Can f 1 | | 25 | C | 78, 79 |
| dog | Can f 2 | | 27 | C | 78,79 |
| | Can f 3 | albumin | | C | S72946 |
| Equus caballus | | | | | |
| domestic horse | Equ c 1 | lipocalin | 25 | C | U70823 |
| | Equ c 2 | lipocalin | 18 | P | 79A, 79B |
| | Equ c 3 | Ag3-X | 67 | C | 79C,X74045 |
| | Equ c 4 | | 17 | P | 79D |
| | Equ c 5 | AgX | 17 | P | |
| Felis domesticus | | | | | |
| cat (saliva) | Fel d 1 | cat-1 | 38 | C | 15 |
| | Fel d 2 | albumin | | C | 79E, X84842 |
| | Fel d 3 | cystatin | 11 | C | 79F, AF238996 |
| Mus musculus | | | | | |
| mouse (urine) | Mus m 1 | MUP | 19 | C | 80, 81 |
| Rattus norvegius | | | | | |
| rat (urine) | Rat n 1 | | 17 | C | 82,83 |
| **Fungi (moulds)** | | | | | |
| Ascomycota | | | | | |
| Dothidiales | | | | | |
| Alternaria alternata | Alt a 1 | | 28 | C | U82633 |
| | Alt a 2 | | 25 | C | 83A, U62442 |
| | Alt a 3 | heat shock prot. 70 | | C | U87807, U87808 |
| | Alt a 4 | prot. disulfideisomerase | 57 | C | X84217 |
| | Alt a 6 | acid ribosomal prot. P2 | 11 | C | X78222, U87806 |
| | Alt a 7 | YCP4 protein | 22 | C | X78225 |
| | Alt a 10 | aldehyde dehydrogenase | 53 | C | X78227, P42041 |
| | Alt a 11 | enolase | 45 | C | U82437 |
| | Alt a 12 | acid ribosomal prot. P1 | 11 | C | X84216 |
| Cladosporium herbarum | Cla h 1 | | 13 | | 83B, 83C |
| | Cla h 2 | | 23 | | 83B, 83C |
| | Cla h 3 | aldehyde dehydrogenase | 53 | C | X78228 |
| | Cla h 4 | acid ribosomal prot. P2 | 11 | C | X78223 |
| | Cla h 5 | YCP4 protein | 22 | C | X78224 |
| | Cla h 6 | enolase | 46 | C | X78226 |
| | Cla h 12 | acid ribosomal prot. P1 | 11 | C | X85180 |
| Eurotiales | | | | | |
| Aspergillus flavus | Asp fl 13 | alkaline serine protease | 34 | | 84 |
| Aspergillus fumigatus | Asp f 1 | | 18 | C | M83781, S39330 |
| | Asp f 2 | | 37 | C | U56938 |
| | Asp f 3 | peroxisomal protein | 19 | C | U20722 |
| | Asp f 4 | | 30 | C | AJ001732 |
| | Asp f 5 | metalloprotease | 40 | C | Z30424 |
| | Asp f 6 | Mn superoxide dismut. | 26.5 | C | U53561 |
| | Asp f 7 | | 12 | C | AJ223315 |
| | Asp f 8 | ribosomal prot. P2 | 11 | C | AJ224333 |
| | Asp f 9 | | 34 | C | AJ223327 |
| | Asp f 10 | aspartic protease | 34 | C | X85092 |
| | Asp f 11 | peptidyl-prolyl isomeras | 24 | | 84A |
| | Asp f 12 | heat shock prot. P90 | 90 | C | 85 |
| | Asp f 13 | alkaline serine protease | 34 | | 84B |
| | Asp f 15 | | 16 | C | AJ002026 |
| | Asp f 16 | | 43 | C | g3643813 |
| | Asp f 17 | | | C | AJ224865 |
| | Asp f 18 | vacuolar serine protease | 34 | | 84C |
| Aspergillus niger | Asp n 14 | beta-xylosidase | 105 | C | AF108944 |
| | Asp n 18 | vacuolar serine protease | 34 | C | 84B |
| | Asp n ? | | 85 | C | Z84377 |
| Aspergillus oryzae | Asp o 13 | alkaline serine protease | 34 | C | X17561 |
| | Asp o 21 | TAKA-amylase A | 53 | C | D00434, M33218 |
| Penicillium | Pen b 13 | alkaline serine protease | 33 | | 86A |
| brevicompactum | | | | | |
| Penicillium citrinum | Pen c 3 | peroxisomal mem prot. | 18 | | 86B |
| | Pen c 13 | alkaline serine protease | 33 | | 86A |
| | Pen c 19 | heat shock prot. P70 | 70 | C | U64207 |
| | Pen c 22w | enolase | 46 | C | AF254643 |
| Penicillium notatum | Pen n 13 | alkaline serine protease | 34 | | 89 |
| | Pen n 18 | vacuolar serine protease | 32 | | 89 |
| | Pen n 20 | N-acetyl glucosaminidas | 68 | | 87 |
| Penicillium oxalicum | Pen o 18 | vacuolar serine protease | 34 | | 89 |
| Onygenales | | | | | |
| Trichophyton rubrum | Tri r 2 | | | C | 90 |
| | Tri r 4 | serine protease | | C | 90 |
| Trichophyton tonsurans | Tri t 1 | | 30 | P | 91 |
| | Tri t 4 | serine protease | 83 | C | 90 |
| Saccharomycetales | | | | | |
| Candida albicans | Cand a 1 | | 40 | C | 88 |
| Candida boidinii | Cand b 2 | | 20 | C | J04984, J04985 |
| Basidiomycota | | | | | |
| Basidiolelastomycetes | | | | | |
| Malassezia furfur | Mala f 1 | | | | 91A |
| | Mala f 2 | MF1, peroxisomal | 21 | C | AB011804 |
| | | membrane protein | | | |
| | Mala f3 | MF2, peroxisomal | 20 | C | AB011805 |
| | | membrane protein | | | |
| | Mala f 4 | | 35 | C | |
| | Mala f 5 | | 18* | C | AJ011955 |
| | Mala f 6 | | 17* | C | AJ011956 |
| Basidiomycetes | | | | | |
| Psilocybe cubensis | Psi c 1 | | | | |
| | Psi c 2 | cyclophilin | 16 | | 91B |
| Coprinus comatus | | | | | |
| shaggy cap | Cop c 1 | leucine zipper protein | 11 | C | AJ132235 |
| | Cop c 2 | | | | AJ242791 |
| | Cop c 3 | | | | AJ242792 |
| | Cop c 5 | | | | AJ242793 |
| | Cop c 7 | | | | AJ242794 |
| Insects | | | | | |
| Aedes aegyptii | | | | | |
| mosquito | Aed a 1 | apyrase | 68 | C | L12389 |
| | Aed a 2 | | 37 | C | M33157 |
| Apis mellifera | | | | | |
| honey bee | Api m 1 | phospholipase A2 | 16 | C | 92 |
| | Api m 2 | hyaluronidase | 44 | C | 93 |
| | Api m 4 | melittin | 3 | C | 94 |
| | Api m 6 | | 7-8 | P | |
| Bombus pennsylvanicus | | | | | |
| bumble bee | Bom p 1 | phospholipase | 16 | P | 95 |
| | Bom p 4 | protease | | P | 95 |
| Blattella germanica | | | | | |
| German cockroach | Bla g 1 | Bd90k | | C | |
| | Bla g 2 | aspartic protease | 36 | C | 96 |
| | Bla g 4 | calycin | 21 | C | 97 |
| | Bla g 5 | glutathione transferase | 22 | C | 98 |
| | Bla g 6 | troponin C | 27 | C | 98 |
| Periplaneta americana | | | | | |
| American cockroach | Per a 1 | Cr-PII | | C | |
| | Per a 3 | Cr-PI | 72-78 | C | 98A |
| | Per a 7 | tropomyosin | 37 | C | Y14854 |
| Chironomus thummi | | | | | |
| thummi | Chi t 1-9 | hemoglobin | 16 | C | 99 |
| midges | Chi t 1.01 | component III | 16 | C | P02229 |
| | Chi t 1.02 | component IV | 16 | C | P02230 |
| | Chi t 2.0101 | component I | 16 | C | P02221 |
| | Chi t 2.0102 | component IA | 16 | C | P02221 |
| | Chi t 3 | component II-beta | 16 | C | P02222 |
| | Chi t 4 | component IIIA | 16 | C | P02231 |
| | Chi t 5 | component VI | 16 | C | P02224 |
| | Chi t 6.01 | component VIIA | 16 | C | P02226 |
| | Chi t 6.02 | component IX | 16 | C | P02223 |
| | Chi t 7 | component VIIB | 16 | C | P02225 |
| | Chi t 8 | component VIII | 16 | C | P02227 |
| | Chi t 9 | component X | 16 | C | P02228 |
| Dolichovespula maculata | | | | | |
| white face hornet | Dol m 1 | phospholipase A1 | 35 | C | 100 |
| | Dol m 2 | hyaluronidase | 44 | C | 101 |
| | Dol m 5 | antigen 5 | 23 | C | 102, 103 |
| Dolichovespula arenaria | | | | | |
| yellow hornet | Dol a 5 | antigen 5 | 23 | C | 104 |
| Polistes annularies | | | | | |
| wasp | Pol a 1 | phospholipase A1 | 35 | P | 105 |
| | Pol a 2 | hyaluronidase | 44 | P | 105 |
| | Pol a 5 | antigen 5 | 23 | C | 104 |
| Polistes dominulus | | | | | |
| Mediterranean paper wasp | Pol d 1 | | | | |
| | Pol d 4 | serine protease | 32-34 | C | |
| | Pol d 5 | | | | P81656 |
| Polistes exclamans | | | | | |
| wasp | Pol e 1 | phospholipase A1 | 34 | P | 107 |
| | Pol e 5 | antigen 5 | 23 | C | 104 |
| Polistes fuscatus | | | | | |
| wasp | Pol f 5 | antigen 5 | 23 | C | 106 |
| Polistes metricus | | | | | |
| wasp | Pol m 5 | antigen 5 | 23 | C | 106 |
| Vespa crabo | | | | | |
| European hornet | Vesp c 1 | phospholipase | 34 | P | 107 |
| | Vesp c 5 | antigen 5 | 23 | C | 106 |
| Vespa mandarina | | | | | |
| giant asian hornet | Vesp m 1 | | | | |
| | Vesp m 5 | | | | P81657 |
| Vespula flavopilosa | | | | | |
| yellowjacket | Ves f 5 | antigen 5 | 23 | C | 106 |
| Vespula germanica | | | | | |
| yellowjacket | Ves g5 | antigen 5 | 23 | C | 106 |
| Vespula maculifrons | | | | | |
| yellowjacket | Ves m 1 | phospholipase A1 | 33.5 | C | 108 |
| | Ves m 2 | hyaluronidase | 44 | P | 109 |
| | Ves m 5 | antigen 5 | 23 | C | 104 |
| Vespula pennsylvanica | | | | | |
| yellowjacket | Ves p 5 | antigen 5 | 23 | C | 106 |
| Vespula squamosa | | | | | |
| yellowjacket | Ves s 5 | antigen 5 | 23 | C | 106 |
| Vespula vidua | | | | | |
| wasp | Ves vi 5 | antigen 5 | 23 | C | 106 |
| Vespula vulgaris | | | | | |
| yellowjacket | Ves v 1 | phospholipase A1 | 35 | C | 105A |
| | Ves v 2 | hyaluronidase | 44 | P | 105A |
| | Ves v 5 | antigen 5 | 23 | C | 104 |
| Myrmecia pilosula | | | | | |
| Australian jumper ant | | Myr p 1 C | | | X70256 |
| | Myr p 2 | | | C | S81785 |
| Solenopsis geminata | | | | | |
| tropical fire ant | Sol g 2 | | | | |
| | Sol g 4 | | | | |
| Solenopsis invicta | | | | | |
| fire ant | Sol i 2 | | 13 | C | 110, 111 |
| | Sol i 3 | | 24 | C | 110 |
| | Sol i 4 | | 13 | C | 110 |
| Solenopsis saevissima | | | | | |
| Brazilian fire ant | Sol s 2 | | | | |
| Foods | | | | | |
| Gadus callarias | | | | | |
| cod | Gad c 1 | allergen M | 12 | C | 112, 113 |
| Salmo salar | | | | | |
| Atlantic salmon | Sal s 1 | parvalbumin | 12 | C | X97824 |
| Bos domesticus | | | | | |
| domestic cattle | Bos d 4 | alpha-lactalbumin | 14.2 | C | M18780 |
| (milk) | Bos d 5 | beta-lactoglobulin | 18.3 | C | X14712 |
| | Bos d 6 | serum albumin | 67 | C | M73993 |
| | Bos d 7 | immunoglobulin | 160 | | 77 |
| | Bos d 8 | caseins | 20-30 | | 77 |
| Gallus domesticus | | | | | |
| chicken | Gal d 1 | ovomucoid | 28 | C | 114, 115 |
| | Gal d 2 | ovalbumin | 44 | C | 114, 115 |
| | Gal d 3 | Ag22, conalbumin | 78 | C | 114, 115 |
| | Gal d 4 | lysozyme | 14 | C | 114, 115 |
| | Gal d 5 | serum albumin | 69 | C | X60688 |
| Metapenaeus ensis | | | | | |
| shrimp | Met e 1 | tropomyosin | C | | U08008 |
| Penaeus aztecus | | | | | |
| shrimp | Pen a 1 | tropomyosin | 36 | P | 116 |
| Penaeus indicus | | | | | |
| shrimp | Pen i 1 | tropomyosin | 34 | C | 117 |
| Todarodes pacificus | | | | | |
| squid | Tod p 1 | tropomyosin | 38 | P | 117A |
| Haliotis midae | | | | | |
| abalone | Hal m 1 | | 49 | | 117B |
| Apium graveolens | | | | | |
| celery | Api g 1 | hom: Bet v 1 | 16* | C | Z48967 |
| | Api g 4 | profilin | | | AF129423 |
| | Api g 5 | | 55/58 | P | P81943 |
| Brassica juncea | | | | | |
| oriental mustard | Bra j 1 | 2S albumin | 14 | C | 118 |
| Brassica rapa | | | | | |
| turnip | Bra r 2 | hom: prohevein | 25 | | P81729 |
| Hordeum vulgare | | | | | |
| barley | Hor v 15 | BMAI-1 | 15 | C | 119 |
| Zea mays | | | | | |
| maize, corn | Zea m 14 | lipid transfer protein | 9 | P | P19656 |
| Oryza sativa | | | | | |
| rice | | Ory s 1 | | | U31771 |
| Corylus avellana | | | | | |
| hazelnut | Cor a 1.0401 | hom: Bet v 1 | 17 | C | AF136945 |
| Malus domestica | | | | | |
| apple | Mal d 1 | hom: Bet v 1 | | C | X83672 |
| | Mal d 2 | hom: thaumatin | | C | AJ243427 |
| | Mal d 3 | lipid transfer protein | 9 | C | |
| Pyrus communis | | | | | |
| pear | Pyr c 1 | hom: Bet v 1 | 18 | C | AF05730 |
| | Pyr c 4 | profilin | 14 | C | AF129424 |
| | Pyr c 5 | hom: isoflavone reductas | 33.5 | C | AF071477 |
| Persea americana | | | | | |
| avocado | Pers a 1 | endochitinase | 32 | C | Z78202 |
| Prunus anneniaca | | | | | |
| apricot | Pru ar 1 | hom: Bet v 1 | | C | U93165 |
| | Pru ar 3 | lipid transfer protein | 9 | P | |
| Prunus avium | | | | | |
| sweet cherry | Pru av 1 | hom: Bet v 1 | | C | U66076 |
| | Pru av 2 | hom: thaumatin | | C | U32440 |
| | Pru av 3 | lipid transfere protein | 10 | C | AF221501 |
| | Pru av 4 | profilin | 15 | C | AF129425 |
| Prunus domestica | | | | | |
| European plum | Pru d 3 | lipid transfer protein | 9 | P | 119A |
| Prunus persica | | | | | |
| peach | Pru p 3 | lipid transfer protein | 10 | P | P81402 |
| Vitis vinifera | | | | | |
| grape | Vit v 1 | lipid transfer protein | 9 | P | P80274 |
| Musa x paradisiaca | | | | | |
| banana | Mus xp 1 | profilin | 15 | C | AF377948 |
| Ananas comosus | | | | | |
| pineapple | Ana c 1 | profilin | 15 | C | AF377949 |
| Lichti chinensis | | | | | |
| litchi | Lit c 1 | profilin | 15 | C | AY049013 |
| Sinapis alba | | | | | |
| yellow mustard | Sin a 1 | 2S albumin | 14 | C | 120 |
| Glycine max | | | | | |
| soybean | Gly m 1 | HPS | 7 | P | 121 |
| | Gly m 2 | | 8 | P | A57106 |
| | Gly m 3 | profilin | 14 | C | AJ223982 |
| Arachis hypogaea | | | | | |
| peanut | Ara h 1 | vicilin | 63.5 | C | L34402 |
| | Ara h 2 | conglutin | 17 | C | L77197 |
| | Ara h 3 | glycinin | 60 | C | AF093541 |
| | Ara h 4 | glycinin | 37 | C | AF086821 |
| | Ara h 5 | profilin | 15 | C | AF059616 |
| | Ara h 6 | hom: conglutin | 15 | C | AF092846 |
| | Ara h 7 | hom: conglutin | 15 | C | AF091737 |
| Actinidia chinensis | | | | | |
| kiwi | Act c 1 | cysteine protease | 30 | P | P00785 |
| Capsicum annum | | | | | |
| bell pepper | Cap a 1w | osmotin-like protein | 23 | c | AJ297410 |
| Solanum tuberosum | | | | | |
| potato | Sola t 1 | patatin | 43 | P | P15476 |
| | Sola t 2 | cathepsin D inhibitor | 21 | P | P16348 |
| | Sola t 3 | cys. protease inhibitor | 21 | P | P20347 |
| | Sola t 4 | asp. protease inhibitor | 16+4 | P | P30941 |
| Bertholletia excelsa | | | | | |
| Brazil nut | Ber e | 2S albumin | 9 | C | P04403, M17146 |
| Juglans regia | | | | | |
| English walnut | Jug r 1 | 2S albumin | | C | U66866 |
| | Jug r 2 | vicilin | 44 | C | AF066055 |
| Ricinus communis | | | | | |
| Castor bean | Ric c 1 | 2S albumin | | C | P01089 |
| Sesamum indicum | | | | | |
| sesame | Ses i 1 | 2S albumin | 9 | C | 121A, AF240005 |
| | Ses i 2 | 2S albumin | 7 | C | AF091841 |
| | Ses i 3 | 7S vicilin-like globulin | 45 | C | AF240006 |
| Cucumis melo | | | | | |
| muslanelon | Cuc m 1 | serine protease | 66 | C | D32206 |
| **Additional:** | | | | | |
| Anisakis simplex | Ani s 1 | | 24 | P | 121B, A59069 |
| nematode | Ani s 2 | paramyosin | 97 | C | AF173004 . |
| | Ani s 3 | tropomyosin | 41 | C | 121C, Y19221 |
| Ascaris suum | | | | | |
| worm | Asc s 1 | | 10 | P | 122 |
| Dendronephthya nipponica | | | | | |
| soft coral | Den n 1 | | 53 | P | 122A |
| Hevea brasiliensis | | | | | |
| rubber (latex) | Hev b 1 | elongation factor | 58 | P | 123, 124 |
| | Hev b 2 | 1,3-glucanase | 34/36 | C | 125 |
| | Hev b 3 | | 24 | P | 126, 127 |
| | Hev b 4 | component of microhelix complex | 100-115 | P | 128 |
| | Hev b 5 | | 16 | C | U42640 |
| | Hev b 6.01 | hevein precursor | 20 | C | M36986, p02877 |
| | Hev b 6.02 | hevein | 5 | C | M36986, p02877 |
| | Hev b 6.03 | C-terminal fragment | 14 | C | M36986, p02877 |
| | Hev b 7.01 | hom: patatin from B-serum | 42 | C | U80598 |
| | Hev b 7.02 | hom: patatin from C-serum | 44 | C | AJ223038 |
| | Hev b 8 | profilin | 14 | C | Y15042, |
| | | | | | AJ132397, |
| | | | | | AF 119365, |
| | | | | | AF119366 |
| | Hev b 9 | enolase | 51 | C | AJ132580 |
| | Hev b 10 | Mn superoxide dismut. | 26 | C | AJ249148 |
| | Hev b 11w | class 1 chitinase | | C | AJ238579 |
| | Hev b 12 | lipid transfer protein | 9.3 | C | |
| Ctenocephalides felis felis | | | | | |
| cat flea | Cte f 1 | | | | |
| | Cte f 2 | M1b | 27 | C | AF231352 |
| Homo sapiens | | | | | |
| human autoallergens | Hom s 1 | | 73* | C | Y14314 |
| | Hom s 2 | | 10.3* | C | X80909 |
| | Hom s 3 | | 20.1* | C | X89985 |
| | Hom s 4 | | 36* | C | Y17711 |
| | Hom s 5 | | 42.6* | C | P02538 |

| | | | | | |
|---|---|---|---|---|---|
| ^{a}Clone (C) or Protein (P) data | | | | | |

### References to Appendix I

1. Marsh, D.G., and L.R. Freidhoff. 1992. ALBE, an allergen database. IUIS, Baltimore, MD, Edition 1.0.
2. Marsh, D. G., L. Goodfriend, T. P. King, H. Lowenstein, and T. A. E. Platts-Mills. 1986. Allergen nomenclature. Bull WHO 64:767-770.
3. King, T.P., P.S. Norman, and J.T. Cornell. 1964. Isolation and characterization of allergen from ragweed pollen. II. Biochemistry 3:458-468.
4. Lowenstein, H. 1980. Timothy pollen allergens. Allergy 35:188-191.
5. Aukrust, L. 1980. Purification of allergens in Cladosporium herbarum. Allergy 35:206-207.
6. Demerec, M., E. A. Adelberg, A. J. Clark, and P. E. Hartman. 1966. A proposal for a uniform nomenclature in bacterial genetics. Genetics 54:61-75.
7. Bodmer, J. G., E. D. Albert, W. F. Bodmer, B. Dupont, H. A. Erlich, B. Mach, S. G. E. Marsh, W. R. Mayr, P. Parham, T. Sasuki, G. M. Th. Schreuder, J. L. Strominger, A. Svejgaard, and P. I. Terasaki. 1991. Nomenclature for factors of the HLA system, 1990. Immunogenetics 33:301-309.
8. Griffith, I.J., J. Pollock, D.G. Klapper, B.L. Rogers, and A.K. Nault. 1991. Sequence polymorphism of Amb a I and Amb a II, the major allergens in Ambrosia artemisiifolia (short ragweed). Int. Arch. Allergy Appl. Immunol. 96:296-304.
9. Roebber, M., D. G. Klapper, L. Goodfriend, W. B. Bias, S. H. Hsu, and D. G. Marsh. 1985. Immunochemical and genetic studies of Amb t V (Ra5G), an Ra5 homologue from giant ragweed pollen. J. Immunol. 134:3062-3069.
10. Metzler, W. J., K. Valentine, M. Roebber, M. Friedrichs, D. G. Marsh, and L. Mueller. 1992. Solution structures of ragweed allergen Amb t V. Biochemistry 31:5117-5127.
11. Metzler, W. J., K. Valentine, M. Roebber, D. G. Marsh, and L. Mueller. 1992. Proton resonance assignments and three-dimensional solution structure of the ragweed allergen Amb a V by nuclear magnetic resonance spectroscopy. Biochemistry 31:8697-8705.
12. Goodfriend, L., A.M. Choudhury, J. Del Carpio, and T.P. King. 1979. Cytochromes C: New ragweed pollen allergens. Fed. Proc. 38:1415.
13. Ekramoddoullah, A. K. M., F. T. Kisil, and A. H. Sehon. 1982. Allergenic cross reactivity of cytochrome c from Kentucky bluegrass and perennial ryegrass pollens. Mol. Immunol. 19:1527-1534.
14. Ansari; A. A., E. A. Killoran, and D. G. Marsh. 1987. An investigation of human response to perennial ryegrass (Lolium perenne) pollen cytochrome c (Lol p X). J. Allergy Clin. Immunol. 80:229-235.
15. Morgenstern, J.P., I.J. Griffith, A.W. Brauer, B.L. Rogers, J.F. Bond, M.D. Chapman, and M. Kuo. 1991. Amino acid sequence of Fel d I, the major allergen of the domestic cat: protein sequence analysis and cDNA cloning. Proc. Natl. Acad. Sci. USA 88:9690-9694.
16. Griffith, I.J., S. Craig, J. Pollock, X. Yu, J.P. Morgenstern, and B.L.Rogers. 1992. Expression and genomic structure of the genes encoding FdI, the major allergen from the domestic cat. Gene 113:263-268.
17. Weber, A., L. Marz, and F. Altmann. 1986. Characteristics of the asparagine-linked oligosaccharide from honey-bee venom phospholipase A2. Comp. Biochem. Physiol. 83B:321-324.
18. Weber, A., H. Schroder, K. Thalberg, and L. Marz. 1987. Specific interaction of IgE antibodies with a carbohydrate epitope of honey bee venom phospholipase A2. Allergy 42:464-470.
19. Stanworth, D. R., K. J. Dorrington, T. E. Hugli, K. Reid, and M. W. Turner. 1990. Nomenclature for synthetic peptides representative of immunoglobulin chain sequences. Bulletin WHO 68:109-111.
20. Rafnar, T., I. J. Griffith, M. C. Kuo, J. F. Bond, B. L. Rogers, and D.G. Klapper. 1991. Cloning of Amb a I (Antigen E), the major allergen family of short ragweed pollen. J. Biol. Chem. 266: 1229-1236.
21. Rogers, B.L., J.P. Morgenstern, I.J. Griffith, X.B. Yu, C.M. Counsell, A.W. Brauer, T.P. King, R.D. Garman, and M.C. Kuo. 1991. Complete sequence of the allergen Amb a II: recombinant expression and reactivity with T cells from ragweed allergic patients. J. Immunol. 147:2547-2552.
22. Klapper, D.G., L. Goodfriend, and J.D. Capra. 1980. Amino acid sequence of ragweed allergen Ra3. Biochemistry 19:5729-5734.
23. Ghosh, B., M.P. Perry, T. Rafnar, and D.G. Marsh. 1993. Cloning and expression of immunologically active recombinant Amb a V allergen of short ragweed (Ambrosia artemisiifolia) pollen. J. Immunol. 150:5391-5399.
24. Roebber, M., R. Hussain, D. G. Klapper, and D. G. Marsh. 1983. Isolation and properties of a new short ragweed pollen allergen, Ra6. J. Immunol. 131:706-711.
25. Lubahn, B., and D.G. Klapper. 1993. Cloning and characterization of ragweed allergen Amb a VI (abst). J. Allergy Clin. Immunol. 91:338.
26. Roebber, M., and D.G. Marsh. 1991. Isolation and characterization of allergen Amb a VII from short ragweed pollen. J. Allergy Clin. Immunol. 87:324.
27. Rogers, B.L., J. Pollock, D.G. Klapper, and I.J. Griffith. 1993. Cloning, complete sequence, and recombinant expression of a novel allergen from short ragweed pollen (abst). J. Allergy Clin. Immunol. 91:339.
28. Goodfriend, L., A.M. Choudhury, D.G. Klapper, K.M. Coulter, G. Dorval, J. DelCarpio, and C.K. Osterland. 1985. Ra5G, a homologue of Ra5 in giant ragweed pollen: isolation, HLA-DR-associated activity and amino acid sequence. Mol. Immunol. 22:899-906.
28A. Breitenbach M, pers. comm.
29. Nilsen, B. M., K. Sletten, M. O'Neill, B. Smestead Paulsen, and H. van Halbeek. 1991. Structural analysis of the glycoprotein allergen Art v II from pollen of mugwort (Artemesia vulgaris). J. Biol. Chem. 266:2660-2668.
29A Jimenez A, Moreno C, Martinez J, Martinez A, Bartolome B, Guerra F, Palacios R 1994. Sensitization to sunflower pollen: only an occupational allergy? Int Arch Allergy Immunol 105:297-307.
29B. Carnés·J, Fernández-Caldas E, Casanovas M, Lahoz C, Colás C. Immunochemical characterization of Salsola kali pollen extracts. Allergy 56, Supplement 68: 274, 2001.
29C. Giuliani A, Pini C, Bonini S, Mucci N, Ferroni L, Vicari G: Isolation and purification of a major allergen from Parietaria officinalis pollen. Allergy 42: 434-440, 1987.
30. Smith,P.M., Suphioglu,C., Griffith,I.J., Theriault,K., Knox,R.B. and Singh,M.B. 1996. Cloning and expression in yeast Pichia pastoris of a biologically active form of Cyn d 1, the major allergen of Bermuda grass pollen. J. Allergy Clin. Immunol. 98:331-343.
31. Suphioglu,C., Ferreira,F. and Knox,R.B. 1997. Molecular cloning and immunological characterisation of Cyn d 7, a novel calcium-binding allergen from Bermuda grass pollen. FEBS Lett. 402:167-172.
31a. Asturias JA, Arilla MC, Gomez-Bayon N, Martinez J, Martinez A, and Palacios R. 1997. Cloning and high level expression of Cynodon dactylon (Bermuda grass) pollen profilin (Cyn d 12) in Escherichia coli: purification and characterization of the allergen. Clin Exp Allergy 27:1307-1313.
32. Mecheri, S., G. Peltre, and B. David. 1985. Purification and characterization of a major allergen from Dactylis glomerata pollen: The Ag Dg 1. Int. Arch. Allergy Appl. Immunol. 78:283-289.
33. Roberts, A.M., L.J. Bevan, P.S. Flora, I. Jepson, and M.R. Walker. 1993. Nucleotide sequence of cDNA encoding the Group II allergen of Cocksfoot/Orchard grass (Dactylis glomerata), Dac g II. Allergy 48:615-623.
33a. Guerin-Marchand,C., Senechal,H., Bouin,A.P., Leduc-Brodard,V., Taudou,G., Weyer,A., Peltre,G. and David,B. 1996. Cloning, sequencing and immunological characterization of Dac g 3, a major allergen from Dactylis glomerata pollen. Mol. Immunol. 33:797-806.
34. Klysner, S., K. Welinder, H. Lowenstein, and F. Matthiesen. 1992. Group V allergens in grass pollen IV. Similarities in amino acid compositions and amino terminal sequences of the group V allergens from Lolium perenne, Poa pratensis and Dactylis glomerata. Clin. Exp. Allergy 22:491-497.
35. Perez, M., G. Y. Ishioka, L. E. Walker, and R. W. Chesnut. 1990. cDNA cloning and immunological characterization of the rye grass allergen Lol p I. J. Biol. Chem. 265:16210-16215.
36. Griffith, I. J., P. M. Smith, J. Pollock, P. Theerakulpisut, A. Avjioglu, S. Davies, T. Hough, M. B. Singh, R. J. Simpson, L. D. Ward, and R. B. Knox. 1991. Cloning and sequencing of Lol p I, the major allergenic protein of rye-grass pollen. FEBS Letters 279:210-215.
37. Ansari, A. A., P. Shenbagamurthi, and D.G. Marsh. 1989. Complete amino acid sequence of a Lolium perenne (perennial rye grass) pollen allergen, Lol p II. J. Biol. Chem. 264:11181-11185.
37a. Sidoli,A., Tamborini,E., Giuntini,I., Levi,S., Volonte,G., Paini,C., De Lalla,C., Siccardi,A.G., Baralle,F.E., Galliani,S. and Arosio,P. 1993. Cloning, expression, and immunological characterization of recombinant Lolium perenne allergen Lol p II. J. Biol. Chem. 268:21819-21825.
38. Ansari, A. A., P. Shenbagamurthi, and D. G. Marsh. 1989. Complete primary structure of a Lolium perenne (perennial rye grass) pollen allergen, Lol p III: Comparison with known Lol p I and II sequences. Biochemistry 28:8665-8670.
39. Singh, M. B., T. Hough, P. Theerakulpisut, A. Avjioglu, S. Davies, P. M. Smith, P. Taylor, R. J. Simpson, L. D. Ward, J. McCluskey, R. Puy, and R.B. Knox. 1991. Isolation of cDNA encoding a newly identified major allergenic protein of rye-grass pollen: Intracellular targeting to the amyloplost. Proc. Nat1. Acad. Sci. 88:1384-1388.
39a. van Ree R, Hoffman DR, van Dijk W, Brodard V, Mahieu K, Koeleman CA, Grande M, van Leeuwen WA, Aalberse RC. 1995. Lol p XI, a new major grass pollen allergen, is a member of a family of soybean trypsin inhibitor-related proteins. J Allergy Clin Immunol 95:970-978.
40. Suphióglu, C. and Singh,M.B. 1995. Cloning, sequencing and expression in Escherichia coli of Pha a 1 and four isoforms of Pha a 5, the major allergens of canary grass pollen. Clin. Exp. Allergy 25:853-865.
41. Dolecek,C., Vrtala,S., Laffer,S., Steinberger,P., Kraft,D., Scheiner,O. and Valenta,R. 1993. Molecular characterization of Phl p II, a major timothy grass (Phleum pratense) pollen allergen. FEBS Lett. 335:299-304.
41A. Fischer S, Grote M, Fahlbusch B, Muller WD, Kraft D, Valenta R. 1996. Characterization of Phl p 4, a major timothy grass (Phleum pratense) pollen allergen. J Allergy Clin Immunol 98:189-198.
42. Matthiesen, F., and H. Lowenstein. 1991. Group V allergens in grass pollens. I. Purification and characterization of the group V allergen from Phleum pratense pollen, Phl p V. Clin. Exp. Allergy 21:297-307.
43. Petersen,A., Bufe,A., Schramm,G., Schlaak,M. and Becker,W.M. 1995. Characterization of the allergen group VI in timothy grass pollen (Phl p 6). II. cDNA cloning of Phl p 6 and structural comparison to grass group V. Int. Arch. Allergy Immunol. 108:55-59.
44. Valenta,R., Ball,T., Vrtala,S., Duchene,M., Kraft,D. and Scheiner,O. 1994. cDNA cloning and expression of timothy grass (Phleum pratense) pollen profilin in Escherichia coli: comparison with birch pollen profilin. Biochem. Biophys. Res. Commun. 199:106-118.
46. Esch, R. E., and D. G. Klapper. 1989. Isolation and characterization of a major cross-reactive grass group I allergenic determinant. Mol. Immunol. 26:557-561.
47. Olsen, E., L. Zhang, R. D. Hill, F. T. Kisil, A. H. Sehon, and S. Mohapatra. 1991. Identification and characterization of the Poa p IX group of basic allergens of Kentucky bluegrass pollen. J. Immunol. 147:205-211.
48. Avjioglu, A., M. Singh, and R.B. Knox. 1993. Sequence analysis of Sor h I, the group I allergen of Johnson grass pollen and it comparison to rye-grass Lol p I (abst). J. Allergy Clin. Immunol. 91:340.
49. Breiteneder H, Pettenburger K, Bito A, Valenta R, Kraft D, Rumpold H, Scheiner O, Breitenbach M. The gene coding for themajor birch pollen allergen BetvI, is highly homologus to a pea disease resistance response gene. EMBO J. 8: 1935-1938,1989.
50. Swoboda I, Jilek A, Ferreira F, Engel E, Hoffman-Sommergruber K, Scheiner O, Kraft D, Breiteneder H, Pittenauer E,Schmid E. Isoforms of Bet v1, the major birch pollen allergen, analyzed by liquid chromatography, mass spectrometry, and cDNA cloning. J. Biol. Chem. 270: 2607-2613, 1995.
51. Larsen JN, Stroman P, Ipsen H. PCR based cloning and sequencing of isogenes encoding the tree pollen major allergen Car b I from Carpinus betulus, hornbeam. Mol. Immunol. 29: 703-711, 1992.
52. Kos T, Hoffmann-Sommergruber K, Ferreira F, Hirschwehr R, Ahorn H, Horak F, Jager S, Sperr W, Kraft D, Scheiner O. 1993. Purification, characterization and N-terminal amino acid sequence of a new major allergen from European chestnut pollen--Cas s 1. Biochem Biophys Res Commun 196:1086-92.
53. Díaz-Perales A, Lombardero M, Sánchez-Monge R, García-Sellés FJ, Pemas M, Femández-Rivas M, Barber D, Salcedo G. 2000. Lipid-transfer proteins as potential plant panallergens: cross-reactivity among proteins of Artemisia pollen, Castaneae nut and Rosaceae fruits, with different IgE-binding capacities. Clin Exp Allergy 30:1403-1410.
54. Ipsen, H., and O.C. Hansen. 1991. The NH2-terminal amino acid sequence of the immunochemically partial identical major allergens of alder (Alnus glutinosa) Aln g I, birch (Betula verrucosa) Bet v I, hornbeam (Carpinus betulus) Car b I and oak (Quercus alba) Que a I pollens. Mol. Immunol. 28:1279-1288.
55. Taniai, M., S. Ando, M. Usui, M. Kurimoto, M. Sakaguchi, S. Inouye, and T. Matuhasi. 1988. N-terminal amino acid sequence of a major allergen of Japanese cedar pollen (Cry j I). FEBS Lett. 239:329-332.
56. Griffith, I.J., A. Lussier, R. Garman, R. Koury, H. Yeung, and J. Pollock. 1993. The cDNA cloning of Cry j I, the major allergen of Cryptomeria japonica (Japanese cedar) (abst). J. Allergy Clin. Immunol. 91:339.
57. Sakaguchi, M., S. Inouye, M. Taniai, S. Ando, M. Usui, and T. Matuhasi. 1990. Identification of the second major allergen of Japanese cedar pollen. Allergy 45:309-312.
57A. Yokoyama M, Miyahara M, Shimizu K, Kino K, Tsunoo H. 2000. Purification, identification, and cDNA cloning of Jun a 2, the second major allergen of mountain cedar pollen. Biochem Biophys Res Commun 275:195-202.
57B. Midoro-Horiuti T, Goldblum RM, Kurosky A, Wood TG, Brooks EG. 2000. Variable Expression of Pathogenesis-Related Protein Allergen in Mountain Cedar (Juniperus ashei) Pollen. J Immunol 164:2188-2192.
57C. Tinghino R., Barletta B., Palumbo S., Afferni C., Iacovacci P., Mari A., Di Felice G., Pini,C. 1998. Molecular characterization of a cross-reactive Juniperus oxycedrus pollen allergen, Jun o 2: a novel calcium-binding allergen J. Allergy Clin. Immunol. 101:772-777.
58 Gross GN, Zimburean JM, Capra JD 1978. Isolation and partial characterization of the allergen in mountain cedar pollen. Scand J Immunol 8:437-41.
58A Obispo TM, Melero JA, Carpizo JA, Carreira J, Lombardero M 1993. The main allergen of Olea europaea (Ole e I) is also present in other species of the oleaceae family. Clin Exp Allergy 23:311-316.
59. Lombardero M., Barbas J.A., Moscoso del Prado J., Carreira J. 1994. cDNA sequence analysis of the main olive allergen, Ole e I. Clin. Exp. Allergy 24:765-770.
60. Villalba, M., E. Batanero, C. Lopez-Otin, L.M. Sanchez, R.I. Monsalve, M.A. Gonzalez de la Pena, C. Lahoz, and R. Rodriguez. 1993. Amino acid sequence of Ole e I, the major allergen from olive tree pollen (Olea europaea). Eur. J. Biochem. 216:863-869.
60A. Asturias JA, Arilla MC, Gomez-Bayon N, Martinez J, Martinez A, Palacios R 1997. Cloning and expression of the panallergen profilin and the major allergen (Ole e 1) from olive tree pollen. J Allergy Clin Immunol 100:365-372.
60B. Batanero E, Villalba M, Ledesma A Puente XS, Rodriguez R. 1996. Ole e 3, an olive-tree allergen, belongs to a widespread family of pollen proteins. Eur J Biochem 241: 772-778.
60C. Batanero E, Ledesma A, Villalba M, Rodriguez R. 1997. Purification, amino acid sequence and immunological characterization of Ole e 6, a cysteine-enriched allergen from olive tree pollen. FEBS Lett. 410:293-296.
60D. Tejera ML, Villalba M, Batanero E, Rodriguez R. 1999. Identification, isolation, and characterization of Ole e 7, a new allergen of olive tree pollen. J Allergy Clin Immunol 104:797-802.
60E. Ledesma,A., Villalba,M. and Rodriguez,R. 2000. Cloning, expression and characterization of a novel four EF-hand Ca(2+)-binding protein from olive pollen with allergenic activity. FEBS Lett. 466:192-196.
61. Chua, K. Y., G. A. Stewart, and W. R. Thomas. 1988. Sequence analysisof cDNA encoding for a major house dust mite allergen, Der p I. J. Exp. Med. 167:175-182.
62. Chua, K. Y., C. R. Doyle, R. J. Simpson, K. J. Turner, G. A. Stewart, and W. R. Thomas. 1990. Isolation of cDNA coding for the major mite allergen Der p II by IgE plaque immunoassay. Int. Arch. Allergy Appl. Immunol. 91:118-123.
63. Smith WA, Thomas WR. 1996. Comparative analysis of the genes encoding group 3 allergens from Dermatophagoides pteronyssinus and Dermatophagoides farinae. Int Arch Allergy Immunol 109: 133-40.
64. Lake, F.R., L.D. Ward, R.J. Simpson, P.J. Thompson, and G.A. Stewart. 1991. House dust mite-derived amylase: Allergenicity and physicochemical characterisation. J. Allergy Clin. Immunol. 87:1035-1042.
65. Tovey, E. R., M. C. Johnson, A. L. Roche, G. S. Cobon, and B. A. Baldo. 1989. Cloning and sequencing of a cDNA expressing a recombinant house dust mite protein that binds human IgE and corresponds to an important low molecular weight allergen. J. Exp. Med. 170:1457-1462.
66. Yasueda, H., T. Shida, T. Ando, S. Sugiyama, and H. Yamakawa. 1991. Allergenic and proteolytic properties of fourth allergens from Dermatophagoides mites. In: "Dust Mite Allergens and Asthma. Report of the 2nd international workshop" A. Todt, Ed., UCB Institute of Allergy, Brussels, Belgium, pp. 63-64.
67. Shen, H.-D., K.-Y. Chua, K.-.L. Lin, K.-H. Hsieh, and W.R. Thomas. 1993. Molecular cloning of a house dust mite allergen with common antibody binding specificities with multiple components in mite extracts. Clin. Exp. Allergy 23:934-40.
67A. O'Neil GM, Donovan GR, Baldo BA. 1994. Cloning and charaterisation of a major allergen of the house dust mite Dermatophagoides pteronyssinus, homologous with glutathione S-transferase. Biochim Biophys Acta,1219:521-528.
67B. King C, Simpson RJ, Moritz RL, Reed GE, Thompson PJ, Stewart GA. 1996. The isolation and characterization of a novel collagenolytic serine protease allergen (Der p 9) from the dust mite Dermatophagoides pteronyssinus. J Allergy Clin Immunol 98:739-47.
68. Lind P, Hansen OC, Horn N. 1988. The binding of mouse hybridoma and human IgE antibodies to the major fecal allergen, Der p I of D. pteronyssinus. J. Immunol. 140:4256-4262.
69. Dilworth, R. J., K. Y. Chua, and W. R. Thomas. 1991. Sequence analysis of cDNA coding for a mojor house dust allergn Der fI. Clin. Exp. Allergy 21:25-32.
70. Nishiyama, C., T. Yunki, T. Takai, Y. Okumura, and H. Okudaira. 1993. Determination of three disulfide bonds in a major house dust mite allergen, Der f II. Int. Arch. Allergy Immunol. 101:159-166.
71. Trudinger, M., K. Y. Chua, and W. R. Thomas. 1991. cDNA encoding the major dust mite allergen Der f II. Clin. Exp. Allergy 21:33-38.
71A. Tategaki A, Kawamoto S, Aki T, Jyo T, Suzuki O, Shigeta S, Ono K. Newly described house dust mite allergens. ACI International suppl. 1: 74-76, 2000.
72. Aki T, Kodama T, Fujikawa A, Miura K, Shigeta S, Wada T, Jyo T, Murooka Y, Oka S, Ono K. 1995. Immunochemical characteristion of recombinant and native tropomyosins as a new allergen from the house dust mite Dermatophagoides farinae. J Allergy Clin Immunol 96:74-83.
72A. Tsai L, Sun Y, Chao P, Ng H, Hung M, Hsieh K, Liaw S, Chua K, 1999. Sequence analysis and expression of a cDNA clone encoding a 98-kDa allergen in Dermatophagoides farinae. Clin Exp Allergy 29:1606-1613.
72B. Gafvelin G, Johansson E, Lundin A, Smith AM, Chapman MD, Benjamin DC, Derewenda U, Van Hage-Hamsten M. 2001. Cross-reactivity studies of a new group 2 allergen from the dust mite Glycyphagus domesticus, Gly d 2, and group 2 allergens from Dermatophagoides pteronyssinus, Lepidoglyphus destructor, and Tyrophagus putrescentiae with recombinant allergens. J Allergy Clin Immunol 107:511-518.
73. van Hage-Hamsten, M., T. Bergman, E. Johansson, B. Persson, H. Jornvall, B. Harfast, and S.G.O. Johansson. 1993. N-terminal amino acid sequence of major allergen of the mite lepidoglyphus destructor (abst). J. Allergy Clin. Immunol. 91:353.
74. Varela J, Ventas P, Carreira J, Barbas JA, Gimenez-Gallego G, Polo F. Primary structure of Lep d I, the main Lepidoglyphus destructor allergen. Eur J Biochem 225:93-98, 1994.
75. Schmidt M, van der Ploeg I, Olsson S, van Hage Hamsten M. The complete cDNA encoding the Lepidoglyphus destructor major allergen Lep d 1. FEBS Lett 370:11-14, 1995.
75A. Eriksson TLJ, Rasool O, Huecas S, Whitley P, Crameri R, Appenzeller U, Gafvelin G, van Hage-Hamsten M. 2001. Cloning of three new allergens from the dust mite Lepidoglyphus destructor using phage surface display technology. Eur. J. Biochem. 268:287-294.
75B. Eriksson TL, Johansson E, Whitley P, Schmidt M, Elsayed S, van Hage-Hamsten M. 1998. Cloning and characterisation of a group II allergen from the dust mite Tyrophagus putrescentiae. Eur. J. Biochem. 251 (1-2), 443-447.
76. Rautiainen J, Rytkonen M, Pelkonen J, Pentikainen J, Perola O, Virtanen T, Zeiler T, Mantyjarvi R. BDA20, a major bovine dander allergen characterized at the sequence level is Bos d 2. Submitted.
77. Gjesing B, Lowenstein H. Immunochemistry of food antigens. Ann Allergy 53:602, 1984.
78. de Groot, H., K.G.H. Goei, P. van Swieten, and R.C. Aalberse. 1991. Affinity purification of a major and a minor allergen from dog extract: Serologic activity of affiity-purified Can f I and Can fI-depleted extract. J. Allergy Clin. Immunol. 87:1056-1065.
79. Konieczny, A. Personal communication; Immunologic Pharmaceutical Corp.
79A. Bulone, V. 1998. Separation of horse dander allergen proteins by two-dimensional electrophoresis. Molecular characterisation and identification of Equ c 2.0101 and Equ c 2.0102 as lipocalin proteins. Eur J Biochem 253:202-211.
79B. Swiss-Prot acc. P81216, P81217.
79C. Dandeu J. P., Rabillon J., Divanovic A., Carmi-Leroy A., David B. (1993). Hydrophobic interaction chromatography for isolation and purification of Equ c 1, the horse major allergen. J. Chromatogr. 621:23-31.
79D. Goubran Botros H., Rabillon J., Grégoire C., David B., Dandeu J.P. 1998. Thiophilic absorption chromatography: purification of Equ c 2 and Equ c 3, two horse allergens from horse sweat. J. Chromatogr. B 710:57-65.
79E. Hilger C, Kohnen M, Grigioni F, Lehners C, Hentges F. Allergic cross-reactions between cat and pig serum albumin. Allergy 52:179-187, 1997; and Hilger C, Grigioni F, Hentges F. Sequence of the gene encoding cat (Felis domesticus) serum albumin. Gene 169:295-296, 1996.
79F. Ichikawa K, Vailes LD, Pomes A, Chapman MD. Molecular cloning, expression and modeling of cat allergen, cystatin (Fel d 3), a cysteine protease inhibitor. Clin Exp Allergy, In Press 2001.
80. McDonald, B., M. C. Kuo, J. L. Ohman, and L. J. Rosenwasser. 1988. A 29 amino acid peptide derived from rat alpha 2 euglobulin triggers murine allergen specific human T cells (abst). J. Allergy Clin. Immunol. 83:251.
81. Clarke, A. J., P. M. Cissold, R. A. Shawi, P. Beattie, and J. Bishop. 1984. Structure of mouse urinary protein genes: differential splicing configurations in the 3'-non-coding region. EMBO J 3:1045-1052.
82. Longbottom, J. L. 1983. Chracterization of allergens from the urines of experimental animals. McMillan Press, London, pp. 525-529.
83. Laperche, Y., K. R. Lynch, K. P. Dolans, and P. Feigelsen. 1983. Tissue-specific control of alpha 2u globulin gene expression: constitutive synthesis in submaxillary gland. Cell 32:453-460.
83A. Bush RK, Sanchez H, Geisler D. 1999. Molecular cloning of a major Alternaria altemata allergen, rAlt a 2. J Allergy Clin Immunol 104:665-671.
83B. Aukrust L, Borch SM. 1979. Partial purification and characterization of two Cladosporium herbanun allergens. Int Arch Allergy Appl Immunol 60:68-79.
83C. Sward-Nordmo M, Paulsen BS, Wold JK. 1988. The glycoprotein allergen Ag-54 (Cla h II] from Cladosporium herbarum. Structural studies of the carbohydrate moiety. Int Arch Allergy Appl Immunol 85:288-294.
84. Shen, et al. J. Allergy Clin. Immunol. 103:S157, 1999.
84A. Crameri R. Epidemiology and molecular basis of the involvement of Aspergillus fumigatus in allergic diseases. Contrib. Microbiol. Vol. 2, Karger, Basel (in press).
84B. Shen, et al. (manuscript submitted), 1999
84C. Shen HD, Ling WL, Tan MF, Wang SR, Chou H, Han SIH. Vacuolar serine proteinase: A major allergen of Aspergillus fumigatus. 10th International Congress of Immunology, Abstract, 1998.
85. Kumar, A., L.V. Reddy, A. Sochanik, and V.P. Kurup. 1993. Isolation and characterization of a recombinant heat shock protein of Aspergillus fumigatus. J. Allergy Clin. Immunol. 91:1024-1030.
86A. Shen HD, Lin WL, Tsai JJ, Liaw SF, Han SH. 1996. Allergenic components in three different species of Penicillium: crossreactivity among major allergens. Clin Exp Allergy 26:444-451.
86B. Shen, et al. Abstract; The XVIII Congress of the European Academy of Allergology and Clinical Immunology, Brussels, Belgium, 3-7 July 1999.
87. Shen HD, Liaw SF, Lin WL, Ro LH, Yang HL, Han SH. 1995. Molecular cloning of cDNA coding for the 68 kDa allergen of Penicillium notatum using MoAbs. Clin Exp Allergy 25:350-356.
88. Shen, H.D., K.B. Choo, H.H. Lee, J.C. Hsieh, and S.H. Han. 1991. The 40 kd allergen of Candida albicans is an alcohol dehydrogenease: molecular cloning and immunological analysis using monoclonal antibodies. Clin. Exp. Allergy 21:675-681.
89. Shen, et al. Clin. Exp. Allergy (in press), 1999.
90. Woodfolk JA, Wheatley LM, Piyasena RV, Benjamin DC, Platts-Mills TA.1998. Trichophyton antigens associated with IgE antibodies and delayed type hypersensitivity. Sequence homology to two families of serine proteinases. J Biol Chem 273:29489-96.
91. Deuell, B., L.K. Arruda, M.L. Hayden, M.D. Chapman and T.A.E. Platts-Mills. 1991. Trichophyton tonsurans Allergen I. J. Immunol. 147:96-101.
91A. Schmidt M, Zargari A, Holt P, Lindbom L, Hellman U, Whitley P, van der Ploeg I, Harfast B, Scheynius A. 1997. The complete cDNA sequence and expression of the first major allergenic protein of Malassezia furfur, Mal f 1. Eur J Biochem 246:181-185.
91B. Horner WE, Reese G, Lehrer SB. 1995. Identification of the allergen Psi c 2 from the basidiomycete Psilocybe cubensis as a fungal cyclophilin. Int Arch Allergy Immunol 107:298-300.
91C. Hoffman DR, Schmidt JO. 2000. Phospholipases from Asian honeybees. J Allergy Clin Immunol 105:S56 (abs).
92. Kuchler, K., M. Gmach1, M. J. Sippl, and G. Kreil. 1989. Analysis of the cDNA for phospholipase A2 from honey bee venom glands: The deduced amino acid sequence reveals homology to the corresponding vertebrate enzymes. Eur. J. Biochem. 184:249-254.
93. Gmach1, M., and G. Kreil. 1993. Bee venom hyaluronidase is homologous to a membrane protein of mammalian sperm. Proc. Natl. Acad. Sci. USA 90:3569-3573.
93A. Hoffman DR. 1977. Allergens in bee venom III. Identification of allergen B as an acid phosphatase. J Allergy Clin. Immunol. 59:364-366.
94. Habermann, E. 1972. Bee and wasp venoms. Science 177:314-322.
95. Hoffman DR, Jacobson RS. 1996. Allergens in Hymenoptera venom XXVII: Bumblebee venom allergy and allergens. J. Allergy Clin. Immunol. 97:812-821.
95A. Hoffman DR, El-Choufani AE, Smith MM, de Groot H. 2001. Occupational allergy to bumblebee venom: Allergens of Bombus terrestris. J Allergy Clin Immunol In press.
95B. Helm R, Cockrell G, Stanley JS, Brenner RJ, Burks W, Bannon GA. 1996. Isolation and characterization of a clone encoding a majore allergen (Bla g Bd90K) involved in IgE mediated cockroach hypersensitivity. J Allerg Clin Immunol 98:172-180.
95C. Pomes A, Melen E, Vailes LD, Retief JD, Arruda LK, Chapman MD. 1998. Novel allergen structures with tandem amino acid repeats derived from German and American cockroach. J Biol Chem 273:30801-30807.
96. Arruda LK, Vailes LD, Mann BJ, Shannon J, Fox JW, Vedvick TS, Hayden ML, Chapman MD. Molecular cloning of a major cockroach (Blattella germanica) allergen, Bla g 2. Sequence homology to the aspartic proteases. J Biol Chem 270:19563-19568, 1995.
97. Arruda LK, Vailes LD, Hayden ML, Benjamin DC, Chapman MD. Cloning of cockroach allergen, Bla g 4, identifies ligand binding proteins (or calycins) as a cause of IgE antibody responses. J Biol Chem 270:31196-31201, 1995.
98. Arruda LK, Vailes LD, Benjamin DC, Chapman MD. Molecular cloning of German Cockroach (Blattella germanica) allergens. Int Arch Allergy Immunol 107:295-297, 1995.
98A. Wu CH, Wang NM, Lee MF, Kao CYY, Luo SF. 1998. Cloning of the American cockroach Cr-PII allergens: Evidence for the existence of cross-reactive allergens between species. J Allergy Clin Immunol 101:832-840.
98B. Melen E, Pomés A, Vailes LD, Arruda LK, Chapman MD. 1999. Molecular cloning of Per a 1 and definition of the cross-reactive Group 1 cockroach allergens. J Allergy Clin Immunol 103:859-64.
98C. Wu CH, Lee MF, Liao SC, Luo SF. Sequencing analysis of cDNA clones encoding the American cockroach Cr-PI allergens. J Biol Chem 271:17937-17943, 1996.
98D. Wu CH, Lee MF, Wang NM, Luo SF. Sequencing and immunochemical characterization of the American cockroach Per a 3 (Cr-PI) isoallergenic variants. Molecular Immunol 34:1-8,1997.
98E. Santos ABR, Chapman MD, Aalberse RC, Vailes LD, Ferriani VPL, Oliver C, Rizzo MC, Naspitz CK, Arruda LK. 1999. Cockroach allergens and asthma in Brazil: Identification of tropomyosin as a major allergen with potential cross-reactivity with mite and shrimp allergens. J Allergy Clin Immunol 104:329-337.
98F. Asturias JA, Gómez-Bayón N, Arilla MC, Martinez A, Palacios R, Sánchez-Gascón, Martinez J. 1999. Molecular characterization of American cockroach tropomyosin (Periplaneta americana allergen 7), a cross-reactive allergen. J Immunol 162:4342-4348.
99. Mazur, G., X. Baur, and V. Liebers. 1990. Hypersensitivity to hemoglobins of the Diptera family Chironomidae: Structural and functional studies of their immunogenic/allergenic sites. Monog. Allergy 28:121-137.
100. Soldatova, L., L. Kochoumian, and T.P. King. 1993. Sequence similarity of a hornet (D. maculata) venom allergen phospholipase A1 with mammalian lipases. FEBS Letters 320:145-149.
101. Lu, G., L. Kochoumian and T.P. King. Whiteface hornet venom allergen hyaluronidase: cloning and its sequence similarity with other proteins (abst.). 1994. J. Allergy Clin. Immunol. 93:224.
102. Fang, K. S. F., M. Vitale, P. Fehlner, and T. P. King. 1988. cDNA cloning and primary structure of a white-faced hornet venom allergen, antigen 5. Proc. Natl. Acad. Sci., USA 85:895-899.
103. King, T. P., D. C. Moran, D. F. Wang, L. Kochoumian, and B.T. Chait. 1990. Structural studies of a hornet venom allergen antigen 5, Dol m V and its sequence similarity with other proteins. Prot. Seq. Data Anal. 3:263-266.
104. Lu, G., M. Villalba, M.R. Coscia, D.R. Hoffman, and T.P. King. 1993. Sequence analysis and antigen cross reactivity of a venom allergen antigen 5 from hornets, wasps and yellowjackets. J.Immunol. 150:2823-2830.
105. King, T. P. and Lu, G. 1997. Unpublished data.
105A. King TP, Lu G, Gonzalez M, Qian N and Soldatova L. 1996. Yellow jacket venom allergens, hyaluronidase and phospholipase: sequence similarity and antigenic cross-reactivity with their hornet and wasp homologs and possible implications for clinical allergy. J. Allergy Clin. Immunol. 98:588-600.
106. Hoffman, D.R. 1993. Allergens in hymenoptera venom XXV: The amino acid sequences of antigen 5 molecules and the structural basis of antigenic cross-reactivity. J. Allergy Clin. Immunol. 92:707-716.
107. Hoffman, D.R. 1992. Unpublished data.
108. Hoffman, D.R. 1993. The complete amino acid sequence of a yellowjacket venom phospholipase (abst). J. Allergy Clin. Immunol. 91:187.
109. Jacobson, R.S., D.R. Hoffman, and D.M. Kemeny. 1992. The cross-reactivity between bee and vespid hyaluronidases has a structural basis (abst). J. Allergy Clin. Immunol. 89:292.
110. Hoffman, D.R. 1993. Allergens in Hymenoptera venom XXIV: The amino acid sequences of imported fire ant venom allergens Sol i II, Sol i III, and Sol i IV. J. Allergy Clin. Immunol. 91:71-78.
111. Schmidt, M., R.B. Walker, D.R. Hoffman, and T.J. McConnell. 1993. Nucleotide sequence of cDNA encoding the fire ant venom protein Sol i II. FEBS Letters 319:138-140.
112. Elsayed S, Bennich H. The primary structure of Allergen M from cod. Scand J Immunol 3:683-686, 1974.
113. Elsayed S, Aas K, Sletten K, Johansson SGO. Tryptic cleavage of a homogeneous cod fish allergen and isolation of two active polypeptide fragments. Immunochemistry 9:647-661, 1972.
114. Hoffman, D.R. 1983. Immunochemical identification of the allergens in egg white. J. Allergy Clin. Immunol. 71:481-486.
115. Langeland, T. 1983. A clinical and immunological study of allergy to hen's egg white. IV. specific IgE antibodies to individual allergens in hen's egg white related to clinical and immunolgical parameters in egg-allergic patients. Allergy 38:493-500.
116. Daul, C.B., M. Slattery, J.E. Morgan, and S.B. Lehrer. 1993. Common crustacea allergens: identification of B cell epitopes with the shrimp specific monoclonal antibodies. In: "Molecular Biology and Immunology of Allergens" (D. Kraft and A. Sehon, eds.). CRC Press, Boca Raton. pp. 291-293.
117. K.N. Shanti, B.M. Martin, S. Nagpal, D.D. Metcalfe, P.V. Subba Rao. 1993. Identification of tropomyosin as the major shrimp allergen and characterization of its IgE-binding epitopes. J.Immunol. 151:5354-5363.
117A. M. Miyazawa, H. Fukamachi, Y. Inagaki, G. Reese, C.B. Daul, S.B. Lehrer, S. Inouye, M. Sakaguchi. 1996. Identification of the first major allergen of a squid (Todarodes pacificus). J. Allergy Clin. Immunol. 98:948-953.
117B Lopata AL, Zinn C, Potter PC. Characteristics of hypersensitivity reactions and identification of a unique 49 kd IgE-binding protein (Hal-m-1) in abalone (Haliotis midae). J. Allergy Clin. Immunol. 100: 642-648, 1997.
117C. K. Hoffmann-Sommergruber, G. O'Riordain, H. Ahorn, C. Ebner, M. Laimer Da Camara Machado, H. Pühringer, O. Scheiner, H. Breiteneder. 1999. Molecular characterization of Dau c 1, the Bet v1 homologous protein from carrot and its cross-reactivity with Bet v1 and Api g 1 Clin Exp Allergy 29:840-847.
118. Monsalve, R.I., M.A. Gonzalez de la Pena, L. Menendez-Arias, C. Lopez-Otin, M. Villalba, and R. Rodriguez. 1993. Characterization of a new mustard allergen, Bra j IE. Detection of an allergenic epitope. Biochem. J. 293:625-632.
118A. Monsalve RI, Gonzalez de la Pena MA, Lopez-Otin C, Fiandor A, Fernandez C, Villalba M, Rodriguez R. 1997. Detection, isolation and complete amino acid sequence of an aeroallergenic protein from rapeseed flour. Clin Exp Allergy 27:833-841.
119. Mena, M., R. Sanchez?Monge, L. Gomez, G. Salcedo, and P. Carbonero. 1992. A major barley allergen associated with baker's asthma disease is a glycosylated monomeric inhibitor of insect alpha-amylase: cDNA cloning and chromosomal location of the gene. Plant Molec. Biol. 20:451-458.
119A. Xu H, Theerakulpisut P, Goulding N, Suphioglu C, Singh M. B. Bhalla P. L. Cloning expression and immunological characterization of Ory s 1, the major allergen of rice pollen. Gene 164:255-259, 1995.
119B. Pastorello EA, Ortolani C, Farioli L, Pravettoni V, Ispano M, Borga A, Bengtsson A, Incorvaia C, Berti C, Zanussi C. 1994. Allergenic cross-reactivity among peach, apricot, plum, and cherry in patients with oral allergy syndrome: an in vivo and in vitro study. J. Allergy Clin. Immunol. 94:699-707.
120. Menendez-Arias, L., I. Moneo, J. Dominguez, and R. Rodriguez. 1988. Primary structure of the major allergen of yellow mustard (Sinapis alba L.) seed, Sin a I. Eur. J. Biochem. 177:159-166.
121. Gonzalez R, Varela J, Carreira J, Polo F. Soybean hydrophobic protein and soybean hull allergy. Lancet 346:48-49, 1995.
121A. Pastorello EA, Varin E, Farioli L, Pravettoni V, Ortolani C, Trambaioli C, Fortunato D, Giuffrida MG, Rivolta F, Robino A, Calamari AM, Lacava L, Conti A. The major allergen of sesame seeds (Sesamum indicum) is a 2S albumin. J. Chromatogr. B Biomed. Sci. Appl. 756: 85-93, 2001.
121B. Moneo I, Caballero ML, Gomez F, Ortega E, Alonso MJ. Isolation and characterization of a major allergen from the fish parasite Anisakis simplex. J. Allergy Clin. Immunol. 106: 177-182, 2000.
121C. Asturias JA, Eraso E, Martínez A. 2000. Is tropomysoin an allergen in Anisakis? Allergy 55:898-890.
122. Christie, J. F., B. Dunbar, I. Davidson, and M. W. Kennedy. 1990. N-terminal amino acid sequence identity between a major allergen of Ascaris lumbricoides and Ascaris suum and MHC-restricted IgE responses to it. Immunology 69:596-602.
122A. Onizuka R, Kamiya H, Muramoto K, Goto R, Inoue K, Kumamoto K, Nakajima Y, Iida S, Ishigami F. Purification of major allergens from red soft coral (Dendrophythya nipponica). Int Arch Allergy Immunol, In press 2001.
123. Czuppon AB, Chen Z, Rennert S, Engelke T, Meyer HE, Heber M, Baur X. The rubber elongation factor of rubber trees (Hevea brasiliensis) is the major allergen in latex. J Allergy Clin Immunol 92:690-697, 1993.
124. Attanayaka DPSTG, Kekwick RGO, Franklin FCH. 1991. Molecular cloning and nucleotide sequencing of the rubber elongation factor gene from hevea brasiliensis. Plant Mol Biol 16:1079-1081.
125. Chye ML, Cheung KY. 1995. J 1,3-glucanase is highly expressed in Laticifers of Hevea brasiliensis. Plant Mol Biol 26:397-402.
126. Alenius H, Palosuo T, Kelly K, Kurup V, Reunala T, Makinen-Kiljunen S, Turjanmaa K Fink J. 1993. IgE reactivity to 14-kD and 27-kD natural rubber proteins in Latex-allergic children with Spina bifida and other congenital anomalies. Int Arch Allergy Immunol 102:61-66.
127. Yeang HY, Cheong KF, Sunderasan E, Hamzah S, Chew NP, Hamid S, Hamilton RG, Cardosa MJ. 1996. The 14.6 kD (REF, Hev b 1) and 24 kD (Hev b 3) rubber particle proteins are recognized by IgE from Spina Bifida patients with Latex allergy. J Allerg Clin Immunol in press.
128. Sunderasan E, Hamzah S, Hamid S, Ward MA, Yeang HY, Cardosa MJ. 1995. Latex B-serum J-1,3-glucanase (Hev b 2) and a component of the microhelix (Hev b 4) are major Latex allergens. J nat Rubb Res 10:82-99.

**Table 9. Selected allergens with structures available in Protein Database (PDB)**

| | |
|---|---|
| **ID NO: 1A0K** | |
| Deposited: 02-Dec-1997 Exp. Method: X-ray Diffraction Resolution: 2.20 Å | |
| Title | Profilin I From Arabidopsis Thaliana |
| Classification | Cytoskeleton |
| Compound | Mol_Id: 1; Molecule: Profilin; Chain: Null; Engineered: Recombinant Plant Protein; Biological_Unit: Monomer |
| | |
| **ID NO: 1A9V** | |
| Deposited: 10-Apr-1998 Exp. Method: NMR, 10 Structures | |
| Title | Tertiary Structure Of The Major House Dust Mite Allergen Der P 2, NMR, 10 Structures |
| Classification | Allergen |
| Compound | Mol_Id: 1; Molecule: Mite Allergen Der P 2; Chain: Null; Engineered: Yes; Mutation: D1S; Other_Details:D1S Mutant Made To Enhance N-Terminal Met Removal |
| | |
| **ID NO: 1AHK** | |
| Deposited: 07-Apr-1997 Exp. Method: NMR, Minimized Average Structure | |
| Title | Der F 2, The Major Mite Allergen From Dermatophagoides Farinae, NMR, Minimized Average Structure |
| Classification | Allergen |
| Compound | Mol_Id: 1; Molecule: Der F 2; Chain: Null; Synonym: Der F II; Engineered: Yes |
| | |
| **ID NO: 1AHM** | |
| Deposited: 07-Apr-1997 Exp. Method: NMR,10 Structures | |
| Title | Der F 2, The Major Mite Allergen From Dermatophagoides Farinae, NMR, 10 Structures |
| Classification Allergen | |
| Compound | Mol_Id: 1; Molecule: Der F 2; Chain: Null; Synonym: Der F II; Engineered: Yes |
| | |
| **ID NO: 1B6F** | |
| Deposited: 13-Jan-1999 Exp. Method: NMR, 23 Structures | |
| Title | Birch Pollen Allergen Bet V 1 |
| Classification | Plant Protein |
| Compound | Mol_Id: 1; Molecule: Major Pollen Allergen Bet V 1-A; Chain: A; Engineered: Yes; Mutation: Yes |
| | |
| **ID NO: 1BBG** | |
| Deposited: 24-Apr-1998 Exp. Method: NMR, Minimized Average Structure | |
| Title | Ragweed Pollen Allergen From Ambrosia Trifida V, NMR, Minimized Average Structure |
| Classification | Allergen |
| Compound | Mol_Id: 1; Molecule: Pollen Allergen 5; Chain: Null |
| | |
| **ID NO: 1BJ7** | |
| Deposited: 02-Jul-1998 Exp. Method: X-ray Diffraction Resolution: 1.80 Å | |
| Title | Bovine Lipocalin Allergen Bos D 2 |
| Classification | Allergen |
| Compound | Mol_Id: 1; Molecule: D 2; Chain: Null; Synonym: Dander Major Allergen Bda20, Dermal Allergen Bda20; Engineered: Yes; Biological_Unit: Monomer |
| | |
| **ID NO: 1BMW** | |
| Deposited: 27-Jul-1998 Exp. Method: NMR, 38 Structures | |
| Title | A Fibronectin Type III Fold In Plant Allergens: The Solution Structure Of Phl Pii From Timothy Grass Pollen, NMR, 38 Structures |
| Classification | Allergen |
| Compound | Mol_Id: 1; Molecule: Pollen Allergen Phl P2; Chain: Null; Synonym: Phl P II; Engineered: Yes; Biological_Unit: Monomer |
| | |
| **ID NO: 1BTV** | |
| Deposited: 30-Jan-1997 Exp. Method: NMR, 20 Structures | |
| Title | Structure Of Bet V1, NMR, 20 Structures |
| Classification | Major Birch Pollen Allergen |
| Compound | Mol_Id: 1; Molecule: Bet V 1; Chain: Null; Engineered: Yes |
| | |
| **ID NO: 1BV1** | |
| Deposited: 08-Jul-1997 Exp. Method: X-ray Diffraction Resolution: 2.00 Å | |
| Title | Birch Pollen Allergen Bet V 1 |
| Classification | Allergen |
| Compound | Mol_Id: 1; Molecule: Bet V 1; Chain: Null; Synonym: Major Pollen Allergen Bet V 1-A; Engineered: Yes |
| | |
| **ID NO: 1BWH** | |
| Deposited: 24-Sep-1998 Exp. Method: X-ray Diffraction Resolution: 1.80 Å | |
| Title | The 1.8 A Structure Of Ground Control Grown Tetragonal Hen Egg White Lysozyme |
| Classification | Hydrolase |
| Compound | Mol_Id: 1; Molecule: Lysozyme; Chain: A; Synonym: Gal D IV, Allergen Gal D 4; Ec: 3.2.1.17 |
| | |
| **ID NO: 1BWI** | |
| Deposited: 24-Sep-1998 Exp. Method: X-ray Diffraction Resolution: 1.80 Å | |
| Title | The 1.8 A Structure Of Microbatch Oil Drop Grown Tetragonal Hen Egg White Lysozyme |
| Classification | Hydrolase |
| Compound | Mol_Id: 1; Molecule: Lysozyme; Chain: A; Synonym: Gal D IV, Allergen Gal D 4; Ec: 3.2.1.17 |
| | |
| **ID NO: 1BWJ** | |
| Deposited: 18-Sep-1998 Exp. Method: X-ray Diffraction Resolution: 1.80 Å | |
| Title | The 1.8 A Structure Of Microgravity Grown Tetragonal Hen Egg White Lysozyme |
| Classification | Hydrolase |
| Compound | Mol_Id: 1; Molecule: Lysozyme; Chain: A; Synonym: Gal D IV, Allergen Gal D 4; Ec: 3.2.1.17. |
| | |
| **ID NO: 1CQA** | |
| Deposited: 26-Jul-1996 Exp. Method: X-ray Diffraction Resolution: 2.40 Å | |
| Title | Birch Pollen Profilin |
| Classification | Contractile Protein |
| Compound | Mol_Id: 1; Molecule: Profilin; Chain: Null; Engineered: Yes |
| | |
| **ID NO: 1E09** | |
| Deposited: 15-Mar-2000 Exp. Method: NMR, 22 Structures | |
| Title | Solution Structure Of The Major Cherry Allergen Pru Av 1 |
| Classification | Allergen |
| Compound | Mol_Id: 1; Molecule: Pru Av 1; Chain: A; Engineered: Yes |
| | |
| **ID NO: 1EW3** | |
| Deposited: 21-Apr-2000 Exp. Method: X-ray Diffraction Resolution: 2.30 Å | |
| Title Crystal | Structure Of The Major Horse Allergen Equ C 1 |
| Classification | Allergen |
| Compound | Mol_Id: 1; Molecule: Allergen Equ C 1; Chain: A; Engineered: Yes |
| | |
| **ID NO: 1F2K** | |
| Deposited: 26-May-2000 Exp. Method: X-ray Diffraction Resolution: 2.30 Å | |
| Title | Crystal Structure Of Acanthamoeba Castellanii Profilin II, Cubic Crystal Form |
| Classification | Structural Protein |
| Compound | Mol_Id: 1; Molecule: Profilin II; Chain: A, B; Engineered: Yes |
| | |
| **ID NO: 1FCQ** | |
| Deposited: 19-Jul-2000 Exp. Method: X-ray Diffraction Resolution: 1.60 Å | |
| Title | Crystal Structure (Monoclinic) Of Bee Venom Hyaluronidase |
| Classification | Hydrolase |
| Compound | Mol_Id: 1; Molecule: Hyaluronoglucosaminidase; Chain: A; Synonym: Hyaluronidase, Api M II; Ec: 3.2.1.35; Engineered: Yes |
| | |
| **ID NO: 1FCU** | |
| Deposited: 19-Jul-2000 Exp. Method: X-ray Diffraction Resolution: 2.10 Å | |
| Title | Crystal Structure (Trigonal) Of Bee Venom Hyaluronidase |
| Classification | Hydrolase |
| Compound | Mol_Id: 1; Molecule: Hyaluronoglucosaminidase; Chain: A; Synonym: Hyaluronidase, Api M II; Ec: 3.2.1.35; Engineered: Yes |
| | |
| **ID NO: 1FCV** | |
| Deposited: 19-Jul-2000 Exp. Method: X-ray Diffraction Resolution: 2.65 Å | |
| Title | Crystal Structure Of Bee Venom Hyaluronidase In Complex With Hyaluronic Acid Tetramer |
| Classification | Hydrolase |
| Compound | Mol_Id: 1; Molecule: Hyaluronoglucosaminidase; Chain: A; Synonym: Hyaluronidase, Api M II; Ec: 3.2.1.35; Engineered: Yes |
| | |
| **ID NO: 1FLQ** | |
| Deposited: 15-Aug-2000 Exp. Method: X-ray Diffraction Resolution: 1.80 Å | |
| Title | Hen Egg White Lysozyme Mutant With Alanine Substituted For Glycine |
| Classification | Hydrolase |
| Compound | Mol_Id: 1; Molecule: Lysozyme; Chain: A; Synonym: 1,4--N-Acetylmuramidase C, Allergen Gal D 4, Gal D IV; Ec: 3.2.1.17; Engineered: Yes; Mutation: Yes |
| | |
| **ID NO: 1FLU** | |
| Deposited: 15-Aug-2000 Exp. Method: X-ray Diffraction Resolution: 1.78 Å | |
| Title | Hen Egg White Lysozyme Mutant With Alanine Substituted For Glycine |
| Classification | Hydrolase |
| Compound | Mol_Id: 1; Molecule: Lysozyme; Chain: A; Synonym: 1,4-N-Acetylmuramidase C, Allergen Gal D 4, Gal D IV; Ec: 3.2.1.17; Engineered: Yes; Mutation: Yes |
| | |
| **ID NO: 1FLW** | |
| Deposited: 15-Aug-2000 Exp. Method: X-ray Diffraction Resolution: 1.81 Å | |
| Title | Hen Egg White Lysozyme Mutant With Alanine Substituted For Glycine |
| Classification | Hydrolase |
| Compound | Mol_Id: 1; Molecule: Lysozyme; Chain: A; Synonym: 1,4--N-Acetylmuramidase C, Allergen Gal D 4, Gal D IV; Ec: 3.2.1.17; Engineered: Yes; Mutation: Yes |
| | |
| **ID NO: 1FLY** | |
| Deposited: 15-Aug-2000 Exp. Method: X-ray Diffraction Resolution: 1.83 Å | |
| Title | Hen Egg White Lysozyme Mutant With Alanine Substituted For Glycine |
| Classification | Hydrolase |
| Compound | Mol_Id: 1; Molecule: Lysozyme; Chain: A; Synonym: 1,4--N-Acetylmuramidase C, Allergen Gal D 4, Gal D IV; Ec: 3.2.1.17; Engineered: Yes; Mutation: Yes |
| | |
| **ID NO: 1FN5** | |
| Deposited: 21-Aug-2000 Exp. Method: X-ray Diffraction Resolution: 1.78 Å | |
| Title | Hen Egg White Lysozyme Mutant With Alanine Substituted For Glycine |
| Classification | Hydrolase |
| Compound | Mol_Id: 1; Molecule: Lysozyme; Chain: A; Synonym: 1,4--N-Acetylmuramidase C, Allergen Gal D 4, Gal D IV; Ec: 3.2.1.17; Engineered: Yes; Mutation: Yes |
| | |
| **ID NO: 1FSK** | |
| Deposited: 11-Sep-2000 Exp. Method: X-ray Diffraction Resolution: 2.90 Å | |
| Title | Complex Formation Between A Fab Fragment Of A Monoclonal IgG Antibody and The Major Allergen From Birch Pollen Bet V 1 |
| Classification | Immune System |
| Compound | Mol_Id: 1; Molecule: Major Pollen Allergen Bet V 1-A; Chain: A, D, G, J; Synonym: Bet V I-A, Betvi Allergen; Engineered: Yes Mol_Id: 2; Molecule: Immunoglobulin Light Chain; Chain: B, E, H, K; Synonym: Bv16 Fab-Fragment, Mopc21 Coding Sequence; Engineered: Yes Mol_Id: 3; Molecule: Antibody Heavy Chain Fab; Chain: C, F, I, L; Synonym: Heavy Chain Of The Monoclonal Antibody Mst2; Engineered: Yes |
| | |
| **ID NO: 1G5U** | |
| Deposited: 02-Nov-2000 Exp. Method: X-ray Diffraction Resolution: 3.10 Å | |
| Title | Latex Profilin Hevb8 |
| Classification | Allergen |
| Compound | Mol_Id: 1; Molecule: Profilin; Chain: A, B; Engineered: Yes |
| | |
| **ID NO: 1H6M** | |
| Deposited: 19-Jun-2001 Exp. Method: X-ray Diffraction Resolution: 1.64 Å | |
| Title | Covalent Glycosyl-Enzyme Intermediate Of Hen Egg White Lysozyme |
| Classification | Hydrolase (O-Glycosyl) |
| Compound | Mol_Id: 1; Molecule: Lysozyme C; Synonym: 1,4--N-Acetylmuramidase C, Allergen Gal D 4, Gal D IV; Chain: A; Ec: 3.2.1.17; Engineered: Yes; Mutation: Yes; Other_Details: Covalent 2-Fluorochitobiosyl Enzyme Intermediate |
| | |
| **ID NO: 1JTI** | |
| Deposited: 21-Aug-2001 Exp. Method: X-ray Diffraction Resolution: 2.30 Å | |
| Title | Loop-Inserted Structure Of P1-P1' Cleaved Ovalbumin Mutant R339T |
| Classification | Allergen |
| Compound | Mol_Id: 1; Molecule: Ovalbumin; Chain: A, B; Engineered: Yes; Mutation: Yes |
| | |
| **ID NO: 1JTT** | |
| Deposited: 22-Aug-2001 Exp. Method: X-ray Diffraction Resolution: 2.10 Å | |
| Title | Degenerate Interfaces In Antigen-Antibody Complexes |
| Classification | Immune System, Lysozyme |
| Compound | Mol_Id: 1; Molecule: Vh Single-Domain Antibody; Chain: A; Fragment: Vh Domain Fragment; Engineered: Yes Mol_Id: 2; Molecule: Lysozyme; Chain: L; Fragment: Enzyme; Synonym: 1,4--N-Acetylmuramidase C, Allergen Gal D IV; Ec: 3.2.1.17 |
| | |
| **ID NO: 1K0K** | |
| Deposited: 19-Sep-2001 Exp. Method: X-ray Diffraction Resolution: 2.35 Å | |
| Title | Yeast Profilin, Cubic Crystal Form |
| Classification | Contractile Protein |
| Compound | Mol_Id: 1; Molecule: Profilin; Chain: A; Engineered: Yes |
| | |
| **ID NO: 1KKC** | |
| Deposited: 07-Dec-2001 Exp. Method: X-ray Diffraction Resolution: 2.00 Å | |
| Title | Crystal Structure Of Aspergillus Fumigatus Mnsod |
| Classification | Oxidoreductase |
| Compound | Mol_Id: 1; Molecule: Manganese Superoxide Dismutase; Chain: A, B, X, Y; Synonym: Mnsod; Ec: 1.15.1.1; Engineered: Yes |
| | |
| **ID NO: 1KUR** | |
| Deposited: 22-Jan-2002 Exp. Method: Theoretical Model | |
| Title | Theoretical Model Of The Allergen Jun A 3 From Mountain Cedar Pollen |
| Classification | Allergen |
| Compound | Mol_Id: 1; Molecule: Allergen Jun A 3; Chain: A; Synonym: Pathogenesis-Related Protein |
| | |
| **ID NO: 1PLM** | |
| Deposited: 09-Jan-1998 Exp. Method: Theoretical Model | |
| Title | Arabidopsis Profilin 1 Complexed With Poly-L-Proline, Theoretical Model |
| Classification | Complex (Protein/Peptide) |
| Compound | Mol_Id: 1; Molecule: Profilin 1; Chain: A; Engineered: Yes Mol_Id: 2; Molecule: Poly-L-Proline; Chain: B; Engineered: Yes |
| | |
| **ID NO: 1PRQ** | |
| Deposited: 18-Aug-1997 Exp. Method: X-ray Diffraction Resolution: 2.50 Å | |
| Title | Acanthamoeba Castellanii Profilin Ia |
| Classification | Contractile Protein |
| Compound | Mol_Id: 1; Molecule: Profilin Ia; Chain: Null; Engineered: Yes |
| | |
| **ID NO: 1QMR** | |
| Deposited: 06-Oct-1999 Exp. Method: X-ray Diffraction Resolution: 2.15 Å | |
| Title | Birch Pollen Allergen Bet V 1 Mutant N28T, K32Q, E45S, P108G |
| Classification | Allergen |
| Compound | Mol_Id: 1; Molecule: Major Pollen Allergen Bet V 1-A; Chain: A; Synonym: Bet V1; Engineered: Yes; Mutation: Yes |
| | |
| **ID NO: 1QNX** | |
| Deposited: 25-Oct-1999 Exp. Method: X-ray Diffraction Resolution: 1.90 Å | |
| Title | Ves V 5, An Allergen From Vespula Vulgaris Venom |
| Classification | Allergen |
| Compound | Mol_Id: 1; Molecule: Ves V 5; Chain: A; Synonym: Antigen 5; Engineered: Yes |
| | |
| **ID NO: 1WHO** | |
| Deposited: 04-Apr-1997 Exp. Method: X-ray Diffraction Resolution: 1.90 Å | |
| Title | Allergen Phl P 2 |
| Classification | Allergen |
| Compound | Mol_Id: 1; Molecule: Allergen Phl P 2; Chain: Null; Synonym: Phl P II; Engineered: Yes |
| | |
| **ID NO: 1WHP** | |
| Deposited: 04-Apr-1997 Exp. Method: X-ray Diffraction Resolution: 3.00 Å | |
| Title | Allergen Phl P 2 |
| Classification | Allergen |
| Compound | Mol_Id: 1; Molecule: Allergen Phl P 2; Chain: Null; Synonym: Phl P II; Engineered: Yes |
| | |
| **ID NO: 2BBG** | |
| Deposited: 24-Apr-1998 Exp. Method: NMR, 30 Structures | |
| Title | Ragweed Pollen Allergen From Ambrosia Trifida V, NMR, 30 Structures |
| Classification | Allergen |
| Compound | Mol_Id: 1; Molecule: Pollen Allergen 5; Chain: Null |
| | |
| **ID NO: 3BBG** | |
| Deposited: 24-Apr-1998 Exp. Method: NMR, 2 Structures | |
| Title | Multi-Conformer Structure Of Ragweed Pollen Allergen From Ambrosia Trifida V, NMR, 2 Structures |
| Classification | Allergen |
| Compound | Mol_Id: 1; Molecule: Pollen Allergen 5; Chain: Null |
| | |
| **ID NO: 3NUL** | |
| Deposited: 27-Nov-1996 Exp. Method: X-ray Diffraction Resolution: 1.60 Å | |
| Title | Profilin I From Arabidopsis Thaliana |
| Classification | Actin-Binding Protein |
| Compound | Mol_Id: 1; Molecule: Profilin I; Chain: Null; Engineered: Selenomethionyl Protein |

## Claims

1. An allergen hybrid protein having reduced allergenicity but retaining immunogenicity in respect of a native allergen protein, comprising a peptide epitope sequence of the allergen protein and a scaffold protein that is structurally homologous to the allergen protein, wherein the peptide epitope sequence is substituted for the homologous region of the scaffold protein; and the scaffold protein in the hybrid protein has a native conformation and the peptide epitope sequence is present in a surface accessible loop or corner region of the hybrid protein corresponding to its position in the allergen protein, wherein the scaffold protein has SEQ ID NO: 17, and the peptide epitope sequence comprises a sequence selected from the group consisting of
NNYCKIKCLKGGVHTACK (SEQ ID NO: 2);
LTGSTAAKYDD (SEQ ID NO: 10);
PKKKFSGND (SEQ ID NO: 11); and
FKNEELYQTK (SEQ ID NO: 13).

2. The hybrid protein of claim 1, wherein the allergen has SEQ ID NO: 16.

3. The hybrid protein of claim 1 further comprising a signal peptide.

4. The hybrid protein of claim 1 further comprising a protease processing site.

5. A nucleic acid encoding the allergen hybrid protein of any one of claims 1-4.

6. A method for preparing a nucleic acid that encodes an allergen hybrid protein; which method comprises substituting a nucleotide sequence encoding a peptide epitope sequence of an allergen protein into a nucleotide sequence encoding a scaffold protein that is structurally homologous to the allergen protein, wherein the nucleotide sequence encoding the peptide epitope sequence is in-frame with the nucleotide sequence encoding the scaffold protein and is in a location such that in the allergen hybrid protein the peptide epitope sequence is present in a surface accessible loop or corner region of the hybrid protein corresponding to its position in the allergen protein, wherein the scaffold protein has SEQ ID NO: 17, and the peptide epitope sequence comprises a sequence selected from the group consisting of
NNYCKIKCLKGGVHTACK (SEQ ID NO: 2);
LTGSTAAKYDD (SEQ ID NO: 10);
PKKKFSGND (SEQ ID NO: 11); and
FKNEELYQTK (SEQ ID NO: 13).

7. The method according to claim 6, wherein the nucleotide sequence encoding the scaffold protein is mutated to substitute the nucleotide sequence encoding the peptide epitope sequence.

8. The method according to claim 6, wherein the nucleotide encoding the peptide epitope sequence is substituted by ligating fragments from nucleic acids comprising the nucleotide sequence encoding the peptide epitope sequence and the nucleotide sequence encoding the scaffold protein treated with an endonuclease.

9. An expression vector comprising the nucleic acid of claim 5 operationally associated with a promoter.

10. Use of a therapeutically effective amount of the hybrid protein according to any one of claims 1 to 4 or the expression vector of claim 9 in the preparation of a medicament for the treatment or prevention of an allergic condition, which medicament is for administration to a patient who is allergic to the allergen protein or the scaffold protein, or both.

11. The use of claim 10, wherein the hybrid protein or expression vector is for oral, pulmonary, nasal, topical or parenteral administration.

12. A pharmaceutical composition comprising the hybrid protein according to any one of claims 1 to 4 or the expression vector of claim 9 and a pharmaccutically acceptable diluent or carrier.

## Patentansprüche

1. Allergenes Hybridprotein, das verringerte Allergenität, aber beibehaltene Immunogenität hinsichtlich eines nativen allergenen Proteins aufweist, umfassend eine Peptidepitopsequenz des allergenen Proteins und ein zum allergenen Protein strukturell homologes Gerüstprotein, worin die Peptidepitopsequenz die homologe Region des Gerüstproteins ersetzt; und das Gerüstprotein im Hybridprotein weist eine native Konformation auf, und die Peptidepitopsequenz ist in einer von der Oberfläche zugänglichen Schleife oder in Eckenregion des Hybridproteins in einer im allergenen Protein entsprechenden Position vorhanden, worin das Gerüstprotein SEQ ID NO: 17 aufweist, und die Peptidepitopsequenz eine Sequenz umfasst, die aus der Gruppe ausgewählt ist, bestehend aus:
NNYCKIKCLKGGVHTACK (SEQ ID NO: 2);
LTGSTAAKYDD (SEQ ID NO: 10);
PKKKFSGND (SEQ ID NO: 11); und
FKNEELYQTK (SEQ ID NO: 13).

2. Hybridprotein nach Anspruch 1, worin das Allergen SEQ ID NO: 16 aufweist.

3. Hybridprotein nach Anspruch 1, welches außerdem ein Signalpeptid umfasst.

4. Hybridprotein nach Anspruch 1, welches außerdem eine Protease bearbeitende Stelle umfasst.

5. Nukleinsäure, die das allergene Hybridprotein nach irgendeinem der Ansprüche 1-4 kodiert.

6. Verfahren zur Herstellung einer ein allergenes Hybridprotein kodierenden Nukleinsäure; welches Verfahren das Substituieren einer eine Peptidepitopsequenz kodierenden Nukleotidsequenz eines allergenen Proteins in eine ein zum allergenen Protein strukturell homologes Gerüstprotein kodierende Nukleinsäuresequenz umfasst, worin die
die Peptidepitopsequenz kodierende Nukleotidsequenz mit der das Gerüstprotein kodierenden Nukleotidsequenz in-frame ist, und sich an einer Stelle befindet, so dass die Peptidepitopsequenz im allergenen Hybridprotein in einer von der Oberfläche zugänglichen Schleife oder Eckenregion des Hybridproteins in einer im allergenen Protein entsprechenden Position vorhanden ist, worin das Gerüstprotein SEQ ID NO: 17 aufweist, und die Peptidepitopsequenz eine Sequenz umfasst, die aus der Gruppe ausgewählt ist, bestehend aus
NNYCKIKCLKGGVHTACK (SEQ ID NO: 2);
LTGSTAAKYDD (SEQ ID NO: 10);
PKKKFSGND (SEQ ID NO: 11); und
FKNEELYQTK (SEQ ID NO: 13).

7. Verfahren nach Anspruch 6, worin die das Gerüstprotein kodierende Nukleotidsequenz zum Ersetzen der die Peptidepitopsequenz kodierenden Nukleotidsequenz mutiert wird.

8. Verfahren nach Anspruch 6, worin das die Peptidepitopsequenz kodierende Nukleotid durch bindende Fragmente von Nukleinsäuren ersetzt wird, umfassend die die Peptidepitopsequenz kodierende Nukleotidsequenz und die das Gerüstprotein kodierende Nukleotidsequenz, welche mit einer Endonuklease behandelt werden.

9. Expressionsvektor, der die Nukleinsäure nach Anspruch 5 umfasst, welche mit einem Promoter operativ verbunden ist.

10. Verwendung einer therapeutisch wirksamen Menge des Hybridproteins nach irgendeinem der Ansprüche 1 bis 4 oder des Expressionsvektors nach Anspruch 9 bei der Herstellung eines Medikaments zur Behandlung oder Vorbeugung eines allergischen Zustands, welches Medikament an einen Patienten verabreicht wird, der gegenüber dem allergenen Protein oder dem Gerüstprotein oder beiden allergisch ist.

11. Verwendung nach Anspruch 10, worin das Hybridprotein oder der Expressionsvektor für orale, pulmonale, nasale, topische oder parenterale Verabreichung bestimmt ist.

12. Pharmazeutische Zusammensetzung, die das Hybridprotein nach irgendeinem der Ansprüche 1 bis 4 oder den Expressionsvektor nach Anspruch 9 umfasst, und ein pharmazeutisch akzeptabler Verdünner oder Träger.

## Revendications

1. Protéine hybride allergène ayant une allergénicité réduite mais conservant une immunogénicité par rapport à une protéine allergène native, comprenant une séquence épitope peptidique de la protéine allergène et une protéine échafaudage qui est homologue du point de vue de la structure à la protéine allergène, dans laquelle la séquence épitope peptidique remplace la région homologue de la protéine échafaudage ; et la protéine échafaudage dans la protéine hybride a une conformation native et la séquence épitope peptidique est présente dans une boucle accessible en surface ou une région de coin de la protéine hybride correspondant à sa position dans la protéine allergène, dans laquelle la protéine échafaudage a la SEQ ID N° : 17 et la séquence épitope peptidique comprend une séquence choisie dans le groupe constitué par
NNYCKIKCLKGGVHTACK (SEQ ID N° : 2) ;
LTGSTAAKYDD (SEQ ID N° 10) ;
PKKKFSGND (SEQ ID N° : 11) ; et
FKNEELYQTK (SEQ ID N° : 13).

2. Protéine hybride de la revendication 1, dans laquelle l'allergène a la SEQ ID N° : 16.

3. Protéine hybride de la revendication 1 comprenant en outre un peptide signal.

4. Protéine hybride de la revendication 1, comprenant en outre un site de traitement par des protéases.

5. Acide nucléique codant pour la protéine hybride allergène de l'une quelconque des revendications 1-4.

6. Procédé pour préparer un acide nucléique qui code pour une protéine hybride allergène ; lequel procédé comprend la substitution d'une séquence nucléotidique codant pour une séquence épitope peptidique d'une protéine allergène dans une séquence nucléotidique codant pour une protéine échafaudage qui est homologue du point de vue de la structure à la protéine allergène, dans lequel la séquence nucléotidique codant pour la séquence épitope peptidique est en phase avec la séquence nucléotidique codant pour la protéine échafaudage et se situe dans un endroit tel que dans la protéine hybride allergène la séquence épitope peptidique est présente dans une boucle accessible en surface ou une région de coin de la protéine hybride correspondant à sa position dans la protéine allergène, dans lequel la protéine échafaudage a la SEQ ID N° : 17 et la séquence épitope peptidique comprend une séquence choisie dans le groupe constitué par
NNYCKIKCLKGGVHTACK (SEQ ID N° : 2) ;
LTGSTAAKYDD (SEQ ID N° 10) ;
PKKKFSGND (SEQ ID N° : 11) ; et
FKNEELYQTK (SEQ ID N° : 13).

7. Procédé selon la revendication 6, dans lequel la séquence nucléotidique codant pour la protéine échafaudage est mutée pour substituer la séquence nucléotidique codant pour la séquence épitope peptidique.

8. Procédé selon la revendication 6, dans lequel le nucléotide codant pour la séquence épitope peptidique est substitué par ligature de fragments provenant d'acides nucléiques comprenant la séquence nucléotidique codant pour la séquence épitope peptidique et la séquence nucléotidique codant pour la protéine échafaudage traités par une endonucléase.

9. Vecteur d'expression comprenant l'acide nucléique de la revendication 5 associé de façon fonctionnelle à un promoteur.

10. Utilisation d'une quantité efficace du point de vue thérapeutique de la protéine hybride selon l'une quelconque des revendications 1 à 4 ou du vecteur d'expression de la revendication 9 dans la préparation d'un médicament pour le traitement ou la prévention d'une affection allergique, lequel médicament est destiné à l'administration à un patient qui est allergique à la protéine allergène ou la protéine échafaudage, ou les deux.

11. Utilisation de la revendication 10, dans laquelle la protéine hybride ou le vecteur d'expression est destiné à une administration orale, pulmonaire, nasale, topique ou parentérale.

12. Composition pharmaceutique comprenant la protéine hybride selon l'une quelconque des revendications 1 à 4 ou le vecteur d'expression de la revendication 9 et un diluant ou véhicule acceptable du point de vue pharmaceutique.
